# EUROPÄISCHE PATENTSCHRIFT

(11) **EP 1 216 223 B1**
(45) Veröffentlichungstag und Bekanntmachung des Hinweises auf die Patenterteilung: **09.11.2005**
(21) Anmeldenummer: 00964067.3
(22) Anmeldetag: 31.08.2000
(51) Int. Cl.: C07C 69/94, C07C 69/76, C07C 65/28, C07C 63/33, C07C 63/72, C07C 65/24, C07C 69/92, C07D 241/04, C07C 323/62, C07C 323/56, C07C 317/46, C07C 229/52, C07C 229/38, C07D 213/64, C07C 235/84

(54) **NEUARTIGE DICARBONSÄUREDERIVATE MIT PHARMAZEUTISCHEN EIGENSCHAFTEN**
NOVEL DICARBOXYLIC ACID DERIVATIVES WITH PHARMACEUTICAL PROPERTIES
DERIVES D'ACIDE DICARBOXYLIQUE A PROPRIETES PHARMACEUTIQUES

(30) Priorität: 13.09.1999 DE 19943636
(43) Veröffentlichungstag der Anmeldung: 26.06.2002
(73) Patentinhaber: Bayer HealthCare AG, 51368 Leverkusen (DE)
(72) Erfinder: ALONSO-ALIJA, Cristina, 42781 Haan (DE); HEIL, Markus, 42799 Leichlingen (DE); FLUBACHER, Dietmar, 40724 Hilden (DE); NAAB, Paul, 42287 Wuppertal (DE); STASCH, Johannes-Peter, 42651 Solingen (DE); WUNDER, Frank, 42115 Wuppertal (DE); DEMBOWSKY, Klaus, 69198 Schriesheim (DE); PERZBORN, Elisabeth, 42327 Wuppertal (DE); STAHL, Elke, 51467 Bergisch Gladbach (DE)
(86) Internationale Anmeldenummer: PCT/EP2000/008466
(87) Internationale Veröffentlichungsnummer: WO 2001/019778

(56) Entgegenhaltungen:
- EP-A- 0 341 551

## Beschreibung

Die vorliegende Erfindung betrifft neue chemische Verbindungen, welche die lösliche Guanylatcyclase auch über einen neuartigen, ohne Beteiligung der Häm-Gruppe des Enzyms verlaufenden Wirkmechanismus stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NOunabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587), sowie verschiedene substituierte Pyrazolderivate (WO 98/16223, WO 98/16507 und WO 98/23619).

Die bisher bekannten Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung (Gerzer et al., FEBS Lett. 132(1981), 71), oder über einen Häm-abhängigen Mechanismus, der unabhängig von NO ist, aber zu einer Potenzierung der stimulierenden Wirkung von NO oder CO führt (z.B. YC-1, Hoenicka et al., J. Mol. Med. (1999) 14; oder die in der WO 98/16223, WO 98/16507 und WO 98/23619 beschriebenen Pyrazolderivate).

Die in der Literatur behauptete stimulierende Wirkung von Isoliquiritigenin und von Fettsäuren, wie z. B. Arachidonsäure, Prostaglandinendoperoxide und Fettsäurehydroperoxide auf die lösliche Guanylatcyclase konnte nicht bestätigt werden (vgl. z.B. Hoenicka et al., J. Mol. Med. 77 (1999), 14).

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben (Ignarro et al., Adv. Pharmacol. 26 (1994), 35). Allerdings kann Protoporphyrin IX als Mimik für das NO-Häm-Addukt angesehen werden, weshalb die Zugabe von Protoporphyrin IX zur Häm-freien löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird (Mülsch et al., Naunyn Schmiedebergs Arch. Phanmacol. 355, R47).

Bislang wurden somit keine Verbindungen beschrieben, welche die lösliche Guanylatcyclase unabhängig von der im Enzym befindlichen Häm-Gruppe stimulieren können.

Es war die Aufgabe der vorliegenden Erfindung, Arzneimittel zur Behandlung von Herz-Kreislauferkrankungen oder anderen über eine Beeinflussung des cGMP-Signalweges in Organismen therapierbaren Erkrankungen zu entwickeln.

Überraschend wurde gefunden, dass es Verbindungen gibt, welche die lösliche Guanylatcyclase auch unabhängig von der im Enzym befindlichen Häm-Gruppe stimulieren können. Die biologische Aktivität dieser Stimulatoren beruht auf einem völlig neuen Mechanismus der Stimulierung der löslichen Guanylatcyclase. Im Gegensatz zu den vorstehend beschriebenen, aus dem Stand der Technik als Stimulatoren der löslichen Guanylatcyclase bekannten Verbindungen sind die erfindungsgemäßen Verbindungen in der Lage, sowohl die Häm-haltige als auch die Häm-freie Form der löslichen Guanylatcyclase zu stimulieren. Die Stimulierung des Enzyms verläuft bei diesen neuen Stimulatoren also über einen Häm-unabhängigen Weg, was auch dadurch belegt wird, dass die neuen Stimulatoren am Häm-haltigen Enzym einerseits keine synergistische Wirkung mit NO zeigen und andererseits sich die Wirkung dieser neuartigen Stimulatoren nicht durch den Häm-abhängigen Inhibitor der löslichen Guanylatcyclase, 1*H*-1,2,4-Oxadiazol-(4,3a)-chinoxalin-1-on (ODQ), blockieren lässt.

Dies stellt einen neuen Therapieansatz zur Behandlung von Herz-Kreislauferkrankungen und anderen über eine Beeinflussung des cGMP-Signalweges in Organismen therapierbaren Erkrankungen dar.

In der EP-A-0 341 551 sind Alkan- und Alkensäurederivate wie beispielsweise (1) beschrieben, die potente Leukotrien-Antagonisten sind und daher beispielsweise als Medikamente zur Behandlung von Asthma oder Durchblutungsstörungen geeignet sind (S. 18, Z. 56-58). Eine stimulierende Wirkung dieser Verbindungen auf die lösliche Guanylatcyclase und die sich daraus ergebende Verwendung dieser Verbindungen zur Herstellung von Arzneimitteln, welche den cGMP-Signalweg beeinflussen können, ist jedoch nicht beschrieben.

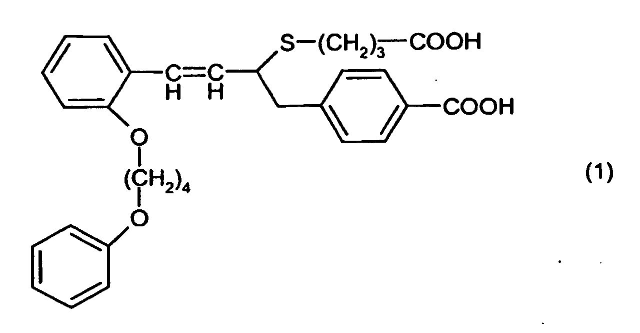

In der EP-A-0 410 241 sind weitere Alkan- und Alkensäurederivate wie beispielsweise (2) mit LTD₄-, LTC₄- oder LTE₄-antagonistischer Wirkung beschrieben.

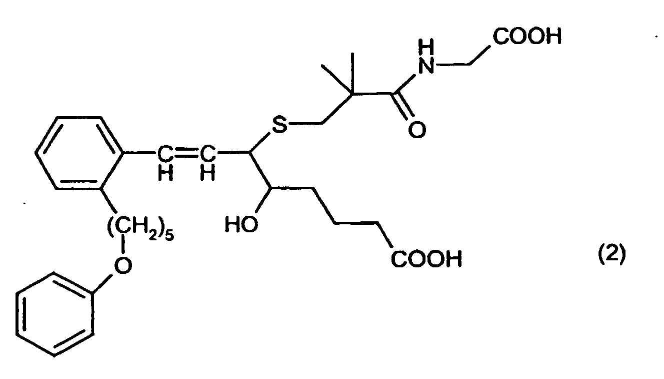

In der EP-A-0 494 621 sind schwefelhaltige Alkensäurederivate wie beispielsweise (3) beschrieben, welche bei allergischen Erkrankungen, Entzündungen und Herz-Kreislauf-Erkrankungen eingesetzt werden können.

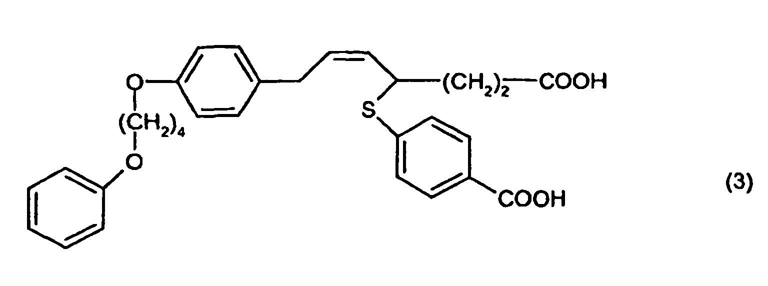

In der EP-A-0 791 576 sind Benzoesäurederivate wie beispielsweise (4) beschrieben, welche zur Behandlung von Atemwegserkrankungen verwendet werden können.

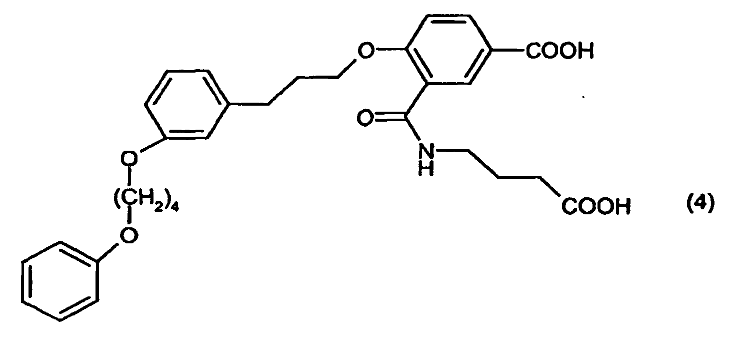

Es ist jedoch nicht beschrieben, dass irgendeine der vorstehend genannten, aus dem Stand der Technik bekannten Verbindungen eine stimulierende Wirkung auf die lösliche Guanylatcyclase besitzt und somit zur Behandlung von Erkrankungen eingesetzt werden könnte, welche durch Beeinflussung des cGMP-Spiegels therapierbar sind.

Die vorliegende Erfindung betrifft Verbindungen der allgemeinen Formel (I) 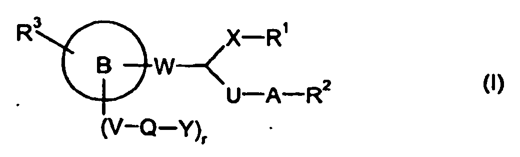
- B: Aryl mit 6 bis 10 Kohlenstoffatomen bedeutet,
- r: 0 oder 1 bedeutet,
- V: fehlt, O, NR⁴, NR⁴CONR⁴, NR⁴CO, NR⁴SO₂, COO, CONR⁴ oder S(O)ₒ bedeutet,
worin
R⁴ unabhängig von einem weiteren gegebenenfalls vorhandenen Rest R⁴ Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder Arylalkyl mit 7 bis 18 Kohlenstoffatomen bedeutet, wobei der Arylrest seinerseits ein- oder mehrfach durch Halogen, Alkyl, Alkoxy mit bis zu 6 Kohlenstoffatomen substituiert sein kann,
o 0, 1 oder 2 bedeutet,
- Q: fehlt, geradkettiges oder verzweigtes Alkylen, geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CO, OCO, S-CO-, CONR⁵ oder NR⁵SO₂ oder eine oder mehrere Alken- oder Alkingruppen enthalten können, und ein oder mehrfach durch Halogen, Hydroxy oder Alkoxy mit bis zu 4 Kohlenstoffatomen substituiert sein kann, wobei gegebenenfalls zwei beliebige Atome der vorstehenden Kette unter Bildung eines drei- bis achtgliedrigen Rings miteinander verbunden sein können, oder CONR⁵ bedeutet,
worin
R⁵ Wasserstoff, geradkettiges oder verzweigtes Alkyl mit bis zu 8 Kohlenstoffatomen oder Cycloalkyl mit 3 bis 8 Kohlenstoffatomen bedeutet, das durch Halogen oder Alkoxy mit bis zu 4 Kohlenstoffatomen substituiert sein kann,
p 0, 1 oder 2 bedeutet,
- Y: Wasserstoff, NR⁶R⁷, Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O oder geradkettiges oder verzweigtes Cycloalkyl mit 3 bis 8 Kohlenstoffatomen bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 8 Kohlenstoffatomen, geradkettiges oder verzweigtes Cycloalkyl mit 3 bis 8 Kohlenstoffatomen, Halogen, Hydroxy, COR⁸, CN, SR⁸, NO₂, NR¹⁰R¹¹, NR⁹COR¹² , NR⁹CONR⁹R¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy, geradkettiges oder verzweigtes Alkyloxyalkyl mit bis zu 8 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 8 Kohlenstoffatomen, geradkettiges oder verzweigtes Halogenalkyl mit bis zu 8 Kohlenstoffatomen oder Cycloalkyl mit 3 bis 8 Kohlenstoffatomen bedeutet,
R⁹ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁰, R¹¹, R¹³ und R¹⁴ unabhängig voneinander Wasserstoff, geradkettiges oder verzweigtes Alkyl, geradkettiges oder verzweigtes Alkenyl mit bis zu 8 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O, Arylalkyl mit 8 bis 18 Kohlenstoffatomen, Cycloalkyl mit 3 bis 8 Kohlenstoffatomen oder einen Rest der Formel SO₂R¹⁵ bedeuten,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
oder zwei Substituenten aus R¹⁰ und R¹¹ oder R¹³ und R¹⁴ miteinander unter Bildung eines fünf- oder sechsgliedrigen Rings verbunden sein können, der O oder N enthalten kann,
worin
R¹⁵ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, CN, NO₂, Alkyl, Alkoxy, Halogenalkyl oder Halogenalkoxy mit bis zu 6 Kohlenstoffatomen substituiert sein kann,
R¹² Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können; und/oder die cyclischen Reste jeweils ein- bis dreifach durch Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O substituiert sein können, die auch über N gebunden sein können,
welche direkt oder über eine Gruppe aus O, S, SO, SO₂, NR⁹, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 8 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl, geradkettiges oder verzweigtes Halogenalkoxy, Carbonylalkyl oder geradkettiges oder verzweigtes Alkenyl mit jeweils bis zu 6 Kohlenstoffatomen, Phenyl, Benzyl, Halogen, SR⁸, CN, NO₂, NR¹⁷R¹⁸, CONR¹⁷R¹⁸oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder einen Rest der Formel SO₂R²⁰ bedeuten, wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
worin
R²⁰ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann, und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff, OH, Halogen, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
- W: geradkettiges oder verzweigtes Alkylen, geradkettiges oder verzweigtes Alkendiyl mit jeweils bis zu 4 Kohlenstoffatomen bedeutet, die eine Gruppe aus O oder NR²³ enthalten können,
worin
R²³ Wasserstoff, geradkettiges oder verzweigtes Alkyl mit bis zu 8 Kohlenstoffatomen oder Cycloalkyl mit 3 bis 8 Kohlenstoffatomen bedeutet,
- U: geradkettiges oder verzweigtes Alkylen mit bis zu 4 Kohlenstoffatomen, O, NH, S, SO oder SO₂ bedeutet,
- A: fehlt, Phenyl oder einen aromatischen Heterocyclus mit 1 bis 9 Kohlenstoffatomen und bis zu 3 Heteroatomen aus der Reihe S, N und/oder O bedeutet,
welche gegebenenfalls ein- bis dreifach durch Halogen, geradkettiges oder verzweigtes Alkyl, geradkettiges oder verzweigtes Halogenalkyl oder geradkettiges oder verzweigtes Alkoxy mit jeweils bis zu 4 Kohlenstoffatomen substituiert sein können,
- R²: COOR²⁶ oder CN bedeutet,
worin
R²⁶ Wasserstoff oder geradkettiges oder verzweigtes Alkyl mit bis zu 8 Kohlenstoffatomen bedeutet;
- X: geradkettiges oder verzweigtes Alkylen, geradkettiges oder verzweigtes Alkendiyl mit jeweils bis zu 8 Kohlenstoffatomen bedeutet, die eine Gruppe aus O, S(O)ᵣ, NR³⁰, eine oder mehrere Alkengruppen oder einen drei- bis sechsgliedrigen gesättigten oder ungesättigten, gegebenenfalls einen oder mehrere geradkettige oder verzweigte Alkylreste mit 1 bis 6 Kohlenstoffatomen aufweisenden Carbocyclus mit gegebenenfalls einem oder zwei Heteroatomen aus der Reihe S(O)ᵣ, NR³² und/oder O enthalten können,
worin
r 0, 1 oder 2 bedeutet,
R³⁰ Wasserstoff, geradkettiges oder verzweigtes Alkyl mit bis zu 6 Kohlenstoffatomen, Phenyl oder Arylalkyl mit 7 bis 12 Kohlenstoffatomen bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
- R¹: CN oder COOR³⁵ bedeutet,
worin
R³⁵ Wasserstoff oder geradkettiges oder verzweigtes Alkyl mit bis zu 6 Kohlenstoffatomen bedeutet;
mit der Maßgabe, dass Y nicht Phenyl oder ausschließlich mit einem oder zwei Resten aus der Gruppe, bestehend aus geradkettigem oder verzweigtem Alkyl oder geradkettigem oder verzweigtem Alkoxy mit jeweils bis zu 8 Kohlenstoffatomen, Halogen, CF₃, OCF₃ oder CN, substituiertes Phenyl sein kann, wenn gleichzeitig B Phenyl ist, V fehlt oder O bedeutet, Q geradkettiges Alkylen mit 1 bis 10 Kohlenstoffatomen bedeutet und gegebenenfalls über ein Sauerstoffatom mit Y verknüpft ist, W eine Alkylengruppe oder Alkendiylgruppe mit jeweils 1 bis 4 Kohlenstoffatomen ist, U eine Alkylengruppe mit bis zu 4 Kohlenstoffatomen, O, S, SO oder SO₂ ist, A Phenyl ist und X geradkettiges Alkylen mit 1 bis 8 Kohlenstoffatomen ist und gegebenenfalls direkt über O, S, SO oder SO₂ an das die Gruppen W und U tragende Kohlenstoffatom gebunden ist;

Insbesondere bevorzugt sind hierbei Verbindungen der Formel (I), bei denen
- B: Phenyl oder Naphthyl bedeutet
- r: 0 oder 1 bedeutet,
- V: fehlt oder O, NR⁴ oder S(O)ₙ bedeutet
worin
R⁴ Wasserstoff bedeutet,
n 0 bedeutet,
- Q: fehlt, geradkettiges oder verzweigtes Alkylen, geradkettiges oder verzweigtes Alkendiyl mit jeweils bis zu 15 Kohlenstoffatomen bedeutet, die eine oder mehrere Gruppen aus O, S(O)ₚ, NR⁵, CONR⁵ , S-CO-, OCO enthalten können, und ein- oder zweifach durch Halogen oder Hydroxy substituiert sein können, oder CONR⁵ bedeutet,
worin
R⁵ Wasserstoff bedeutet,
p 0 oder 1 bedeutet,
- Y: Wasserstoff, NR⁶R⁷, Phenyl, Napthyl oder einen Heterocyclus aus der Gruppe 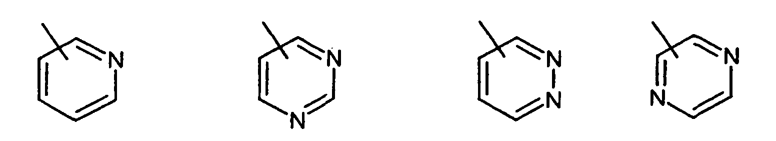 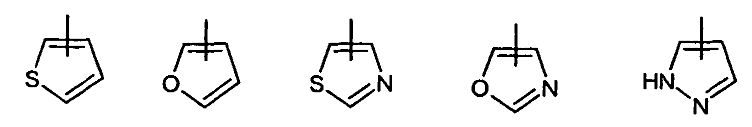 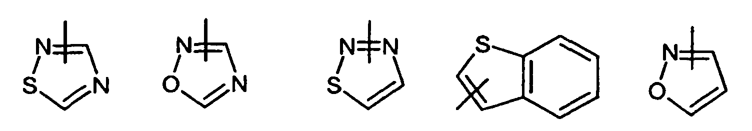 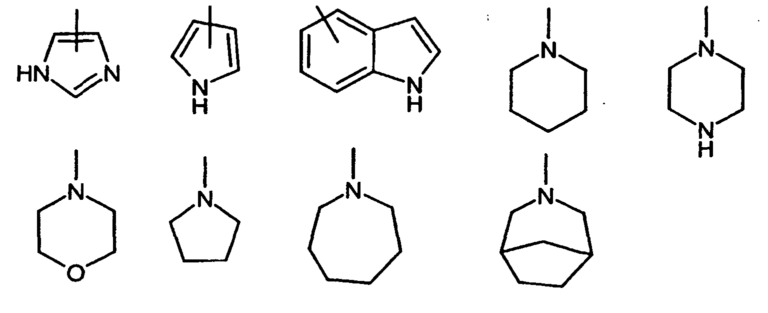 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist,bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann; und/oder die cyclischen Reste jeweils ein- bis dreifach durch Phenyl oder einen Heterocyclus aus der Gruppe 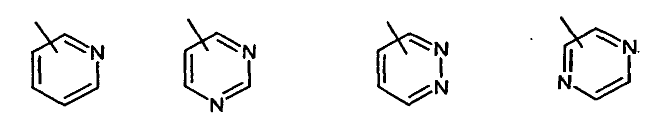 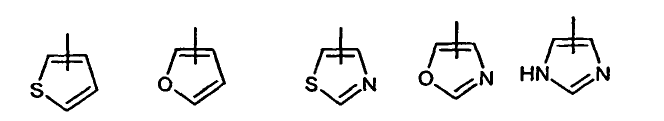 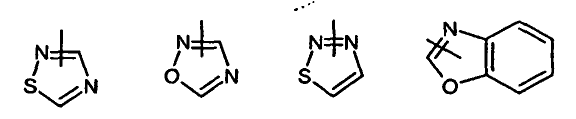 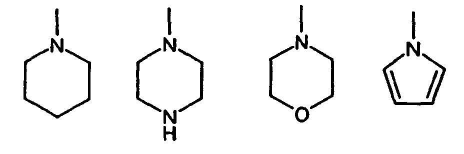 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet, und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff, OH, F, Cl, Br, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
- W: CH₂CH₂, CH=CH, CH₂O, OCH₂, CH₂OCH₂, CH₂NH, NHCH₂ oder CH₂NHCH₂ bedeutet,
- U: geradkettiges Alkylen mit bis zu 4 Kohlenstoffatomen, O, NH, S, SO oder SO₂ bedeutet,
- A: fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
- R²: COOR²⁶ oder CN bedeutet,
worin
R²⁶ Wasserstoff oder geradkettiges oder verzweigtes Alkyl mit bis zu 4 Kohlenstoffatomen bedeutet;
- X: geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls einem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
- R¹: CN oder COOR³⁵ bedeutet,
worin
R³⁵ Wasserstoff oder geradkettiges oder verzweigtes Alkyl mit bis zu 6 Kohlenstoffatomen bedeutet; mit der Maßgabe, dass Y nicht Phenyl oder ausschließlich mit einem oder zwei Resten aus der Gruppe, bestehend aus geradkettigem oder verzweigtem Alkyl oder geradkettigem oder verzweigtem Alkoxy mit jeweils bis zu 4 Kohlenstoffatomen, Halogen, CF₃, oder OCF₃, substituiertes Phenyl sein kann, wenn gleichzeitig V fehlt oder O bedeutet, Q geradkettiges Alkylen mit 1 bis 10 Kohlenstoffatomen bedeutet und gegebenenfalls über ein Sauerstoffatom mit Y verknüpft ist, W eine Ethylengruppe oder eine Ethandiylgruppe mit jeweils 1 bis 6 Kohlenstoffatomen ist, U eine Alkylengruppe mit bis zu 4 Kohlenstoffatomen, O, S, SO oder SO₂ ist, A Phenyl ist und X geradkettiges Alkylen mit 1 bis 4 Kohlenstoffatomen ist und gegebenenfalls direkt über O, S, SO oder SO₂ an das die Gruppen W und U tragende Kohlenstoffatom gebunden ist;

Erfindungsgemäß ebenfalls bevorzugt sind Verbindungen der Formel (I), worin
- B: einen aromatischen Heterocyclus mit 1 bis 9 Kohlenstoffatomen und bis zu 3 Heteroatomen aus der Reihe S, N und/oder O bedeutet, und die anderen Substituenten wie vorstehend definiert sind.

Besonders bevorzugt sind hierbei Verbindungen der Formel (I), bei denen
- B: einen aromatischen Heterocyclus mit 1 bis 9 Kohlenstoffatomen und bis zu 3 Heteroatomen aus der Reihe S, N und/oder O bedeutet,
- r: 0 oder 1 bedeutet,
- V: fehlt, O, NR⁴, NR⁴CONR⁴, NR⁴CO, NR⁴SO₂, COO, CONR⁴ oder S(O)ₒ bedeutet,
worin
R⁴ unabhängig von einem weiteren gegebenenfalls vorhandenen Rest R⁴ Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder Arylalkyl mit 7 bis 18 Kohlenstoffatomen bedeutet, wobei der Arylrest seinerseits ein- oder mehrfach durch Halogen, Alkyl, Alkoxy mit bis zu 6 Kohlenstoffatomen substituiert sein kann,
o 0, 1 oder 2 bedeutet,
- Q: fehlt, geradkettiges oder verzweigtes Alkylen, geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CO, OCO, S-CO-, CONR⁵ oder NR⁵SO₂ enthalten können, und ein oder mehrfach durch Halogen, Hydroxy oder Alkoxy mit bis zu 4 Kohlenstoffatomen substituiert sein können, wobei gegebenenfalls zwei beliebige Atome der vorstehenden Kette unter Bildung eines drei- bis achtgliedrigen Rings miteinander verbunden sein können, oder CONR⁵ bedeutet,
worin
R⁵ Wasserstoff, geradkettiges oder verzweigtes Alkyl mit bis zu 8 Kohlenstoffatomen oder Cycloalkyl mit 3 bis 8 Kohlenstoffatomen bedeutet, das durch Halogen oder Alkoxy mit bis zu 4 Kohlenstoffatomen substituiert sein kann,
p 0, 1 oder 2 bedeutet,
- Y: Wasserstoff, NR⁶R⁷, Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O oder geradkettiges oder verzweigtes Cycloalkyl mit 3 bis 8 Kohlenstoffatomen bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 8 Kohlenstoffatomen, geradkettiges oder verzweigtes Cycloalkyl mit 3 bis 8 Kohlenstoffatomen, Halogen, Hydroxy, COR⁸, CN, SR⁸, NO₂, NR¹⁰R¹¹, NR⁹COR¹² , NR⁹CONR⁹R¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy, geradkettiges oder verzweigtes Alkyloxyalkyl mit bis zu 8 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 8 Kohlenstoffatomen, geradkettiges oder verzweigtes Halogenalkyl mit bis zu 8 Kohlenstoffatomen oder Cycloalkyl mit 3 bis 8 Kohlenstoffatomen bedeutet,
R⁹ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁰, R¹¹, R¹³ und R¹⁴ unabhängig voneinander Wasserstoff, geradkettiges oder verzweigtes Alkyl, geradkettiges oder verzweigtes Alkenyl mit bis zu 8 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O, Arylalkyl mit 8 bis 18 Kohlenstoffatomen, Cycloalkyl mit 3 bis 8 Kohlenstoffatomen oder einen Rest der Formel SO₂R¹⁵ bedeuten,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
oder zwei Substituenten aus R¹⁰ und R¹¹ oder R¹³ und R¹⁴ miteinander unter Bildung eines fünf- oder sechsgliedrigen Rings verbunden sein können, der O oder N enthalten kann,
worin
R¹⁵ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, CN, NO₂, Alkyl, Alkoxy, Halogenalkyl oder Halogenalkoxy mit bis zu 6 Kohlenstoffatomen substituiert sein kann,
- R¹²: Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können;
und/oder die cyclischen Reste jeweils ein- bis dreifach durch Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O substituiert sein können, die auch über N gebunden sein können,
welche direkt oder über eine Gruppe aus O, S, SO, SO₂, NR⁹, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 8 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl, geradkettiges oder verzweigtes Halogenalkoxy, Carbonylalkyl oder geradkettiges oder verzweigtes Alkenyl mit jeweils bis zu 6 Kohlenstoffatomen, Phenyl, Benzyl, Halogen, SR⁸, CN, NO₂, NR¹⁷R¹⁸, CONR¹⁷R¹⁸oder NR¹⁶COR¹⁹ substituiert sein können,
worin
- R¹⁶: Wasserstoff, geradkettiges oder verzweigtes Alkyl mit bis zu 8 Kohlenstoffatomen oder Cycloalkyl mit 3 bis 8 Kohlenstoffatomen bedeutet,
- R¹⁷, R¹⁸: unabhängig voneinander Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder einen Rest der Formel SO₂R²⁰ bedeuten, wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
worin
R²⁰ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann, und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff, Halogen, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
- W: geradkettiges oder verzweigtes Alkylen, geradkettiges oder verzweigtes Alkendiyl mit jeweils bis zu 4 Kohlenstoffatomen bedeutet, die eine Gruppe aus O oder NR²³ enthalten können,
worin
R²³ Wasserstoff, geradkettiges oder verzweigtes Alkyl mit bis zu 8 Kohlenstoffatomen oder Cycloalkyl mit 3 bis 8 Kohlenstoffatomen bedeutet,
- U: geradkettiges oder verzweigtes Alkylen mit bis zu 4 Kohlenstoffatomen, O, NH, S, SO oder SO₂ bedeutet,
- A: fehlt, Phenyl oder einen aromatischen Heterocyclus mit 1 bis 9 Kohlenstoffatomen und bis zu 3 Heteroatomen aus der Reihe S, N und/oder O bedeutet,
welche gegebenenfalls ein- bis dreifach durch Halogen, geradkettiges oder verzweigtes Alkyl, geradkettiges oder verzweigtes Halogenalkyl oder geradkettiges oder verzweigtes Alkoxy mit jeweils bis zu 4 Kohlenstoffatomen substituiert sein können,
- R²: COOR²⁶ oder CN bedeutet,
worin
R²⁶ Wasserstoff oder geradkettiges oder verzweigtes Alkyl mit bis zu 8 Kohlenstoffatomen bedeutet;
- X: geradkettiges oder verzweigtes Alkylen, geradkettiges oder verzweigtes Alkendiyl mit jeweils bis zu 8 Kohlenstoffatomen bedeutet, die eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 6 Kohlenstoffatomen aufweisenden Carbocyclus mit gegebenenfalls einem oder zwei Heteroatomen aus der Reihe S(O)ᵣ, NR³² und/oder O enthalten können,
worin
r 0, 1 oder 2 bedeutet,
R³⁰ Wasserstoff, geradkettiges oder verzweigtes Alkyl mit bis zu 6 Kohlenstoffatomen, Phenyl oder Arylalkyl mit 7 bis 12 Kohlenstoffatomen bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
- R¹: CN oder COOR³⁵ bedeutet,
worin
R³⁵ Wasserstoff oder geradkettiges oder verzweigtes Alkyl mit bis zu 6 Kohlenstoffatomen bedeutet.

Insbesondere bevorzugt sind hierbei Verbindungen der Formel (I), bei denen
- B: einen Heterocyclus aus der Reihe 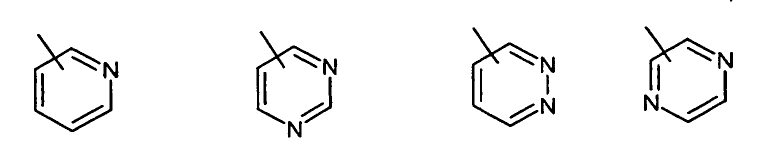 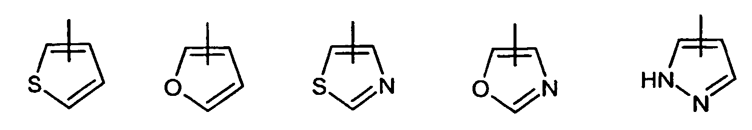 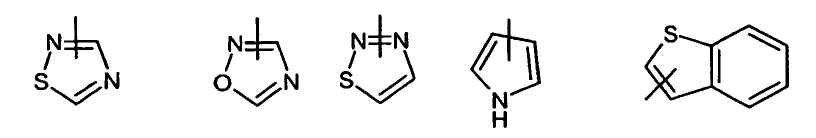 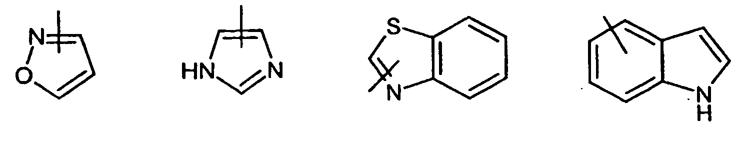 bedeutet
- r: 0 oder 1 bedeutet,
- V: fehlt oder O, NR⁴ oder S(O)ₙ bedeutet
worin
R⁴ Wasserstoff bedeutet,
n 0 bedeutet,
- Q: fehlt, geradkettiges oder verzweigtes Alkylen, geradkettiges oder verzweigtes Alkendiyl mit jeweils bis zu 15 Kohlenstoffatomen bedeutet, das eine oder mehrere Gruppen aus O, S(O)ₚ, NR⁵, CONR⁵ , S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
- Y: Wasserstoff, NR⁶R⁷, Phenyl, Napthyl oder einen Heterocyclus aus der Gruppe 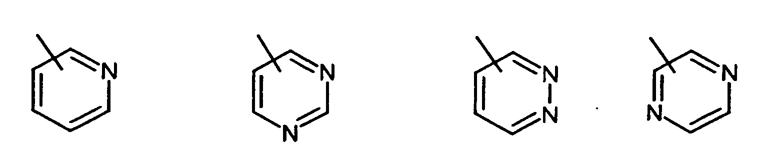 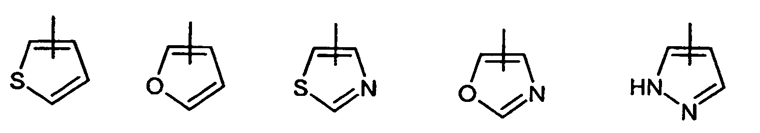 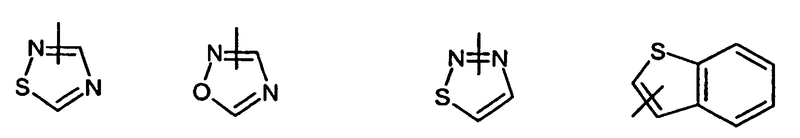 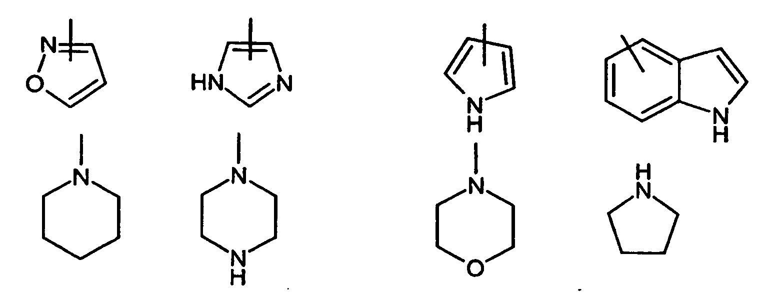 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann; und/oder die cyclischen Reste jeweils ein- bis dreifach durch Phenyl oder einen Heterocyclus aus der Gruppe 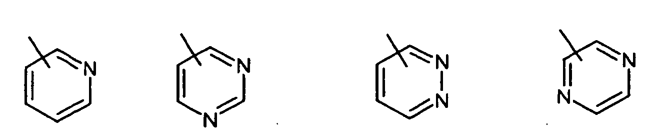 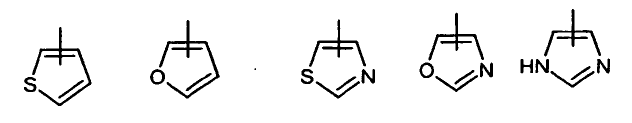 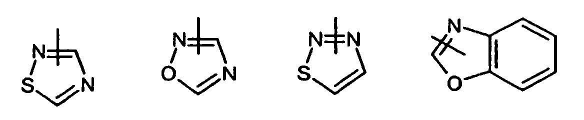 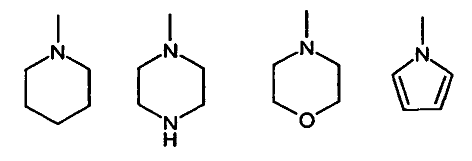 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl, Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet, und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können; und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
- R³: Wasserstoff, F, Cl, Br, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
- W: CH₂CH₂, CH=CH, CH₂O, OCH₂, CH₂OCH₂, CH₂NH, NHCH₂ oder CH₂NHCH₂ bedeutet,
- U: geradkettiges Alkylen mit bis zu 4 Kohlenstoffatomen, O, NH, S, SO oder SO₂ bedeutet,
- A: fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
- R²: COOR²⁶ oder CN bedeutet,
worin
R²⁶ Wasserstoff oder geradkettiges oder verzweigtes Alkyl mit bis zu 4 Kohlenstoffatomen bedeutet;
- X: geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls enem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
- R¹: CN oder COOR³⁵ bedeutet,
worin
R³⁵ Wasserstoff oder geradkettiges oder verzweigtes Alkyl mit bis zu 6 Kohlenstoffatomen bedeutet.

Erfindungsgemäß ganz besonders bevorzugt sind Verbindungen der Formel (I), bei denen R¹ und R² jeweils für COOH stehen.

Insbesondere bevorzugt sind hierbei Verbindungen, bei denen B für Phenyl, R³ für H, W für CH₂CH₂ oder CH=CH, X für (CH₂)₄, U für CH₂, A für Phenyl und R¹ und R² für COOH stehen, wobei V, Q, Y und r wie vorstehend definiert sind.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch Racematspaltung oder durch chromatographische Trennung, in die stereoisomer einheitlichen Bestandteile trennen. In den erfindungsgemäßen Verbindungen vorhandene Doppelbindungen können in der cis- oder trans- Konfiguration (Z- oder E-Form) vorliegen.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, α-Methylethylen, β-Methylethylen, α-Ethylethylen, β-Ethylethylen, Butylen, α-Methylpropylen, β-Methylpropylen, γ-Methylpropylen, α-Ethylpropylen, β-Ethylpropylen, γ-Ethylpropylen, Pentylen, Hexylen, Heptylen, Octylen, Nonylen, Decylen, Dodeylen und Eicosylen genannt.
Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.
Alkinyl steht im allgemeinen für einen geradkettigen oder verzweigten Kohlenwasserstoffrest mit 2 bis 20 Kohlenstoffatomen und einer oder mehreren, bevorzugt mit einer oder zwei Dreifachbindungen. Beispielsweise seien Ethinyl, 2-Butinyl, 2-Pentinyl und 2-Hexinyl benannt.
Alkendiyl steht im allgemeinen für eine geradkettige oder verzweigte Kohlenwasserstoffbrücke mit 2 bis 20 Kohlenstoffatomen und einer oder mehreren, bevorzugt mit einer oder zwei Doppelbindungen. Beispielsweise seien Ethen-1,2-diyl, Propen-1,3-diyl, Propen-1,2-diyl, 1-Buten-1,4-diyl, 1-Buten-1,3-diyl, 1-Buten-1,2-diyl, 2-Buten-1,4-diyl, 2-Buten-1,3-diyl, 2-Buten-2,3-diyl genannt.
Alkindiyl steht im allgemeinen für eine geradkettige oder verzweigte Kohlenwasserstoffbrücke mit 2 bis 20 Kohlenstoffatomen und einer oder mehreren, bevorzugt mit einer oder zwei Dreifachbindungen. Beispielsweise seien Ethin-1,2-diyl, Propin-1,3-diyl, 1-Butin-1,4-diyl, 1-Butin-1,3-diyl, 2-Buten-1,4-diyl genannt.
Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Alkoxyalkyl steht im allgemeinen für einen Alkylrest mit bis zu 8 Kohlenstoffatomen, der durch einen Alkoxyrest mit bis zu 8 Kohlenstoffatomen substituiert ist.
Alkoxycarbonyl kann beispielsweise durch die Formel 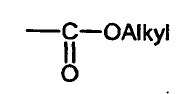 dargestellt werden.
Alkyl steht hierbei im allgemeinen für einen geradkettigen oder verzweigten Kohlenwasserstoffrest mit 1 bis 13 Kohlenstoffatomen. Beispielsweise seien die fölgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkoxy steht im Rahmen der Erfindung für einen Alkoxyrest, dessen Kohlenwasserstoffrest ein Cycloalkylrest ist. Der Cycloalkylrest hat im allgemeinen bis zu 8 Kohlenstoffatome. Als Beispiele seien genannt: Cyclopropyloxy und Cyclohexyloxy. Die Begriffe "Cycloalkoxy" und "Cycloalkyloxy" werden synonym verwendet.
Aryl steht im allgemeinen für einen aromatischen Rest mit 6 bis 10 Kohlenstoffatomen. Bevorzugte Arylreste sind Phenyl und Naphthyl.
Halogen steht im Rahmen der Erfindung für Fluor, Chlor, Brom und lod.
Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Piperidyl. Bevorzugt sind Thiazolyl, Furyl, Oxazolyl, Pyrazolyl, Triazolyl, Pyridyl, Pyrimidinyl, Pyridazinyl und Tetrahydropyranyl. Der Begriff "Heteroaryl" (bzw. "Hetaryl") steht für einen aromatischen heterocyclischen Rest. Bei den in der vorliegenden Anmeldung gezeigten Heterocyclenstrukturen ist jeweils nur eine Bindung zur benachbarten Gruppe angedeutet, z.B. bei den Heterocyclenstrukturen, die für Y in Frage kommen, die Bindung zur Einheit Q.

Unabhängig davon können diese Heterocyclenstrukturen jedoch wie angegeben weitere Substituenten tragen.

Die vorliegende Erfindung betrifft weiterhin ein Verfahren zur Herstellung der Verbindungen der Formel (I) 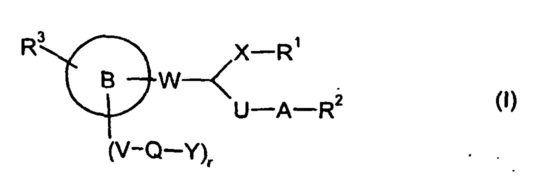 umfassend
[A] die Umsetzung von Aldehyden der allgemeinen Formel (II) 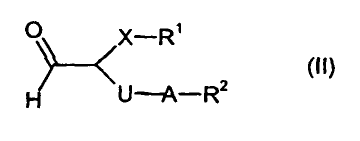 worin
   - R¹, R², A, U und X: die vorstehend angegebene Bedeutung haben, mit der Maßgabe, dass R¹ und R² nicht für freie Carbonsäuregruppen stehen dürfen,
   mit Phosphorverbindungen der allgemeinen Formel (III) 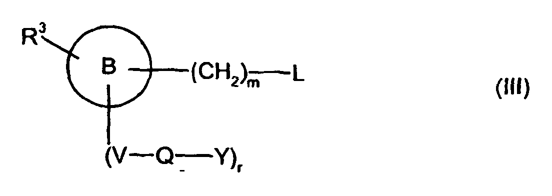 worin
   - R³, B, V, Q, Y und r: die vorstehend angegebenen Bedeutungen haben,
   - m: eine ganze Zahl von 1 bis 5 bedeutet, und
   - L: für einen Rest der Formel 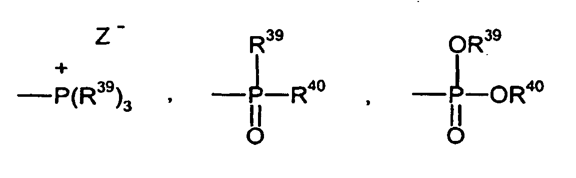 steht, worin
   R³⁹ und R⁴⁰ unabhängig voneinander geradkettiges oder verzweigtes Alkyl mit bis zu 12 Kohlenstoffatomen oder Phenyl bedeuten, und
   Z ein Halogenidanion oder Tosylatanion bedeutet, in inerten Lösungsmitteln in Gegenwart einer Base,
   und gegebenenfalls die anschließende teilweise oder vollständige Hydrolyse der Reste R¹ und R² zu freien Carbonsäuregruppen;
   oder
[B] Verbindungen der Formel (IV), 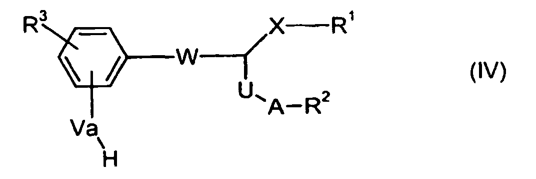 worin
   - Va: für O oder S steht
   - R¹, R², R³, U, W,A, X: die vorstehend angegebene Bedeutung haben
   mit Verbindungen der Formel (V) 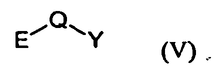 umsetzt,
   worin
   - Q, Y: die gleichen Bedeutungen wie vorstehend definiert haben,
   - E: entweder eine Abgangsgruppe bedeutet, die in Gegenwart einer Base substituiert wird, oder eine gegebenenfalls aktivierte Hydroxyfunktion ist;
   oder
[C] Verbindungen der Formel (VI), 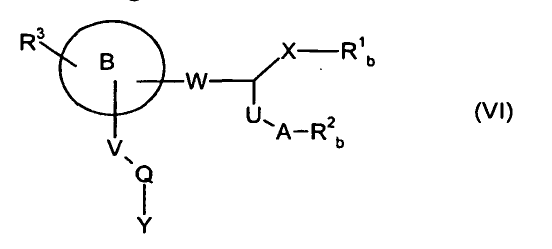 worin
   - R³, V, Q, Y, W, U, A, B: die gleichen Bedeutungen wie vorstehend definiert haben,
   - R¹_{b} und R²_{b}: jeweils unabhängig für CN oder COOAlk stehen, wobei Alk für einen geradkettigen oder verzweigten Alkylrest mit bis zu 6 Kohlenstoffatomen steht,
   mit wässrigen Lösungen starker Säuren oder starker Basen in die entsprechenden freien Carbonsäuren überführt.
   oder
[D] Verbindungen der Formel (VII) 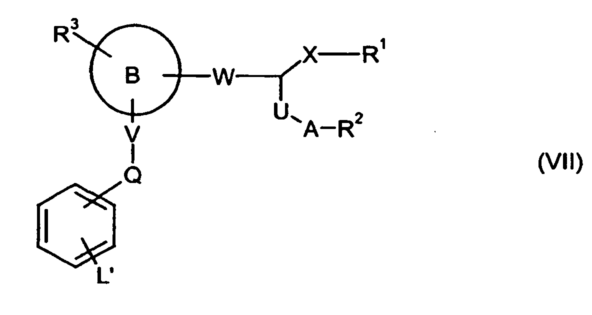 worin
   - R¹, R², R³, V, Q, X, W, U, A, B: die gleichen Bedeutungen wie vorstehend definiert haben,
   - L': für Br, I oder die Gruppe CF₃SO₂-O steht,
   mit Verbindungen der Formel (VIII)

   M-Z' (VIII)

   worin
   - M: für einen Aryl oder Heteroarylrest, einen geradkettigen oder verzweigten Alkyl-, Alkenyl- oder Alkinylrest oder Cycloalkylrest oder für einen Arylalkyl-, einen Arylalkenyl- oder einen Arylalkinylrest steht,
   - Z': für die Gruppierungen -B(OH)₂, -CH≡CH, -CH=CH₂ oder -Sn(nBu)₃ steht
   in Gegenwart einer Palladiumverbindung, gegebenenfalls zusätzlich in Gegenwart eines Reduktionsmittels und weiterer Zusatzstoffe und in Gegenwart einer Base umsetzt;
   oder
[E] Verbindungen der Formel (VII) 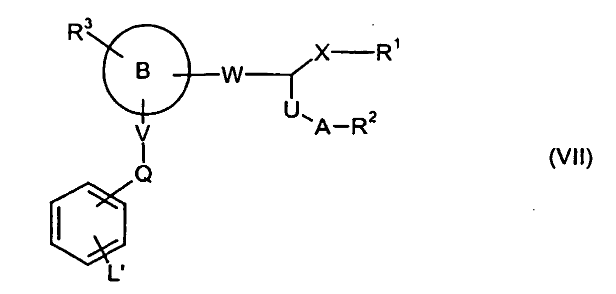 worin
   - R¹, R², R³, V, Q, X, W, U, A, B: die gleichen Bedeutungen wie vorstehend definiert haben,
   - L': für Br, I oder die Gruppe CF₃SO₂-O steht,
   mit Verbindungen der Formel (IX)

   NHR^{a}R^{b} (IX)

   worin
   - R^{a} und R^{b}: unabhängig voneinander für Wasserstoff oder einen geradkettigen oder verzweigten Alkylrest mit bis zu 8 Kohlenstoffatomen stehen oder zusammen mit dem Stickstoffatom, an das sie gebunden sind, einen einen aromatischen Heterocyclus mit 1 bis 9 Kohlenstoffatomen und bis zu 3 Heteroatomen aus der Reihe S, N und/oder O bilden können, in Gegenwart einer Palladiumverbindung, gegebenenfalls zusätzlich in Gegenwart eines Reduktionsmittels und weiterer Zusatzstoffe und in Gegenwart einer Base umsetzt;
   oder
[F] Verbindungen der Formel (IV), 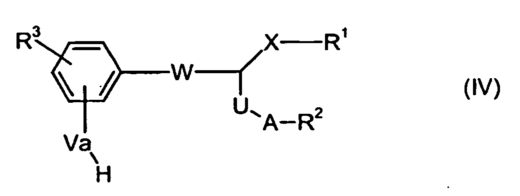 worin
   - Va: für O oder S steht
   - R¹, R², R³, U, W,A, X: die vorstehend angegebene Bedeutung haben
   mit Verbindungen der Formel (X) 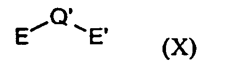 umsetzt,
   worin
   - Q': die gleichen Bedeutung wie Q hat oder Phenyl bedeutet,
   - E und E': jeweils unabhängig voneinander entweder eine Abgangsgruppe bedeutet, die in Gegenwart einer Base substituiert wird, oder eine gegebenenfalls aktivierte Hydroxyfunktion ist, oder ein eine derartige Gruppe enthaltender Rest ist;
   und die so erhaltenen Verbindungen der Formel (XI) 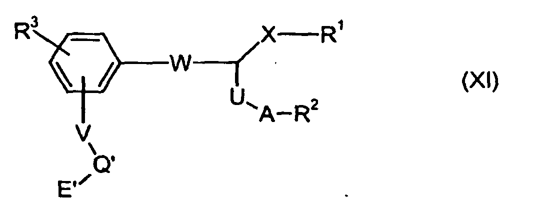 worin
   - R¹, R², R³, A, U, V, W, X und E': die vorstehend angegebenen Bedeutungen haben,
   - Q': die gleiche Bedeutung wie Q hat oder 1,4-CH₂-Ph-CH₂- bedeutet,
   mit Aminen der Formel (XII)

   NHR^{a}R^{b} (XII)

   umsetzt;
   worin
   - R^{a} und R^{b}: unabhängig voneinander für Wasserstoff oder einen geradkettigen oder verzweigten Alkylrest mit bis zu 8 Kohlenstoffatomen stehen oder zusammen mit dem Stickstoffatom, an das sie gebunden sind, einen einen aromatischen Heterocyclus mit 1 bis 9 Kohlenstoffatomen und bis zu 3 Heteroatomen aus der Reihe S, N und/oder O bilden können,
   oder
[G] Verbindungen der Formel (XIII) 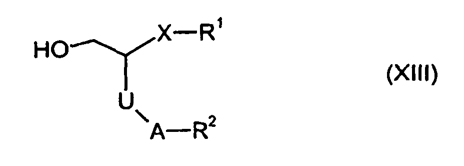 worin
   - R¹, R², A, U, X: die vorstehend angegebenen Bedeutungen haben,
   mit Verbindungen der Formel (XIV) 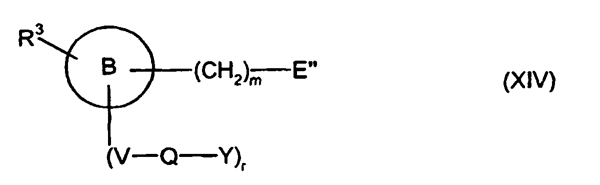 worin
   - R³, V, Q, Y, r und B: die vorstehend angegebenen Bedeutungen haben,
   - m: eine ganze Zahl von 1 bis 5 bedeutet, und
   - E": entweder eine Abgangsgruppe bedeutet, die in Gegenwart einer Base substituiert wird, oder eine gegebenenfalls aktivierte Hydroxyfunktion ist;
   umsetzt;
   oder
[H] Verbindungen der Formel (XV) 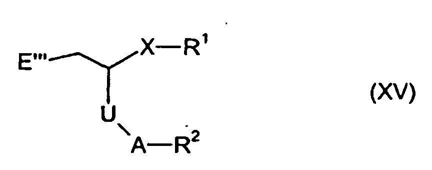 worin
   - R¹, R², A, U, X: die vorstehend angegebenen Bedeutungen haben,
   - E"': entweder eine Abgangsgruppe bedeutet, die in Gegenwart einer Base substituiert wird, oder eine gegebenenfalls aktivierte Hydroxyfunktion ist;
   mit Verbindungen der Formel (XVI) 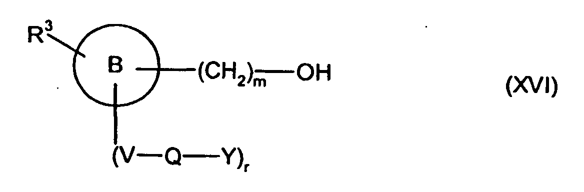 worin
   - R³, V, Q, Y, r und B: die vorstehend angegebenen Bedeutungen haben,
   - m: eine ganze Zahl von 1 bis 5 bedeutet,
   umsetzt;
   oder
[I] Verbindungen der Formel (XVII) 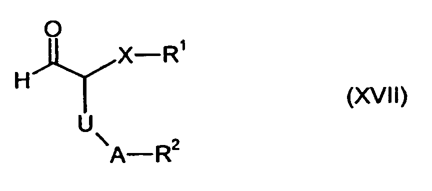 worin
   - R¹, R², A, U, X: die vorstehend angegebenen Bedeutungen haben,
   mit Verbindungen der Formel (XVIII) 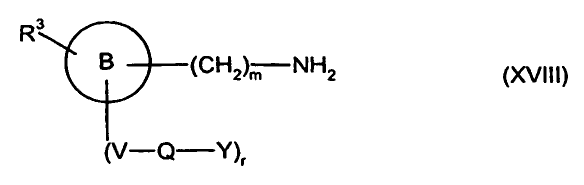 worin
   - R³, V, Q, Y, r und B: die vorstehend angegebenen Bedeutungen haben,
   - m: eine ganze Zahl von 0 bis 5 bedeutet,
   zunächst zu einer Schiffschen Base umsetzt und diese dann mit gängigen Reduktionsmitteln reduziert oder direkt unter den Bedingungen einer reduktiven Alkylierung in Gegenwart eines Reduktionsmittels umsetzt;
   oder
[J] Verbindungen der Formel (XIX) 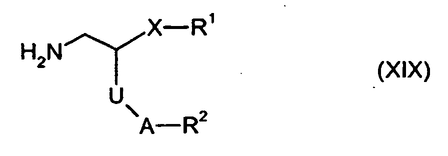 worin
   - R¹, R², A, U, X: die vorstehend angegebenen Bedeutungen haben,
   mit Verbindungen der Formel (XX) 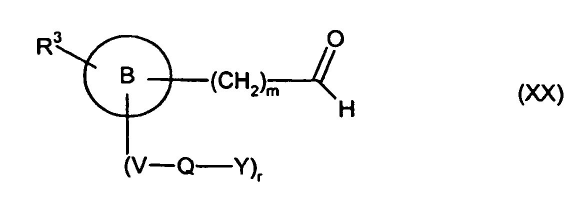 worin
   - R³, V, Q, Y, r und B: die vorstehend angegebenen Bedeutungen haben,
   - m: eine ganze Zahl von 0 bis 5 bedeutet,
   zunächst zu einer Schiffschen Base umsetzt und diese dann mit gängigen Reduktionsmitteln reduziert oder direkt unter den Bedingungen einer reduktiven Alkylierung in Gegenwart eines Reduktionsmittels umsetzt,
   oder
[K] Aldehyde der Formel (XXI) 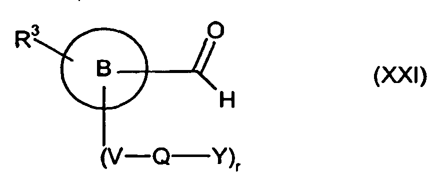 worin
   - R³, V, Q, Y, r und B: die vorstehend angegebenen Bedeutungen haben,
   mit Phosphorverbindungen der Formel (XXII) 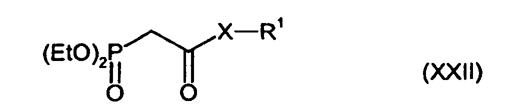 worin
   - X und R¹: die vorstehend angegebenen Bedeutungen haben,
   zu Verbindungen der Formel (XXIII) 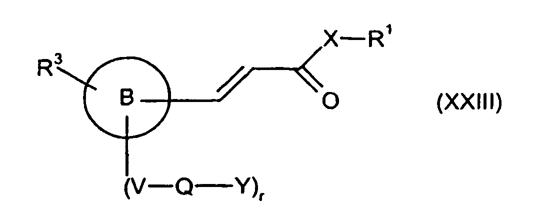 worin
   - R³, V, Q, Y, r, B, X und R¹: die vorstehend angegebenen Bedeutungen haben,
   umgesetzt und anschließend durch aufeinanderfolgende Reduktion der Alkengruppe und der Carbonylgruppe und anschließende Substitution der durch Reduktion der Carbonylgruppe erzeugten Hydroxygruppe beziehungsweise des durch Umsetzung mit Halogenierungsmitteln aus der Hydroxygruppe erzeugten Halogenrestes mit Alkoholen, primären Aminen oder Thiolen sowie gegebenenfalls anschließende Oxidation zu den entsprechenden Sulfoxid- oder Sulfonverbindungen in Verbindungen der Formel (XXIV) überführt, 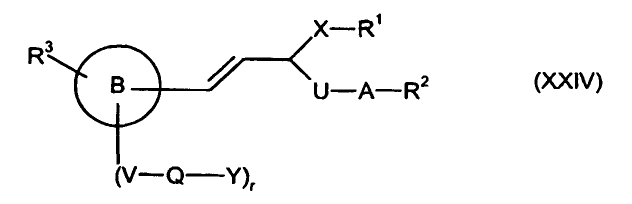 worin
   - R³, V, Q, Y, r, B, X, A, R² und R¹: die vorstehend angegebenen Bedeutungen haben,
   - U: für O, NH oder S steht.

Gemäß der vorliegenden Erfindung bedeutet beim Verfahren [A] Z bevorzugt ein Halogenidanion, insbesondere bevorzugt Chlorid, Bromid oder Iodid.

Gemäß der vorliegenden Erfindung erfolgt die beim Verfahren [A] gegebenenfalls durchzuführende teilweise oder vollständige Hydrolyse zu den entsprechenden freien Carbonsäuregruppen vorzugsweise mit starken Säuren wie z.B. HCl oder mit starken Basen wie z.B. NaOH oder LiOH, die in wässriger Lösung oder Lösungsmittelgemischen aus Wasser mit Alkoholen wie z.B. Methanol oder Ethern vorliegen.

Für das erfindungsgemäße Verfahren [A] bevorzugte inerte Lösungsmittel sind herkömmliche organische Lösungsmittel, welche sich unter den Reaktionsbedingungen nicht verändern. Vorzugsweise können für das erfindungsgemäße Verfahren [A] Ether wie Diethylether, Butylmethylester, Dioxan, Tetrahydrofuran, Glykoldimethylether oder Diethylenglykoldimethylether, oder Kohlenwasserstoffe wie Benzol, Toluol, Xylol oder Petrolether, oder Amide wie Dimethylformamid oder Hexamethylphosphortriamid, oder 1,3-Dimethyl-imidazolidin-2-on, 1,3-Dimethyl-tetrahydropyrimidin-2-on oder Dimethylsulfoxid verwendet werden. Es ist selbstverständlich auch möglich, Gemische der vorstehend genannten Lösungsmittel zu verwenden.

Für das erfindungsgemäße Verfahren [A] bevorzugte Basen umfassen herkömmlicherweise für basische Reaktionen eingesetzte basische Verbindungen. Vorzugsweise können Alkalimetallhydride wie beispielsweise Natriumhydrid oder Kaliumhydrid, oder Alkalimetallalkoholate wie Natriummethanolat, Natriumethanolat, Kaliummethanolat, Kaliumethanolat oder Kalium-t.-butylat, oder Amide wie Natriumamid oder Lithiumdiisopropylamid oder Natriumhexamethyldisilazan, oder Organolithium-Verbindungen wie Phenyllithium, Butyllithium oder Methyllithium verwendet werden. Gegebenenfalls kann beim erfindungsgemäßen Verfahren [A] zur Optimierung der Reaktion ein herkömmlicher Kronenether wie 18-Krone-6 zugegeben werden.

Die Wahl des Lösungsmittels oder Base hängt von der Stabilität, Empfindlichkeit gegenüber Hydrolyse oder der CH-Aktivität der entsprechenden Phosphorverbindung ab. Ether wie Diethylether, Tetrahydrofuran, Dimethoxyethan oder Dioxan, zusammen mit einem Co-Lösungsmittel wie Dimethylformamid oder 1,3-Dimethyltetrahydropyridin-2-on oder 1,3-Dimethylimidazolidin-2-on, werden als Lösungsmittel besonders bevorzugt verwendet. Alkalimetallalkoholate wie Kalium-t.-butylat oder Organolithiumverbindungen wie Phenyllithum oder Butyllithium oder Natriumhydrid werden als Basen besonders bevorzugt verwendet.

Die Reaktion kann im allgemeinen in einem Temperaturbereich von -80°C bis +70°C, vorzugsweise von -80°C bis +20°C ausgeführt werden.

Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Bei der Durchführung der Reaktion werden die Phosphorverbindungen im allgemeinen in einer Menge von 1 - 2 mol, bezogen auf 1 mol Aldehyd eingesetzt. Die Basen werden im allgemeinen in einer Menge von 1 - 5 mol, vorzugsweise von 1 -2 mol, bezogen auf 1 mol Phosphorverbindung eingesetzt.

Das erfindungsgemäße Verfahren [A] kann beispielsweise durchgeführt werden, indem die Base und anschließend das Aldehyd, gegebenenfalls in einem Lösungsmittel, zu der in einem Lösungsmittel gelösten oder suspendierten Phosphorverbindung zugegeben werden, und anschließend gegebenenfalls das Gemisch erhitzt wird. Die Aufarbeitung erfolgt auf herkömmliche Weise, durch Extraktion, Chromatographie und/oder Kristallisation.

Bei der Durchführung des erfindungsgemäßen Verfahrens [A] ist es ebenfalls möglich, anstelle der vorstehend genannten Phosphoniumsalze die entsprechenden Phosphorane (U ist gleich -P(R¹²)₃=CHR) zu verwenden, die vorher in einer getrennten Reaktion aus den entsprechenden Phosphoniumsalzen im basischen Milieu dargestellt wurden. Es hat sich jedoch als vorteilhaft erwiesen, die Reaktion mit den Phosphorverbindungen in Gegenwart von Basen als Eintopf-Verfahren durchzuführen.

Die Phosphorverbindungen der allgemeinen Formel (III) können auf folgenden verschiedenen Wegen hergestellt werden. 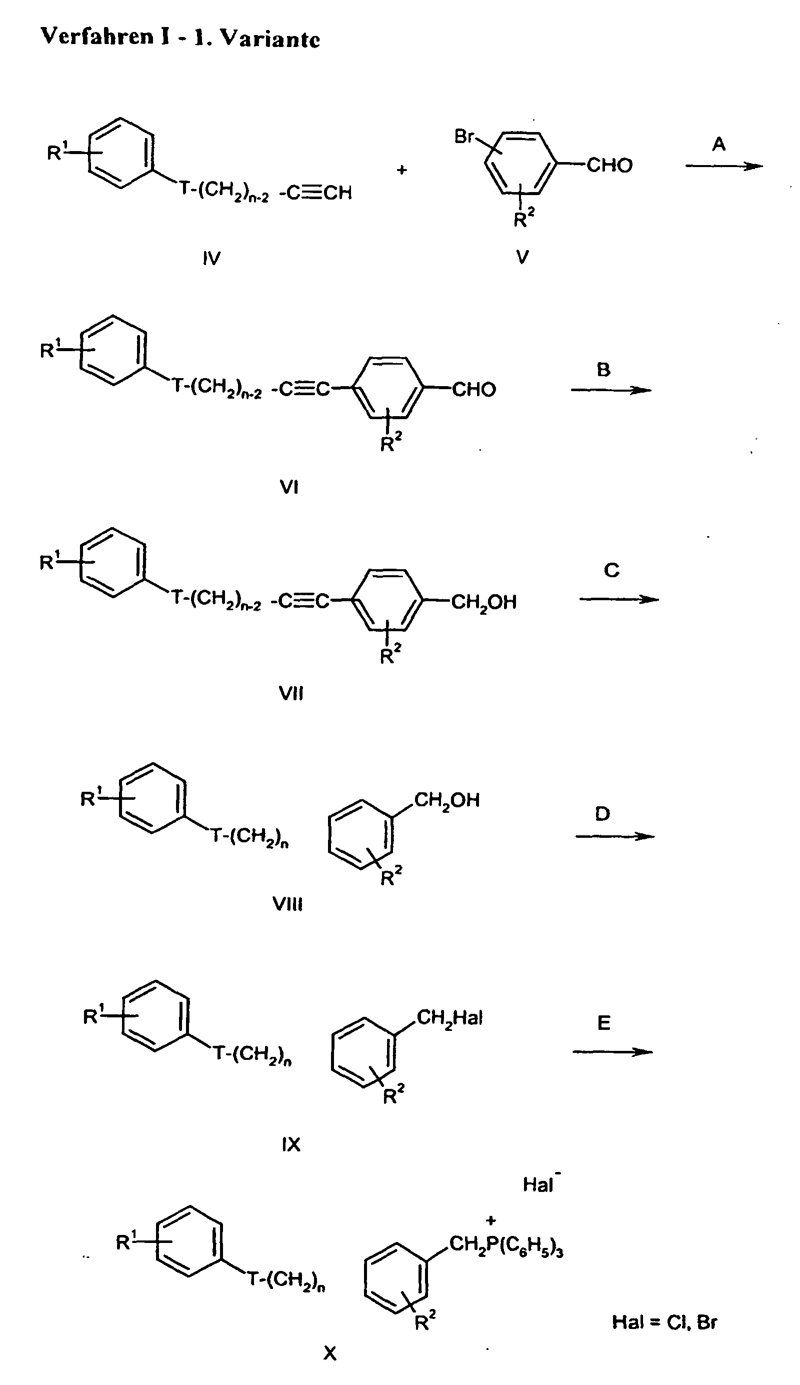 wobei das Verfahren nicht auf die hier beispielhaft dargestellten Verbindungen eingeschränkt ist, bei denen Y und B für Phenyl, Q für eine Alkylenkette steht und V fehlt, sondern grundsätzlich mit Verbindungen mit beliebigen Resten V, Q, Y und B durchgeführt werden kann.

Beim ersten Reaktionsschritt A dieser Variante werden die Acetylenverbindungen (IVa) mit den Brombenzaldehyden (Va) in Lösungsmitteln wie Triethylamin, Acetonitril, Pyridin oder Gemischen davon, vorzugsweise in Triethylamin, in Gegenwart von Kupfer-(I)-Salzen und Palladium-(0)-Verbindungen, vorzugsweise in Gegenwart von Kupfer-(I)-Halogeniden wie beispielsweise Kupferiodid, und bis-(Triphenylphosphan)-Palladium-(II)-chlorid in einem Temperaturbereich von -40°C bis +80°C, vorzugsweise von 0°C bis +40°C umgesetzt.

Im zweiten Reaktionsschritt B wird die Formylverbindung (VIa) in Lösungsmitteln wie Alkoholen, beispielsweise Methanol, Ethanol, Propanol oder Isopropanol, oder Ethern wie Diethylether, Tetrahydrofuran oder Dioxan, oder in basischen Lösungsmitteln wie Triethylamin, Pyridin oder Dimethylformamid, oder in Wasser oder in Gemischen aus den vorstehend genannten Lösungsmitteln unter Verwendung komplexierter Hydride wie beispielsweise Borhydriden oder Aluminiumhydriden, vorzugsweise Natriumborhydrid oder Lithiumaluminiumhydrid, als Reduktionsmittel in einem Temperaturbereich von -40°C bis +60°C, vorzugsweise von 0°C bis +40°C, zu den Hydroxylverbindungen (VIIa) reduziert.

Im dritten Reaktionsschritt C werden die Verbindungen (VIIa) in inerten Lösungsmitteln wie Alkoholen, beispielsweise Methanol, Ethanol, Propanol oder Isopropanol, oder Kohlenwasserstoffen wie Benzol, Toluol oder Xylol oder in Ethern wie Diethylether oder Tetrahydrofuran, oder in Ethylacetat, insbesondere bevorzugt in Methanol, in Gegenwart von Edelmetall-Katalysatoren wie Palladium oder Platin in einem Temperaturbereich von -30°C bis +80°C, vorzugsweise von 0°C bis +40°C, unter einem Druck von 1 bar bis 50 bar, vorzugsweise von 1 bar bis 20 bar hydriert.

Die Schritte B und C können auch in umgekehrter Reihenfolge ausgeführt werden.

Im vierten Schritt D werden die hydrierten Verbindungen VIIIa durch Umsetzung mit Bromierungsmitteln wie beispielsweise Phosphortribromid, Sulfonylbromid, Bromwasserstoff oder Tetrabrommethan/Triphenylphosphan in inerten Lösungsmitteln wie Ether, beispielsweise Diethylether oder Tetrahydrofuran, oder Kohlenwasserstoffen wie Benzol oder Toluol oder besonders bevorzugt chlorierten Kohlenwasserstoffen wie Methylenchlorid oder Chloroform, in einem Temperaturbereich von -20°C bis +60°C, vorzugsweise von 0°C bis +40°C bromiert. Es können aber auch die entsprechenden Chlorverbindungen verwendet werden, die beispielsweise durch Umsetzung der Verbindungen VIIIa mit SOCl₂ erhältlich sind.

Im fünften Reaktionsschritt E werden die bromierten oder chlorierten Verbindungen (IXa) mit Triphenylphosphan in inerten Lösungsmitteln wie Acetonitril oder Kohlenwasserstoffen wie Benzol, Toluol oder Xylol, oder Benzonitril oder Dimethylformamid oder Dimethylsulfoxid oder in einem Alkohol wie Methanol, Ethanol, Propanol, Butanol oder Isopropanol oder ohne Lösungsmittel in einem Temperaturbereich von 0°C bis +200°C, vorzugsweise von +20°C bis +180°C unter Darstellung der Phosphoniumsalze Xa umgesetzt.

Über dieses Verfahren sind die erfindungsgemäßen Verbindungen der Formel (I) zugänglich, bei denen V fehlt. Bei den Verbindungen der Formeln (IVa) bis (Xa) hat der Rest R³ die gleiche Bedeutung wie vorstehend definiert.

Die Acetylenverbindungen der Formel (IVa) sind beispielsweise durch Umsetzung entsprechender Amine oder cyclischer Substrate mit einer nukleophilen Gruppe, beispielsweise Phenolderivate, Anilinderivate oder carbanionische Derivate wie Grignard-Reagenzien, mit ω-Halogenalkinen in Gegenwart von Basen auf bekannte Weise erhältlich. Besonders bevorzugt sind hierbei ω-Chloralkine wie beispielsweise 5-Chlor-1-pentin. Als Basen können beispielsweise Metallhydride wie Natriumhydrid verwendet werden. Die Umsetzung zu den Acetylenverbindungen der Formel (IVa) kann in organischen Lösungsmitteln wie beispielsweise Ethern, insbesondere Tetrahydrofuran, bei Temperaturen von +20°C bis +80°C unter Inertgasatmosphäre, beispielsweise Argon durchgeführt werden. In einigen Fällen kann es vorteilhaft sein, Komplexierungsmittel wie Hexaphosphorsäuretriamid zuzugeben. Alternativ können die Acetylenverbindungen (IVa) durch Umsetzung entsprechender Substrate mit einer nukleophil substituierbaren Gruppe, beispielsweise ω-Halogenalkylphenylverbindungen, vorzugsweise ω-Chloralkylphenylverbindungen, mit Acetyliden wie beispielsweise Natriumacetylid oder Lithiumacetylid unter dem Fachmann bekannten Bedingungen erhalten werden (vgl. z.B. J. March, Advanced Organic Chemistry, 3.Auflage, Wiley, S. 429). 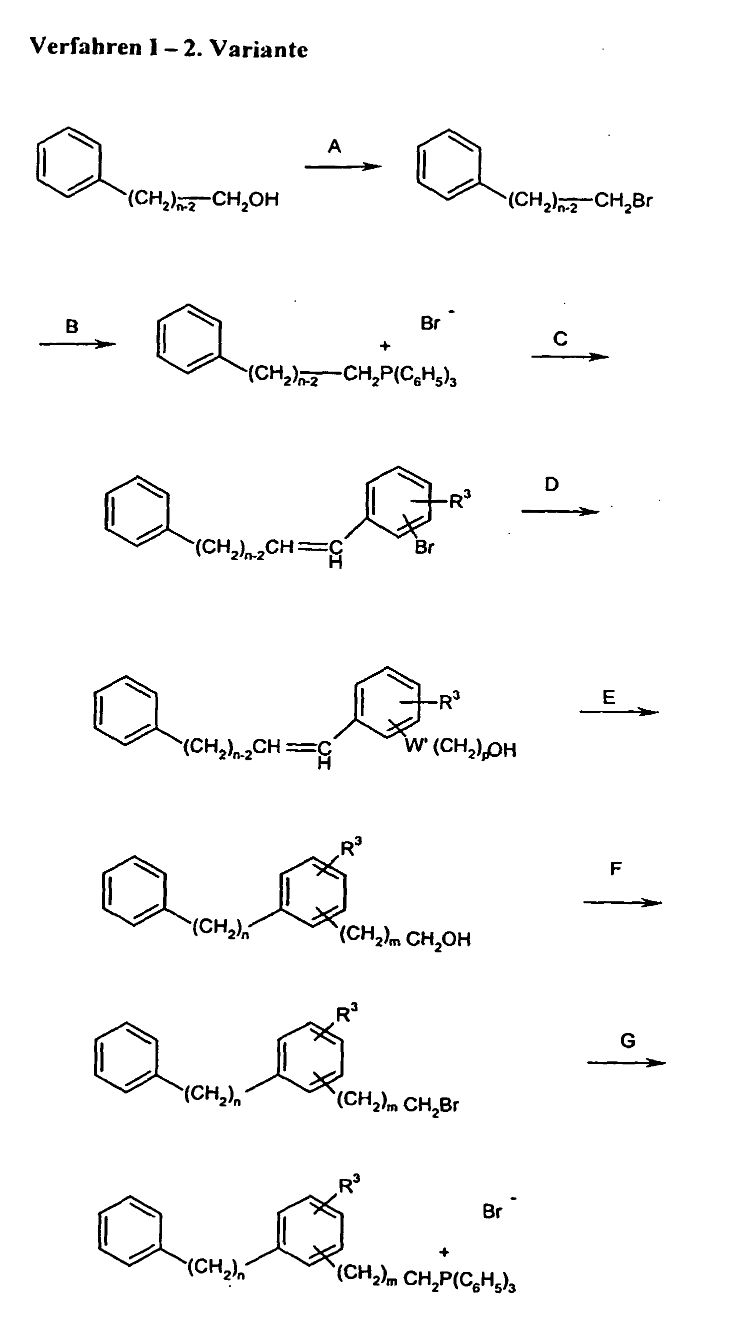 wobei das Verfahren nicht auf die hier beispielhaft dargestellten Verbindungen eingeschränkt ist, bei denen Y und B für Phenyl, Q für eine Alkylenkette steht und V fehlt, sondern grundsätzlich mit Verbindungen mit beliebigen Resten V, Q, Y und B durchgeführt werden kann.

Im ersten Reaktionsschritt werden die als Ausgangsverbindungen verwendeten Alkohole bromiert, wobei als Bromierungsmittel beispielsweise die Verbindungen eingesetzt werden können, die im Schritt D der 1. Variante des Verfahrens 1 aufgeführt sind.

Die so erhaltenen Bromide werden wie im Schritt E der 1. Variante von Verfahren I mit Triphenylphosphan umgesetzt.

Im nächsten Reaktionsschritt wird wie vorstehend erläutert das reaktive Ylid erzeugt und dieses mit einem Brombenzaldehyd mit gewünschtem Substitutionsmuster umgesetzt.

Aus der so erhaltenen Verbindung können durch Umsetzung mit einer Base, vorzugsweise t-Butyllithium in einem inerten Lösungsmittel (Tetrahydrofuran), bei tiefen Temperaturen und anschließender Zugabe eines entsprechenden Elektrophils wie Paraformaldehyd oder Ethylenoxid die entsprechenden primären Alkohole (W' ist eine Direktbindung) erhalten werden. Wahlweise können die so erhaltenen Verbindungen mit einem gegebenenfalls geschützten Hydroxyalkin wie dem Tetrahydropyranylether von Propargylalkohol unter den gleichen Bedingungen wie im Verfahrensschritt I der 1. Variante von Verfahren I umgesetzt (W' bedeutet C=C) und anschließend durch eine Hydrierung, die analog zu Schritt C der 1. Variante von Verfahren I durchgeführt werden kann, zu den primären Alkoholen umgewandelt werden. Die so erhaltenen primären Alkohole werden analog zur 1. Variante des Verfahrens I in die entsprechenden Phosphoniumsalze überführt.

Über dieses Verfahren sind die erfindungsgemäßen Verbindungen der Formel (1) zugänglich, bei denen V fehlt.

Die als Ausgangsverbindungen bei diesem Verfahren verwendeten Alkohole, beispielsweise Hydroxyalkyloxyphenylverbindungen beziehungsweise Hydroxyalkylphenylverbindungen, sind entweder käuflich erhältlich oder durch dem Fachmann bekannte herkömmliche Reaktionen darstellbar.

Bei den im vorstehenden Diagramm aufgeführten Verbindungen hat der Rest R³ die gleiche Bedeutung wie vorstehend definiert. 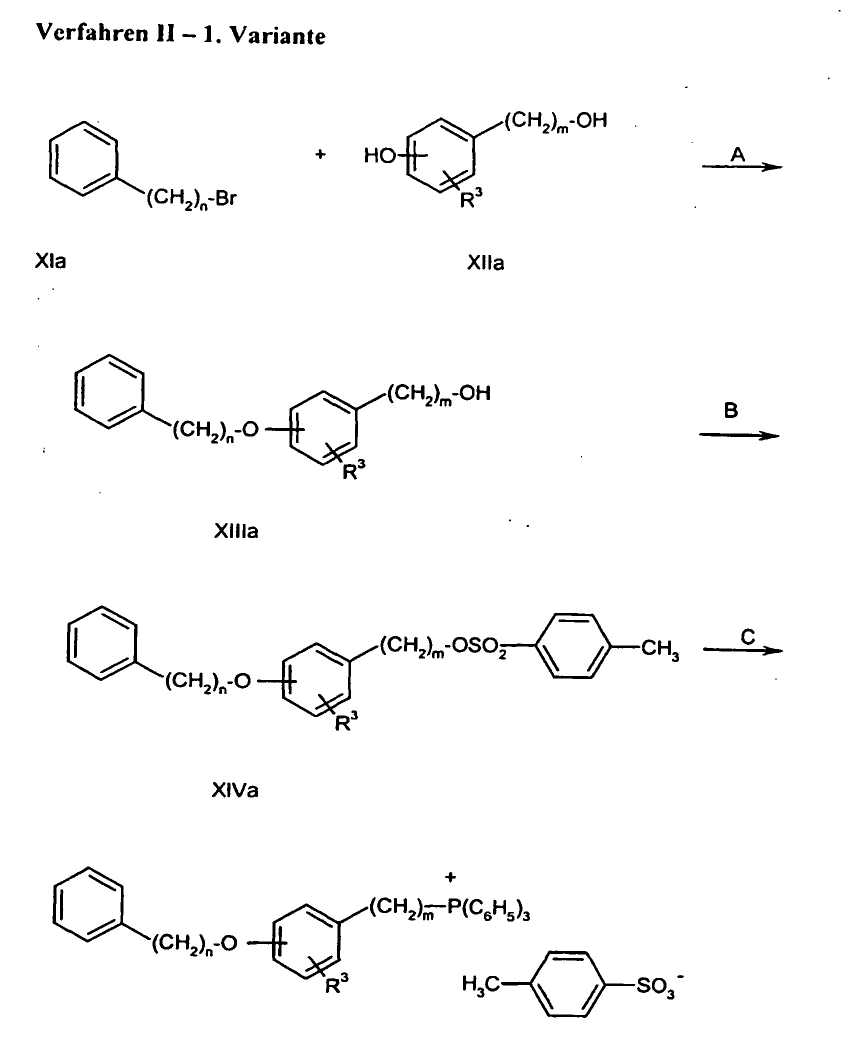 wobei das Verfahren nicht auf die hier beispielhaft dargestellten Verbindungen eingeschränkt ist, bei denen Y und B für Phenyl, Q für eine Alkylenkette steht und V für O steht, sondern grundsätzlich mit Verbindungen mit beliebigen Resten V, Q, Y und B durchgeführt werden kann.

Im ersten Reaktionsschritt dieser Variante werden die Bromverbindungen (XIa) mit den Phenolen (XIIa) in bevorzugten Lösungsmitteln wie Wasser oder Alkoholen wie beispielsweise Methanol, Ethanol, Propanol oder Isopropanol, oder Ethern wie Diethylether, Tetrahydrofuran, Dioxan oder Dimethyloxymethan, oder Dimethylformamid oder Dimethylsulfoxid, oder Acetonitril oder Ketonen wie beispielsweise Aceton, besonders bevorzugt in Isopropanol, in Gegenwart von Basen wie Alkalimetallhydroxiden, Carbonaten oder Alkoholaten wie beispielsweise Natriumcarbonat, Kaliumcarbonat, Cäsiumcarbonat, Natriumhydroxid, Kaliumhydroxid, Natriumethanolat oder Kalium-t.-butylat in einem Temperaturbereich von 0°C bis 200°C, vorzugsweise von +20°C bis +180°C umgesetzt.

Im zweiten Schritt B werden die Phenylether (XIIIa) mit Tosylchlorid in inerten Lösungsmitteln wie Ether, beispielsweise Diethylether, Tetrahydrofuran oder Dioxan, oder Kohlenwasserstoffen wie Benzol oder Toluol, oder chlorierten Kohlenwasserstoffen wie Chloroform oder Methylenchlorid, oder in Ethylacetat, Aceton oder Acetonitril, vorzugsweise in Methylenchlorid, in Gegenwart von Basen wie Triethylamin, Pyridin oder Dimethylaminopyridin, vorzugsweise in Gegenwart von Pyridin, in einem Temperaturbereich von -30°C bis +50°C, vorzugsweise von -10°C bis +30°C umgesetzt.

Im dritten Reaktionsschritt C werden die Tosylverbindungen (XIVa) mit Triphenylphosphan in bevorzugten Lösungsmitteln wie Kohlenwasserstoffen, beispielsweise Benzol oder Toluol, Benzonitril, Acetonitril, Dimethylformamid oder Dimethylsulfoxid, oder ohne Lösungsmittel, besonders bevorzugt in Acetonitril, in einem Temperaturbereich von 0°C bis +200°C, vorzugsweise von +20°C bis +180°C unter Erhalt der Phosphoniumsalze (XVa) umgesetzt.

Bei den Schritten B und C kann die Hydroxyverbindung XIIIa auch analog zu den Schritten D und E der ersten Variante des Verfahrens A zunächst in das Bromid und anschließend in das Phosphoniumsalz überführt werden.

Über dieses Verfahren sind die erfindungsgemäßen Verbindungen der Formel (I) zugänglich, bei denen V für O steht.

Für den Fall, dass B für einen Heterocyclus steht, kann das Verfahren auch dermaßen durchgeführt werden, dass anstelle des Bromids (XIa) der entsprechende Alkohol mit einer Verbindung (XIIa) umgesetzt wird, welche anstatt der direkt am Heterocyclus befindlichen Hydroxygruppe eine geeignete Abgangsgruppe wie beispielsweise einen Halogenrest, eine Tosyl-, Mesyl- oder Triflatgruppe besitzt und zudem anstatt dem Rest (CH₂)ₘOH eine Estergruppe aufweist. Durch anschließende Reduktion der Estergruppe mit gängigen Reduktionsmitteln wie beispielsweise LiAlH₄ kann die Verbindung der Formel (XIIIa) erhalten werden. 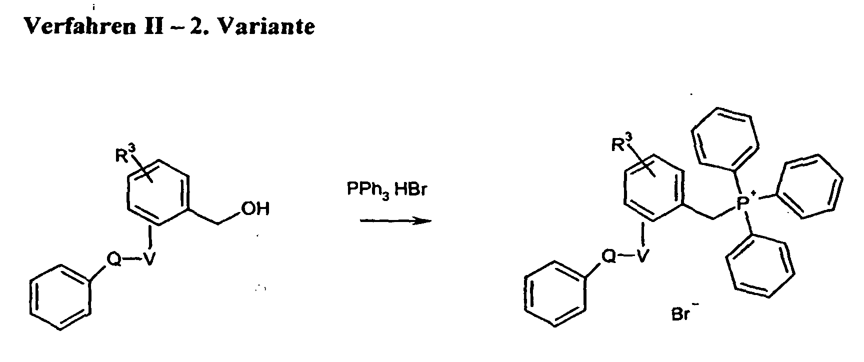 wobei das Verfahren nicht auf die hier beispielhaft dargestellten Verbindungen eingeschränkt ist, bei denen Y und B für Phenyl stehen, sondern grundsätzlich mit Verbindungen mit beliebigen Resten Y und B durchgeführt werden kann.

Bei dieser Variante werden die entsprechenden Alkohole, beispielsweise Hydroxyalkylphenylverbindungen, mit Triphenylphosphoniumhydrobromid in einem organischen Lösungsmittel wie beispielsweise Acetonitril bei einer Temperatur von +30°C bis +100°C, vorzugsweise von +50C bis +90°C umgesetzt. Die Ausgangsverbindungen können auf herkömmliche Weise erhalten werden. Beispielsweise können für den Fall, dass V gleich O ist, durch Umsetzung einer entsprechenden Halogenverbindung, beispielsweise einer Halogenalkylphenylverbindung, vorzugsweise einer Chlor- oder Bromalkylphenylverbindung wie beispielsweise Benzylbromid, mit einem entsprechenden Alkohol, beispielsweise einer Phenolverbindung wie beispielsweise 2-Hydroxybenzylalkohol, in einem organischen Lösungsmittel wie einem Alkohol, vorzugsweise Isopropanol, in Gegenwart einer Base wie beispielsweise Kaliumcarbonat bei einer Temperatur von +30 bis 100°C, vorzugsweise +50 bis 90°C umgesetzt.

Bei den in den vorstehenden Diagrammen des Verfahrens II aufgeführten Verbindungen hat der Rest R³ die gleiche Bedeutung wie vorstehend definiert. Der Rest V kann für O stehen oder fehlen. 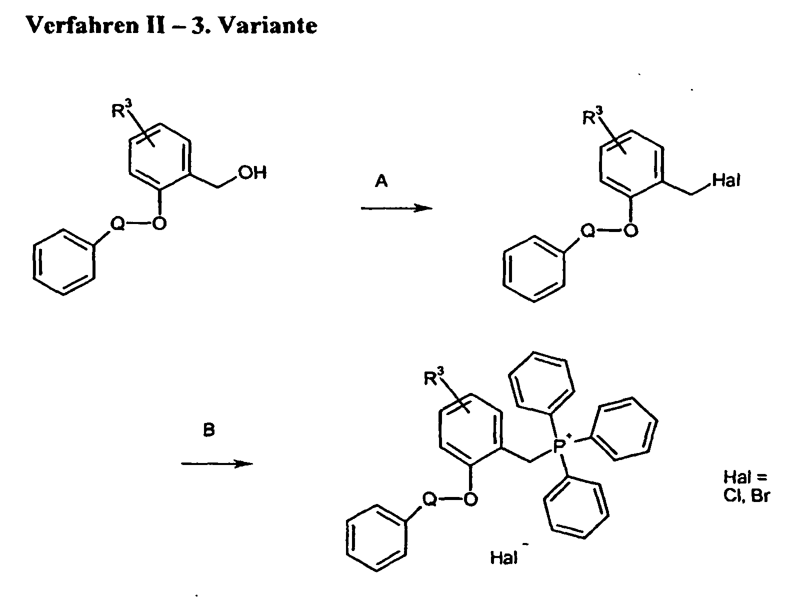 wobei das Verfahren nicht auf die hier beispielhaft dargestellten Verbindungen eingeschränkt ist, bei denen Y und B für Phenyl stehen, sondern grundsätzlich mit Verbindungen mit beliebigen Resten Y und B durchgeführt werden kann.

Bei dieser Variante wird der Alkohol zunächst gemäß dem Schritt D des Verfahrens I, Variante 1,in ein Halogenid überführt, welches anschließend analog zum Schritt E des Verfahrens I, Variante 1, zum gewünschten Phosphoniumsalz umgesetzt werden kann.

Bei dieser Variante haben Q und R³ die vorstehend angegebenen Bedeutungen.

Die Aldehyde der allgemeinen Formel (II) können in Abhängigkeit der Bedeutungen der verschiedenen Reste beispielsweise über folgende Verfahren hergestellt werden.

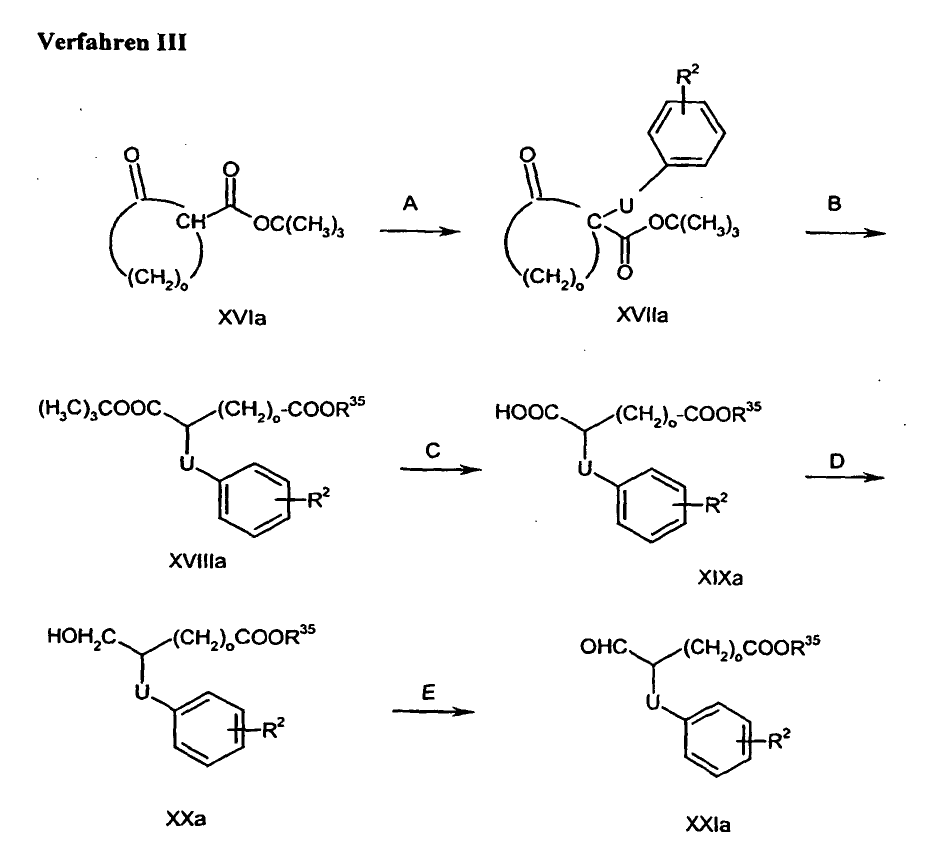

Im ersten Reaktionsschritt A dieser Variante wird das Keton XVIa (wobei o 3, 4 oder 5 bedeutet) mit 4-Halogenmethylbenzoesäureestern oder 4-Halogensulfenylbenzoesäureestem, wobei der Halogenrest vorzugsweise Chlor oder Brom ist, beziehungsweise den entsprechenden Nitrilen in inerten Lösungsmitteln wie einem Ether, beispielsweise Diethylether, Tetrahydrofuran oder Dioxan, oder Dimethylformamid, oder Dimethylsulfoxid, oder in Gemischen davon, besonders bevorzugt in Dimethylformamid, in Gegenwart von Basen wie Alkalimetallhydriden, Amiden oder Alkolaten wie Natriumhydrid, Kaliumhydrid, Lithiumdiisopropylamid, Kaliumethylat, Natriumethylat, Kaliummethylat oder Kalium-t.-butylat, besonders bevorzugt in Gegenwart von Natriumhydrid, in einem Temperaturbereich von -40°C bis +60°C, besonders bevorzugt von -20°C bis +30°C umgesetzt.

Im zweiten Reaktionsschritt B werden die Ketone XVIIa in Lösungsmitteln wie Dimethylformamid oder Alkoholen, beispielsweise Methanol, Ethanol, Propanol oder Isopropanol, oder in Wasser oder in Gemischen davon, besonders bevorzugt in Dimethylformamid oder Ethanol, in Gegenwart von Basen wie Alkalimetallhydroxiden, Alkalimetallcarbonaten oder Alkalimetallalkoholaten wie Natriumhydroxid, Kaliumhydroxid, Natriumcarbonat, Natriummethanolat, Natriumethanolat, Kaliumethanolat oder Kalium-t.-butanolat, besonders bevorzugt in Gegenwart von Kalium-t.-butanolat, in einem Temperaturbereich von 0°C bis +150°C, besonders bevorzugt von +20°C bis +100°C, unter Erhalt der Verbindungen XVIIIa umgesetzt.

Im dritten Reaktionsschritt C werden die Verbindungen XVIIIa in Lösungsmitteln wie Alkoholen, beispielsweise Methanol, Ethanol, Propanol oder Isopropanol, oder in Ethern, beispielsweise Methylether, Tetrahydrofuran oder Dioxan, oder in chlorierten Kohlenwasserstoffen wie Methylenchlorid oder Chloroform, oder Carbonsäuren wie Essigsäure oder Trifluoressigsäure, oder in Gemischen davon, besonders bevorzugt in Trifluoressigsäure, in Gegenwart von Säuren wie Mineralsäuren, beispielsweise Salzsäure, Bromwasserstoffsäure oder Schwefelsäure oder Carbonsäuren, beispielsweise Essigsäure oder Trifluoressigsäure, besonders bevorzugt in Gegenwart von Essigsäure, insbesondere bevorzugt in Gegenwart von Trifluoressigsäure, sowohl als Lösungsmittel als auch als Säure, in einem Temperaturbereich von -20°C bis +60°C, besonders bevorzugt von 0°C bis +30°C unter Erhalt der Carbonsäuren XIXa verseift.

Im vierten Schritt D werden die Carbonsäuren XIXa in Lösungsmitteln wie Ether, beispielsweise Diethylether, Tetrahydrofuran oder Dioxan, oder in chlorierten Kohlenwasserstoffen wie Methylenchlorid oder Chloroform, oder in Gemischen davon, besonders bevorzugt in Tetrahydrofuran, unter Verwendung von Borverbindungen als Reduktionsmittel, beispielsweise Boran oder der Boran-Dimethylsulfid-Komplex, in einem Temperaturbereich von -40 °C bis +60°C, besonders bevorzugt von -20°C bis +30°C, unter Erhalt der Hydroxylverbindungen XXa reduziert.

Im fünften Reaktionsschritt E werden die Hydroxyverbindungen XXa in Lösungsmitteln wie Ether, beispielsweise Diethylether, Dioxan oder Tetrahydrofuran, oder in chlorierten Wasserstoffen wie Methylenchlorid oder Chloroform, oder in Dimethylsulfoxid oder in Gemischen davon, besonders bevorzugt in Dichlormethan, unter Verwendung von Oxidationsmitteln wie Pyridiniumchlorchromat, Chrom-(VI)-Salzen, Dimethylsulfoxid/ Pyridin/SO₃, katalytischen Mengen von Tetraalkylammoniumperruthenat in Gegenwart von N-Methylmorpholinoxid und Molekularsieb, Dimethylsulfoxid/Oxalylchlorid/ Triethylamin, besonders bevorzugt unter Verwendung von Pyridiniumchlorchromat, katalytischen Mengen von Tetraalkylammoniumperruthenat in Gegenwart von N-Methylmorpholin und Molekularsieb oder Dimethylsulfoxid/Oxalylchlorid/ Triethylamin, gegebenenfalls in Anwesenheit von Basen wie Triethylamin, Diisopropylamin, Pyridin oder Dimethylaminopyridin, besonders bevorzugt in Gegenwart von Triethylamin, in einem Temperaturbereich von - 20°C bis +60°C, besonders bevorzugt von 0°C bis +30°C, unter Erhalt der Aldehyde XXIa oxidiert.

Die cyclischen Ketone XVIa sind entweder käuflich oder auf dem Fachmann bekannten herkömmlichen Wegen, beispielsweise durch Dieckmann-Kondensation der entsprechenden Carbonsäuredieester darstellbar.

Die mit den Ketonen XVIa umzusetzenden 4-Chlormethylbenzoesäureester oder 4-Chlorsulfenylbenzoesäureester bzw. die entsprechenden Nitrile sind entweder käuflich oder auf dem Fachmann bekannten herkömmlichen Wegen darstellbar.

Bei den in dem vorstehenden Diagramm des Verfahrens III aufgeführten Verbindungen haben die Reste R², R³⁵ und U die gleichen Bedeutungen wie vorstehend definiert und steht o für eine ganze Zahl von 1 bis 12.

Mit dem Verfahren III können Aldehyde (II) hergestellt werden, bei denen X für eine Alkylenkette, U für -CH₂-, R¹ für COOR³⁵ und R² für CN oder COOR²⁶ steht.

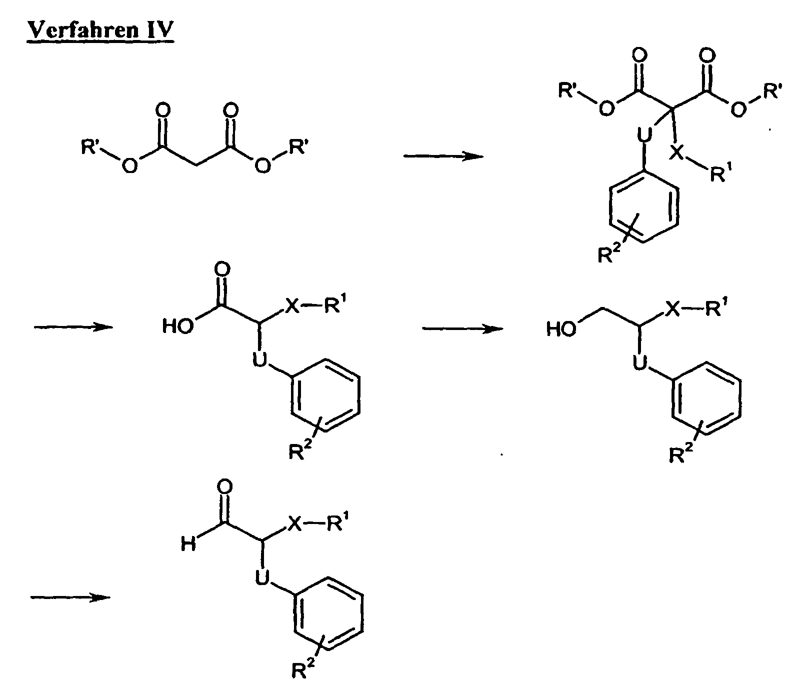

Bei diesem Verfahren wird ein Malonsäurediester (wobei als alkholische Komponente R' ein Allylrest oder niedere Alkylreste wie Methyl, Ethyl, t-Bu oder ein Benzylrest eingesetzt werden können) durch zwei aufeinanderfolgende Umsetzungen mit entsprechenden Elektrophilen in einen 2,2-disubstituierten Malonsäurediester überführt. Beispielsweise kann der als Ausgangsverbindung verwendete Malonsäurediester zunächst in Gegenwart einer Base wie beispielsweise Natriumhydrid, Triethylamin, Kaliumcarbonat, Natriumhydroxid, DABCO, Kaliumhydroxid, Lithiumdiisopropylamid oder Natriumamid, bevorzugt Natriumhydrid, mit einem entsprechenden Elektrophil wie einem entsprechenden Halogenid, Tosylat, Mesylat oder Triflat, zum Beispiel einem Halogenid wie ω-Chlor- oder ω-Bromcarbonsäureester, beispielsweise Bromessigsäuremethylester, in einem Lösungsmittel wie Dioxan bei Temperaturen von 0 bis 50°C umgesetzt werden. In einem zweiten Schritt kann das so erhaltene monsubstituierte Malonsäurediesterderivat durch Umsetzung mit einem entsprechenden Elektrophil wie einem entsprechenden Halogenid, Tosylat, Mesylat oder Triflat, zum Beispiel einem 2-Halogenbenzylderivat wie 2-(Bromomethyl)-benzoesäuremethylester, in Gegenwart einer Base wie beispielsweise Natriumhydrid, Triethylamin, Kaliumcarbonat, Natriumhydroxid, DABCO, Kaliumhydroxid, Lithiumdiisopropylamid oder Natriumamid, bevorzugt Natriumhydrid, in einem Lösungsmittel wie Dimethylformamid bei Temperaturen von 0 bis 50°C umgesetzt werden. Die Umsetzungen mit den beiden Elektrophilen können jedoch auch in umgekehrter Reihenfolge durchgeführt werde.

Das so erhaltene 2,2-disubstituierte Malonsäurediesterderivat kann durch Reaktion mit einer Säure wie beispielsweise Salzsäure, Schwefelsäure oder Trifluoressigsäure, oder durch Reaktion mit einer Base wie Kaliumhydroxid, Natriumhydroxid oder Lithiumhydroxid, oder durch eine Palladium-katalysierte Reaktion wie beispielsweise mit Ameisensäure in Gegenwart eines Pd-Katalysators, vorzugsweise eines Pd(II)-Katalysators wie Palladium-(II)-acetat, und eines Phosphans wie Triphenylphosphan und einer Base wie einem Amin, vorzugsweise Triethylamin, in einem Lösungsmittel wie Dioxan bei Temperaturen von 20 bis 120°C durch Esterspaltung und anschließende Decarboxylierung bei erhöhten Temperaturen in die entsprechenden Carbonsäurederivate überführt werden.

Diese Carbonsäurederivate können ihrerseits durch eine Reduktion mit herkömmlichen Reduktionsmitteln wie beispielsweise Düsobutylaluminiumhydrid (DIBAL), Lithiumaluminiumhydrid oder Borhydriden wie Boran in Tetrahydrofuran zu den entsprechenden Alkoholen umgesetzt werden.

Diese Alkohole können schließlich mit herkömmlichen milden Oxidationsmitteln wie Cr-(VI)-Verbindungen wie PDC oder PCC, Kaliumpermanganat, Dimethylsulfoxid/Oxalylchlorid/Triethalmin (Swern-Oxidation) oder Tetrapropylammoniumperruthenat (TPAP) in Gegenwart einer Base wie N-Methylmorpholinoxid und Molsieb oder durch die Dess-Martin-Oxidation zu den entsprechenden Aldehyden oxidiert werden.

Bei den in dem vorstehenden Diagramm des Verfahrens IV aufgeführten Verbindungen haben die Reste R¹, R², U, X die gleichen Bedeutungen wie vorstehend definiert, wobei jedoch X nicht O und R¹ und R² keine freien Carboxylfunktionen sein dürfen.

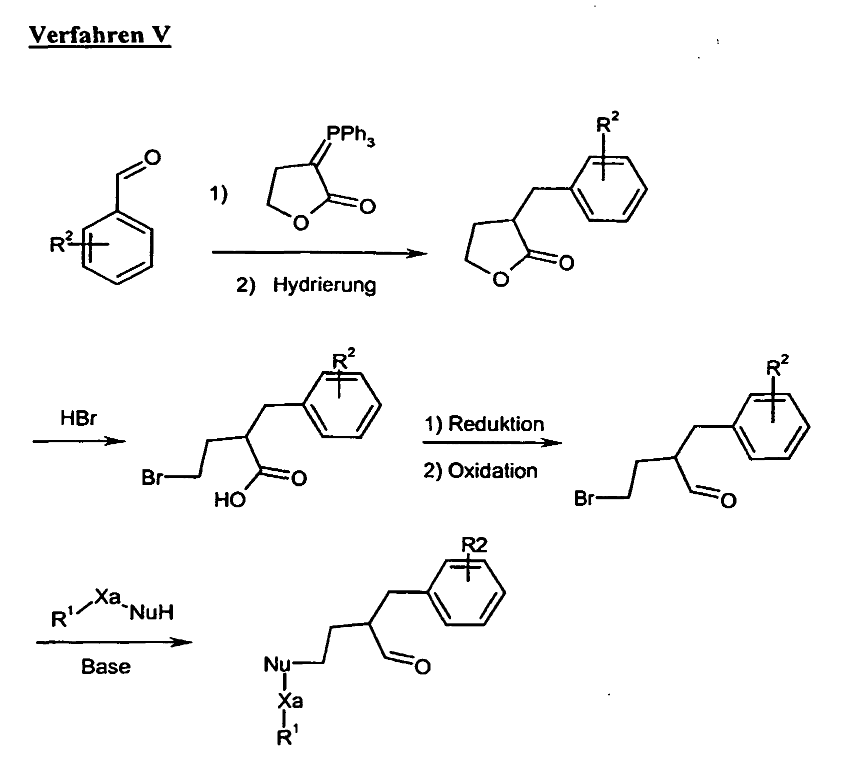

Bei dieser Variante wird zunächst ein Benzaldehydderivat mit einem Tetrahydofuranonphosphoran in einem organischen Lösungsmittel wie Dimethylsulfoxid unter Erhitzen umgesetzt. Das so erhaltene Alken wird anschließend mit herkömmlichen Reduktionsmitteln wie Pd/H₂/C zum entsprechenden 3-Benzoylmethyltetrahydrofuranonderivat umgesetzt. Dieses wird anschließend durch Ringöffnung unter Zugabe einer Säure wie HBr unter Erhitzen in Buttersäurederivat überführt. Die anschließend Reduktion mit hierfür herkömmlich verwendeten Reduktionsmitteln wie Boran in einem organischen Lösungsmittel wie Tetrahydrofuran ergibt zunächst den entsprechenden Alkohol, der anschließend mit einem üblichen Reduktionsmittel, wie Pyridiniumdichromat (PDC) zum Aldehyd oxidiert werden kann. Durch Umsetzung mit einer Verbindung R¹-Xa-Nu in Gegenwart einer herkömmlichen Base wie beispielsweise NaHCO₃ kann die Seitenkette entsprechend verändert werden. Diese Seitenkettenvariation kann aber auch erst nach der Umsetzung des Aldehyds mit einem Phosphoniumsalz gemäß Verfahren A durchgeführt werden.

Bei den im vorstehenden Schema angegebenen Verbindungen haben R¹ und R² die vorstehend angegebenen Bedeutungen. Xa hat die vorstehend angegebene Bedeutung von X, trägt aber zusätzlich eine nukleophile Gruppe Nu wie beispielsweise eine Aminogruppe und ist um die bereits im Molekül in der Seitenkette vorhandenen Kohlenstoffatome vermindert.

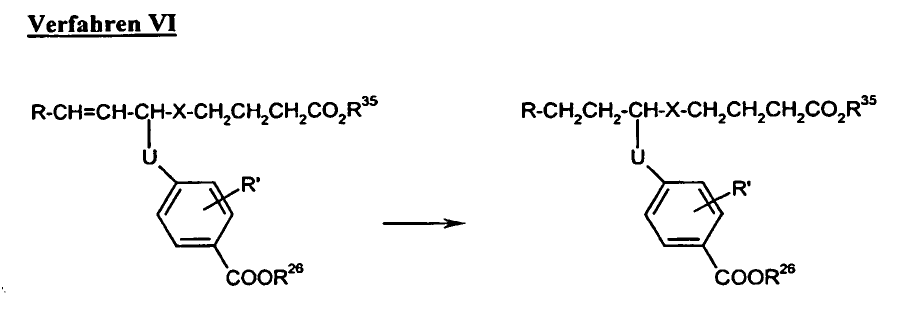

Bei diesem Verfahren wird ein Alkenderivat in Lösungsmitteln wie Alkoholen, Wasser, Benzol, Toluol, Ethern wie Dimethylether, Tetrahydrofuran, Dioxan, Estern wie Ethylacetat, oder in Kohlenwasserstoffen wie Hexan, oder in Aminen wie Triethylamin oder in Ammoniak mit einem Reduktionsmittel wie Wasserstoff in Gegenwart eines Metallkatalysators wie den Oxiden oder löslichen Komplexen von Palladium, Platin, Ruthenium oder Nickel, oder mit einem Metall wie Lithium oder Natrium, oder mit Hydrazin oder Arylaralkoxy-substituierten Hydrazinen umgesetzt. Das Produkt dieser Reaktion ist ein Alkanderivat, worin W der allgemeinen Formel (I) -CH₂CH₂- oder -CH₂CH₂CH₂- bedeutet. Der normale Temperaturbereich für dieses Verfahrens beträgt -20°C bis +30°C.

Bei den in dem vorstehenden Diagramm des Verfahrens VI aufgeführten Verbindungen haben die Reste R²⁶, R³⁵, U und X die gleichen Bedeutungen wie in Anspruch 1 definiert. R' steht für einen der Substituenten, die gemäß Anspruch 1 an U vorhanden sein kann. R steht für den Rest der Verbindungen der allgemeinen Formel (I), wobei R einen Arylrest, aber keine Doppelbindung enthalten darf.

Das erfmdungsgemäße Verfahren B kann vorzugsweise in Acetonitril durch Reaktion der Verbindungen (IV) und (V) in Gegenwart einer Base wie Natriumcarbonat, Et₃N, DABCO, K₂CO₃, KOH, NaOH, Cs₂CO₃ gegebenenfalls mit NaI als Katalysator oder NaH durchgeführt werden. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Beim erfindungsgemäßen Verfahren B wird eine Verbindung der Formel (I) durch nukleophile Substitution einer Abgangsgruppe E in der Verbindung der Formel (V) durch die Hydroxy- oder Thiolfunktion der Verbindung der Formel (IV) dargestellt. Als Abgangsgruppen E kommen hierbei beispielsweise in Frage: Halogen, beispielsweise Cl, Br, I, Tosylat, Mesylat, oder eine durch Reagenzien wie Diisopropylazodicarboxylat/PPh₃ aktivierte Hydroxyfunktion (Mitsonobu-Reaktion).

Die als Ausgangsverbindung verwendete Verbindung der Formel (IV) kann durch Umsetzung einer entsprechenden Phosphoniumverbindung wie beispielsweise 2-Hydroxybenzyltriphenylphosphoniumbromid mit einem entsprechenden Aldehyd (II) analog zum Verfahren A hergestellt werden. Die Verbindungen der Formel (V) sind käuflich erhältlich oder auf dem Fachmann bekannte herkömmliche Weise zugänglich.

Beim erfindungsgemäßen Verfahren C wird eine Verbindung der Formel (I), bei der R¹ und R² jeweils für eine freie Carboxylfunktion stehen, durch Überführung von Ester- und/oder Nitrilfunktionen der Verbindung (VI) in die entsprechenden freien Carboxylfunktionen erhalten. Diese Reaktion kann beispielsweise durch Zugabe wässriger Lösungen starker Säuren wie z.B. HCl oder H₂SO₄, oder starker Basen wie z.B. NaOH, KOH oder LiOH erfolgen. Die Reaktion kann vorzugsweise in einem herkömmlichen organischen Lösungsmittel, welches sich unter den Reaktionsbedingungen nicht verändert, oder in Wasser durchgeführt werden. Vorzugsweise können für das erfindungsgemäße Verfahren C Ether wie Diethylether, Butylmethylester, Dioxan, Tetrahydrofuran, Glykoldimethylether oder Diethylenglykoldimethylether, oder Kohlenwasserstoffe wie Benzol, Toluol, Xylol oder Petrolether, oder Amide wie Dimethylformamid oder Hexamethylphosphortriamid, oder 1,3-Dimethyl-imidazolidin-2-on, 1,3-Dimethyl-tetrahydropyrimidin-2-on, Acetonitril, Essigsäureethylester oder Dimethylsulfoxid verwendet werden. Es ist selbstverständlich auch möglich, Gemische der vorstehend genannten Lösungsmittel zu verwenden.

Erfindungsgemäß bevorzugt ist beispielsweise die Durchführung der Reaktion in einem Gemisch aus Wasser und Methanol. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Die als Ausgangsverbindungen verwendeten Verbindungen der Formel (VI) können auf einen der in der vorliegenden Anmeldung beschriebenen Wege zur Herstellung der Verbindungen der Formel (I) dargestellt werden, beispielsweise gemäß Verfahren A.

Beim erfindungsgemäßen Verfahren D wird eine Verbindung der Formel (I) durch Umsetzung einer Verbindung der Formel (VII), welche eine substituierbare Gruppe L' enthält, mit einer Verbindung der Gruppe (VIII) in Gegenwart einer Palladiumverbindung sowie gegebenenfalls eines Reduktionsmittels und weiterer Zusatzstoffe im basischen Medium dargestellt. Die Reaktion stellt formal eine reduktive Kupplung der Verbindungen der Formeln (VII) und (VIII) dar, wie sie z.B. in L.S. Hegedus, Organometallics in Synthesis, M. Schlosser, Ed., Wiley & Sons, 1994, beschrieben ist.
Als substituierbare Gruppe L' bei den Verbindungen der Formel (VII) kann beispielsweise ein Halogenrest wie Br oder I oder eine herkömmliche Abgangsgruppe wie beispielsweise ein Triflatrest verwendet werden.

Die Verbindungen der Formel (VIII) enthalten eine reaktive Gruppe Z', welche aus der Gruppe, bestehend aus -B(OH)₂, -CH≡CH, -CH=CH₂ oder -Sn(nBu)₃, ausgewählt werden kann.

Als Palladiumverbindung kann eine Palladium(II)-Verbindung wie z.B. Cl₂Pd(PPh₃)₂ oder Pd(OAc)₂ oder eine Palladium(0)-Verbindung wie z.B. Pd(PPh₃)₄ oder Pd₂(dba)₃ verwendet werden. Falls erforderlich, können dem Reaktionsgemisch noch zusätzlich ein Reduktionsmittel wie beispielsweise Triphenylphosphin, BINAP oder andere Zusatzstoffe wie beispielsweise Cu(I)Br, NBu₄NCl, LiCl oder Ag₃PO₄ zugesetzt werden (vgl. hierzu T Jeffery, Tetrahedron lett. 1985, 26, 2667-2670; T. Jeffery, J. Chem. Soc., Chem. Commun. 1984, 1287-1289; S. Bräse, A. deMejiere in "Metalcatalyzied cross-coupling reactions", Ed. F. Diederich, P. J. Stang, Wiley-VCH, Weinheim 1998, 99-166).

Die Reaktion wird in Gegenwart einer herkömmlichen Base wie z.B. Na₂CO₃, NaOH oder Triethylamin durchgeführt. Als Lösungsmittel kommen die vorstehend beim Verfahren C genannten organischen Lösungsmittel in Frage, wobei Ether wie beispielsweise Dimethoxyethan besonders bevorzugt sind. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Die als Ausgangsverbindungen verwendeten Verbindungen der Formel (VII) können auf einen der in der vorliegenden Anmeldung beschriebenen Wege zur Herstellung der Verbindungen der Formel (I) dargestellt werden, beispielsweise gemäß Verfahren A. Die Verbindungen der Formel (VIII) sind käuflich erhältlich oder auf dem Fachmann bekannte herkömmliche Weise darstellbar.

Beim erfindungsgemäßen Verfahren E wird eine Verbindung der Formel (I) durch Umsetzung einer Verbindung der Formel (VII), welche eine substituierbare Gruppe L' enthält, mit einer Verbindung der Gruppe (IX) in Gegenwart einer Palladiumverbindung sowie gegebenenfalls eines Reduktionsmittels und weiterer Zusatzstoffe im basischen Medium dargestellt. Die Reaktion stellt formal eine reduktive Kupplung der Verbindungen der Formeln (VII) und (IX) dar, wie sie z.B. von J. F. Hartwig, Angew. Chem. 1998, 10, 2154, beschrieben ist.

Als substituierbare Gruppe L' bei den Verbindungen der Formel (VII) kann beispielsweise ein Halogenrest wie Br oder I oder eine herkömmliche Abgangsgruppe wie beispielsweise ein Triflatrest verwendet werden.

Als Palladiumverbindung kann eine Palladium(II)-Verbindung wie z.B. Cl₂Pd(PPh₃)₂, Pd₂(dba)₃ (dba=Dibenzylidenaceton) oder Pd(OAc)₂ oder eine Palladium(0)-Verbindung wie z.B. Pd(PPh₃)₄ verwendet werden. Falls erforderlich, können dem Reaktionsgemisch noch zusätzlich ein Reduktionsmittel wie beispielsweise Triphenylphosphin oder Tributylphosphin oder andere Zusatzstoffe wie beispielsweise Cu(I)I zugesetzt werden.

Die Reaktion wird in Gegenwart einer herkömmlichen Base wie z.B. Na₂CO₃, Na-OH, NaOt-Bu oder Triethylamin durchgeführt. Als Lösungsmittel kommen die vorstehend beim Verfahren C genannten organischen Lösungsmittel in Frage, wobei Ether wie beispielsweise Dimethoxyethan besonders bevorzugt sind. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.
Die als Ausgangsverbindungen verwendeten Verbindungen der Formel (VII) können auf einen der in der vorliegenden Anmeldung beschriebenen Wege zur Herstellung der Verbindungen der Formel (I) dargestellt werden, beispielsweise gemäß Verfahren A. Die Verbindungen der Formel (IX) sind käuflich erhältlich oder auf dem Fachmann bekannte herkömmliche Weise darstellbar.

Beim erfindungsgemäßen Verfahren F wird zunächst eine Verbindung der Formel (IV) analog zum Verfahren B mit einer Verbindung der Formel (X) umgesetzt. Die Verbindung der Formel (X) besitzt zwei Abgangsgruppe E und E', welche voneinander unabhängig beispielsweise Halogen, beispielsweise Cl, Br, I, Tosylat, Mesylat, oder eine durch Reagenzien wie Düsopropylazodicarboxylat/PPh₃ aktivierte Hydroxyfunktion (Mitsonobu-Reaktion) oder derartige Gruppen enthaltende Reste wie beispielsweise Halogenalkylreste wie Chlormethyl darstellen können. Die Abgangsgruppen E und E' müssen jedoch so gewählt werden, dass sie selektiv und unabhängig voneinander reagieren können. Man kann aber auch die Verbindung der Formel (X) bei der Umsetzung mit der Verbindung der Formel (IV) im Überschuss einsetzen. In diesem Fall können die Abgangsgruppen E und E' auch gleich sein.

Anschließend wird die so erhaltene Verbindung der Formel (XI) mit einem Amin der Formel (XII) in Gegenwart einer Base umgesetzt. Die. Reaktion wird in Gegenwart einer herkömmlichen Base wie z.B. Na₂CO₃, K₂CO₃, NaOH, NaOt-Bu oder Triethylamin durchgeführt. Als Lösungsmittel kommen die vorstehend beim Verfahren C genannten organischen Lösungsmittel in Frage, wobei Acetonitril besonders bevorzugt ist. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt. Gegebenenfalls kann der Reaktionslösung eine katalytische Menge an Kaliumiodid zugegeben werden.

Die Verbindungen der Formel (X) und (XII) sind käuflich erhältlich oder auf dem Fachmann bekannte herkömmliche Weise darstellbar.

Bei den erfindungsgemäßen Verfahren G und H wird jeweils ein Alkohol (XIII) oder (XVI) mit einer Verbindung mit einer herkömmlichen Abgangsgruppe (XIV) oder (XV) gemäß einer nukleophilen Substitutionsreaktion umgesetzt.

Als Abgangsgruppen E" und E''' in den Verbindungen der Formeln (XIV) und (XV) kommen in Frage: Halogen, beispielsweise Cl, Br, I, Tosylat, Mesylat, oder eine durch Reagenzien wie Düsopropylazodicarboxylat/PPh₃ aktivierte Hydroxyfunktion (Mitsonobu-Reaktion).

Als Basen kommen beispielsweise Natriumcarbonat, Et₃N, DABCO, K₂CO₃, Cs₂CO₃, KOH, NaOH, NaH oder Silberoxid/Molekularsieb in Frage. Die Reaktion kann im allgemeinen in einem Temperaturbereich von -20°C bis +90°C, vorzugsweise von 0°C bis +90°C ausgeführt werden. Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt. Als Lösungsmittel kommen die vorstehend beim Verfahren C genannten organischen Lösungsmittel in Frage, wobei Benzol besonders bevorzugt sind.

Die als Ausgangsverbindungen verwendeten Verbindungen der Formeln (XIII) bis (XVI) können über eines der Verfahren I bis IV hergestellt werden, wo sie als Zwischenverbindungen beschrieben sind. Weiterhin kann die Verbindung XV beispielsweise über Bromierung mit PBr₃ oder CBr₄/PPh₃ aus einer Verbindung der Formel XIII hergestellt werden.

Bei den erfindungsgemäßen Verfahren I und J wird ein Amin der Formel (XVIII) oder (XIX) mit einer Carbonylverbindung der Formel (XVII) oder (XX) umgesetzt. Dies kann entweder unter Bildung einer Schiffschen Base und anschließender Reduktion derselben oder auf direkte Weise unter Bedingungen einer reduktiven Alkylierung erfolgen.

Bei der ersten Variante werden die Reaktanden unter herkömmlichen Bedingungen (vgl. J. March, Advanced organic Chemistry, Wiley, 3^{rd} ed., S. 796 f.) miteinander umgesetzt. Die so erhaltene Schiffsche Base wird anschließend mit einem Reduktionsmittel zur gewünschten Aminoverbindung reduziert. Als Reduktionsmittel können die hierfür herkömmlich verwendeten Reduktionsmittel wie beispielsweise NaBH₄, H₂/Pd/C, NaBH(OAc)₃ oder NaCNBH₃ verwendet werden.

Bei der zweiten Variante werden die Reaktanden unter herkömmlichen Bedingungen (vgl. J. March, Advanced organic Chemistry, Wiley, 3^{rd} ed., S. 798 f.) in Gegenwart eines Reduktionsmittels miteinander umgesetzt. Als Reduktionsmittel können die hierfür herkömmlich verwendeten Reduktionsmittel wie beispielsweise H₂/Pd/C, NaCNBH₃ oder NaBH(OAc)₃ verwendet werden.

Die als Ausgangsverbindungen verwendeten Verbindungen der Formel (XVII) können gemäß einem der Verfahren III oder IV hergestellt werden. Die als Ausgangsverbindungen verwendeten Verbindungen der Formel (XVIII) oder (XX) können beispielsweise aus einer der bei den Verfahren I oder II erhaltenen Zwischenverbindungen durch herkömmliche Verfahren dargestellt werden. So sind die Amine (XVIII) beispielsweise durch Reaktion der entsprechenden Halogenide oder Tosylate mit Phthalimid (Gabriel-Synthese) beziehungsweise die Aldehyde (XX) durch Oxidation der entsprechenden Alkohole auf bekannte Weise zugänglich. Die als Ausgangsverbindungen verwendeten Verbindungen der Formel (XIX) können aus einer der bei den Verfahren III oder IV erhaltenen Zwischenverbindungen dargestellt werden, beispielsweise durch Umsetzung eines aus einem entsprechenden Alkohol erhaltenen Tosylats mit Benzylamin und anschließende hydrogenolytische Abspaltung der Benzylgruppe oder über die Reaktion der Verbindung der Formel (XVII) mit Benzylamin gemäß Verfahren [I] und anschließende hydrogenolytische Abspaltung der Benzylgruppe.

Die erfindungsgemäßen Verbindungen der Formel (I), bei denen U für O, NH, S, SO oder SO₂ steht, können über das erfindungsgemäße Verfahren [K] hergestellt werden. Hierbei werden Aldehyde der Formel (XXI) 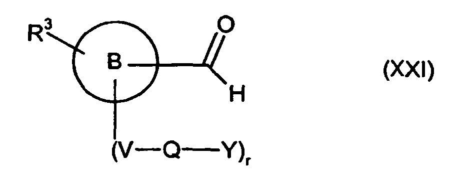 worin
- R³, V, Q, Y, r und B: die vorstehend angegebenen Bedeutungen haben,
mit Phosphorverbindungen der Formel (XXII) 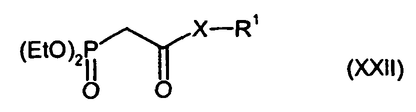 worin
- X und R¹: die vorstehend angegebenen Bedeutungen haben,
zu Verbindungen der Formel (XXIII) 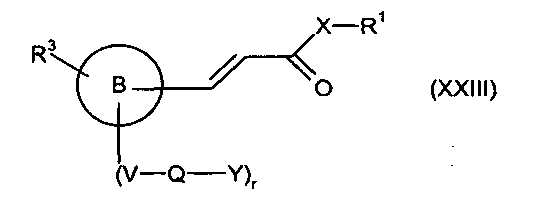 worin
- R³, V, Q, Y, r, B, X und R¹: die vorstehend angegebenen Bedeutungen haben,
umgesetzt und anschließend durch aufeinanderfolgende Reduktion der Alkengruppe und der Carbonylgruppe und anschließende Substitution der durch Reduktion der Carbonylgruppe erzeugten Hydroxygruppe beziehungsweise des durch Umsetzung mit Hälogenierungsmitteln aus der Hydroxygruppe erzeugten Halogenrestes mit Alkoholen, primären Aminen oder Thiolen sowie gegebenenfalls anschließende Oxidation zu den entsprechenden Sulfoxid- oder Sulfonverbindungen in Verbindungen der Formel (XXIV) überführt, 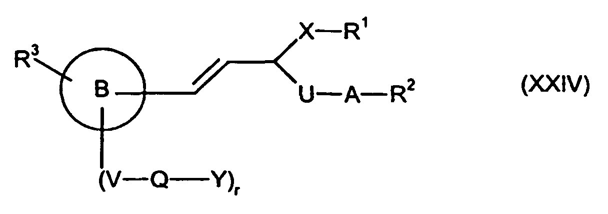 worin
- R³, V, Q, Y, r, B, X, U, A, R² und R¹: die vorstehend angegebenen Bedeutungen haben.

Die Aldehyde der Formel (XXI) sind beispielsweise aus den bei den Verfahren I und II als Zwischenprodukte eingesetzten Alkoholen durch dem Fachmann bekannte herkömmliche Oxidationsreaktionen zugänglich (vgl. z.B. J: March, Advanced organic Chemistry, 3^{rd} ed., S. 1057 ff., Wiley).

Die Phosphorverbindungen der Formel (XXII) können beispielsweise durch Umsetzung von Alkandicarbonsäurederivaten, beispielsweise den entsprechenden Monoestern, mit Phosphonoessigsäurederivaten, beispielsweise den entsprechenden Diestern, hergestellt werden. Möglich ist aber auch die Synthese aus Phosphiten wie beispielsweise Triethylphosphit mit entsprechenden α-Halogenketonderivaten (Arbuzov-Rkt, vgl. z.B. J. March, Advanced organic Chemistry, 3^{rd} ed., S. 848 ff., Wiley).

Die Umsetzung der Verbindungen der Formel (XXI) mit Verbindungen der Formel (XXII) erfolgt in Gegenwart von Basen wie Alkalimetallhydriden, beispielsweise Natriumhydrid, Alkalimetallalkoholaten, beispielsweise Kalium-t-butylat, oder in Gegenwart von Salzen wie beispielsweise MgCl₂ und Basen wie Aminen, beispielsweise Triethylamin, oder der Hünig-Base. Die Reaktion wird vorzugsweise in organischen Lösungsmitteln, besonders bevorzugt in Tetrahydrofuran, bei Raumtemperatur oder unter leichtem Erhitzen durchgerührt.

Die so erhaltenen Carbonylverbindungen der Formel (XXIII) werden nach herkömmlichen, dem Fachmann bekannten Verfahren zu den entsprechenden Alkoholen reduziert (vgl. z.B. J. March, Advanced organic Chemistry, 3^{rd} ed., S. 809 ff., Wiley). Besonders bevorzugt ist die Verwendung von komplexen Metallhydriden wie Diisobutylaluminiumhydrid (DIBAL), NaBH₄ oder NaBH₄/CeCl · 7 H₂O. Die Reaktion wird vorzugsweise in organischen Lösungsmitteln wie beispielsweise Alkoholen wie Methanol unter Kühlung durchgeführt.

Die olefinische Doppelbindung der so erhaltenen Hydroxyverbindungen kann nach herkömmlichen, dem Fachmann bekannten Verfahren hydriert werden (vgl. z.B. J. March, Advanced organic Chemistry, 3^{rd} ed., S. 691 ff., Wiley). Bevorzugt ist die Hydrierung mit Wasserstoff in Gegenwart eines Metallkatalysators wie Pd/C oder Raney-Nickel in einem organischen Lösungsmittel wie beispielsweise Ethylacetat.

Die Einführung des Restes U-A-R² kann auf mehreren Wegen erfolgen. Beispielsweise kann die Hydroxyverbindung unter Mitsunobu-Bedingungen (vgl. O. Mitsunobu, Synthesis, 1981, 1-28) mit entsprechenden Alkoholen, Phenolen, primären Aminen oder Thiolen umgesetzt werden. Die Hydroxygruppe kann aber auch erst in eine Abgangsgruppe überführt werden, welche anschließend durch entsprechende Alkohole, Phenole, primäre Amine oder Thiole in Gegenwart einer Base wie beispielsweise DABCO, Triethylamin, NaH, NaOH, KOH, LDA, Natriumamid oder besonders bevorzugt Kaliumcarbonat substituiert werden kann. Als Abgangsgruppen sind erfindungsgemäß bevorzugt Halogenreste wie Cl, Br oder I, welche durch Umsetzung der Hydroxyverbindung mit beispielsweise SOCl₂, SOBr₂, POCl₃, PCl₃, PCl₅, PBr₃ usw. einführbar sind, der Tosylatrest, welcher beispielsweise durch Umsetzung mit Tosylchlorid einführbar ist, der Mesylatrest, welcher beispielsweise durch Umsetzung mit MsCl einführbar ist, oder der Triflatrest, welcher durch Umsetzung mit beispielsweise Tf₂O oder TfCl einführbar ist.

Die erfindungsgemäßen Verbindungen der allgemeinen Formel (I), zeigen ein nicht vorhersehbares, wertvolles pharmakologisches Wirkspektrum.

Die erfindungsgemäßen Verbindungen der allgemeinen Formel (I), führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, periphereri und kardialen Gefäßerkrankungen, von Arrhythmien, zur Behandlung von thromboembolischen Erkrankungen und Ischämien wie Myokardinfarkt, Himschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, fibrotischen Erkrankungen wie Leberfibrose oder Lungenfibrose, asthmatischen Erkrankungen und Krankheiten des Urogenitalsystems wie beispielsweise Prostatahypertrophie, erektile Dysfunktion, weibliche sexuelle Dysfunktion und Inkontinenz sowie zur Behandlung von Glaucoma eingesetzt werden.

Die in der vorliegenden Erfindung beschriebenen Verbindungen der allgemeinen Formel (I), stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Him-Traumas. Ebenso können die erfindungsgemäßen Verbindungen der allgemeinen Formel (I), zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

### Geräßrelaxierende Wirkung in vitro

Kaninchen werden durch intravenöse Injektion von Thiopental-Natrium narkotisiert bzw. getötet (ca. 50 mg/kg,) und entblutet. Die Arteria Saphena wird entnommen und in 3 mm breite Ringe geteilt. Die Ringe werden einzeln auf je einem triangelförmigen, am Ende offenen Häkchenpaar aus 0,3 mm starkem Spezialdraht (Remanium®) montiert. Jeder Ring wird unter Vorspannung in 5 ml Organbäder mit 37°C warmer, carbogenbegaster Krebs-Henseleit-Lösung folgender Zusammensetzung (mM) gebracht: NaCl: 119; KCl: 4,8; CaCl₂ x 2 H₂O: 1; MgSO₄ x 7 H₂O: 1,4; KH₂PO₄: 1,2; NaHCO₃: 25; Glucose: 10; Rinderserumalbumin: 0,001 %. Die Kontraktionskraft wird mit Statham UC2-Zellen erfasst, verstärkt und über A/D-Wandler (DAS-1802 HC, Keithley Instruments München) digitalisiert, sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50 % zu reduzieren (IC₅₀). Das Standardapplikationsvolumen beträgt 5 µl. Der DMSO-Anteil in der Badlösung entspricht 0,1 %.

Die Ergebnisse sind in Tabelle 1 gezeigt:

**Tabelle 1:**

| Gefäßrelaxierende Wirkung in vitro | |
|---|---|
| **Beispiel** | **IC**_{**50**} **(nM)** |
| 26 | 1,9 |
| 29 | 2,5 |
| 30 | 3500 |
| 34 | 170 |
| 72 | 0,2 |
| 76 | 5,2 |
| 78 | 5,8 |
| 81 | 3,9 |
| 93 | 0,2 |
| 116 | 190 |
| 132 | 220 |
| 150 | 30 |
| 164 | 580 |

### Stimulation der rekombinanten löslichen Guanylatcyclase (sGC) in vitro

Die Untersuchungen zur Stimulation der rekombinanten löslichen Guanylatcyclase (sGC) und die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77 (1999), 14-23.

Die Häm-freie Guanylatcyclase wurde durch Zugabe von Tween 20 zum Probenpuffer (0,5% in der Endkonzentration) erhalten.

Die Aktivierung der sGC durch eine Prüfsubstanz wird als n-fache Stimulation der Basalaktivität angegeben.

Die Ergebnisse sind in Tabelle 2 gezeigt:

**Tabelle 2:**

| Stimulation der rekombinanten löslichen Guanylatcyclase (sGC) in vitro | | | | | |
|---|---|---|---|---|---|
| Bsp. 93 Konzentration (µM) | Stimulation (n-fach) | | | | |
| | Häm-haltige sGC | | | Häm-freie sGC | |
| | Basal | + SNP (0.1 µM) | + ODQ (10 µM) | Basal | + ODQ (10 µM) |
| 0 | 1 | 15 | 1 | 1 | 1 |
| 0.1 | 17 | 45 | 84 | 436 | 392 |
| 1.0 | 23 | 44 | 151 | 476 | 435 |
| 10 | 33 | 54 | 178 | 541 | 500 |

Aus Tabelle 2 ist ersichtlich, dass eine Stimulation sowohl des Häm-haltigen als auch des Häm-freien Enzyms erreicht wird. Weiterhin zeigt eine Kombination aus sGC-Stimulator und Natriumnitroprussid (SNP), einem NO-Donor, keine synergistischen Effekt, d.h. die Wirkung von SNP wird nicht potenziert, wie dies bei über einem Häm-abhängigen Mechanismus wirkenden sGC-Stimulatoren zu erwarten wäre. Darüber hinaus wird die Wirkung des erfindungsgemäßen sGC-Stimulators durch den Häm-abhängigen Inhibitor der löslichen Guanylatcyclase ODQ nicht blockiert. Die Ergebnisse aus Tabelle 2 belegen somit den neuen Wirkmechanismus der erfindungsgemäßen Stimulatoren der löslichen Guanylatcyclase.

Zur vorliegenden Erfindung gehören pharmazeutische Zubereitungen, die neben nichttoxischen, inerten pharmazeutisch geeigneten Trägerstoffen die erfindungsgemäßen Verbindungen, insbesondere die Verbindungen der allgemeinen Formel (I), enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen, insbesondere die Verbindungen der allgemeinen Formel (I), sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können ausser den erfindungsgemäßen Verbindungen, insbesondere den Verbindungen der allgemeinen Formel (I), auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Beispiele

### Abkürzungen:

- RT:: Raumtemperatur
- EE:: Essigsäureethylester
- BABA:: n-Butylacetat/n-Butanol/Eisessig/Phosphatpuffer pH 6 (50:9:25.15; org. Phase)

### Laufmittel für die Dünnschichtchromatographie:

- T1E1:: Toluol - Essigsäureethylester (1:1)
- T1EtOH1:: Toluol - Methanol (1:1)
- C1E1:: Cyclohexan - Essigsäureethylester (1:1)
- C1E2:: Cyclohexan - Essigsäureethylester (1:2)

### Ausgangsverbindungen

### Darstellung der Phosphoniumverbindungen

### Ia: 2-(5-Phenylpentyloxy)nicotinsäure

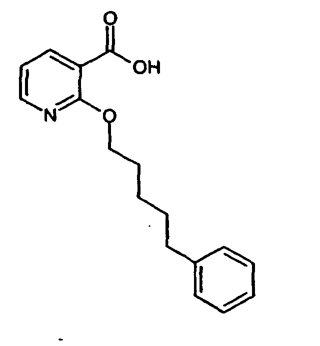

Zu einer Suspension von 635 mg (15,9 mmol) 60%-igem Natriumhydrid in 25 ml DMF bei 0°C wird langsam 1,00 g (6,35 mmol) 2-Chlornicotinsäure zugegeben und anschließend 30 min bei 0°C gerührt. 1,15 g (6,98 mmol) 5-Phenyl-1-pentanol werden in 5ml DMF gelöst und langsam zur vorstehenden Reaktionslösung getropft. Die Lösung wird 3,5 Stunden bei Raumtemperatur gerührt. Es wird dann auf 75°C erhitzt und über Nacht weiter gerührt. Die Substanz wird in Wasser aufgenommen, dann Essigester zugegeben, und die wässrige Phase mit 1M HCl angesäuert. Dann wird mit Essigester extrahiert, mit Wasser gewaschen, über Magnesiumsulfat getrocknet und im Vakuum eingedampft.

Das Produkt wird roh weiter umgesetzt.

### Ib: 2-(5-Benzyloxy)nicotinsäure

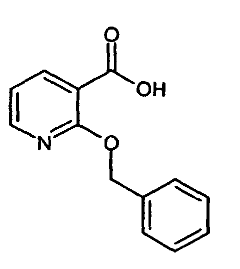

Die Darstellung erfolgte analog Beispiel Ia mit 4,00 g (25,4 mmol) Benzylalkohol als alkolischer Komponente.

Ausbeute: 5,02 g (86,4 % d.Th.)
¹H-NMR (200 MHz, CDCl₃): 8,50 (m, 2H), 7,40 (m, 5H), 7,10 (m, 1H), 5,60 (s, 2H).

### IIa) 2-(5-Phenylpentoxy)-3-pyridinylmethanol

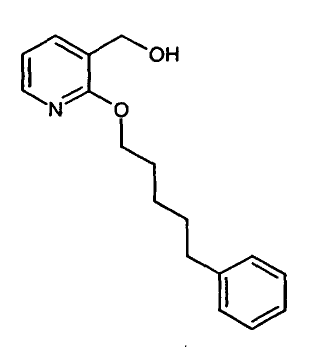

500 mg (1,75 mmol) der Säure aus Bsp. Ia wurden in 20 ml Tetrahydrofuran (THF) bei 0°C unter Argon gelöst. Dann wurden langsam 3,5 ml (3,5 mmol) einer LiAlH₄-Lösung (1M in THF) zugegeben. Es wurde für 3 Stunden unter Rückfluss gekocht. Die Lösung wurde auf 0°C abgekühlt, langsam 1 ml Wasser, 1 ml 1N Natronlauge und 3 ml Wasser zugegeben. Bei Raumtemperatur wurden noch ca. 50ml Wasser hinzugegeben. Dann wurde mit Essigester extrahiert und mit Wasser gewaschen, über Magnesiumsulfat getrocknet und im Vakuum eingedampft.

Ausbeute: 410 mg (86,4 % d.Th.)
¹H-NMR (200 MHz, CDCl₃): 8,00 (m, 1H), 7,50 (m, 1H), 7,20 (m, 5H), 6,80 (m, 1H), 4,60 (s, 2H), 4,40 (t, 2H), 3,60 (t, 1H), 2,60 (m, 2H), 1,90-1,20 (m, 6H).

Analog wurde hergestellt:

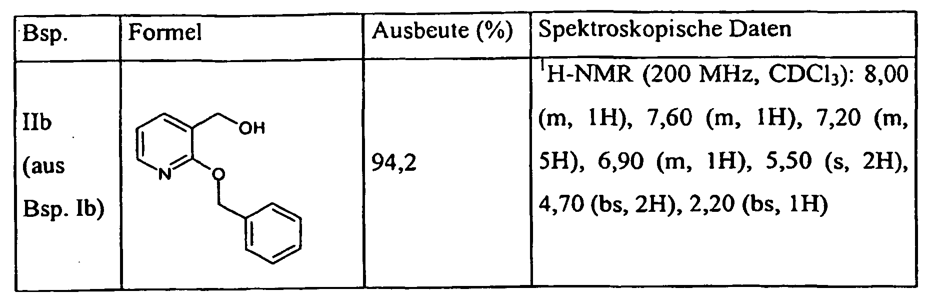

### IIc: 3-(5-Phenylpentoxy)-2-pyridinylmethanol

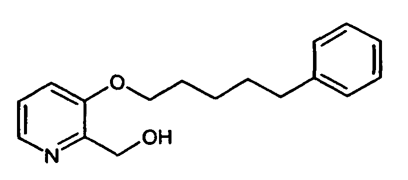

1,9 g (6,01 mmol) Phenylpentylbromid, 1,00 g (8,00 mmol) 2-Hydroxymethyl-3-pyridinol und 1,2 g (8,8 mmol) Kaliumcarbonat werden über Nacht unter Rückfluss erhitzt. Es wird in Essigester aufgenommen, mit Wasser, 2N Natronlauge und Wasser gewaschen, getrocknet und im Vakuum eingedampft.

Ausbeute: 853 mg (52,3 % d. Th.)
¹H-NMR (200 MHz, CDCl₃): 8,10 (m, 1H), 7,40-7,10 (m, 7H), 4,80 (d, 2H), 4,40 (t, 1H), 4,00 (t, 2H), 2,60 (m, 2H), 1,90-1,20 (m, 6H)

### IId) 2-Butyloxybenzylalkohol

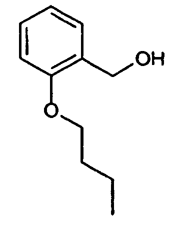

12,4 g (90,5 mmol) Butylbromid, 11,2 g (90,5 mmol) 2-Hydroxybenzylalkohol und 12,5 g (90,5 mmol) Kaliumcarbonat werden in 270 ml 2-Propanol unter Rückfluss über Nacht erhitzt. Die Suspension wird abgekühlt, in Essigester aufgenommen und mit 1N Natronlauge und Wasser gewaschen, über Magnesiumsulfat getrocknet und im Vakuum eingedampft.
Ausbeute: 12,8 g (78,3% d. Th).
R_{f} (SiO₂, C4E1): 0,14

Analog wurden dargestellt:

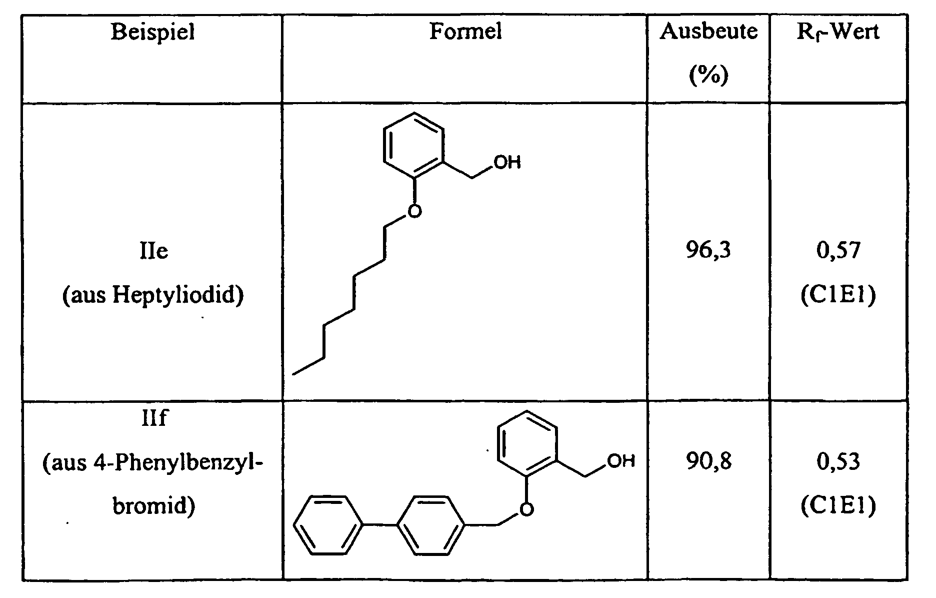

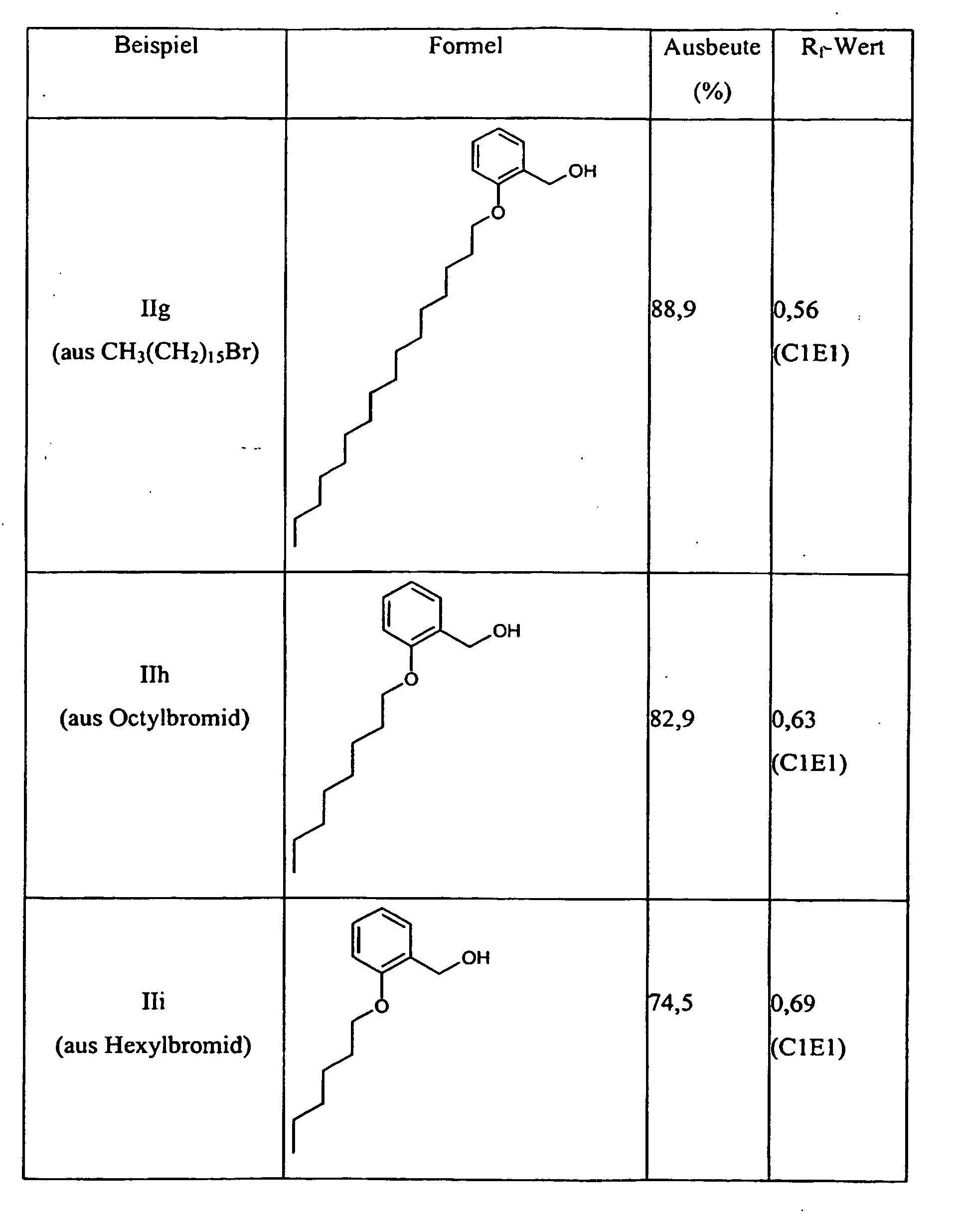

### IIIa: 3-(Bromomethyl)-2-(5-phenylpentoxy)pyridin

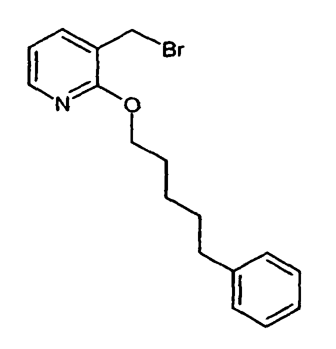

410 mg (1,51 mmol) des Alkoholes aus Bsp. IIa werden in Toluol/Dichlormethan 2:1 gelöst. Dann werden 820 mg (3,03 mmol) Phosphortribromid zugegeben und bei Raumtemperatur 1 Stunde gerührt. Die Substanz wird in gesättigter NaHCO₃-Lösung aufgenommen, mit Essigester extrahiert, mit Wasser gewaschen, über Magnesiumsulfat getrocknet, eingeengt und durch Säulenchromatographie gereinigt.

Ausbeute: 321 mg (63,8 % d.Th.)
¹H-NMR (200 MHz, CDCl₃): 8,10 (m, 1H), 7,60 (m, 1H), 7,20 (m, 5H), 6,80 (m, 1H), 4,50 (s, 2H), 4,40 (t, 2H), 2,60 (m, 2H), 1,90-1,20 (m, 6H).

### IIIb: 2-Benzyloxy-3-chloromethylpyridin

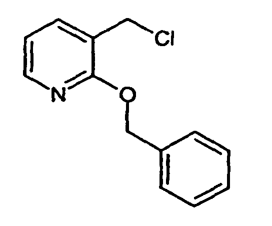

1,48 g (6,88 mmol) des Alkoholes aus Bsp. IIb werden in Dichlormethan gelöst und mit 5 ml (68,8 mmol) Thionylchlorid versetzt. Die Lösung wird 2 h bei Raumtemperatur gerührt, und das Lösungsmittel anschließend in Vakuum eingedampft. Dabei fällt das Produkt als Hydrochlorid aus. Es wird in Wasser und Essigester aufgenommen, mit Natronlauge gewaschen, getrocknet und im Vakuum eingedampft.

Ausbeute: 769 mg (47,9 % d.Th.)
¹H-NMR (400 MHz, CDCl₃): 8,00 (m, 1H), 7,60 (m, 1H), 7,20 (m, 5H), 6,80 (m, 1H), 5,40 (s, 2H), 4,60 (s, 2H).

Analog wurden dargestellt:

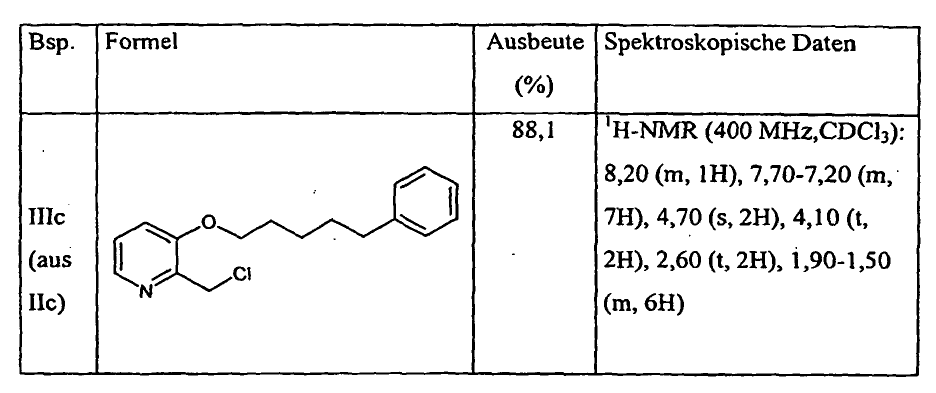

### IVa:(2-(5-Phenylpentoxy)-3-pyridinyl)methyltriphenylphosphoniumbromid

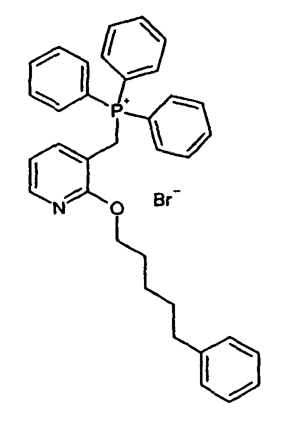

321 mg (0,96 mmol) des Bromids aus Bsp. IIIa und 264 mg (1,00 mmol) Triphenylphosphin werden in 20 ml Toluol 4 Stunden unter Rückfluss erhitzt. Das Lösungsmittel wird in Vakuum eingedampft, der Rückstand wird mit Ethylether zerkleinert, filtriert, getrocknet.
Ausbeute: 322 mg (56,3 % d.Th.)
¹H-NMR (400 MHz, CDCl₃): 7,80-7,10 (m, 21H), 6,80 (m, 2H), 5,45 (d, J=15Hz, 2H), 3,70 (t, 2H), 2,60 (m, 2H), 1,60-1,30 (m, 6H).

Analog wurden dargestellt:

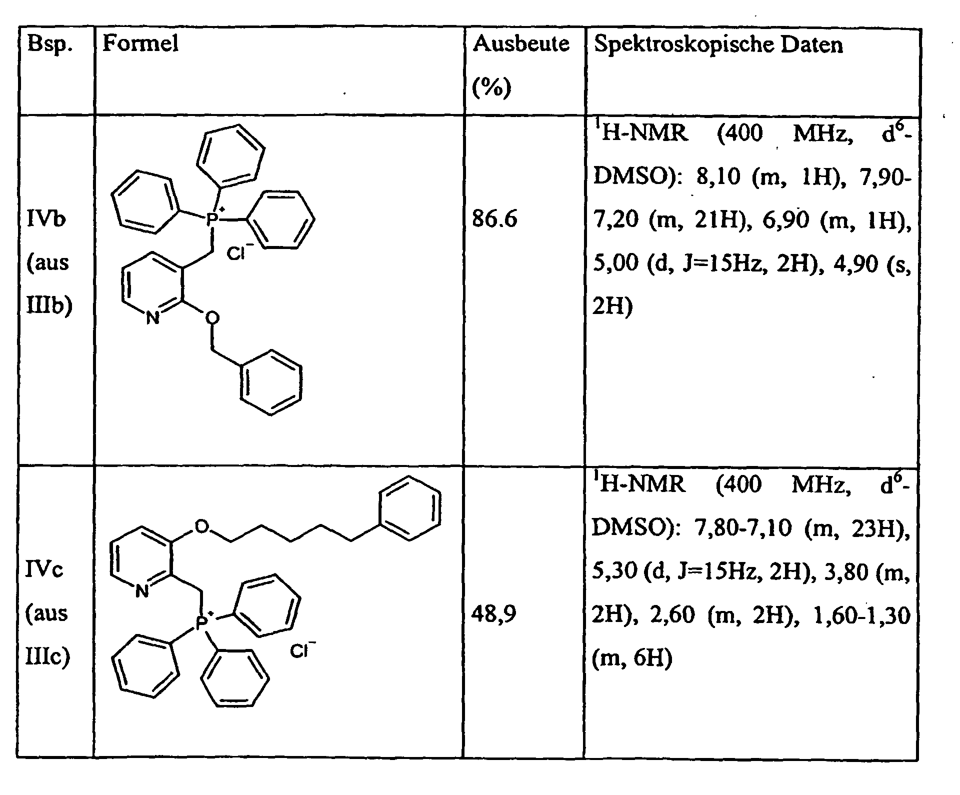

### IVd: 2-(Butyloxy)benzyltriphenylphosphoniumbromid

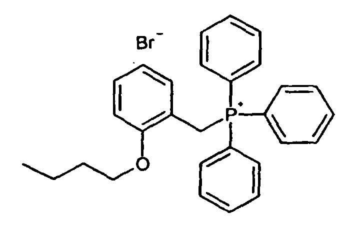

8,2 g (45,5 mmol) des Benzylalkohols IId und 15,6 g (45,5 mmol) Triphenylphosphoniumhydrobromid werden in 100 ml Acetonitril unter Rückfluss 5 Stunden erhitzt. Das Lösungsmittel wird im Vakuum eingedampft, und anschließend wird Diethylether zugegeben. Das Feststoff wird filtriert und im Vakuum getrocknet. Das Produkt wird roh weiter umgesetzt.
¹H-NMR (400 MHz, d⁶-DMSO): 7,80-6,70 (m, 19H), 4,90 (d, J=15Hz, 2H), 3,40 (t, 2H), 1,30 (m, 4H), 0,90 (t, 3H).

Analog wurden hergestellt:

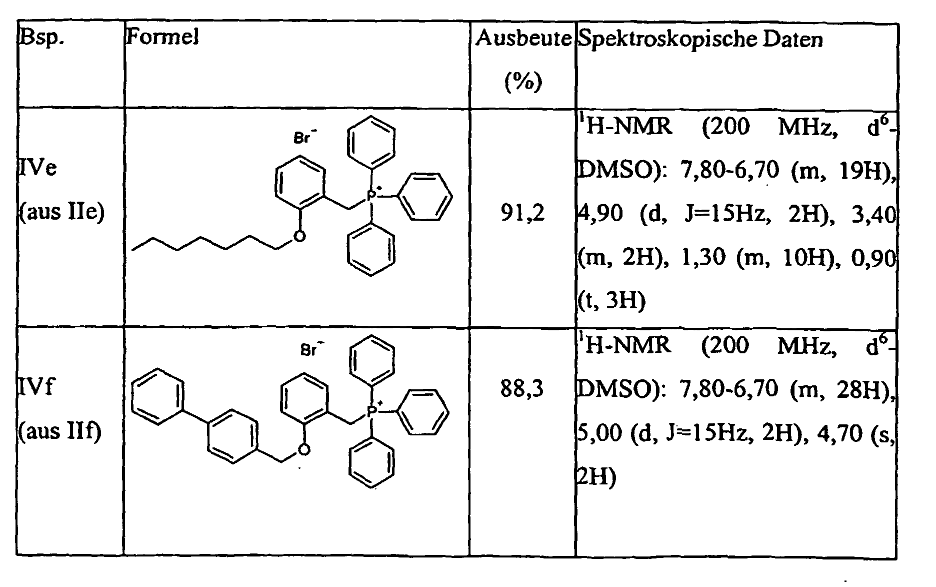

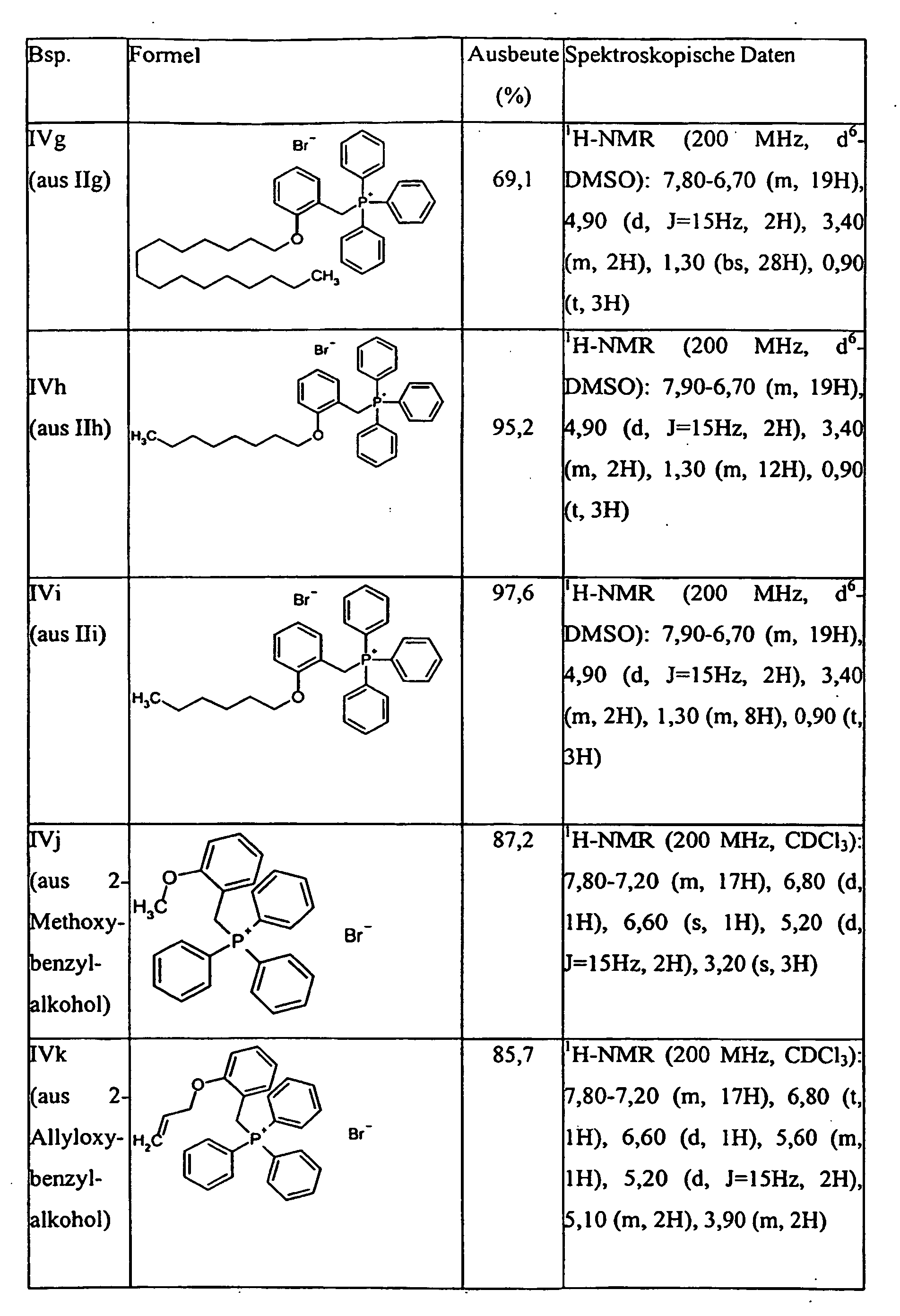

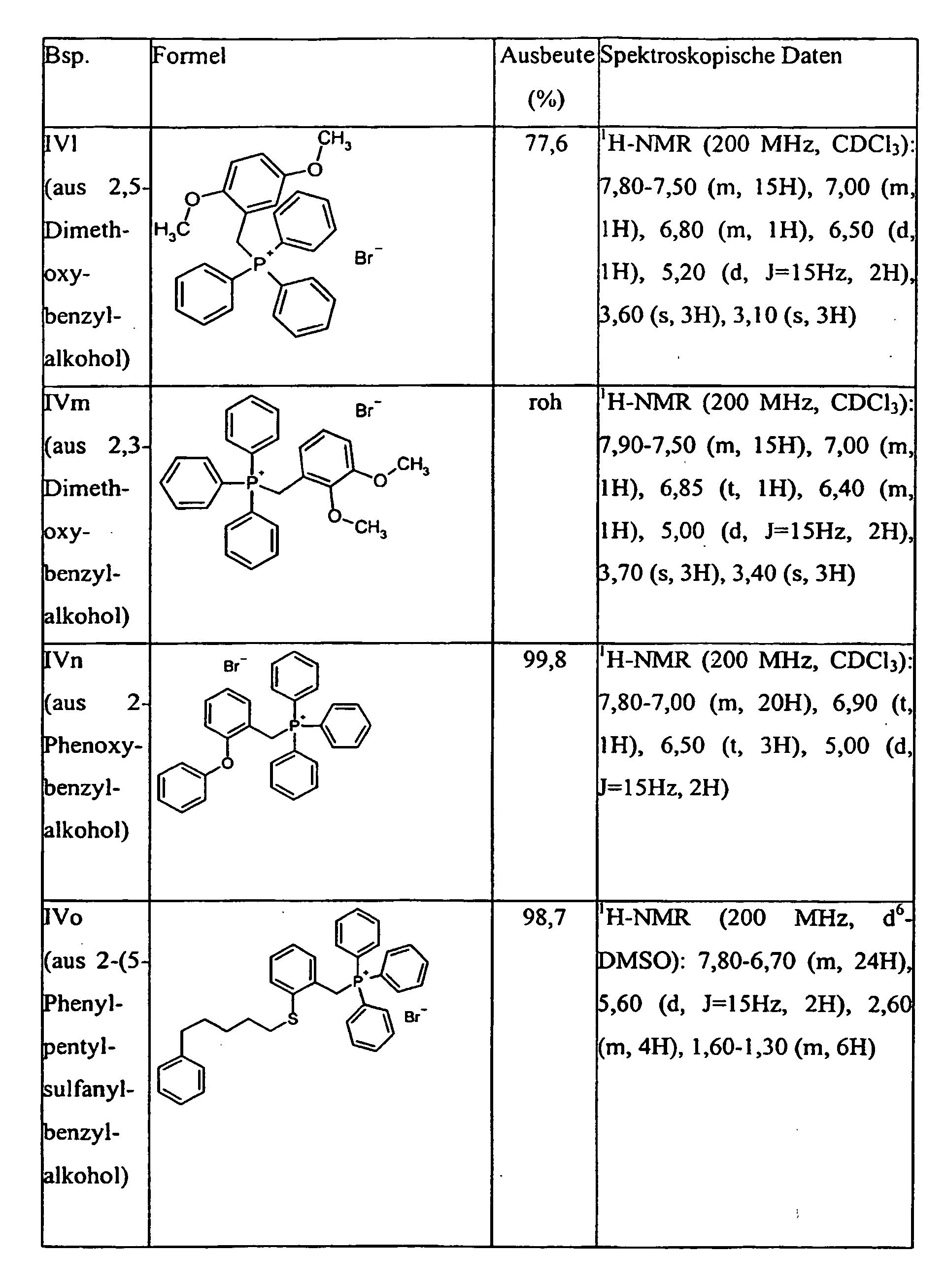

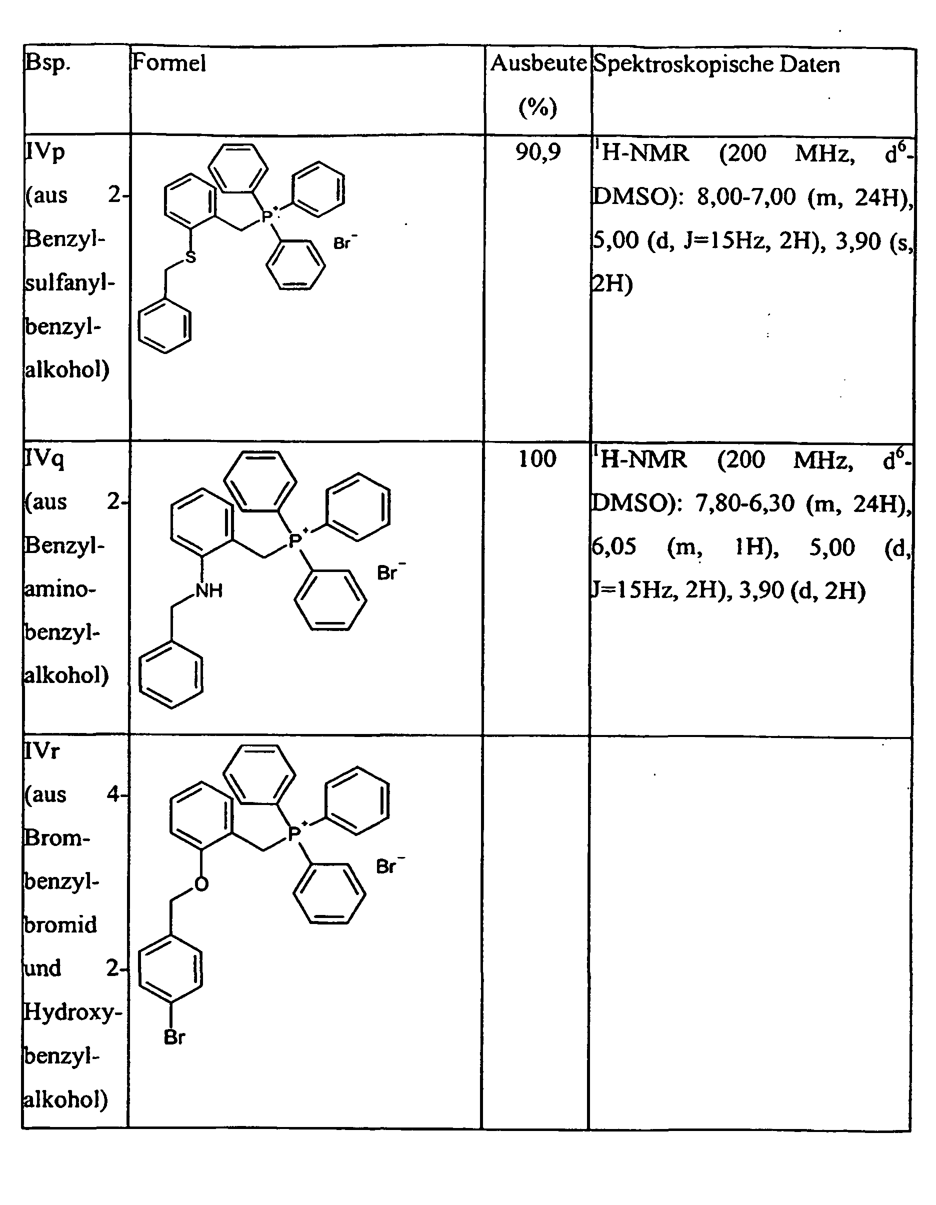

### V: Methyl 4-{[2-oxodihydro-3(2H)-furanyliden]methyl}benzoat

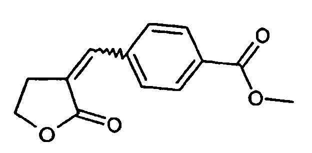

Ein Gemisch aus 40,00 g (0,12 mol) 3-(Triphenylphosphoranyliden)dihydro-2(3H)-furanon und 20,85 g (0,13 mol) Methyl-4-formylbenzoat wird in 240 ml Dimethylsulfoxid 18 Stunden bei 80 °C gerührt. Nach dem Abkühlen wird mit 400 ml Chloroform versetzt und fünfmal mit 200 ml Wasser extrahiert. Die organische Phase wird über Magnesiumsulfat getrocknet und das Solvens unter vermindertem Druck abdestilliert. Der Rückstand wird mit Diethylether verrührt und im Vakuum bei 40°C getrocknet.
Ausbeute: 17,82 g (66,4% der Theorie)
¹H-NMR (300 MHz, d⁶-DMSO): δ= 3,30 (m, 2H), 3,990 (s, 3H),4,45 (t, 2H), 7,25 (d, 2H), 8,03 (d, 2H).

### VI: Methyl 4-[(2-oxotetrahydro-3-furanyl)methyl]benzoat

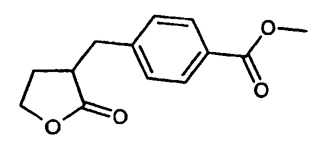

20,00 g (0,09 mol) Methyl 4-{[2-oxodihydro-3(2H)-furanyliden]methyl}benzoat aus Bsp. V werden in 240 ml Eisessig suspendiert mit 2,00 g 10% Palladium-Kohle versetzt und 4 Stunden lang bei Normaldruck hydriert. Das Reaktionsgemisch wird über Kieselgur filtriert und das Lösungsmittel unter vermindertem Druck abdestilliert.
Ausbeute: 19,00 g (92,4 % der Theorie)
¹H-NMR (300 MHz, d⁶-DMSO): δ= 1,9 (m, 1H) 2,15 (m, 1H), 2,8 (m, 1H), 3,0 (m, 1H), 3,1 (m, 1H), 3,85 (s, 3H), 4,1 (m, 1H), 4,2 (m, 1H), 7,25 (d, 2H), 8,03 (d, 2H).

### VII: 4-Bromo-2-[4-(methoxycarbonyl)benzyl]butansäure

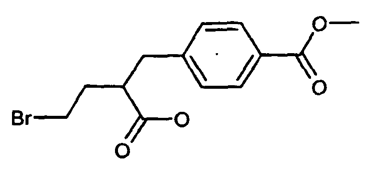

9,00 g (38,42 mmol) Methyl 4-[(2-oxotetrahydro-3-furanyl)methyl]benzoat aus Bsp. VI werden in 54 ml einer 33-prozentigen HBr-Lösung in Eisessig suspendiert und 40 min bei 80°C gerührt. Die Reaktionslösung wird auf Eiswasser gegossen, der entstandene Niederschlag abgesaugt, mit Wasser gewaschen und im Vakuum bei 40°C getrocknet.
Ausbeute: 11,01 g (90,9 % der Theorie).
¹H-NMR (300 MHz, d⁶-DMSO): δ= 1,90 (m, 1H), 2,10 (m, 1H), 2,70 (m, 1H), 2,90 (m, 2H), 3,53 (m, 2H), 3,83 (s, 3H), 7,35 (d, 2H), 7,92 (d, 2H).

### VIII: Methyl 4-(4-bromo-2-formylbutyl)benzoat

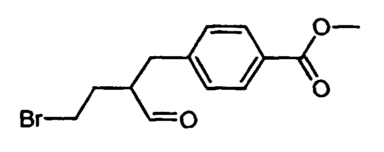

10,7 g (33,95 mmol) 4-Bromo-2-[4-(methoxycarbonyl)benzyl]bulansäure aus Bsp. VII in 200 ml THF werden bei 0°C mit 40,74 ml (40,74 mmol) einer 1M Lösung von Boran in THF versetzt und unter Erwärmen auf Raumtemperatur 2 Stunden gerührt. Überschüssiges Boran wird durch Zugabe von Wasser zerstört. Nach Extraktion mit Ether wird die organische Phase über Magnesiumsulfat getrocknet und das Lösungsmittel unter vermindertem Druck abdestilliert. Es verbleiben 10,23 g (33,92 mmol) des sehr instabilen Methyl 4-[4-bromo-2-(hydroxymethyl)butyl]benzoat, die unmittelbar in 100 ml Methylenchlorid gelöst und zu einer Suspension von 10,98 g (50,92 mmol) Pyridiniumchlorochromat in 200 ml Methylenchlorid getropft werden.

Nach 3,5 Stunden wird die Lösung über Kieselgel filtriert, gut mit Ether nachgewaschen und das Lösungsmittel abdestilliert. Das Rohprodukt wird durch Blitzchromatographie an Kieselgel (0,04-0,063 nm) mit Methylenchlorid/Methanol 3/1 als Laufmittel gereinigt.
Ausbeute: 7,08 g (69,7 % der Theorie)
¹H-NMR (300 MHz, d⁶-DMSO): δ= 1,85 (m, 1H), 2,15 (m, 1H), 2,85 (m, 2H), 3,10 (m, 1 H), 3,53 (m, 2H), 3,85 (s, 3H)7,38 (d, 2H), 7,90 (d, 2H), 9,70 (s, 1H).

### IX: Methyl-4-((E/Z)-2-(2-bromoethyl)-4-{2-[(5-phenylpentyl)oxy]phenyl}-3-butenyl)benzoat

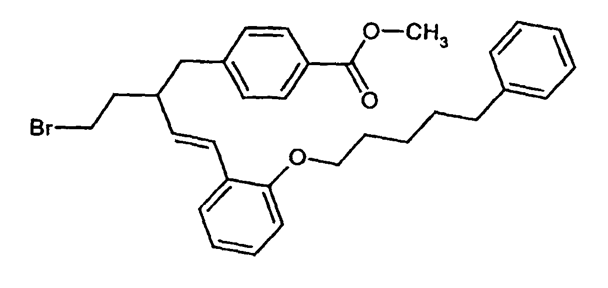

5,97 g (10,03 mmol) Triphenyl {2-[(5-phenylpentyl)oxy]benzyl}phosphoniumbromid (herstellbar analog Bsp. IId bis IVd mit 5-Phenylpentylbromid statt Butylbromid) werden in 80 ml THF suspendiert und bei 0°C mit 7,52 ml einer 1,6M n-Butyllithiumlösung in n-Hexan versetzt. Man rührt 30 Minuten und kühlt dann auf -20°C, woraufhin 3,00 g (10,03 mmol) Methyl 4-(4-bromo-2-formylbutyl)benzoat aus Bsp. VIII, gelöst in 20 ml THF, zugesetzt werden. Nach weiteren 30 min bei -20°C versetzt man mit Wasser und extrahiert mit Ethylacetat. Die organische Phase wird mit gesättigter Kochsalzlösung gewaschen, über Magnesiumsulfat getrocknet und das Solvens unter vermindertem Druck abdestilliert. Das Rohprodukt wird durch Blitzchromatographie an Kieselgel (0,04-0,063 nm) mit Cyclohexan/ Methylenchlorid 1/1 als Laufmittel gereinigt.
Ausbeute: 2,53 g (46,5% der Theorie) des E/Z Isomerengemisches im Verhältnis 15:85
¹H-NMR (300 MHz, d⁶-DMSO): δ= 1,40 (m, 1H), 1,65 (m, 4H9, 1,95 (m, 2H), 2,55 (t, 2H), 2,85 (m, 2H), 3,45 (m, 2H), 3,80 (s, 3H), 3,90 (t, 2H), 6,00 (m, 1H), 6,45 (m, 1H), 6,90 (m, 2H), 7,1-7,4 (m, 10H), 7,85 (d, 2H).

### X: Methyl-4-((E/Z)-2-(2-iodoethyl)-4-{2-[(5-phenylpentyl)oxy]phenyl}-3-butenyl)benzoat

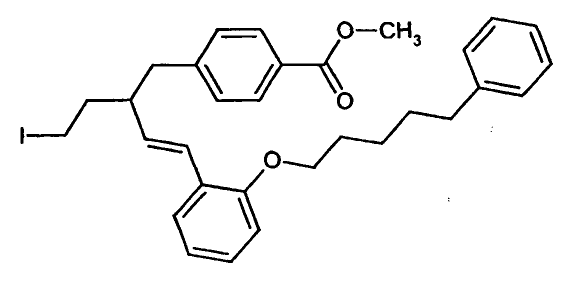

500,0 mg (0,930 mmol) Methyl4-((E/Z)-2-(2-bromoethyl)-4-{2-[(5-phenylpentyl)-oxy]phenyl}-3-butenyl)benzoat aus Bsp. IX und 153,95 mg (1,03 mmol) Natriumiodid werden in 2 ml Aceton 18 Stunden unter Rückfluss erhitzt. Man filtriert vom Feststoff ab, versetzt das Filtrat mit Wasser und extrahiert mit Methylenchlorid. Die Organische Phase wird mit gesättigter Natriumchloridlösung gewaschen, über Magnesiumsulfat getrocknet und das Lösungsmitel im Vakuum abdestilliert.
Ausbeute: 550,3 mg (97 % der Theorie)

### XI: Methyl-4-{4-[(2-ethoxy-2-oxoethyl)(methyl)amino]-2-formylbutyl}benzoat

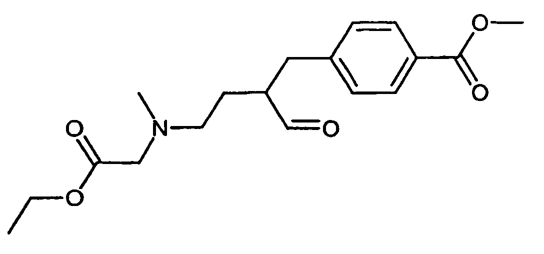

0,500 g (1,67 mmol) Methyl-4-(4-bromo-2-formylbutyl)benzoat aus Bsp. VIII, 0,257 g (1,67 mmol) Sarcosinethylesterhydrochlorid und 0,309 g (3,68 mmol) Natriumhydrogencarbonat werden in 10 ml Acetonitril 1 Stunde unter Rückfluss erhitzt. Die Reaktionsmischung wird abgekühlt, mit 50 ml Wasser versetzt und mit Ethylacetat mehrfach extrahiert. Die vereinigten organischen Phasen werden mit gesättigter Natriumchloridlösung gewaschen, über Magnesiumsulfat getrocknet und das Lösungsmittel im Vakuum abdestilliert. Das Rohprodukt wird durch Chromatographie an Kieselgel (0,04-0,063 nm) mit Methylenchlorid/Methanol 100:3 als Laufmittel gereinigt.
Ausbeute: 0,479 g (85,4 % der Theorie)

### XII: 8-(2-Hydroxyphenyl)-6-(4-Methoxycarbonylphenoxy)-octansäuremethylester

### XIIa: 2-{[tert-Butyl(dimethyl)silyl]oxy}benzaldehyd

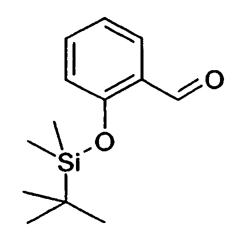

Zu einer Lösung von 10,00 g (81,89 mmol) Salicylaldehyd und 6,13 g (90,07 mmol) Imidazol in 82 ml DMF wurden 13,58 g (90,07 mmol) t-Butyldimethylsilylchlorid (TBDMSCI) zugegeben. Man rührte bei Raumtemperatur und kontrollierte die Reaktion per Dünnschichtchromatographie (Cyclohexan/EE 10:1). Die Mischung wurde mit 1 N NaOH versetzt und mit Petrolether extrahiert. Die vereinten organischen Phasen wurden über Na₂SO₄ getrocknet, das Lösungsmittel entfernt und das Produkt chromatografisch gereinigt (Kieselgel, Cyclohexan/EE 10:1). Man erhielt 16,94 g (87,5 %) einer klaren Flüssigkeit.
¹H-NMR (300 MHz, CDCl₃): δ = 0.18 (s, 6H), 0.92 (s, 9H), 6.78 (d, J = 8.3 Hz, 1H), 6.93 (t, *J =* 7.7 Hz, 1H), 7.36 (dt, *J =* 8.1 Hz, *J =* 1.9 Hz, 1H), 7.71 (dd, *J =* 9.3 Hz, *J* = 1.5 Hz, 1H), 10.37 (s, 1H).

### XIIb: 7-(Diethoxyphosphoryl)-6-oxoheplansäuremethylester

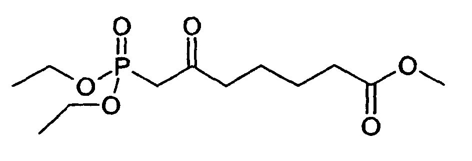

Zu einer Lösung von 15,00 g (74,95 mmol) Phosphonoessigsäurediethylester in 400 ml Toluol wurden bei 0°C 30,34 g (299,79 mmol) Triethylamin sowie 12,21 g (112,42 mmol) Trimethylchlorsilan zugetropft. Man rührte 1 h bei Raumtemperatur und fügte 7,14 g (74,95 mmol) Magnesiumchlorid zu. Man rührte eine Stunde und tropfte 16,56 g (89,94 mmol) Adipinsäuremonomethylesterchlorid zu. Die Mischung wurde 24 h bei Raumtemperatur gerührt. Es wurde mit Wasser versetzt. Man extrahierte mit Diethylether, trocknete die organischen Phasen über Na₂SO₄ und entfernte das Lösungsmittel. Das Produkt wurde chromatografisch gereinigt (Kieselgel, Ethylacetat). Man erhielt 7,83 g (35,5 %) einer klaren Flüssigkeit.
¹H NMR (300 MHz, CDCl₃): δ = 1.34 (t, *J* = 6.9 Hz, 6H), 1.59 - 1.66 (m, 4H), 2.25 - 2.40 (m, 2H), 2.59 - 2.70 (m, 2H), 3.07 (d, *J* = 22.9 Hz, 2H), 3.66 (s, 3H), 4.14 (quint, *J* = 7.2 Hz, 4H).

### XIIc:(E)-8-(2-{[tert-Butyl(dimethyl)silyl]oxy)phenyl)-6-oxo-7-octensäuremethylester

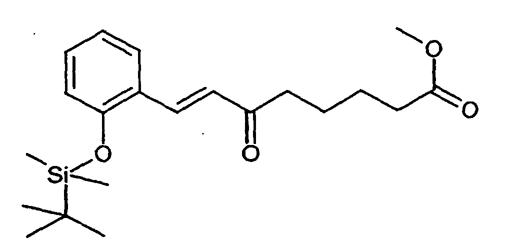

Zu einer Lösung von 3,20 g (10,87 mmol) 7-(Diethoxyphosphoryl)-6-oxoheptansäuremethylester aus Bsp. XIIb in 53 ml THF wurden unter Argon 0,26 g (10,87 mmbl) Natriumhydrid zugegeben. Man rührte 30 min bei Raumtemperatur und tropfte eine Lösung von 9,06 mmol 2-{[tert-Butyl(dimethyl)silyl]oxy}benzaldehyd aus Bsp. XIIa in 20 ml THF zu und rührte 18 h bei Raumtemperatur. Man versetzte mit Wasser, extrahierte mit Ethylacetat, trocknete die vereinten organischen Phasen über Na₂SO₄ und entfernte das Lösungsmittel. Das Produkt wurde chromatografisch gereinigt (Kieselgel, Cyclohexan/EE 10:1). Man erhielt 2.51 g (67.8 %) einer farblosen Flüssigkeit.
¹H-NMR (300 MHz, CDCl₃): δ = 0.24 (s, 6H), 1.05 (s, 9H), 1.62 - 1.77 (m, 4H), 2.29 - 2.41 (m, 2H), 2.62 - 2.73 (m, 2H), 3.66 (s, 3H), 6.67 (d, J = 16.6 Hz, 1H), 6.84 (m_{c} = 1H), 6.96 (t, J = 7.6 Hz, 1H), 7.20 - 7.30 (m, 1H), 7.56 (d, J = 7.7 Hz, 1H), 7.96 (d, *J* = 16.6 Hz, 1H).

### XIId: (E)-8-(2-{[tert-Butyl(dimethyl)silyl]oxy}phenyl)-6-hydroxy-7-octensäuremethylester

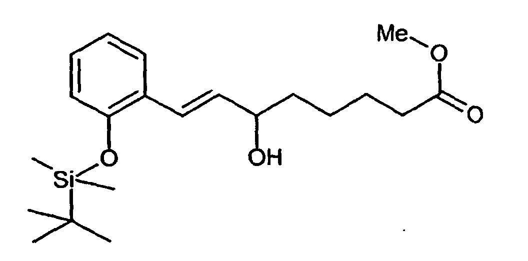

Zu einer Lösung von 1,436 g (3,86 mmol) CeCl₃·7H₂O und 3,67 mmol (E)-8-(2-{[tert-Butyl(dimethyl)silyl]oxy}phenyl)-6-oxo-7-octensäuremethylester aus Bsp. XIIc in 30 ml Methanol wurden bei 0°C 0,146 g (3,86 mmol) Natriumborhydrid zugegeben. Man rührte die Mischung bei 0°C und kontrollierte den Reaktionsverlauf per Dünnschichtchromatografie. Man versetzte mit gesättigter NH₄Cl-Lösung, extrahierte mit Ethylacetat und trocknete die vereinten organischen Phasen über Na₂SO₄. Das Produkt wurde chromatografisch gereinigt (Kieselgel, Cyclohexan/EE 10:2). Man erhielt 1,38 g (91,5 %) einer farblosen Flüssigkeit.
¹H NMR (400 MHz, CDCl₃): δ = 0.01 (s, 6H), 0.80 (s, 9H), 1.13 - 1.54 (m, 7H), 2.11 (t *J=* 7.3 Hz, 2H), 3.44 (s, 3H), 3.99 - 4.11 (m, 1H), 5.93 (*dd, J=* 15.9 *Hz, J =* 6.9 Hz, 1H), 6.57 (dd, *J =* 8.0 Hz, *J* = 1.0 Hz, 1H), 6.63 - 6.73 (m, 2H), 6.90 (dt, *J =* 8.0 Hz, *J* = 1.7 Hz, 1H), 7.23 (dd, *J* = 7.8 Hz, *J* = 1.7 Hz, 1 H).

### XIIe: 8-(2-{[tert-Butyl(dimethyl)silyl]oxy}phenyl)-6-hydroxyoctansäuremethylester

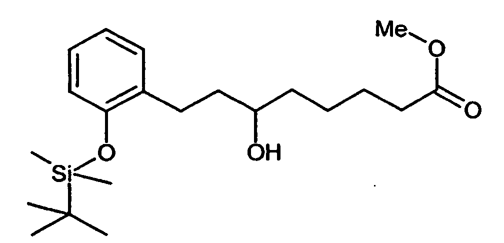

Zu einer Lösung von 4,38 mmol der Verbindung aus Bsp. XIId in 22,5 ml Ethylacetat wurden 30 mg Palladium auf Kohle (10 %) zugegeben. Man rührte unter einer Wasserstoffatmosphäre bis keine weitere Absorption zu beobachten war, filtrierte über Celite und entfernte das Lösungsmittel.
Ausbeute: 82,2 %
¹H NMR (300 MHz, CDCl₃): δ = 0.25 (s, 3H), 0.26 (s, 3H), 1.03 (s, 9H), 1.20 - 1.84 (m, 9H), 2.26 - 2.38 (m, 2H), 2.66 - 2.78 (m, 2H), 3.49 - 3.62 (m, 1H), 3.67 (s, 3H), 6.75 - 6.84 (m, 1H), 6.85 - 6.94 (m, 1H), 7.02 - 7.19 (m, 2H).

### XIIf: 8-(2-{[tert-Butyl(dimethyl)silyl]oxy}phenyl)-6-(4-Methoxycarbonylphenoxy)-octansäuremethylester

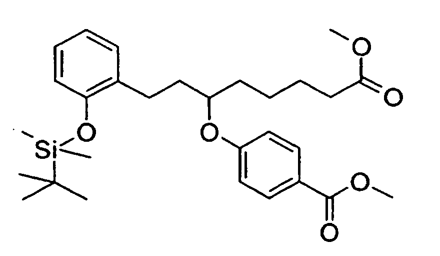

Zu einer Lösung von 55,32 mg (0,36 mmol) 4-Hydroxybenzeosäuremethylester und 95,36 mg (0,36 mmol) Triphenylphosphin in 2,5 ml THF tropfte man innerhalb von 2 h eine Lösung von 0,24 mmol der Verbindung aus Bsp. XIIe und 63,32 mg (0,36 mmol) DEAD in 2,5 ml THF zu. Man rührte 18 h bei Raumtemperatur, versetzte mit 40 ml Diethylether, filtrierte und entfernte das Lösungsmittel. Das Produkt wurde chromatografisch gereinigt (Kieselgel, Cyclohexan/EE 10:1).
Ausbeute: 64,3 %
¹H NMR (400 MHz, CDCl₃): δ = 0.20 (s, 3H), 0.21 (s, 3H), 0.98 (s, 9H), 1.31 - 1.77 (m, 6H), 1.84 - 2.07 (m, 2H), 2.28 (t, *J* = 7.3 Hz, 2H), 2.54 - 2.68 (m, 1H), 2.70 - 2.81 (m, 1H), 3.64 (s, 3H), 3.87 (s, 3H), 4.25 - 4.38 (m, 1H), 6.74 - 6.88 (m, 4H), 7.01 - 7.10 (m, 2H), 7.93 (d, *J* = 8.8 Hz, 2H).

### XII: 8-(2-Hydroxyphenyl)-6-(4-Methoxycarbonylphenoxy)-octansäuremethylester

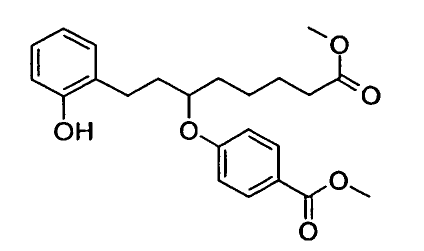

Eine Lösung von 1,30 g (2,53 mmol) der Verbindung aus Bsp. XIIf wurde mit 2,78 ml (2,78 mmol) Tetrabutylammoniumfluorid (TBAF) (1 M In THF) versetzt. Man rührte bei Raumtemperatur und verfolgte den Reaktionsverlauf per DC (Kieselgel, Cyclohexan/EE 10:1, KMnO₄). Nach beendeter Reaktion versetzte man mit Wasser, extrahierte mit Diethylether, trocknete die vereinten organischen Phasen über Na₂SO₄ und entfernte das Lösungsmittel. Das Produkt wurde chromatografisch gereinigt und man erhielt 0,85 g (84,24 %) einer klaren Flüssigkeit.
¹H NMR (400 MHz, CDCl₃): δ = 1.32 - 1.78 (m, 6H), 1.90 - 2.04 (m, 2H), 2.30 (t, *J* = 6.6 Hz, 2H), 2.60 - 2.72 (m, 1H), 2.72 - 2.83 (m, 1H), 3.65 (s, 3H), 3.87 (s, 3H), 4.33 (quint, *J =* 5.9 Hz, 1H), 5.31 (bs, 1H), 6.71 - 6.88 (m, 4H), 7.01 - 7.14 (m, 2H), 7.93 (d, *J* = 9.0 Hz, 2H).

### XIII: (7E)-8-{2-[(4-cyclohexylbenzyl)oxy]phenyl}-6-oxo-7-octensäuremethylester

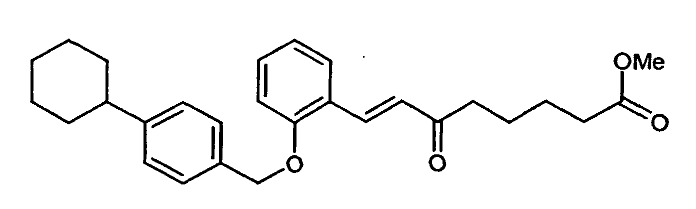

Zu einer Suspension von 0,25 g (10,19 mmol) Natriumhydrid in 20 ml THF wurde unter Argon eine Lösung von 3,00 g (10,19 mmol) 7-(Diethoxyphosphoryl)-6-oxoheptansäuremethylester XIIb in 10 ml THF zugetropft. Nach 30 min wurde eine Lösung von 2,50 g (8,49 mmol) 2-[(4-Cyclohexylbenzyl)oxy]benzaldehyd (erhältlich aus Salicylaldehyd und 4-Cyclohexylbenzylchlorid in 10 ml THF zugetropft. Die Mischung wurde 2 Tage bei Raumtemperatur gerührt. Man versetzt mit Wasser, extrahiert mit Ethylacetat und trocknet die vereinten organischen Phasen über Na₂SO₄. Das Produkt wurde chromatografisch gereinigt (Kieselgel, Cyclohexan/Ethylacetat 10:1).
Ausbeute: 2.82 g (76.41 %).
¹H NMR (200 MHz, CDCl₃): δ = 1.10 - 1.98 (m, 14H), 2.23 - 2.74 (m, 5H), 3.66 (s, 3H), 5.12 (s, 2H), 6.80 (d, *J* = 16.4 Hz, 1H), 6.88 - 7.08 (m, 2H), 7.15 - 7.43 (m, 5H), 7.47 - 7.63 (m, 1 H), 7.95 (d, *J =* 16.4 Hz, 1H).

### XIV: 8-{2-[(4-cyclohexylbenzyl)oxy]phenyl}-6-oxooctansäuremethylester

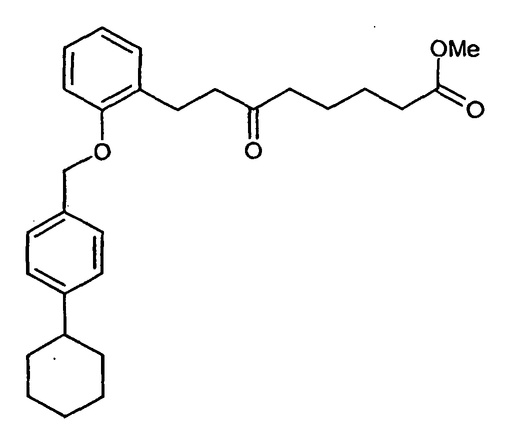

Eine Suspension von 2,80 g (6,44 mmol) (7E)-8-{2-[(4-Cyclohexylbenzyl)-oxy]phenyl}-6-oxo-7-octensäuremethylester XIII und 0,06 g Pd/C (10 % Pd) in 30 ml Ethylacetat wurde 3 h unter einer Wasserstoffatmosphäre gerührt. Der Katalysator wurde durch Filtration über Celite abgetrennt und das Produkt chromatografisch gereinigt (Kieselgel, Cyclohexan/Ethylacetat 20: 1).
Ausbeute: 2,30 g (81,7 %)
¹H NMR (300 MHz, CDCl₃): δ = 1.15 - 1.62 (m, 9H), 1.69 - 1.96 (m, 5H), 2.20-2.39 (m, 4H), 2.51 (m, 1H), 2.70 (t, *J* = 7.0 Hz, 2H), 2.93 (t, *J* = 7.6 Hz, 2H), 3.65 (s, 3H), 5.04 (s, 2H), 6.82 - 6.94 (m, 2H), 7.06 - 7.27 (m, 4H), 7.33 (d, *J* = 7.93 Hz, 2H).

### XV: (7E)-8-{2-[(4-cyclohexylbenzyl)oxy]phenyl}-6-hydroxy-7-octensäuremethylester

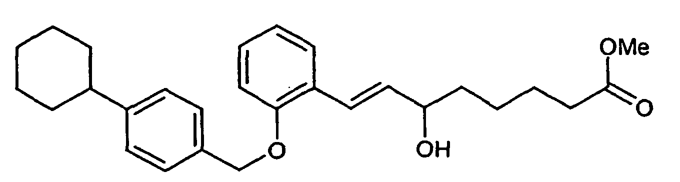

(7E)-8-{2-[(4-cyclohexylbenzyl)oxy]phenyl}-6-oxo-7-octensäuremethylester aus Bsp. XIII wurde analog zu Bsp. XIId mit Natriumborhydrid in den entsprechenden Alkohol überführt. Die Ausbeute betrug 92,2 %.
¹H NMR (300 MHz, CDCl₃): δ = 1.19 - 1.94 (m, 17H), 2.31 (t, *J* = 7.7 Hz, 2H), 2.42 - 2.60 (m, 1H), 3.65 (s, 3H), 4.26 (q, *J* = 6.6 Hz, 1H), 5.05 (s, 2H), 6.22 (dd, *J* = 16.1 Hz, *J* = 7.0 Hz, 1H), 6.87 - 6.97 (m, 3H), 7.13 - 7.26 (m, 3H), 7.30 - 7.37 (m, 2H), 7.40 - 7.48 (m, 1H).

Analog wurde hergestellt:

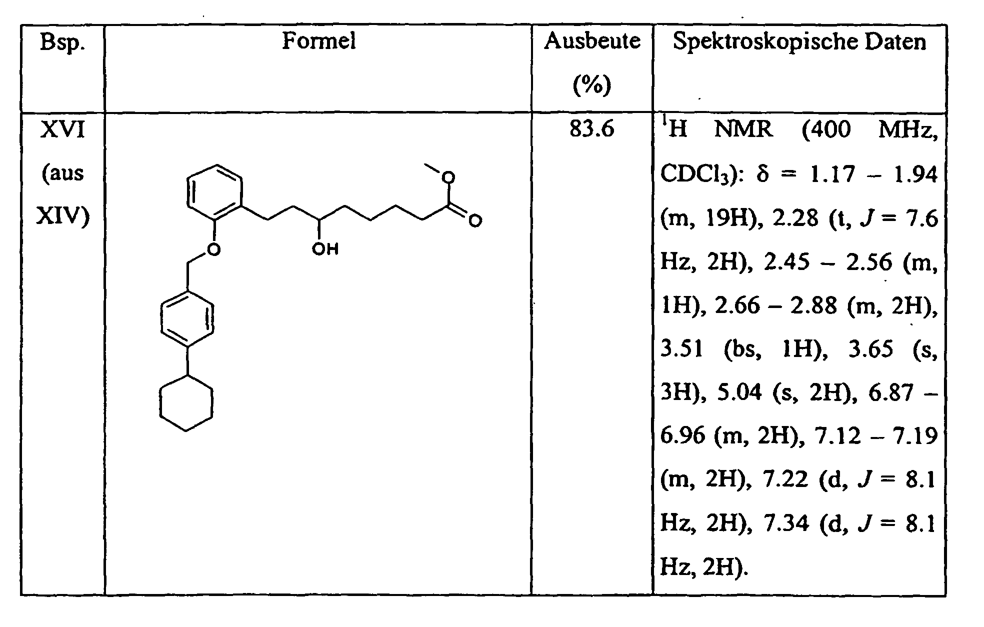

### XVII: 6-Bromo-8-{2-[(4-cyclohexylbenzyl)oxy]phenyl}octanosäuremethylester

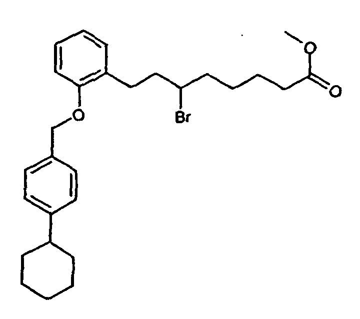

Zu einer Lösung von 500 mg (1,14 mmol) 8-{2-[(4-Cyclohexylbenzyl)oxy]phenyl}-6-hydroxyoctansäuremethylester XVI in 5 ml Diethylether wurden bei 0°C 140 mg (0,51 mmol) Phosphortribromid zugegeben. Man rührte 1 h bei 0°C und weitere 16 h bei Raumtemperatur. Man versetzte mit Wasser, extrahierte mit Cyclohexan und trocknete die vereinten organischen Phasen über Na₂SO₄. Das Produkt wurde chromatografisch gereinigt (Kieselgel, Cyclohexan(Ethylacetat 10: 1).
Ausbeute: 290 mg (50,7 %).
¹H NMR (300 MHz, CDCl₃): δ = 1.17 - 1.94 (m, 16H), 2.05 - 2.17 (m, 2H), 2.28 (t, *J* = 7.2 Hz, 2H), 2.44 - 2.5 8 (m, 1H), 2.68 - 2.81 (m, 1H), 2.88 - 3.01 (m, 1H), 3.65 (s, 3H), 3.98 (quint, *J* = 6.5 Hz, 1H), 5.04 (s, 2H), 6.83 - 6.94 (m, 2H), 7.11 - 7.37 (m, 6H).

### XVIII: Dimethyl 6-[2-(2-hydroxyphenyl)ethyl]undecanedioat

### XVIIIa: 1,1-Diallyl 5-methyl 1,1,5-pentanetricarboxylat

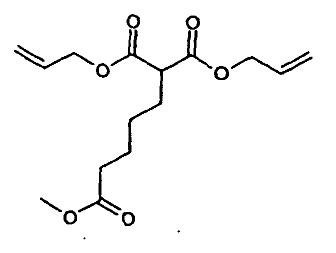

Zu einer Lösung von 2,00 g (69,62 mmol) Malonsäurediallylester in 700 ml Dioxan wurden vorsichtig 1,50 g (52,22 mmol) Natrimhydrid zugegeben. Nach beendeter Gasentwicklung wurde die Mischung 20 min bei Raumtemperatur gerührt und eine Lösung von 7,00 g (34,81 mmol) 5-Bromvaleriansäuremethylester in 120 ml Dioxan zugetropft. Die Lösung wurde 16 h bei 110°C gerührt. Der gebildete Niederschlag wurde abfiltriert, das Lösungsmittel entfernt und der Rückstand in Wasser aufgenommen. Man extrahierte mit Diethylether, trocknete die vereinten organischen Pahsen über Na₂SO₄ und entfernte das Lösungsmittel. Das Produkt wurde chromatographisch gereinigt (Kieselgel, Cylcohexan/Ethylacetat 10:1).
Ausbeute: 4,16 g (40,1 %)
¹H NMR (300 MHz, CDCl₃): δ = 1.37 - 1.49 (m, 2H), 1.58 - 1.78 (m, 2H), 1.87 - 2.03 (m, 2H), 2.33 (t, *J* = 5.5 Hz, 2H), 3.41 (t, *J* = 8.0 Hz, 1H), 3.68 (s, 3H), 4.60 - 4.68 (m, 4H)5.21 - 5.40 (m, 4H), 5.79 - 6.02 (m, 2H).

### XVIIIb: 5,5-Diallyl 1,9-dimethyl 1,5,5,9-nonanetetracarboxylat

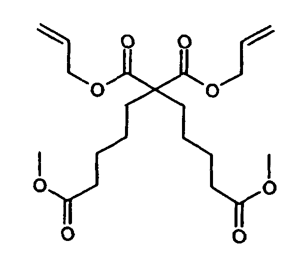

Zu einer Lösung von 2,00 g (6,70 mmol) XVIIIa in 20 ml Dimethylformamid (DMF) wurden vorsichtig 0,182 g (7,37 mmol) Natriumhydrid zugegeben. Nach beendeter Gasentwicklung wurde eine Lösung von 1,75 g (8,71 mmol) 5-Bromvaleriansäueremethylester zugegeben und rührte 16 h bei Raumtemperatur. Man versetzte mit Wasser, extrahierte mit Diethylether, trocknete die vereinten organischen Phasen über Na₂SO₄ und entfernte das Lösungsmittel. Das Produkt wurde chromatographisch gereinigt (Kieselgel, Cyclohexan/Ethylacetat 10:1)
Ausbeute: 2,39 g (86,4 %)
¹H NMR (300 MHz, CDCl₃): δ = 1.34 - 1.45 (m 2H), 1.60 - 1.71 (m, 2H), 1.82 - 1.93 (m, 2H), 2.32 (t, *J* = 7.4 Hz, 2H), 3.52 (s, 2H), 3.67 (s, 3H), 4.56 - 4.70 (m, 4H), 5.21 - 5.34 (m, 4H), 5.79 - 5.94 (m, 2H), 7.25 - 7.66 (m, 4H).

### XVIIIc: 7-Methoxy-2-(5-methoxy-5-oxopentyl)-7-oxoheptansäure

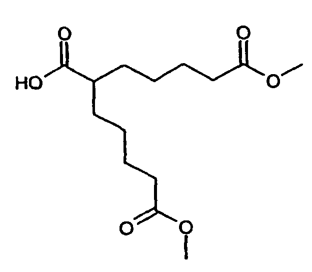

Zu einer Lösung von 10,00 g (24,24 mmol) XVIIIb in 85 mmol Dioxan wurden 0,51 g (1,94 mmol) Triphenylphosphin und 0,11 g (0,48 mmol) Palladiumacetat gegeben. Man versetzte mit einer Lösung aus 3,28 g (60,61 mmol) Ameisensäure und 8,10 g (80,00 mmol) Triethylamin in 255 ml Dioxan. Die Lösung wurde 3 h unter Rückfluss erhitzt. Man entfernte das Lösungsmittel und reinigte das Produkt chromatographisch (Kieselgel, Ethylacetat, dann MeOH).
Ausbeute: 5,84 g (83,5 %)
¹H NMR (300 MHz, CDCl₃): δ = 1.20 - 1.42 (m, 4H), 1.50 - 1.67 (m, 4H), 1.76 - 1.91 (m, 4H), 2.18 - 2.34 (m, 5H), 3.62 (s, 6H).

### XVIIId: Dimethyl 6-(hydroxymethyl)undecanedioat

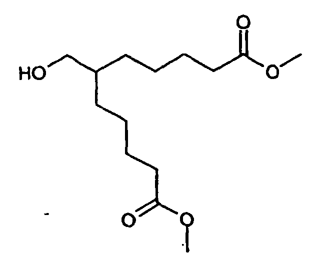

Zu einer Lösung von 1,90 g (6,59 mmol) XVIIIc wurden bei -10°C 8,49 ml 1 M BH₃ in THF zugetropft. Man ließ die Reaktionsmischung auf Raumtemperatur erwärmen und versetzte nach vollständiger Reaktion mit Wasser. Man extrahiert mit Ethylacetat, trocknet die vereinten organischen Phasen über Na₂SO₄ und entfernt das Lösungsmittel. Das Rohprodukt weiter umgesetzt.

### XVIIIe: Dimethyl 6-formylundecanedioat

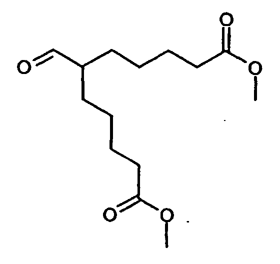

Die Verbindung XVIIId wird unter Bedingungen der Swern-Oxidation (vgl. z.B. J. March, Advanced Organic Chemistry, 3^{rd} ed., Wiley 1985, 1082) in den Aldehyd überführt. Das Rohprodukt weiter umgesetzt.

### XVIIIf: Dimethyl 6-[(E)-2-(2-hydroxyphenyl)ethenyl]undecanedioat

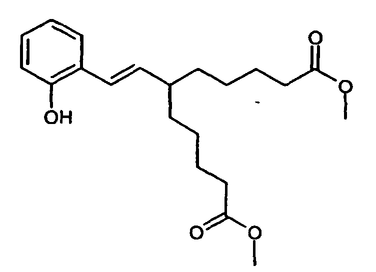

Zu einer Suspension von 3.03 g (6.61 mmol) (2-Hydroxybenzyl)-triphenylphosphoniumbromid in 10 ml Tetrahydrofuran (THF) wurden langsam bei -78°C 8,15 ml n-Butyllithium (1.6 M in Hexan) zugetropft. Man rührte 30 min bei -78°C, entfernte das Kühlbad und ließ die Reaktionsmischung auf Raumtemperatur erwärmen. Man kühlte wieder auf -78°C und gab eine Lösung von 1,50 g (5,51 mmol) XVIIIe zu. Man ließ die Reaktionsmischung auf Raumtemperatur erwärmen und rührte über Nacht. Man versetzte mit Wasser, extrahierte mit Ethylacetat und trocknete die vereinten organischen Phasen über Na₂SO₄. Das Produkt wurde chromatographisch (Kieselgel, Cyclohexan/Ethylacetat 5:1) gereinigt.
Ausbeute: 0,80 g (40,3 %)
¹H NMR (200 MHz, CDCl₃): δ = 1.06 - 1.81 (m, 12H), 2.06 - 2.41 (m, 5H), 3.65 (s, 6H), 5.60 (s, 1H), 5.77 (dd, *J* = 15.9 Hz, *J* = 9.2 Hz, 1H), 6.56 (d, *J* = 15.8 Hz, 1H), 6.76 - 6.94 (m, 2H), 7.05 - 7.17 (m, 1H), 7.21 - 7.38 (m, 1H).

### XVIII: Dimethyl 6-[2-(2-hydroxyphenyl)ethyl]undecanedioat

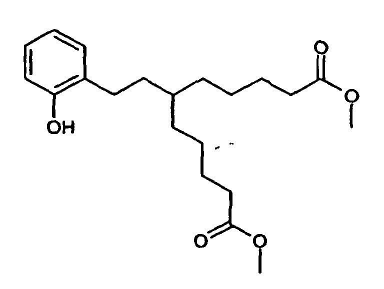

Eine Lösung von 770 mg (2.14 mmol) XVIIIf in15 ml Ethylacetat wurde mit 20 mg (Pd/C (10 % Pd) versetzt. Man rührte über Nacht unter einer Wasserstoffatmosphäre. Die Mischung wurde über Celite abgesaugt und das Lösungsmittel entfernt.
Ausbeute: 766 mg (98,8 %)
¹H NMR (400 MHz, CDCl₃): δ = 1.17 - 1.68 (m, 12H), 2.02 - 2.16 (m, 4H), 2.27 - 2.36 (m, 4H), 2.53 - 2.60 (m, 2H), 3.67 (s, 6H), 6.73 - 6.77 (m, 1H), 6.79 - 6.91 (m, 1H), 7.02 - 7.13 (m, 2H).

### Synthesebeispiele

### Bsp 1: 6-(4-Methoxycarbonylbenzyl)-8-(2-Methoxyphenyl)-7-octensäuremethylester

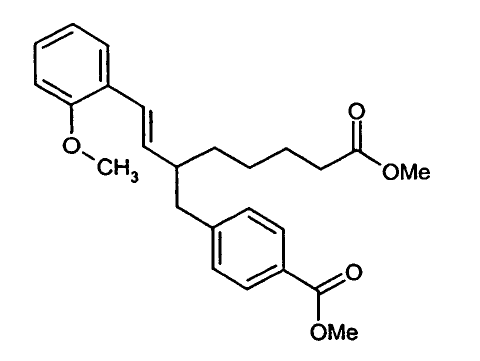

77,4 mg (0,17 mmol) 2-Methoxybenzyltriphenylphosphoniumbromid aus Bsp. IVj werden bei 0°C unter Argon in 20 ml THF suspendiert und mit 0,115 ml Buthyllithium (0,18 mmol, 1,6 M Lösung in Hexan) versetzt. Die tieforange Lösung wird 30 min. bei 0°C gerührt. Eine Lösung von 51,2 mg (0,17 mmol) 6-Formyl-7-(4-methoxycarbonylphenyl)heptansäuremethylester (Synthese analog zu EP-A-0 341 551, S. 32, Bsp. 44) in 15 ml THF wird bei dieser Temperatur tropfenweise hinzugegeben. Die Mischung wird 30 min. bei 0°C gerührt. Es wird bei 0°C mit Wasser versetzt, auf Raumtemperatur erwärmt und mit Essigsäureethylester extrahiert. Die organische Phase wird mit Natriumchloridlösung gewaschen, mit Magnesiumsulfat getrocknet und im Vakuum eingedampft. Die Substanz wird zur Reinigung an Kieselgel 60 (Korngröße 0,040-0,063 mm) mit Cyclohexan/Essigester 9:1 bis 1:1 als Eluenten chromatographiert.
Ausbeute: 17,7 mg (25,8 % d.Th.)
¹H-NMR (400 MHz, CDCl₃): 7,95 (m, 2H), 7,40-6,70 (m, 6H), 6,50 (d, J=16 Hz, 1H), 6,00 (dd, J=16 Hz, J=8Hz, 1H), 3,90 (s, 3H), 3,80 (s, 3H), 3,60 (s, 3H), 2,80-2,50 (m, 3H), 2,30 (m, 2H), 1,80-1,20 (m, 6H)

Analog wurden dargestellt:

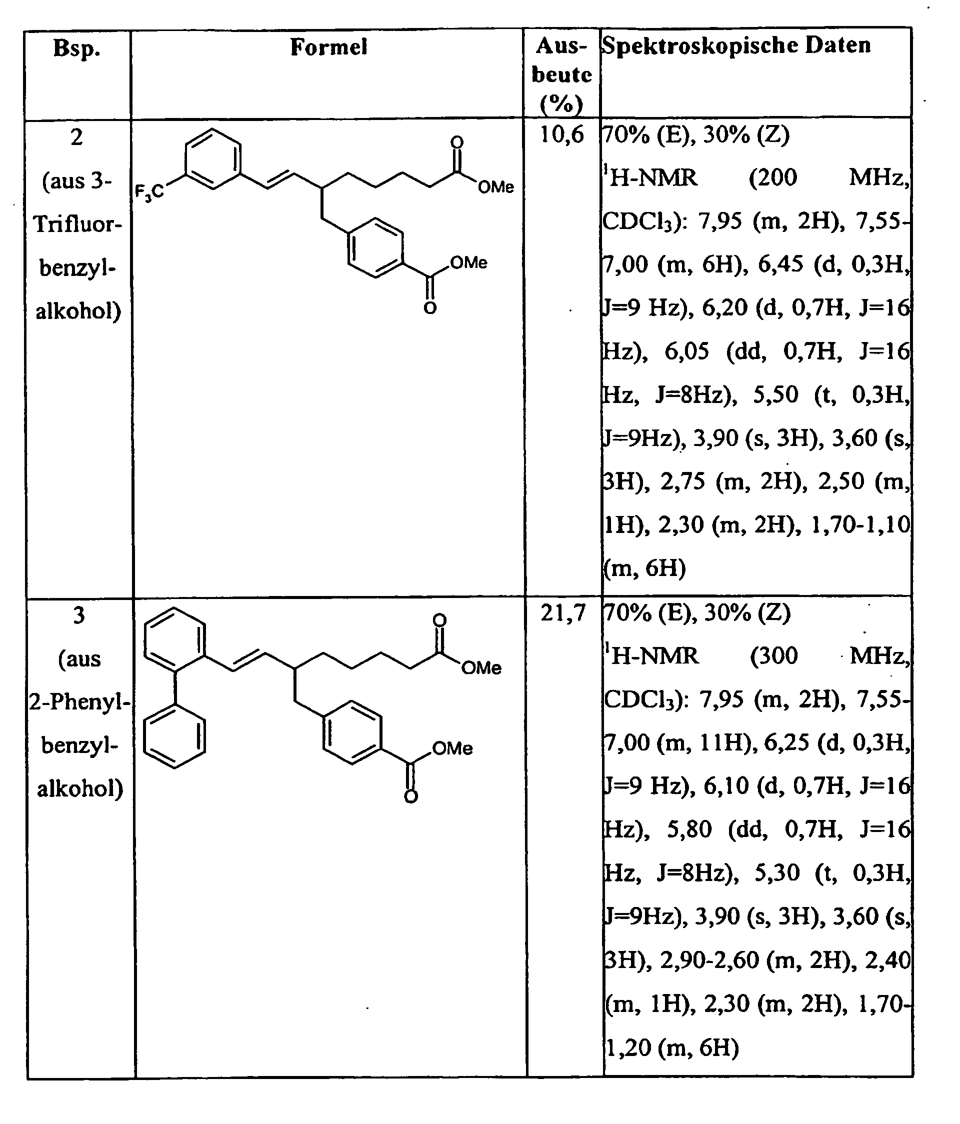

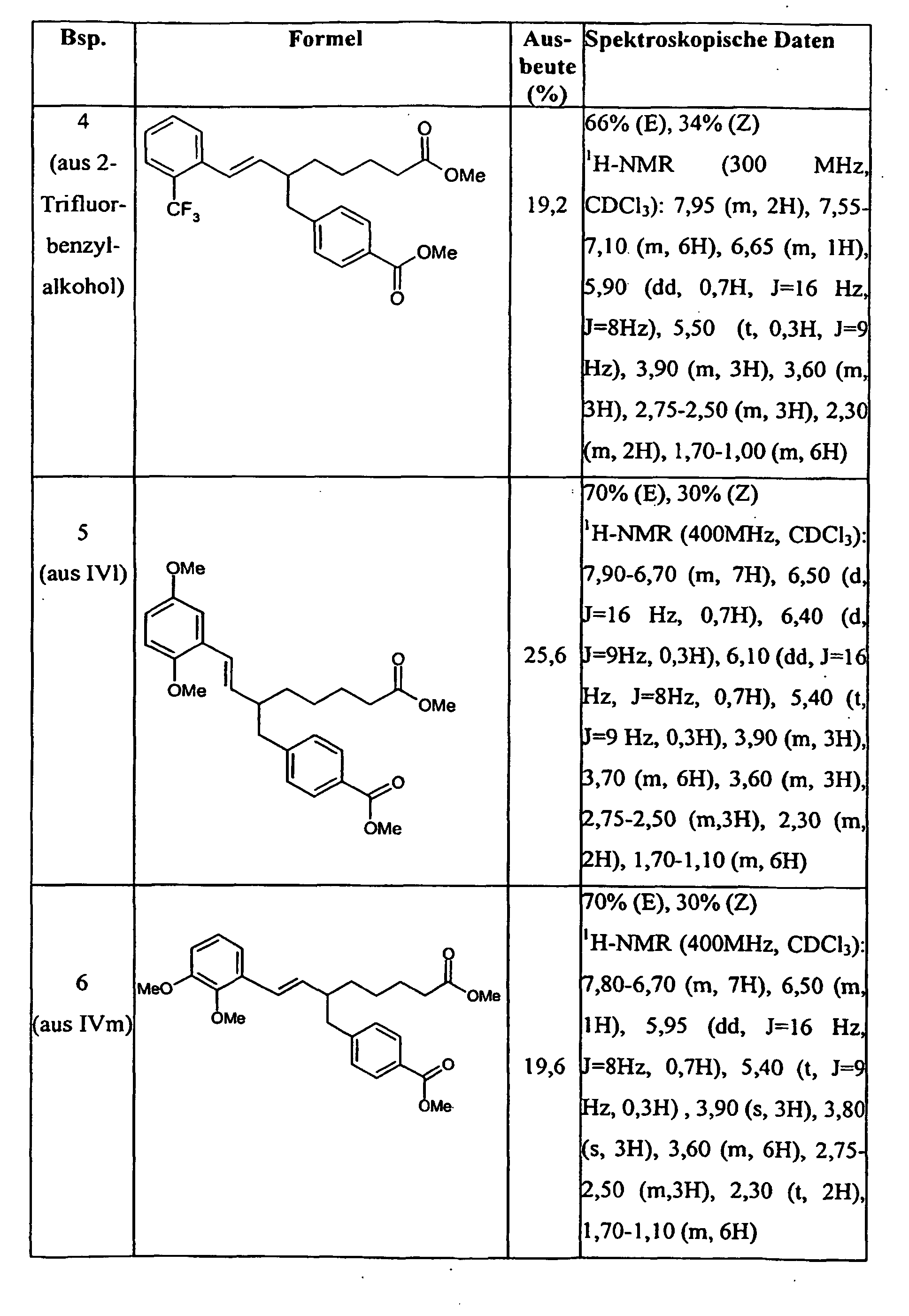

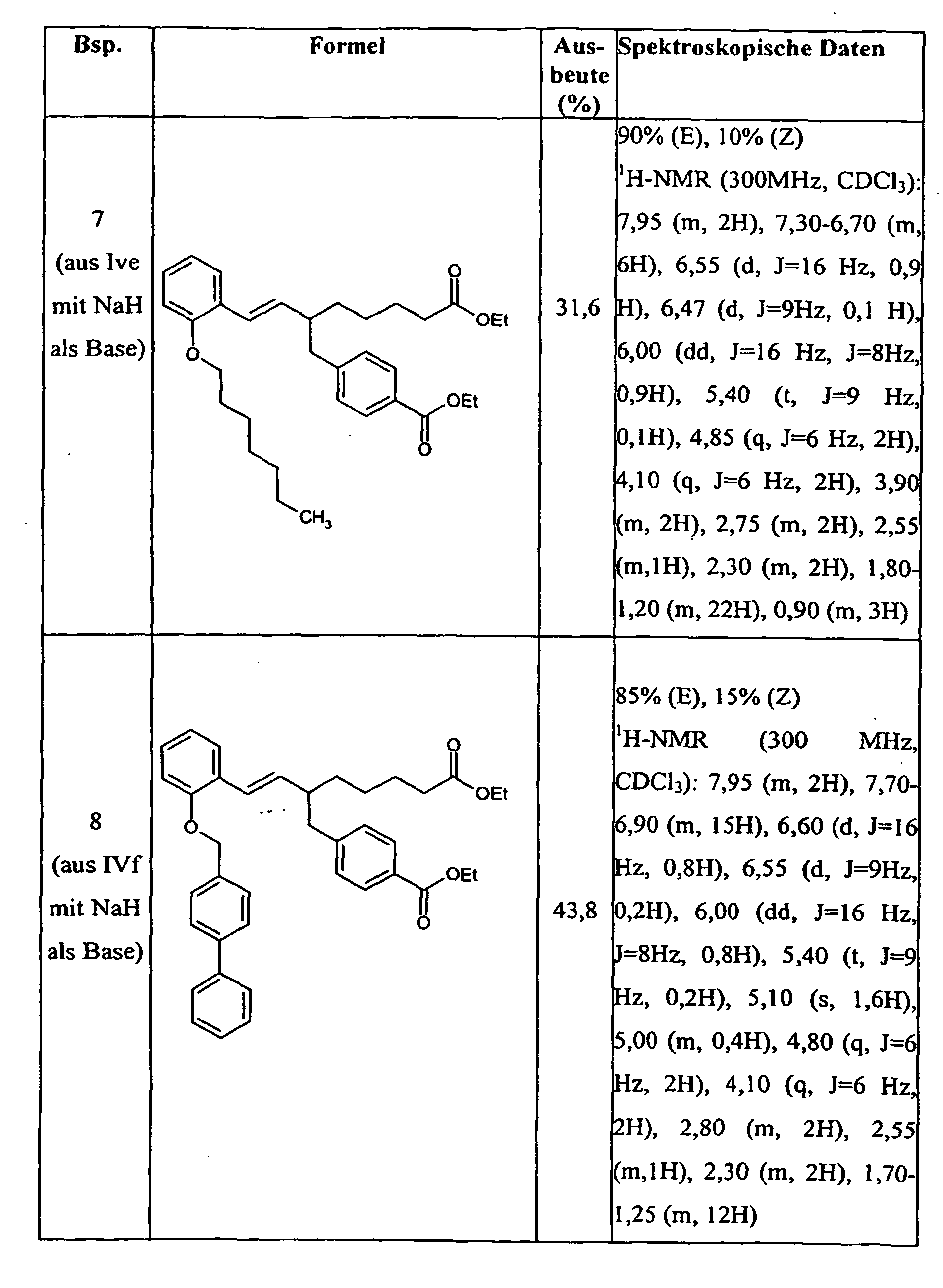

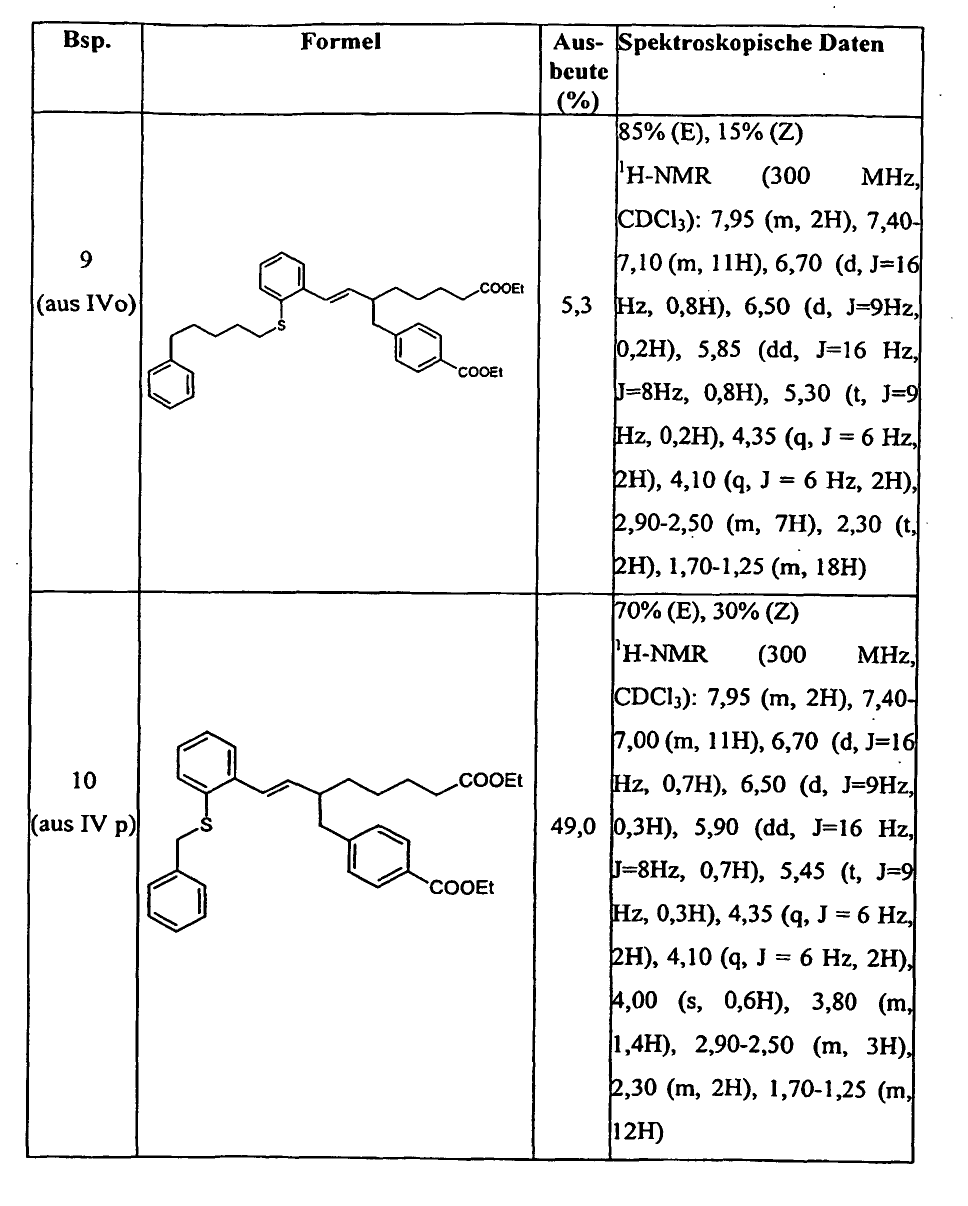

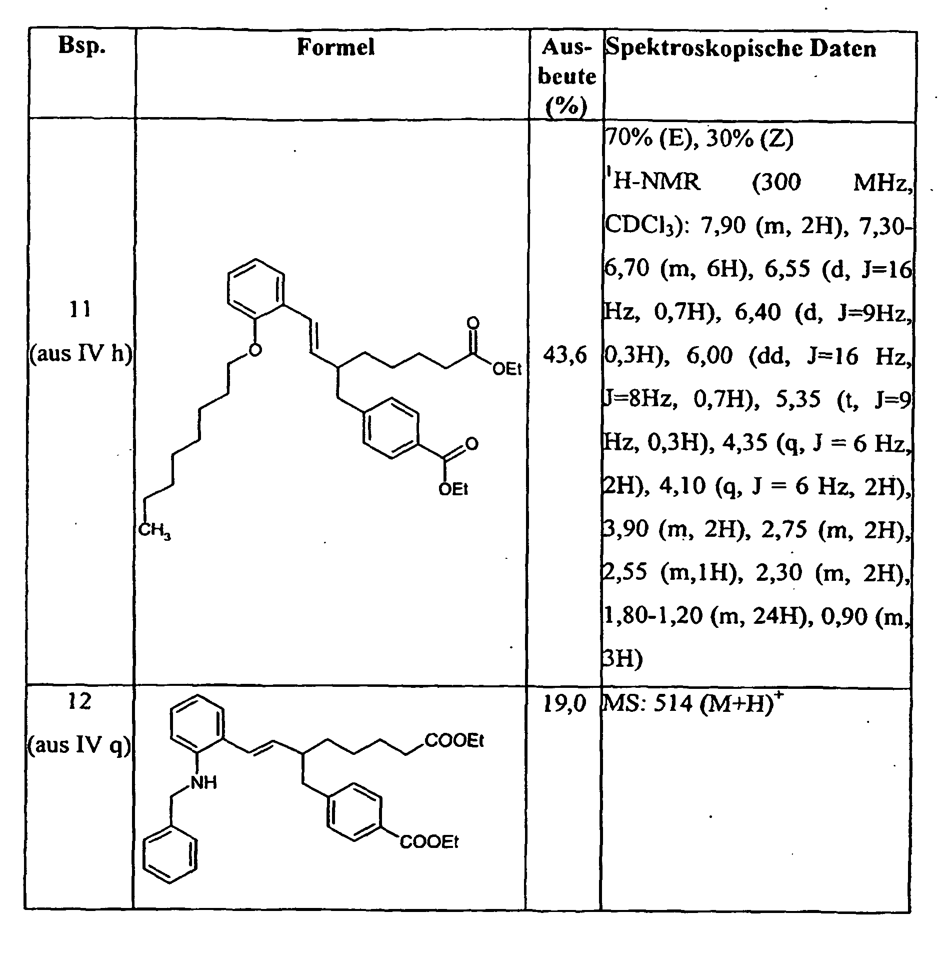

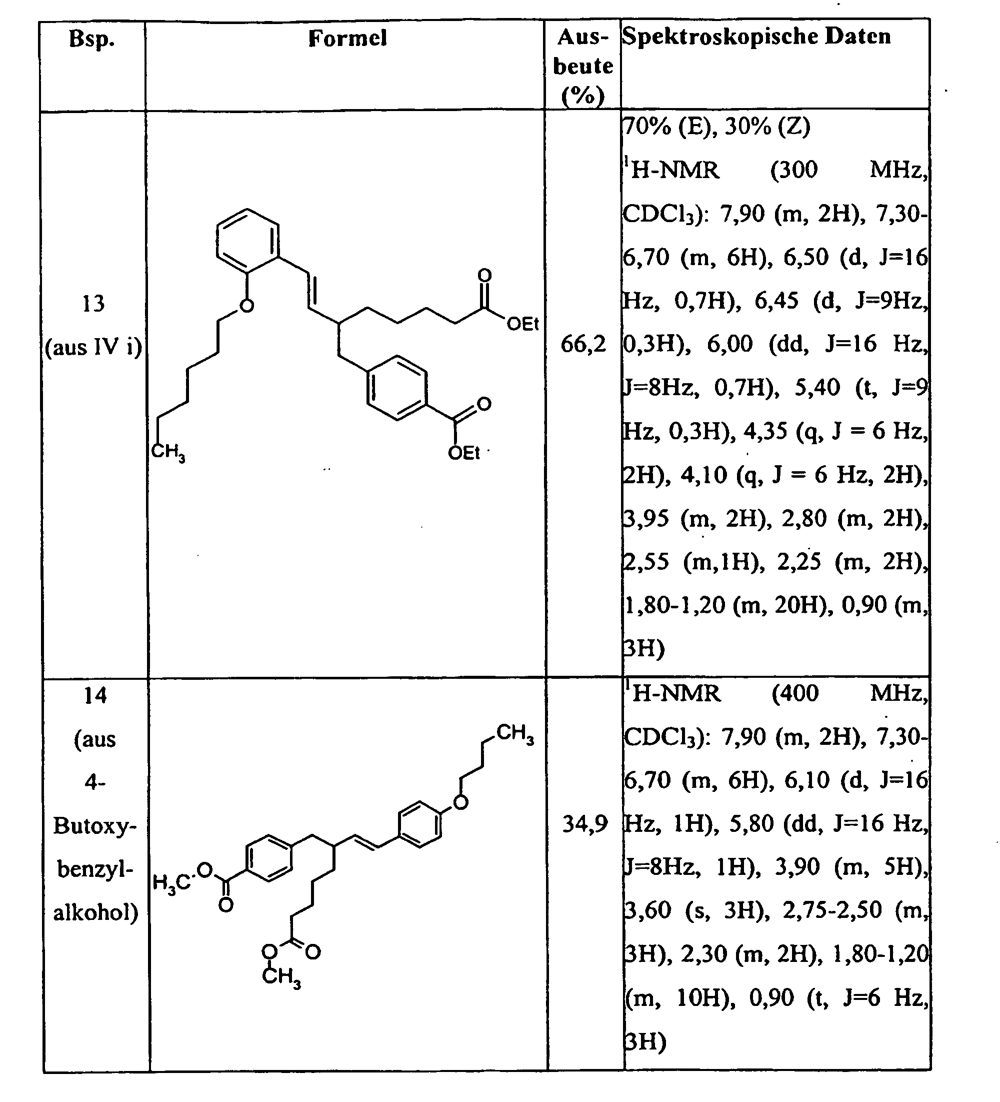

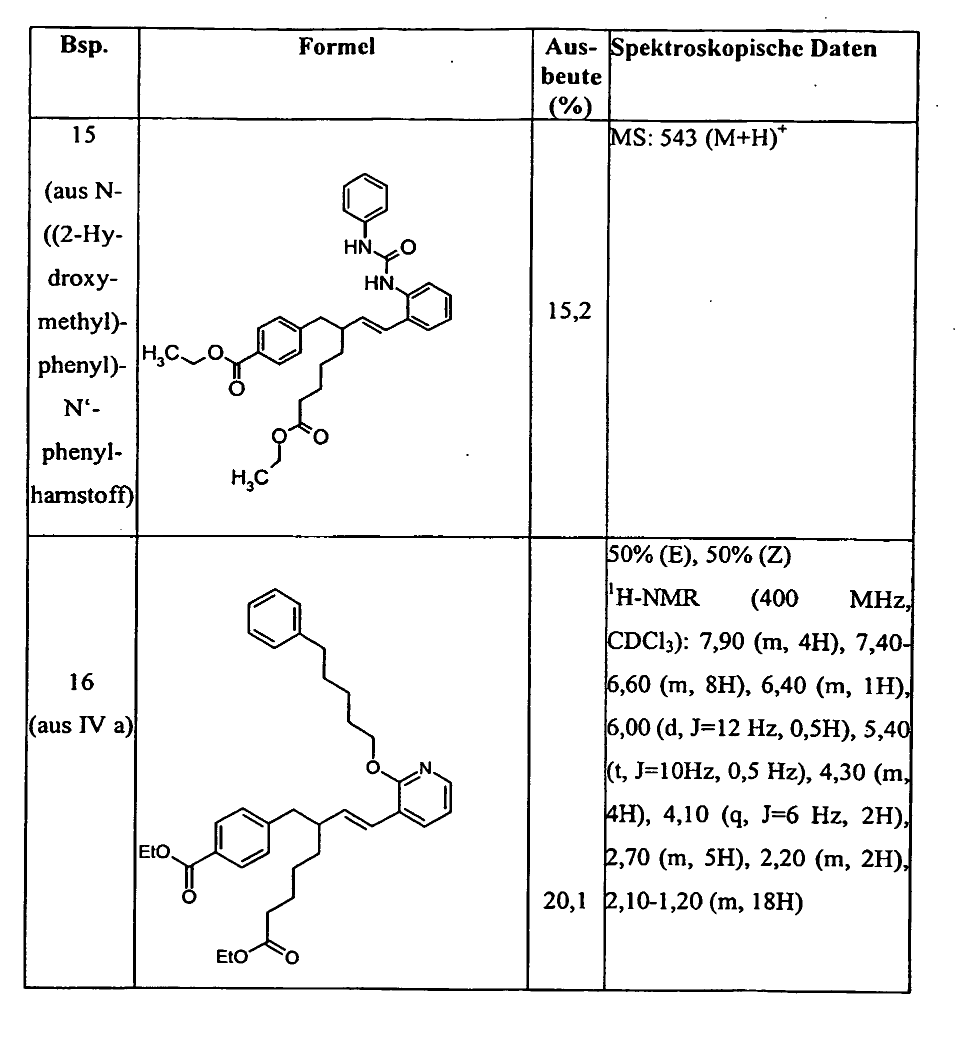

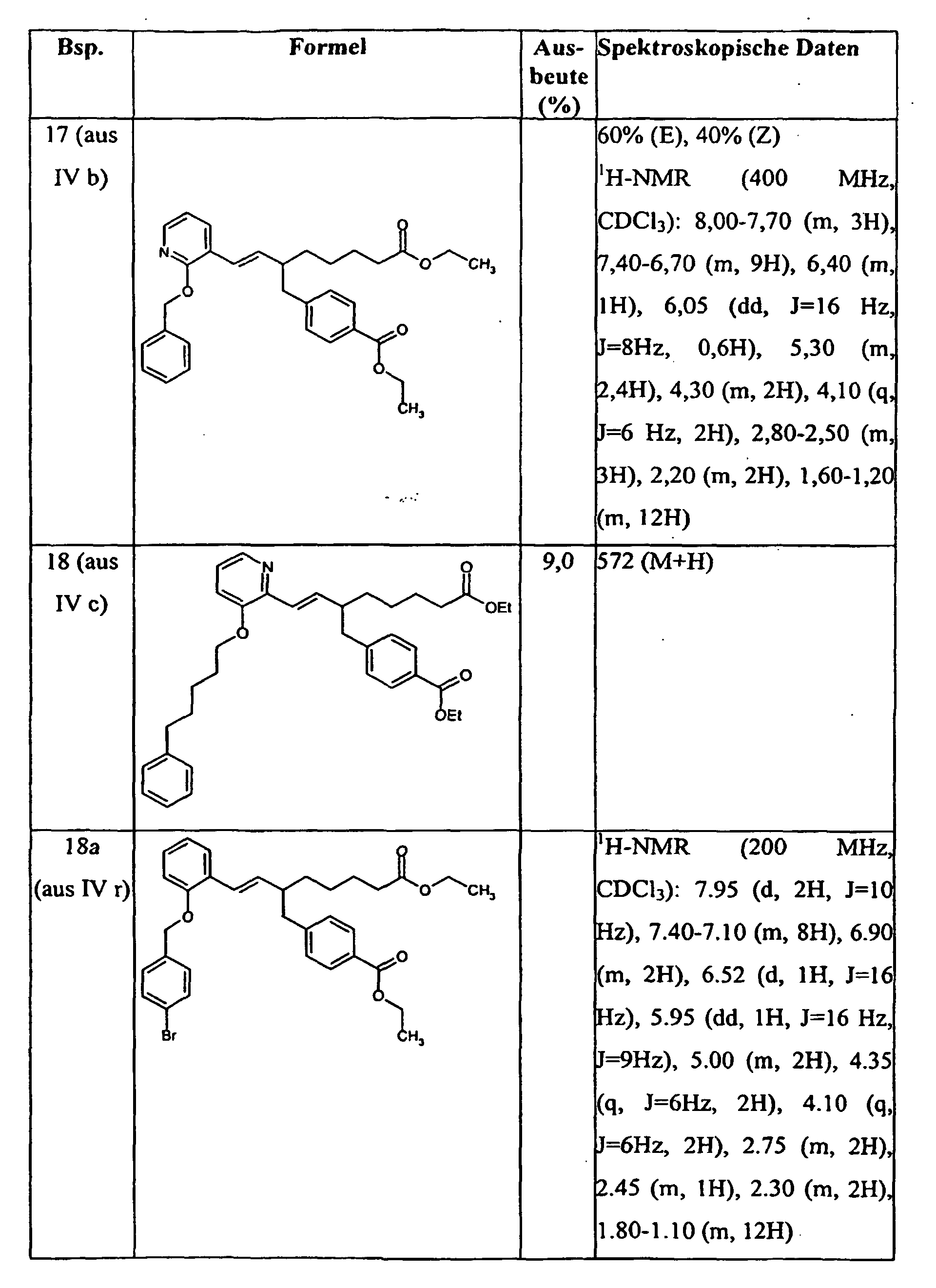

### 19: 6-(4-Carboxybenzyl)-8-(2-methoxyphenyl)oct-7-ensäure

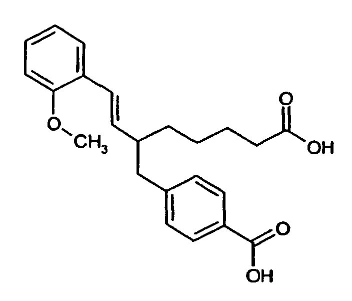

16,0 mg (0,04 mmol) des Diesters aus Beispiel 1 werden in 1 Methanol gelöst und bei 0°C mit 0,5 ml 45%iger Natronlauge versetzt. Bei Raumtemperatur werden 0,2 ml Dichlormethan hinzugegeben. Die Lösung wird 16 Stunden bei Raumtemperatur gerührt, mit etwas Wasser versetzt und mit Ethylether extrahiert. Die wässrige Phase wird mit 10%iger Schwefelsäure auf pH 2-3 gestellt und zweimal mit Essigsäureethylester extrahiert, mit Magnesiumsulfat getrocknet und im Vakuum eingedampft.

Ausbeute: 7,0 mg (47,0 % d.Th.) als eine Mischung: 70,0% Trans / 30,0% Cis.
¹H-NMR (400 MHz, CD₃COCD₃): 7,95 (m, 2H), 7,80-7,10 (m, 6H), 6,60 (d, J=16 Hz, 0,3H), 6,40 (d, J=9Hz, 0,7H), 6,25 (dd, J=16 Hz, J=8Hz, 0,7H), 5,50 (t, J=9 Hz, 0,3H), 3,10-2,50 (m, 3H), 2,30 (m,5H), 1,80-1,20 (m, 6H).

Analog wurden dargestellt:

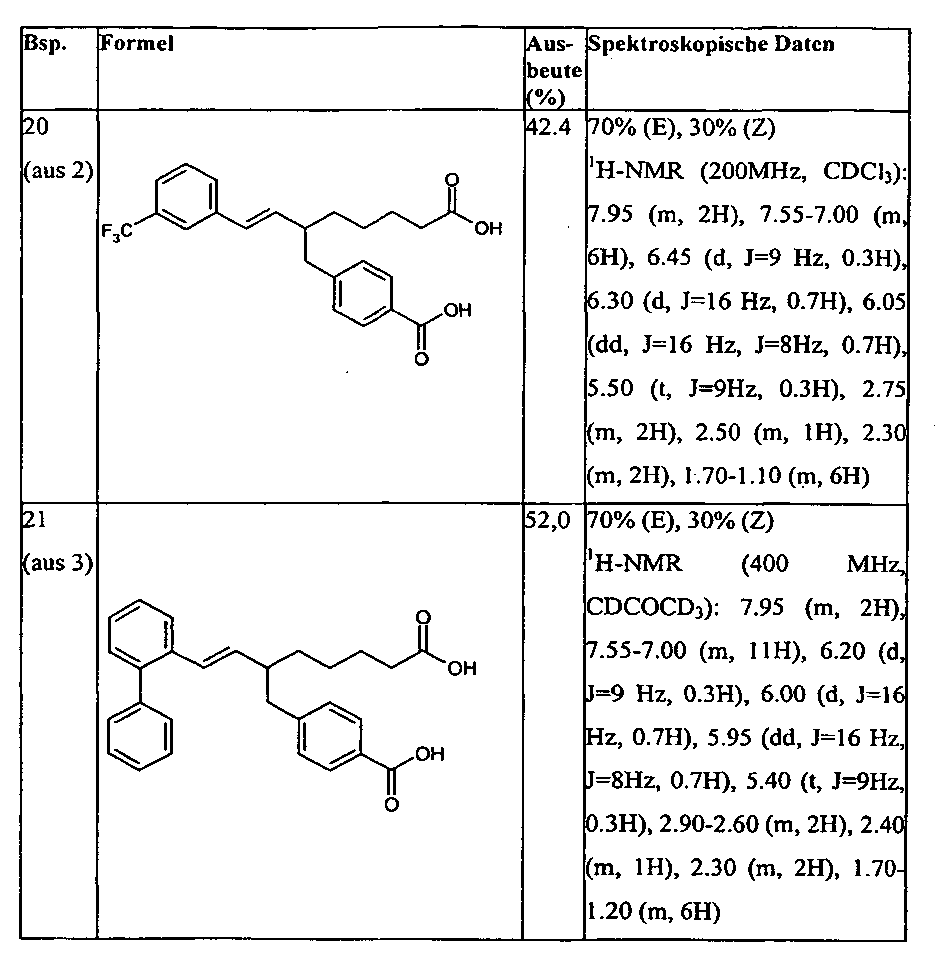

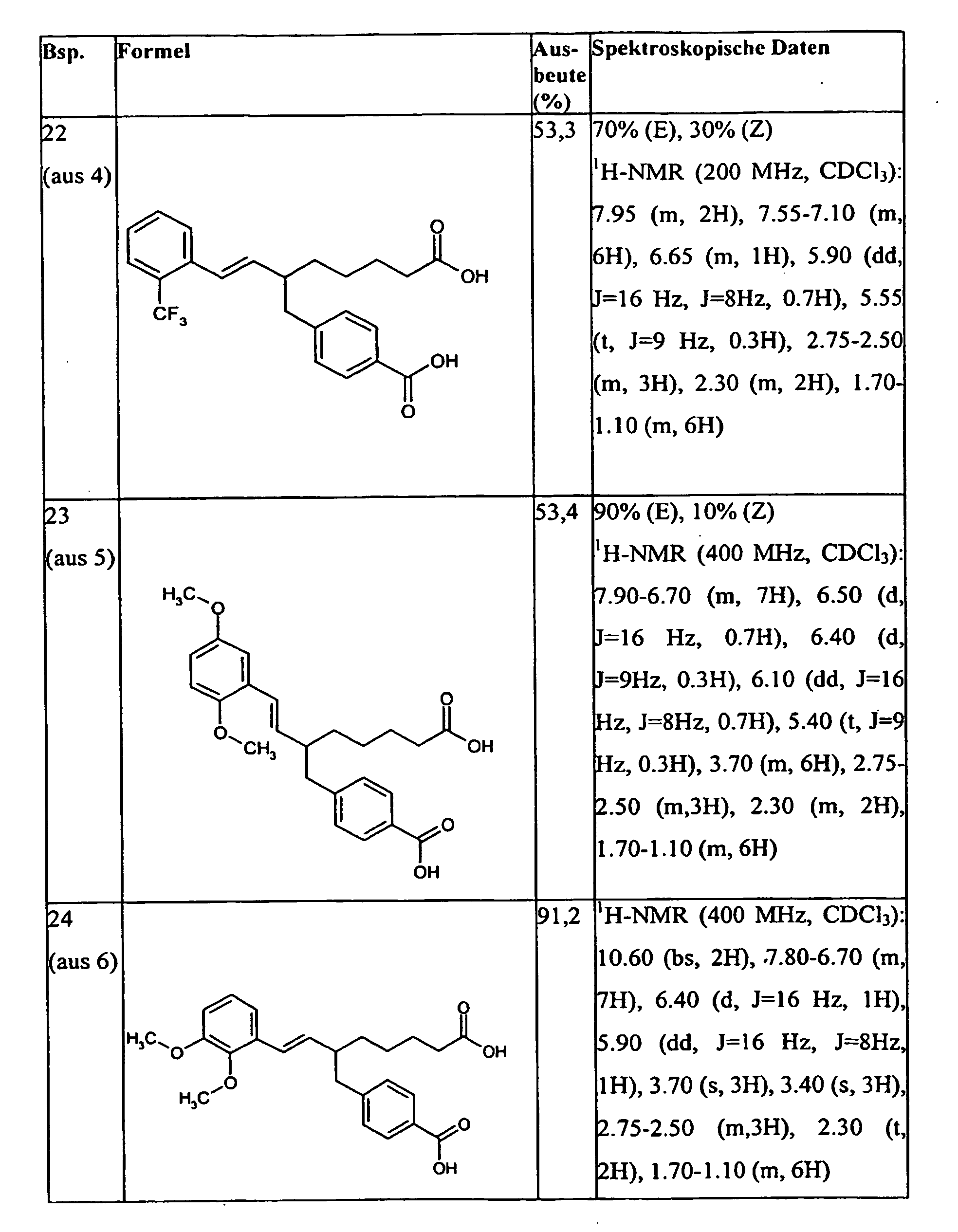

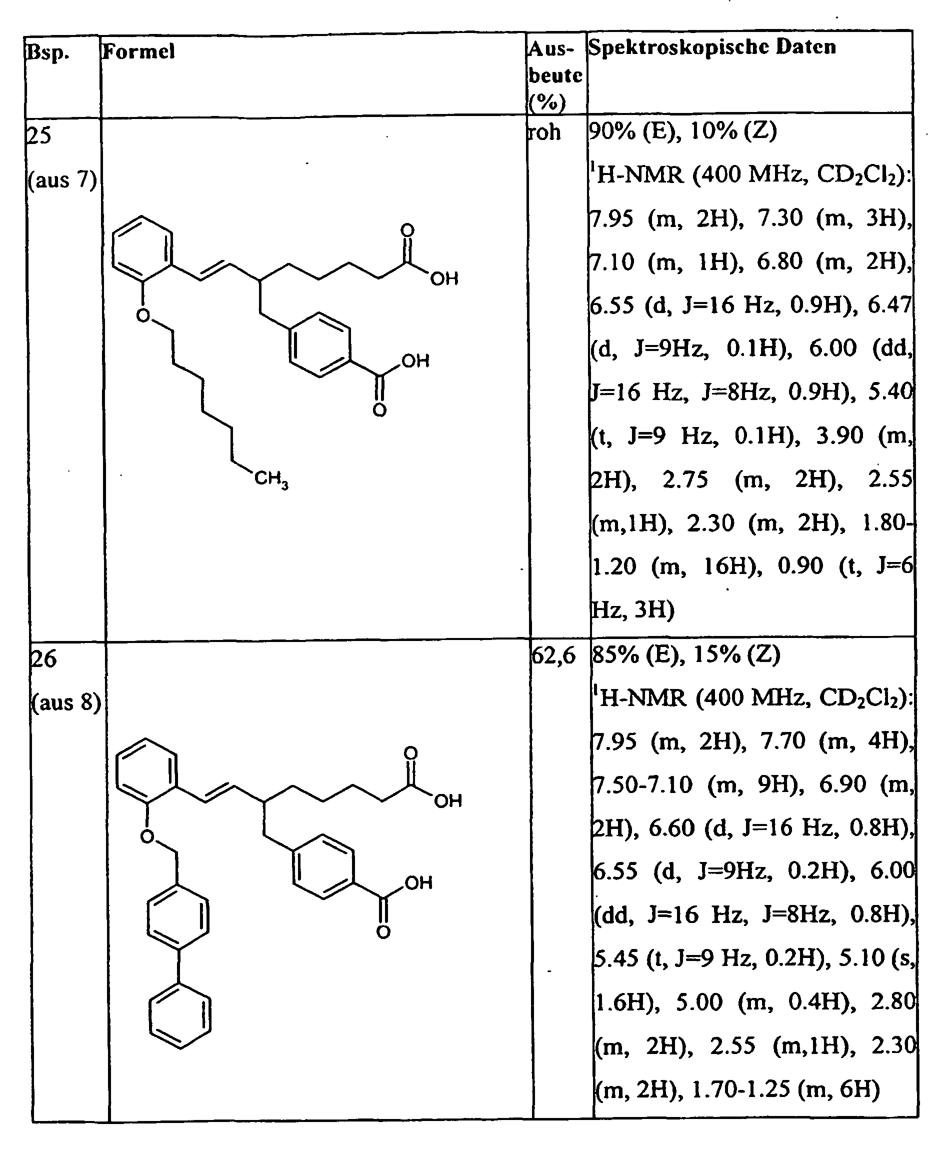

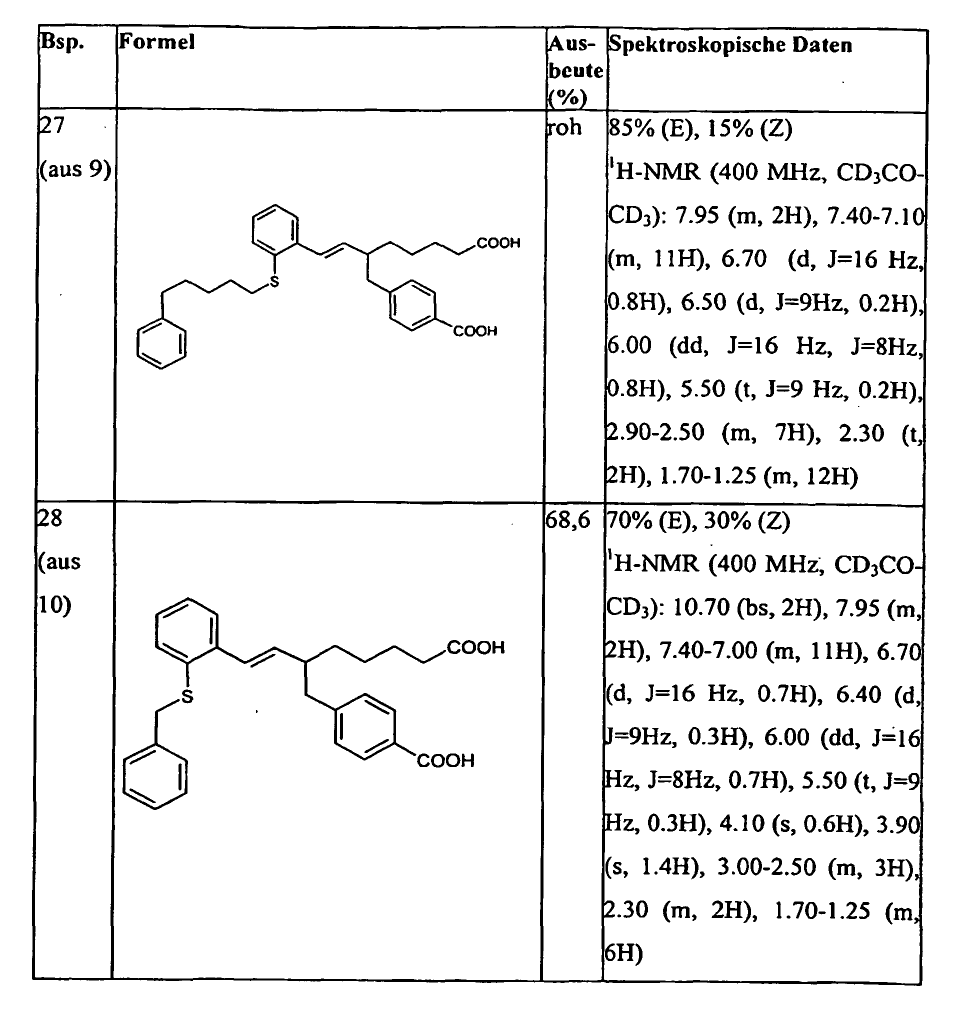

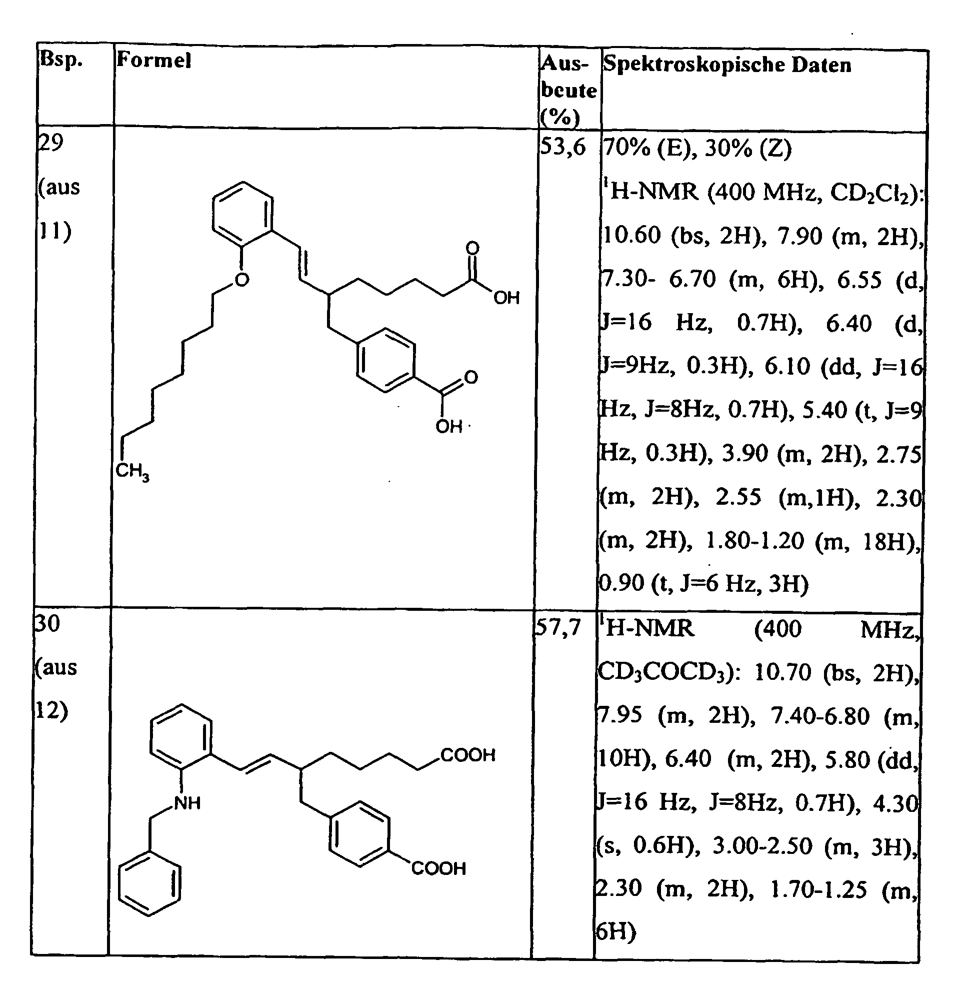

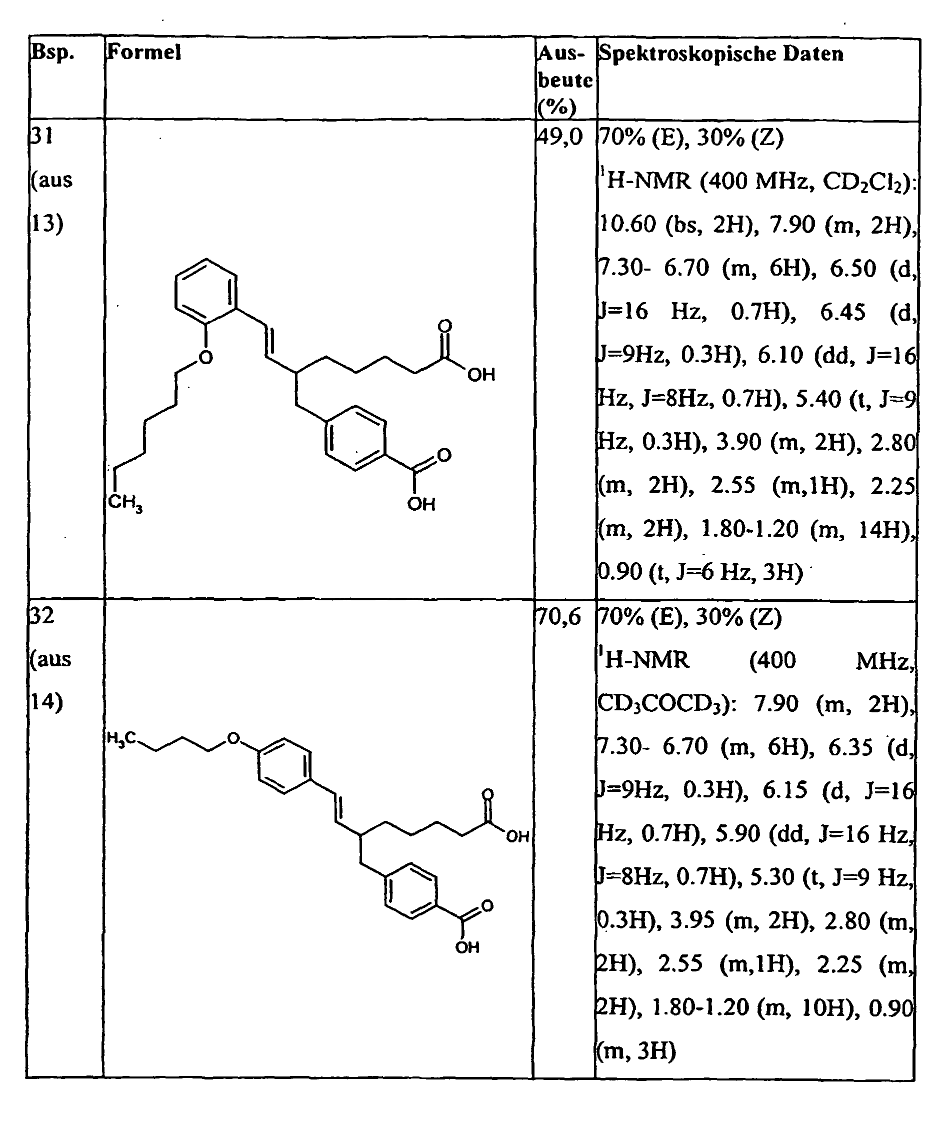

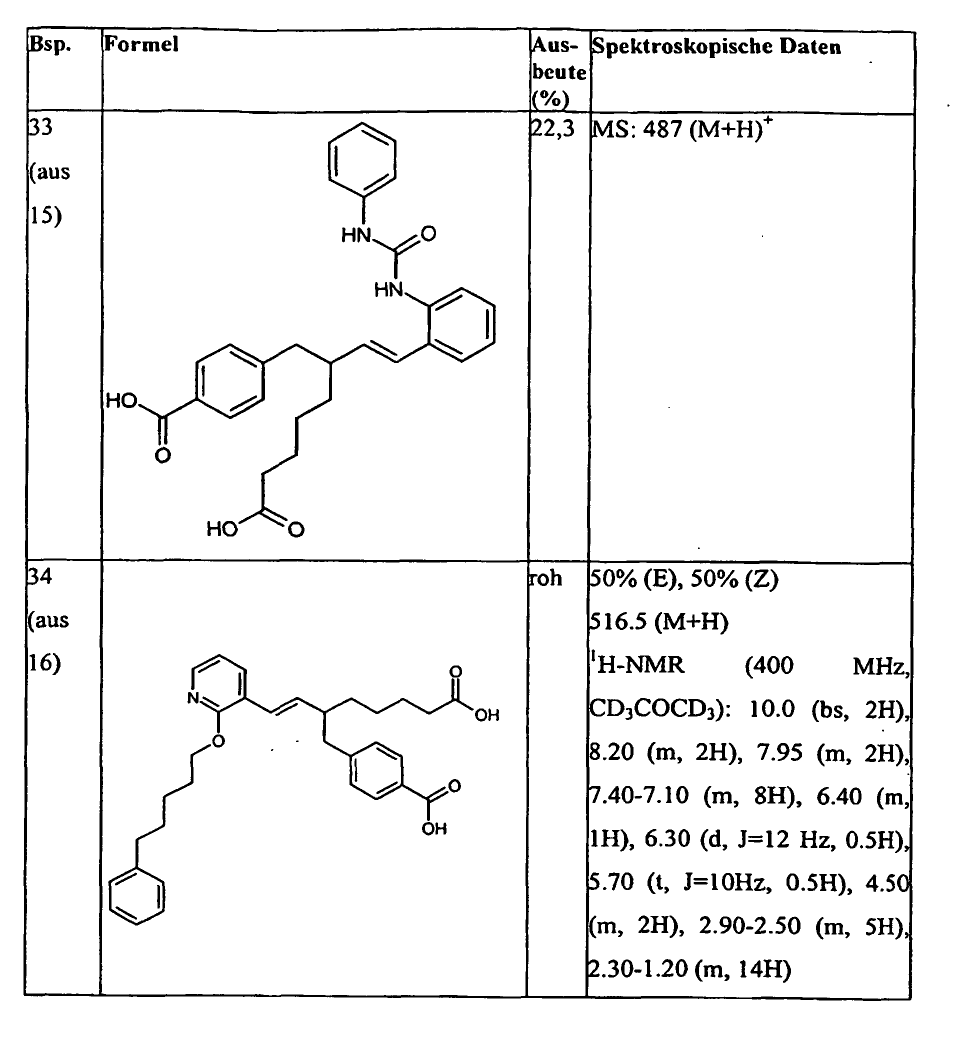

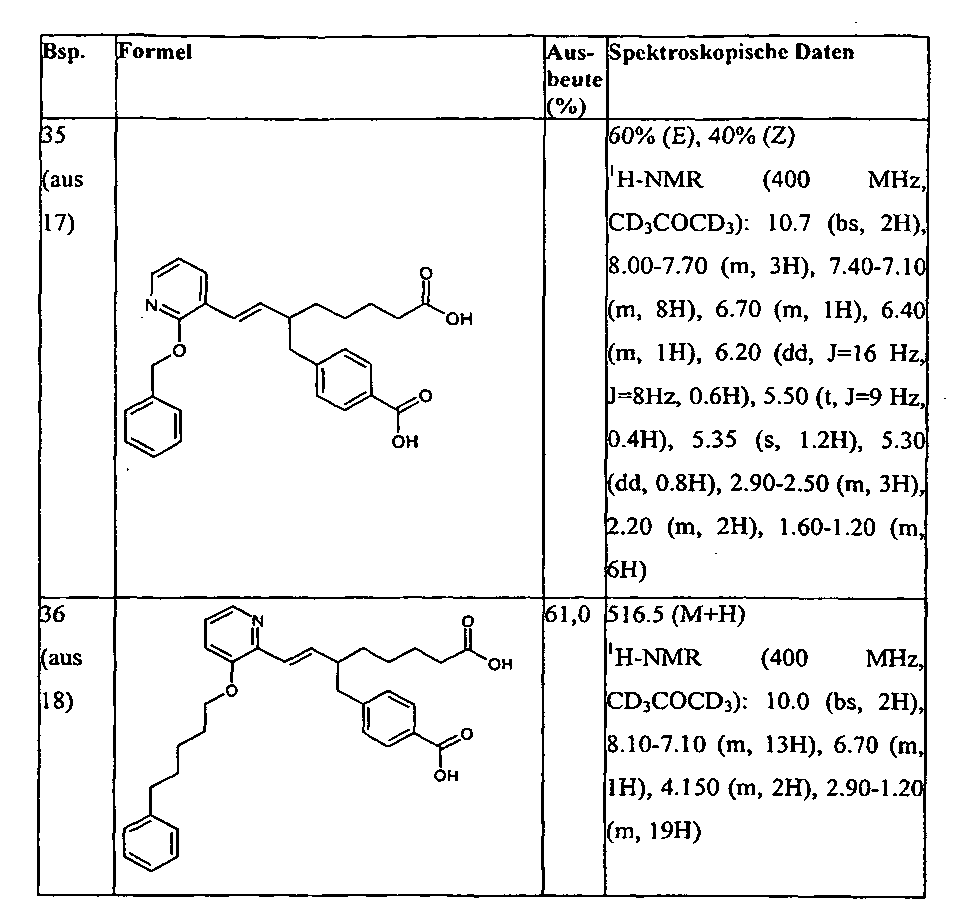

### 37: 6-(4-Carboxybenzyl)-8-(2-phenyloxyphenyl)-7-octensäure

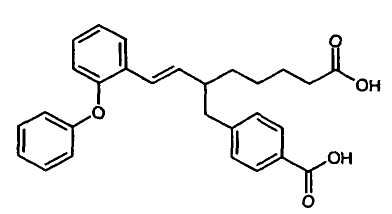

294,5 mg (0,56 mmol) 2-Benzylbenzyltriphenylphosphoniumbromid (hergestellt aus Bsp. IVn) werden bei 0°C unter Argon in 20 ml THF suspendiert und mit 0,42 ml Buthyllithium (0,72 mmol, 1,6M Lösung in Hexan) versetzt. Die tieforange Lösung wird 30 min. bei 0°C gerührt. Eine Lösung von 125 mg (0,37 mmol) 6-Formyl-7-(4-ethoxycarbonylphenyl)heptansäureethylester (vgl. EP-A-0 341 551) in 15 ml THF wird bei dieser Temperatur tropfenweise hinzugegeben. Die Mischung wird 30 min. bei 0°C gerührt. Es wird bei 0°C mit Wasser versetzt, auf Raumtemperatur erwärmt und mit Essigsäureethylester extrahiert. Die organische Phase wird mit Natriumchloridlösung gewaschen, mit Magnesiumsulfat getrocknet und zur Trockne eingedampft. Das Rohprodukt wird in 5 ml Methanol gelöst und bei 0°C mit 1,5 ml 45%iger Natronlauge versetzt. Bei Raumtemperatur werden 0,2 ml Dichlormethan hinzugegeben. Die Lösung wird 16 Stunden bei Raumtemperatur gerührt, mit etwas Wasser versetzt und mit Ethylether extrahiert. Die wässrige Phase wird mit 10%iger Schwefelsäure auf pH 2-3 gestellt und zweimal mit Essigsäureethylester extrahiert, mit Magnesiumsulfat getrocknet und im Vakuum eingedampft.

Ausbeute: 175 mg (roh) als eine Mischung: 70,0 % Trans / 30,0 % Cis.
¹H-NMR (400 MHz, CD₂Cl₂): 9,70 (bs, 2H), 7,95 (m, 2H), 7,70-7,00 (m, 9H), 6,80 (m, 2H), 6,40 (m, 1H), 6,00 (dd, J=16 Hz, J=8Hz, 0,7H), 5,45 (t, J=9 Hz, 0,3H), 3,90 (m, 2H), 2,75 (m, 2H), 2,50-2,20 (m, 3H), 1,80-1,20 (m, 6H)

Analog wurden hergestellt:

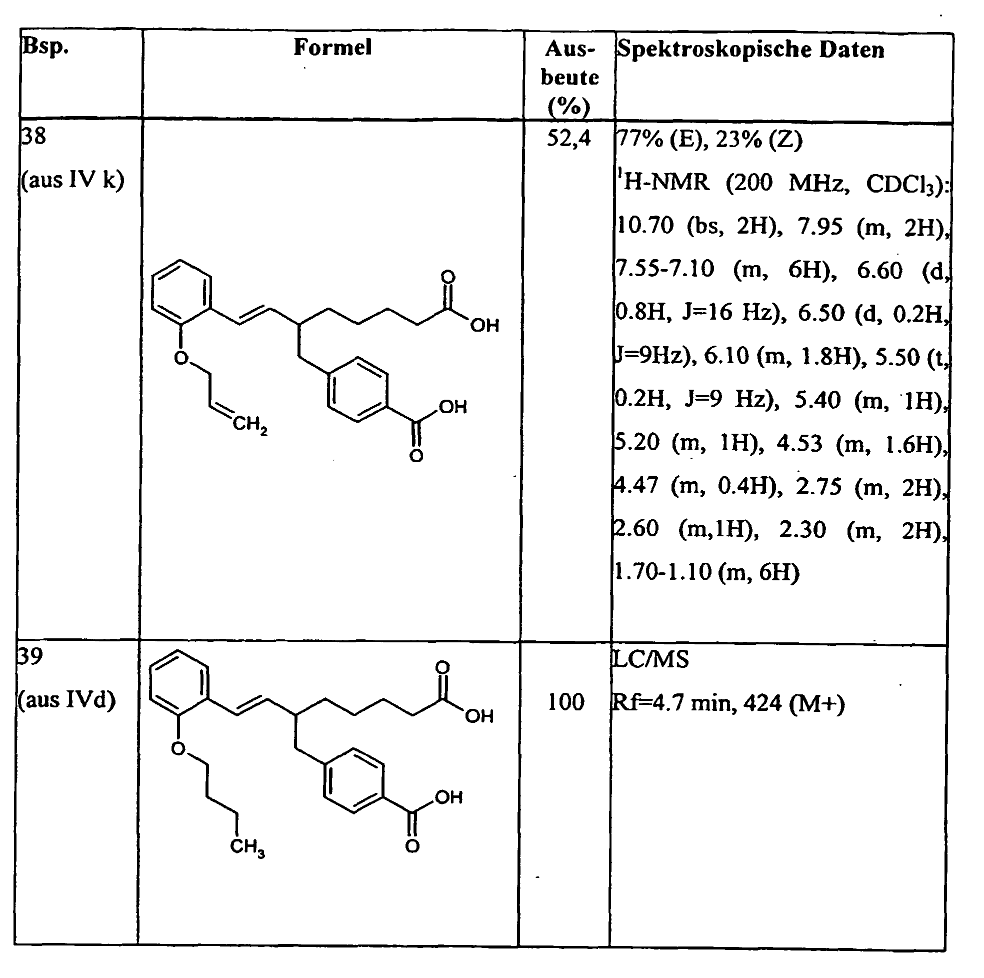

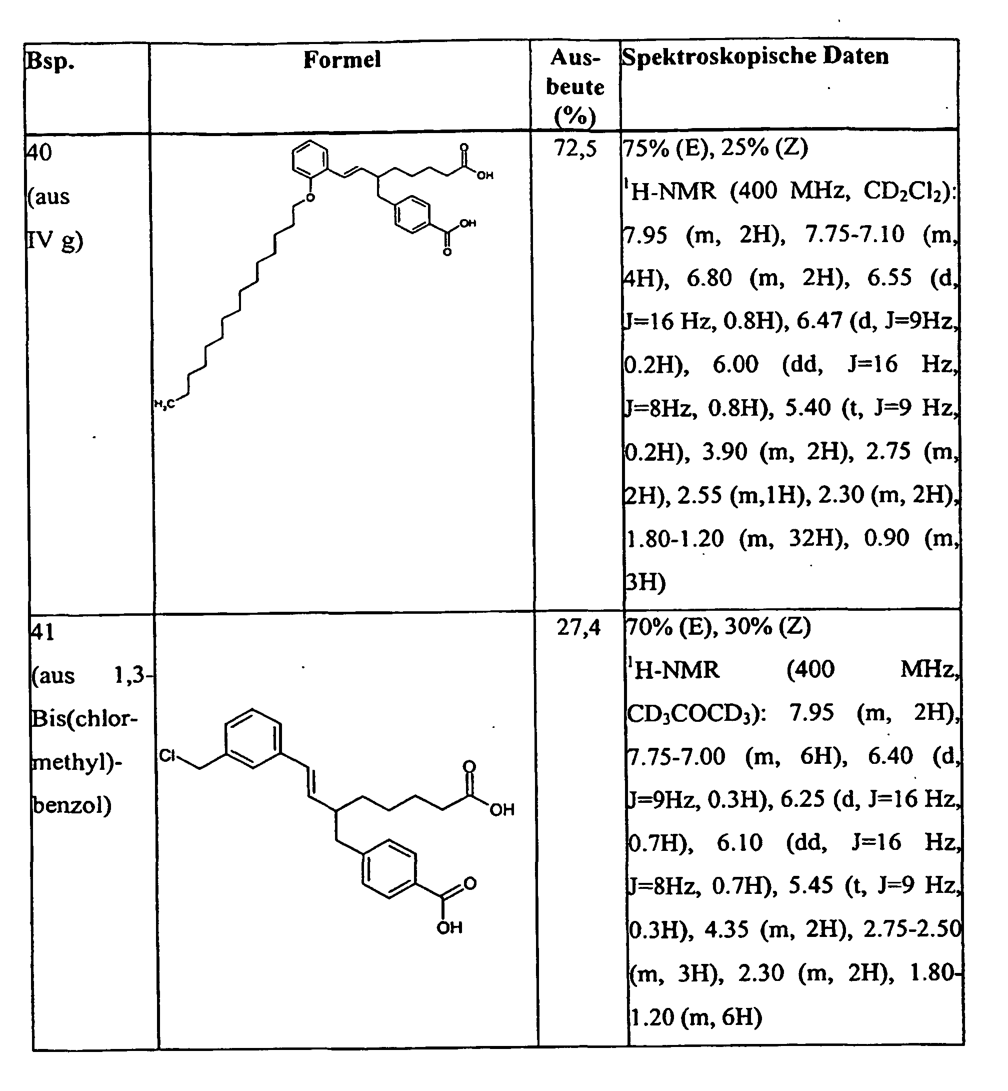

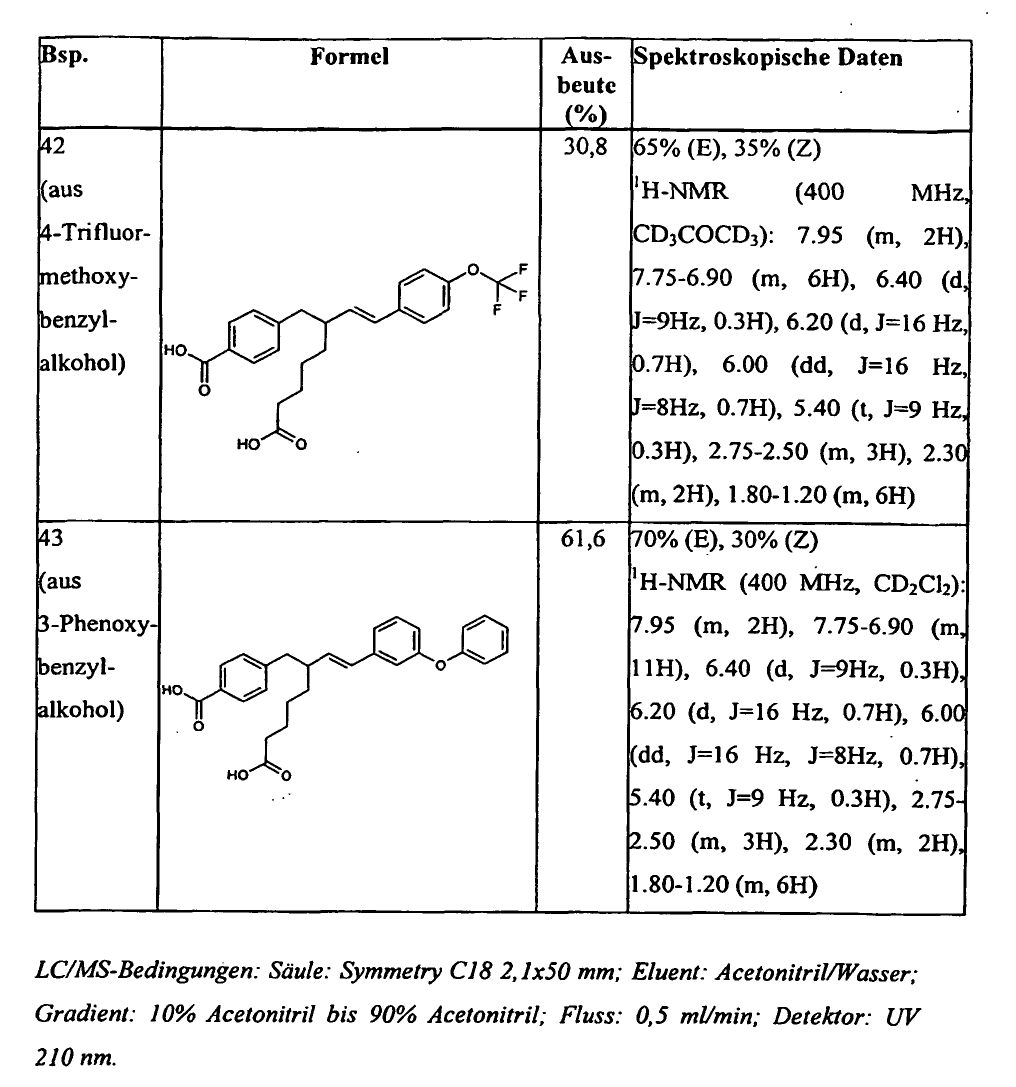

### 44: 6-(4-Ethoxycarbonylbenzyl)-8-(2-hydroxyphenyl)-7(E)-octensäureethylester

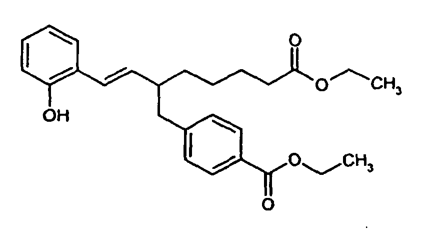

645,2 mg (1,44 mmol) 2-Hydroxybenzyltriphenylphosphoniumbromid werden bei 0°C unter Argon in 25 ml THF suspendiert und mit 2,2 ml Buthyllithium (3,53 mmol, 1,6M Lösung in Hexan) versetzt. Die tieforange Lösung wird 30 min. bei 0°C gerührt. Eine Lösung von 437 mg (1,31 mmol) 6-Formyl-7-(4-ethoxycarbonylphenyl)heptansäureethylester (vgl. EP-A-0 341 551) in 2 ml THF wird bei dieser Temperatur tropfenweise hinzugegeben. Die Mischung wird 30 min. bei 0°C gerührt. Es wird bei 0°C mit Wasser und Dichlormethan versetzt, auf Raumtemperatur erwärmt und mit Salzsäure auf pH 2 eingestellt. Die Mischung wird über Extrelut filtriert und im Vakuum eingedampft. Das rohe Material wird chromatographiert.

Ausbeute 184 mg (33,2 % d. Th.)
¹H-NMR (200 MHz, CDCl₃): 7,95 (d, 2H, J=10 Hz), 7,25 (d, 2H), 7,10 (m, 2H); 6,80 (m, 2H), 6,40 (d, 1H,1=16 Hz), 5,85 (dd, 1H, J=16 Hz, J=9Hz), 5,10 (s, 1H), 4,35 (q, J=6Hz, 2H), 4,10 (m, 2H), 2,75 (m, 2H), 2,50 (m, 1H), 2,30 (m, 2H), 1,80-1,10 (m, 12H).

### 45: 6-(4-Carboxybenzyl)-8-(2-hydroxyphenyl)-7(E)-octensäure

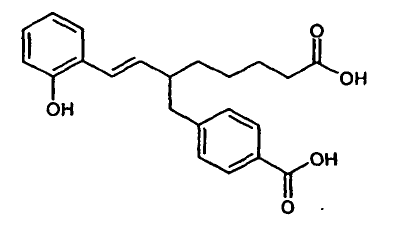

Der Diester aus Bsp. 44 wird in der 50-fachen Menge Methanol gelöst und bei 0°C mit der 12-fachen Menge Natronlauge tropfenweise versetzt. Man lässt auf Raumtemperatur kommen und gibt soviel Methylenchlorid (ca. 0,2 ml) zu, dass die Lösung klar wird. Nach fünf Stunden wird mit etwas Wasser versetzt, mit Ether überschichtet, abgetrennt, die waessrige Phase mit 10%iger Schwefelsäure auf pH 2-3 gestellt, zweimal mit Essigester extrahiert, getrocknet und einrotiert.

¹H-NMR (200 MHz, CDCl₃): 7,90 (d, 2H), 7,75-7,30 (m,4H) 6,80 (m, 2H), 6,55 (d, 1H, J=16 Hz), 6,10 (dd, 1H, J=16 Hz, J=9Hz), 4,70 (s, 1H), 2,75 (m, 2H), 2,50 (m, 1H), 2,30 (m, 1H), 1,80-1,10 (m, 6H).

### 46: 6-(4-Ethoxycarbonylbenzyl)-8-(2-hydroxyphenyl)octansäureethylester

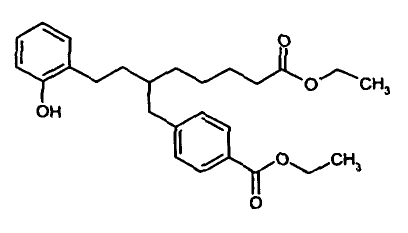

510,2 mg (1,44 mmol) 6-(4-Ethoxycarbonylbenzyl-8-(2-hydroxyphenyl)-7(E)-octensäureethylester aus Bsp. 44 und 250 mg Palladium/A-Kohle 10%ig werden in 20 ml Essigsäureethylester gegeben und bei Raumtemperatur unter Normaldruck mit Wasserstoff hydriert. Nach fünf Stunden wird die Mischung über Celite filtriert und im Vakuum eingedampft.

Ausbeute 507,9 mg (99,1% d. Th.)
¹H-NMR (400 MHz, CDCl₃): 7,95 (d, 2H, J=10 Hz), 7,20 (d, 2H), 7,00 (m, 2H), 6,80 (m, 2H), 4,90 (s, 1H), 4,35 (q, J=6Hz, 2H), 4,10 (m, 2H), 2,65 (m, 4H), 2,30 (m, 2H), 1,80-1,10 (m, 15H)

### 47: 6-(4-Ethoxycarbonylbenzyl)-8-(2-((2-phenyl)-benzyloxy)phenyl)-7(E)-octensäureethylester

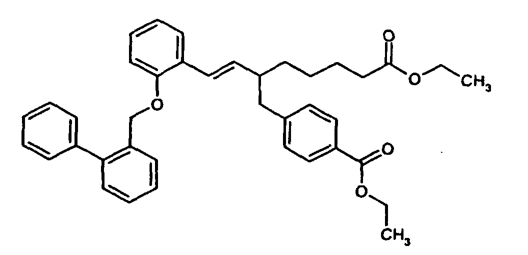

97 mg (0,23 mmol) des Phenols aus Beispiel 44, 67,9 mg (0,27 mmol) 2-Phenylbenzylbromid und 47,5 mg (0,34 mmol) Kaliumcarbonat werden in 5 ml Acetonitril zusammengegeben und unter Rückfluss erhitzt. Die Mischung wird abgekühlt, filtriert, im Vakuum eingedampft und chromatographiert.

Ausbeute: 79 mg (58,4 % d. Th.)
¹H-NMR (400 MHz, CD₂Cl₂): 7,90 (d, 2H), 7,50-6,70 (m, 15H), 6,55 (d, J=16 Hz, 1H), 6,00 (dd, J=16 Hz, J=8Hz, 1H), 4,90 (s, 2H), 4,35 (q, J=6 Hz, 2H), 4,05 (q, J=6 Hz, 2H), 2,75 (m, 2H), 2,50 (m,1H), 2,30 (m, 2H), 1,70-1,20 (m, 12H).

Analog wurden die folgenden Verbindungen synthetisiert:

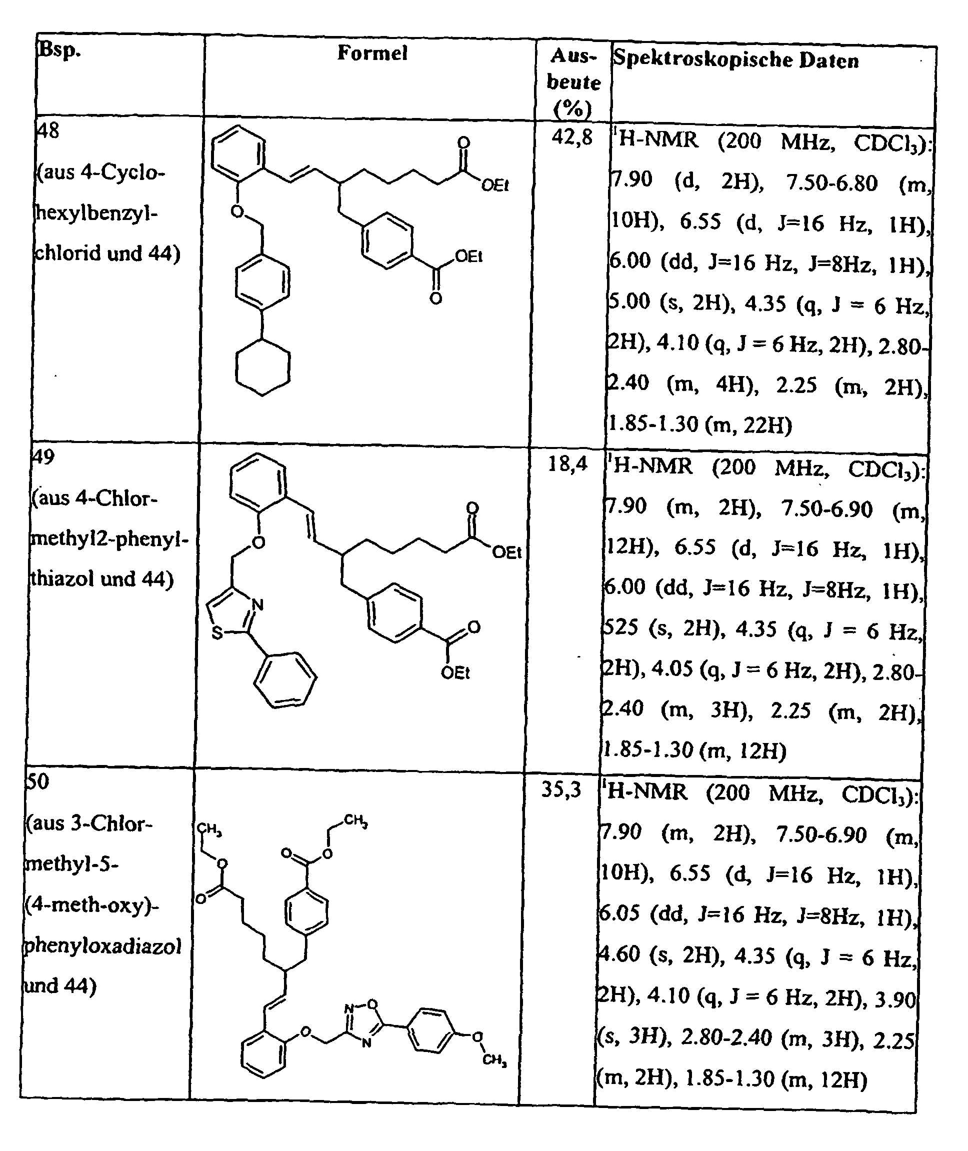

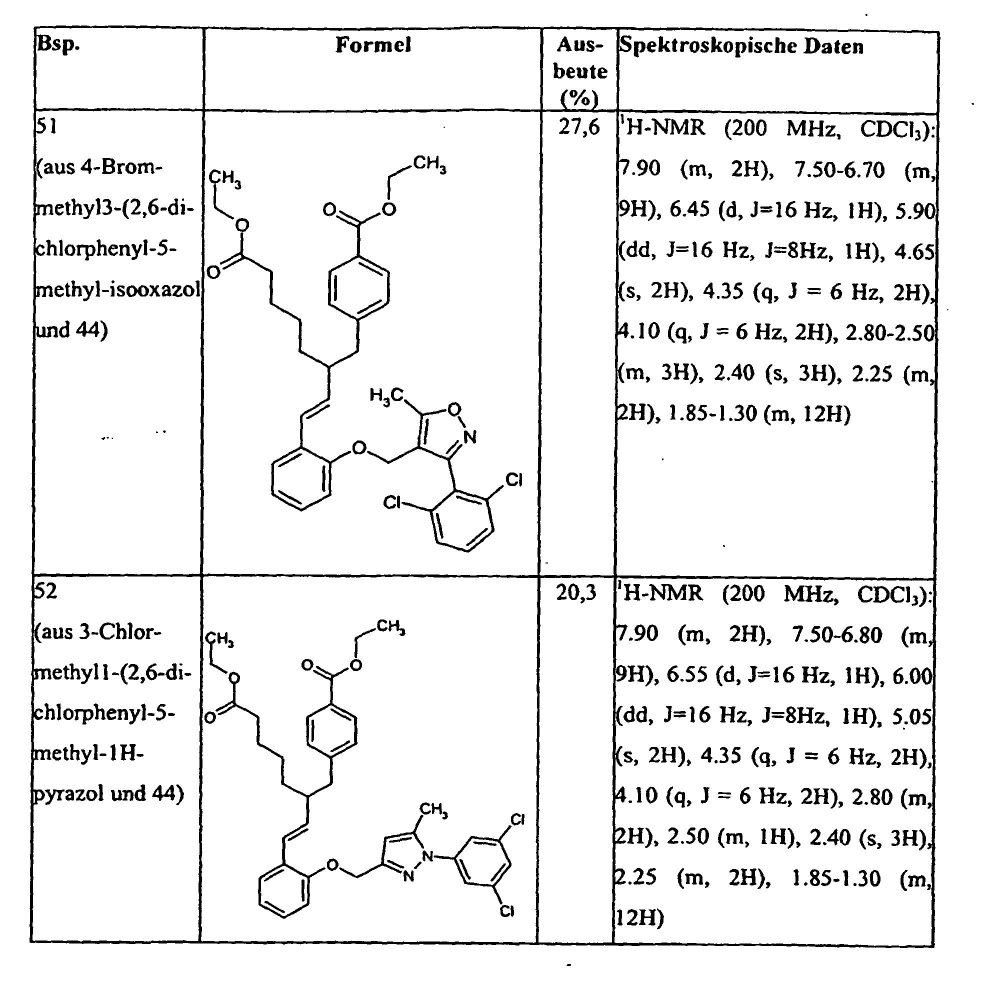

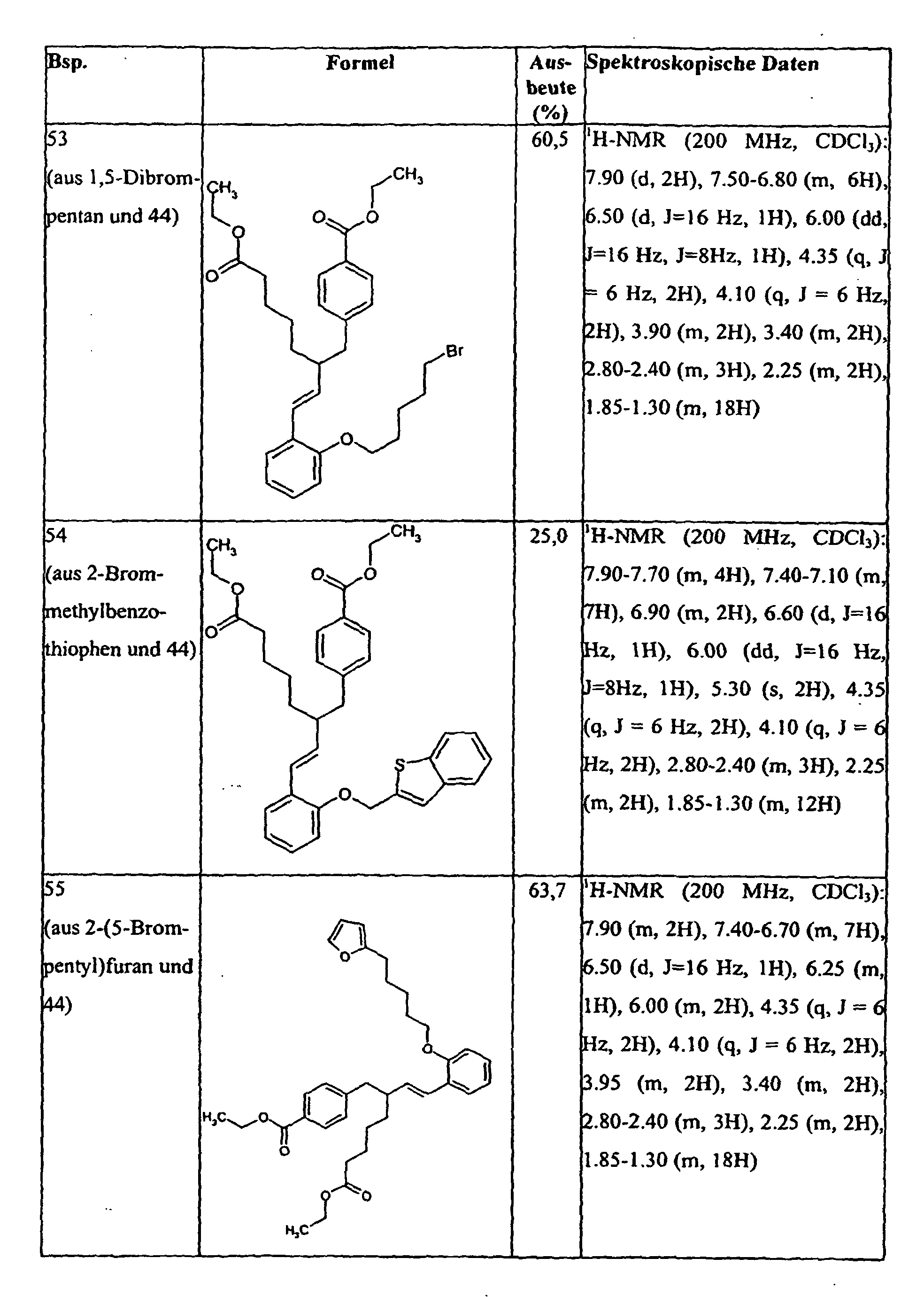

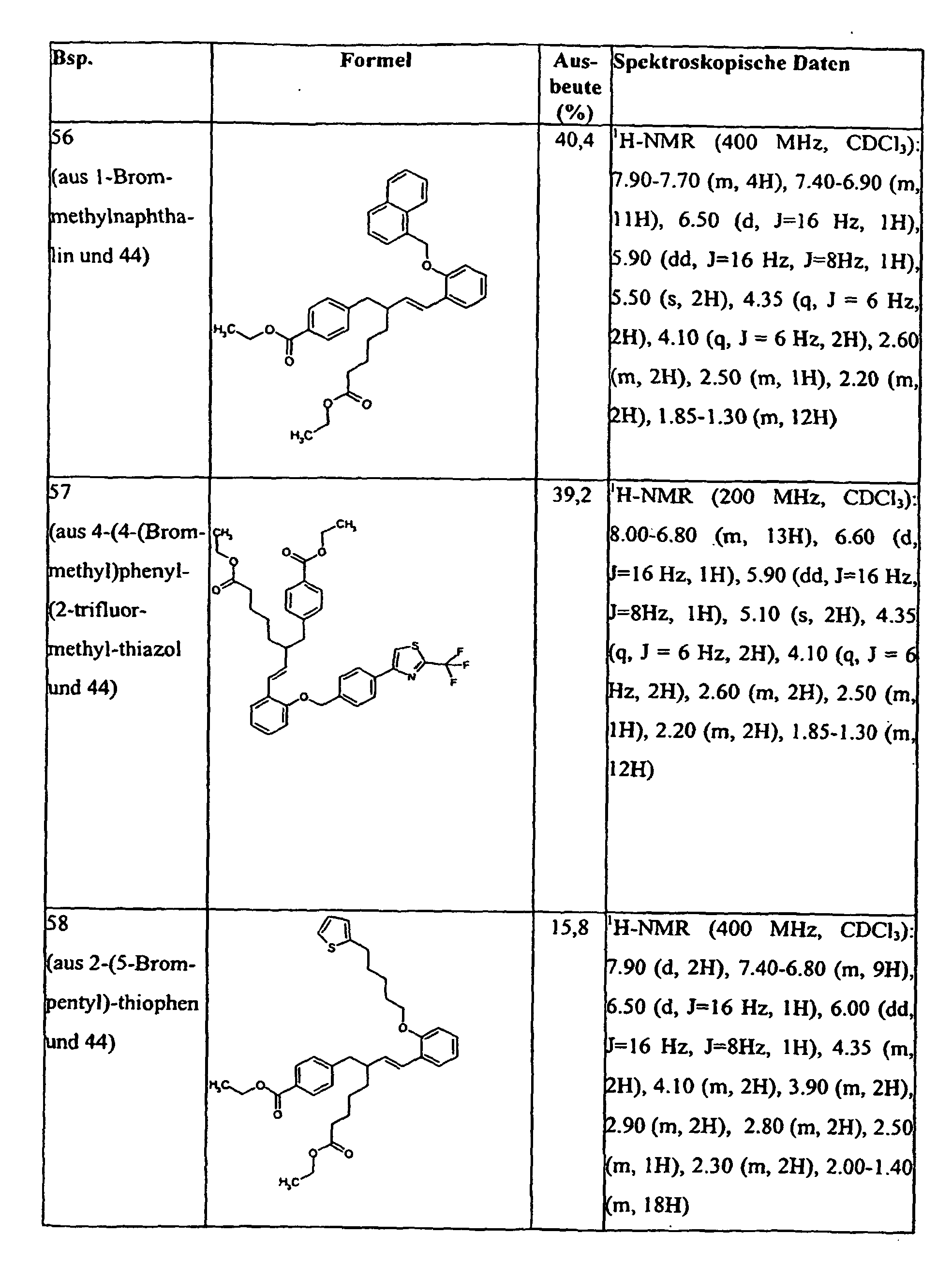

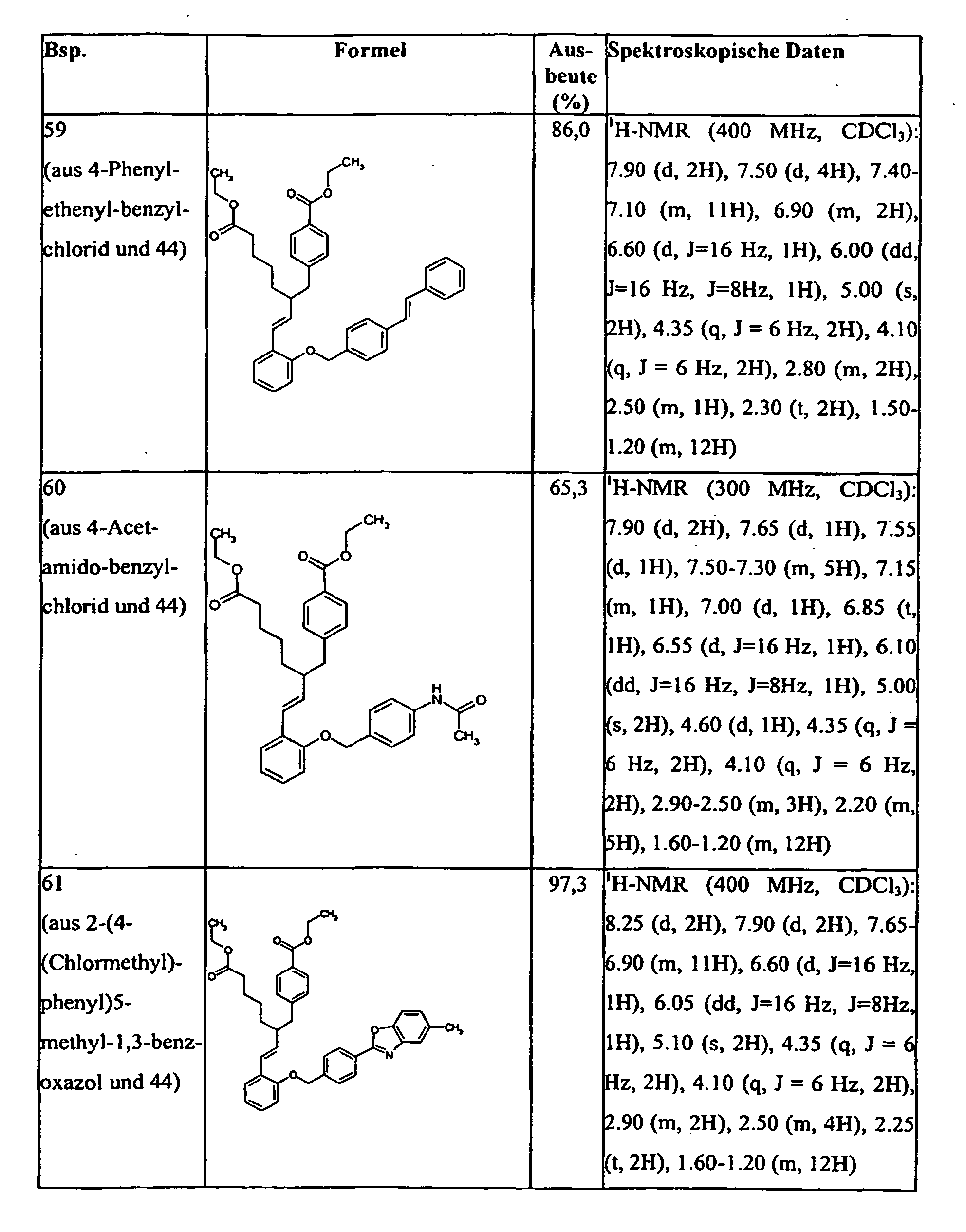

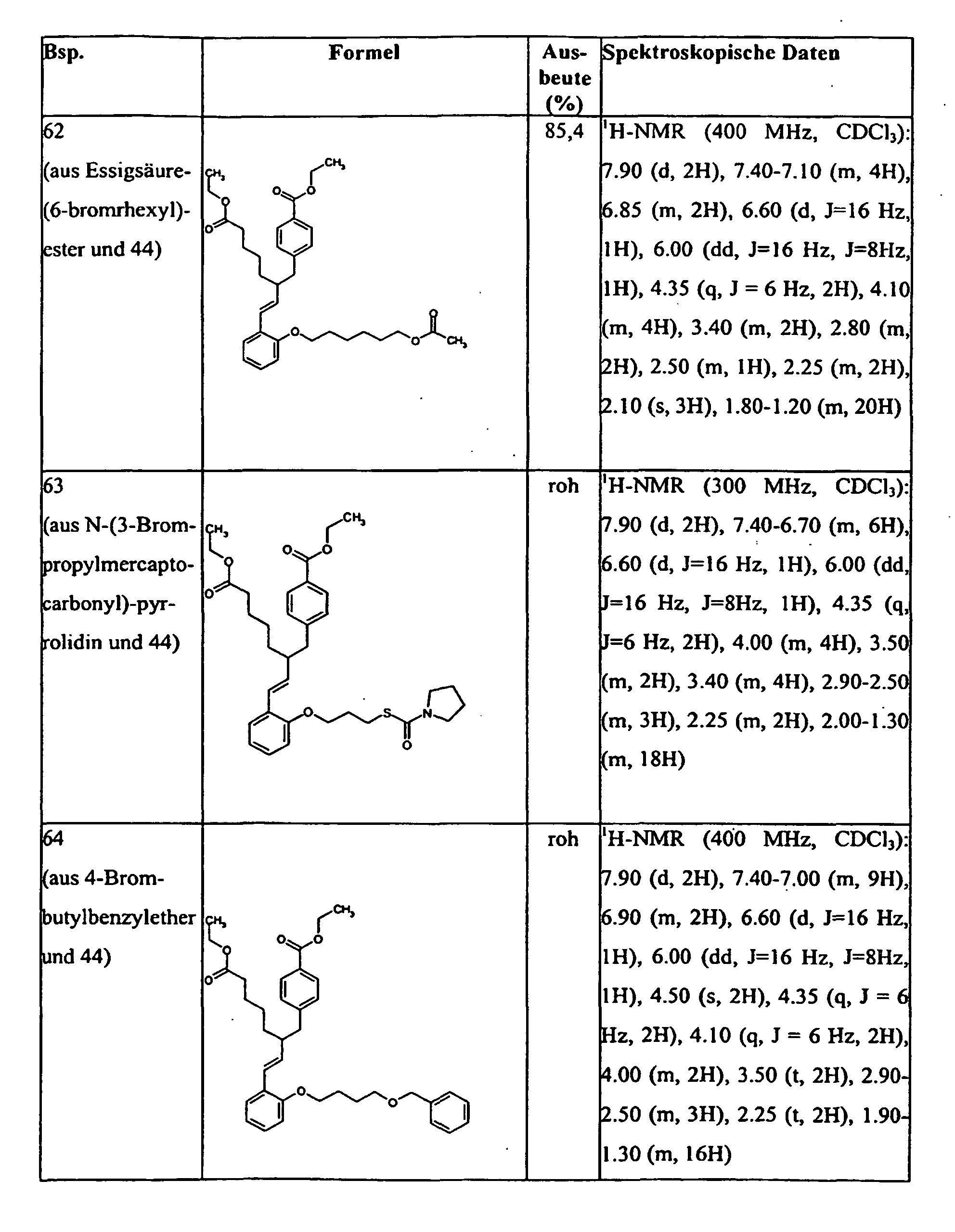

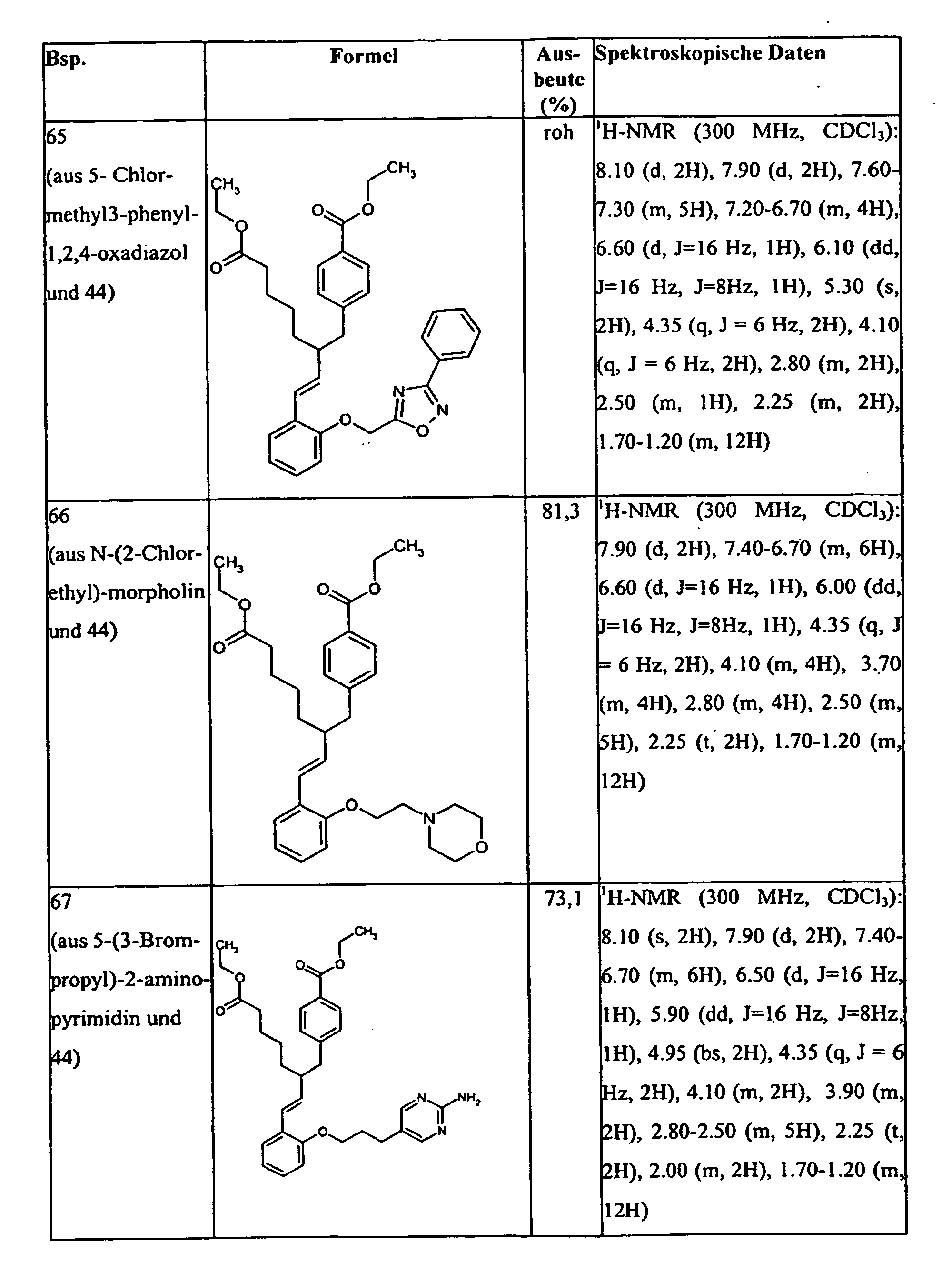

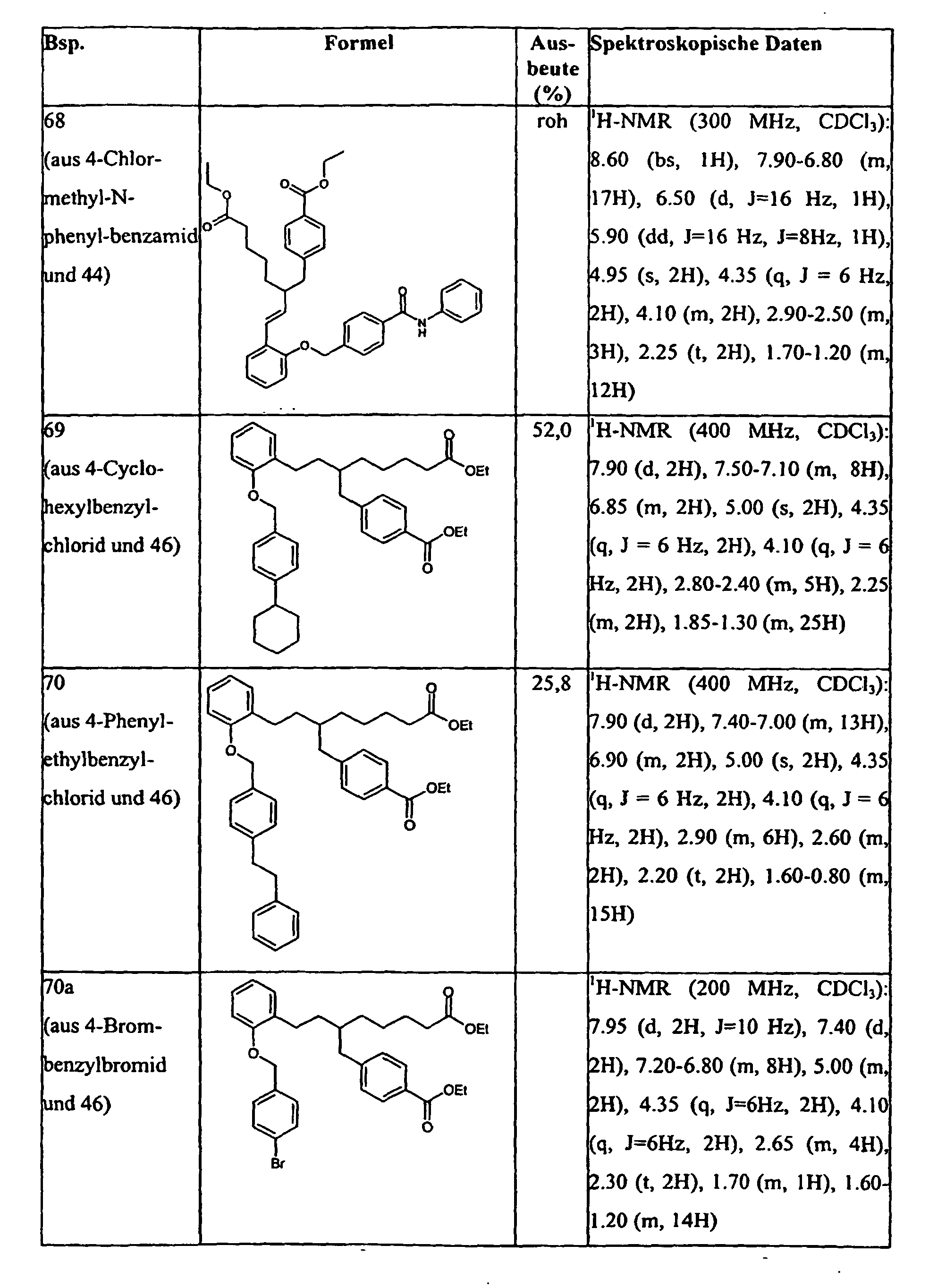

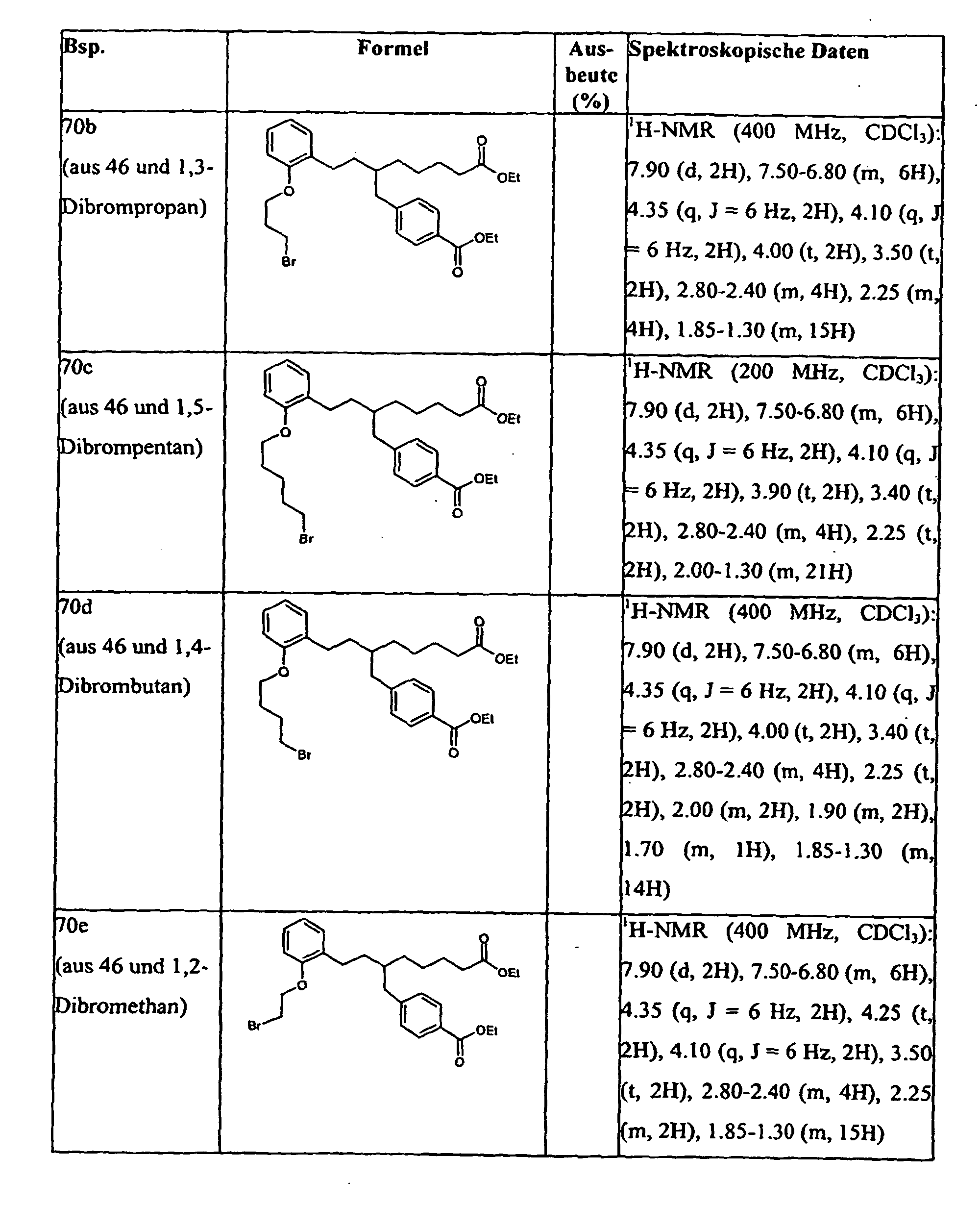

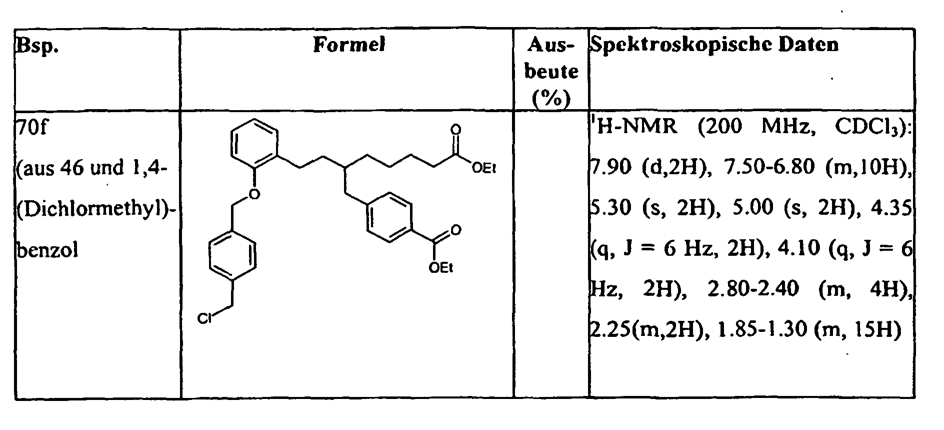

### 71: 6-(4-Carboxybenzyl-8-(2-(2-phenylbenzyloxy)phenyl)-7(E)-octensäure

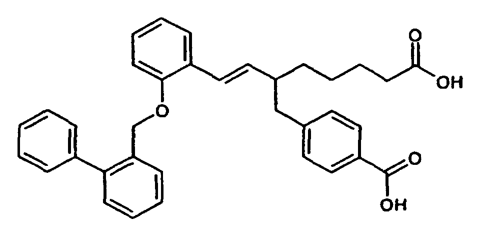

70 mg (0,12 mmol) des Diethylesters aus Bsp. 47 werden in 5 ml Methanol gelöst und mit 0,5 ml 45%iger Natronlauge versetzt. Die Reaktion ist exotherm. Man lässt auf Raumtemperatur kommen und gibt 0,3 ml Dichlormethan hinzu. Nach 20 Stunden bei Raumtemperatur wird die Reaktionslösung einmal mit Ether gewaschen, mit 10 %iger Schwefelsäure sauer gestellt, zweimal mit Essigester extrahiert, die vereinigten organischen Phasen über Extrelut filtriert und eingeengt.
Ausbeute: 15 mg (20,0% d. Th.)
LC/MS: Rf: 5,1 min, 535 (M+1)

Analog werden die folgenden Substanzen synthetisiert:

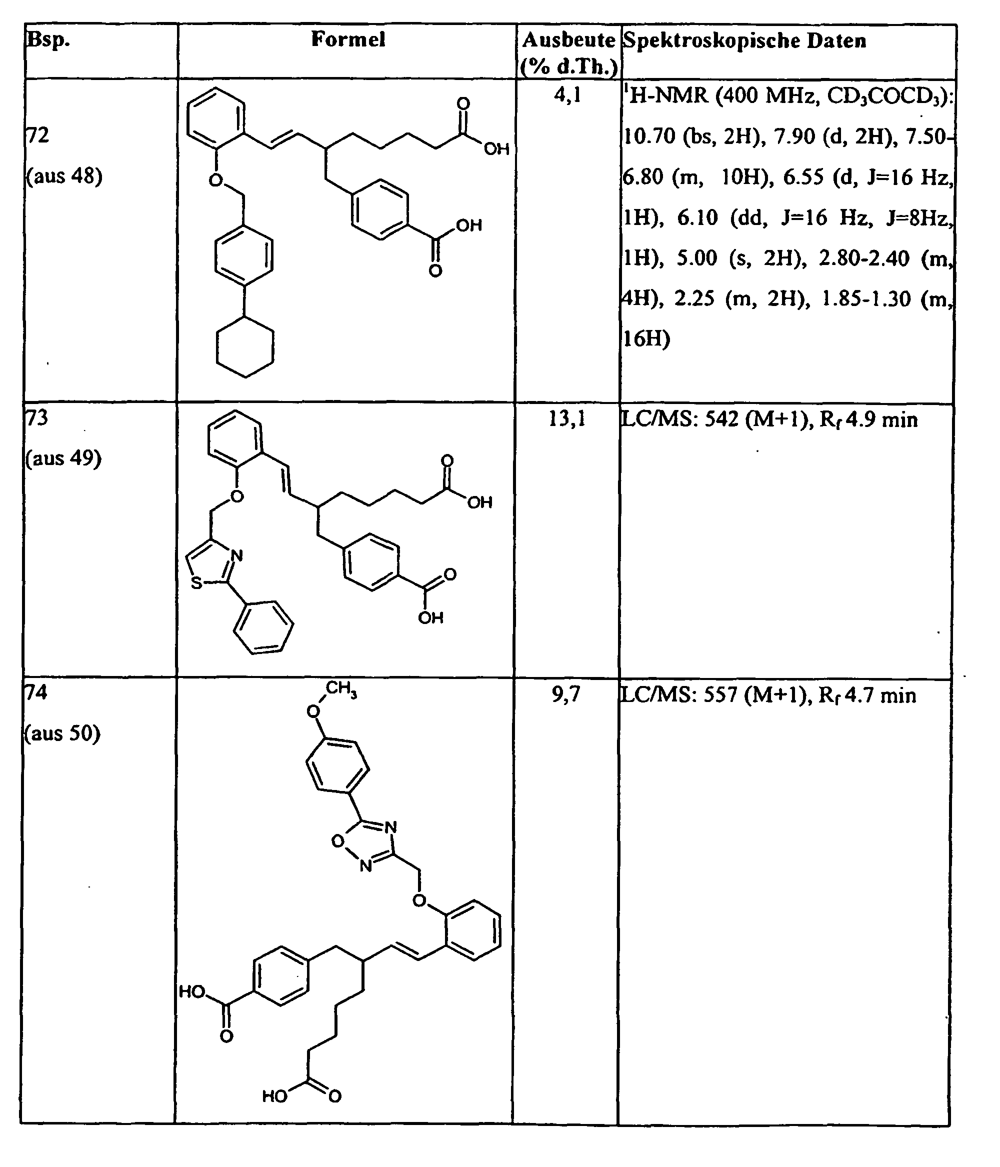

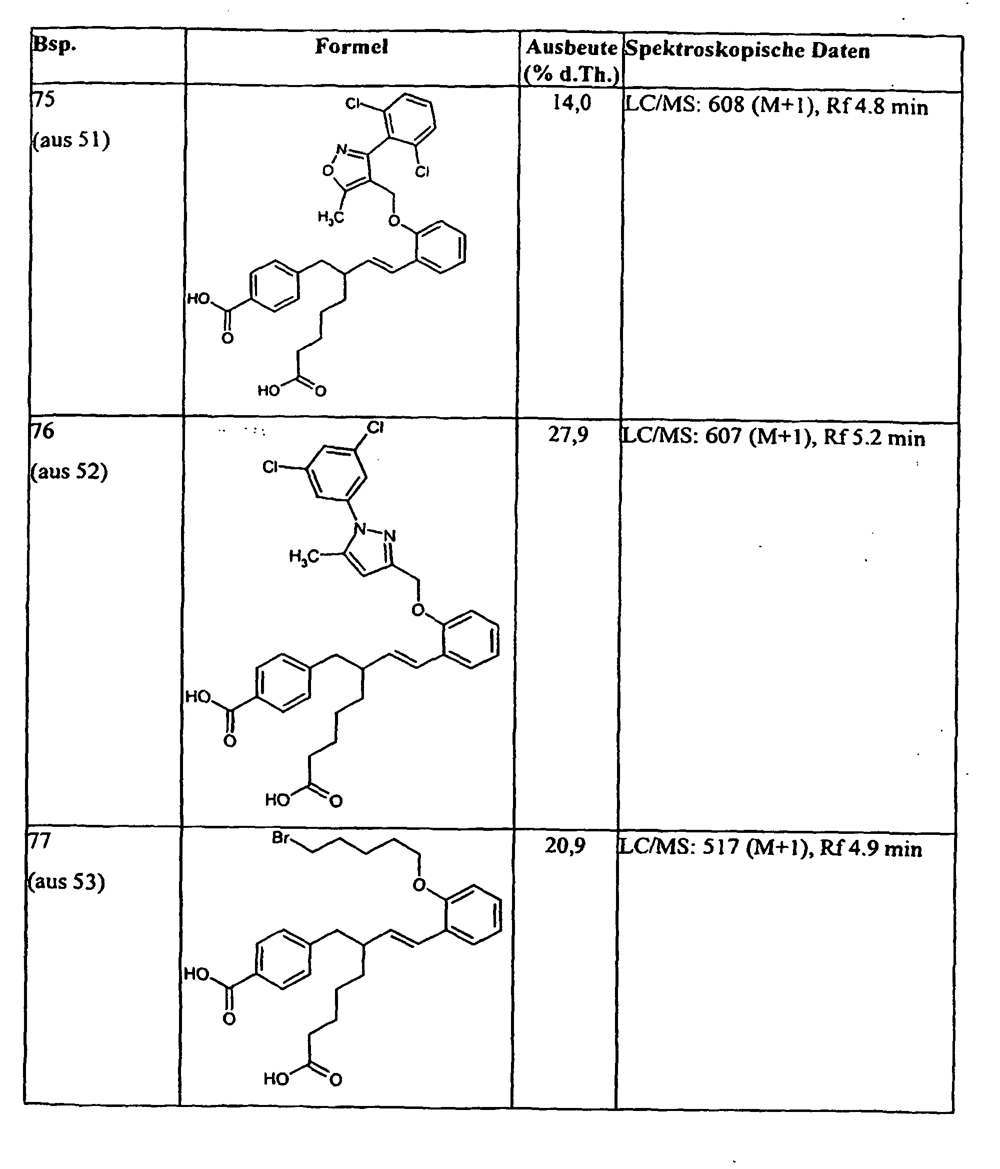

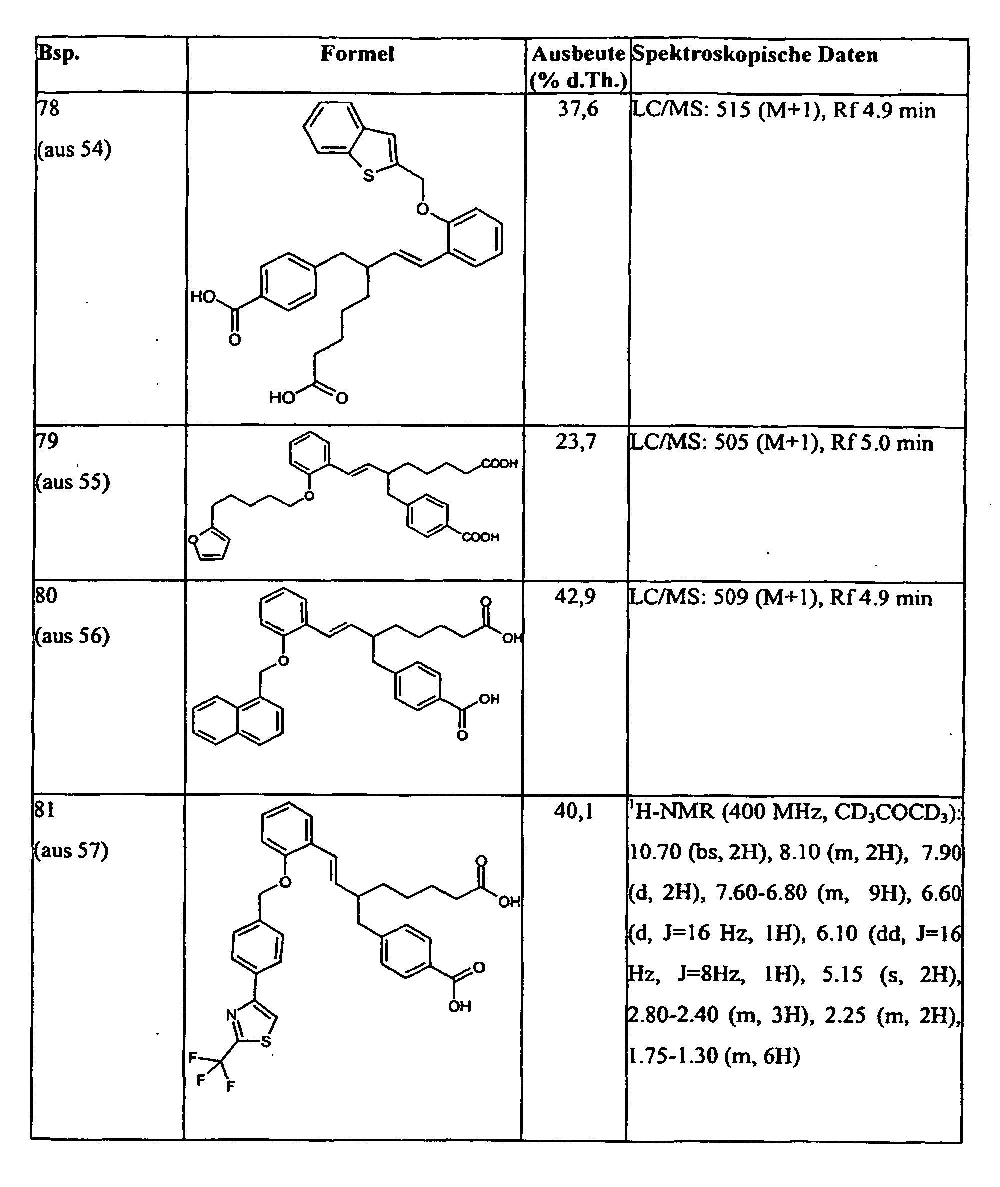

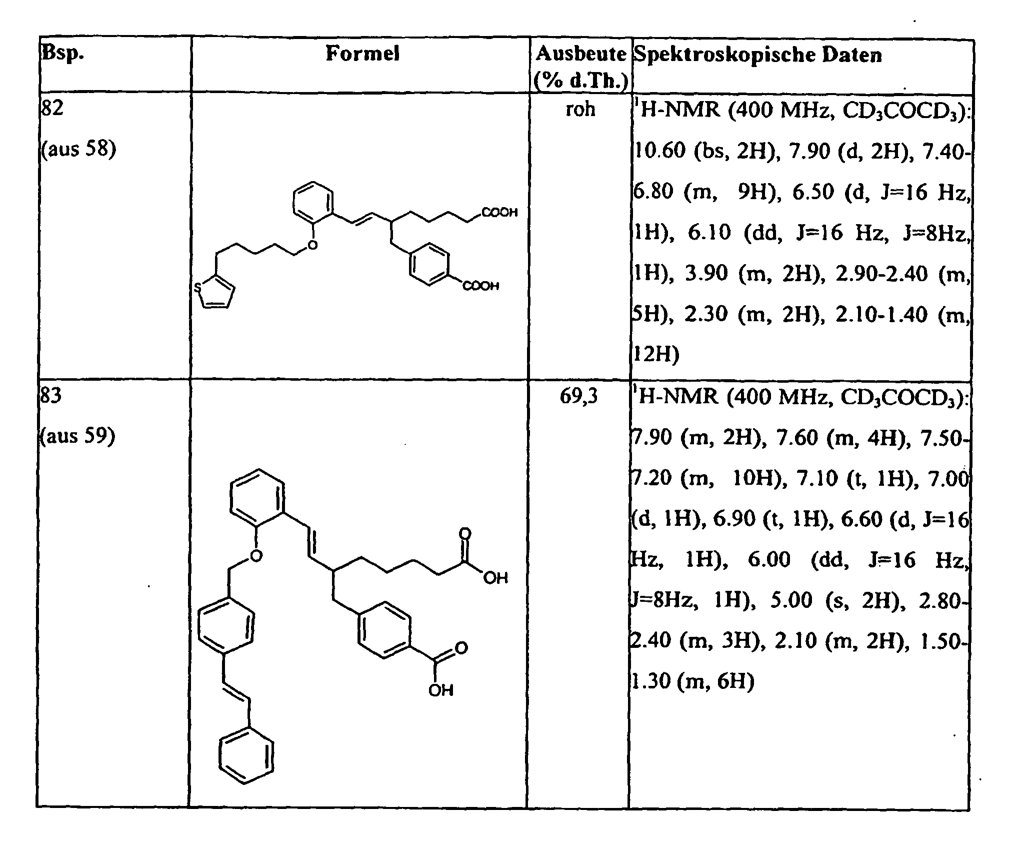

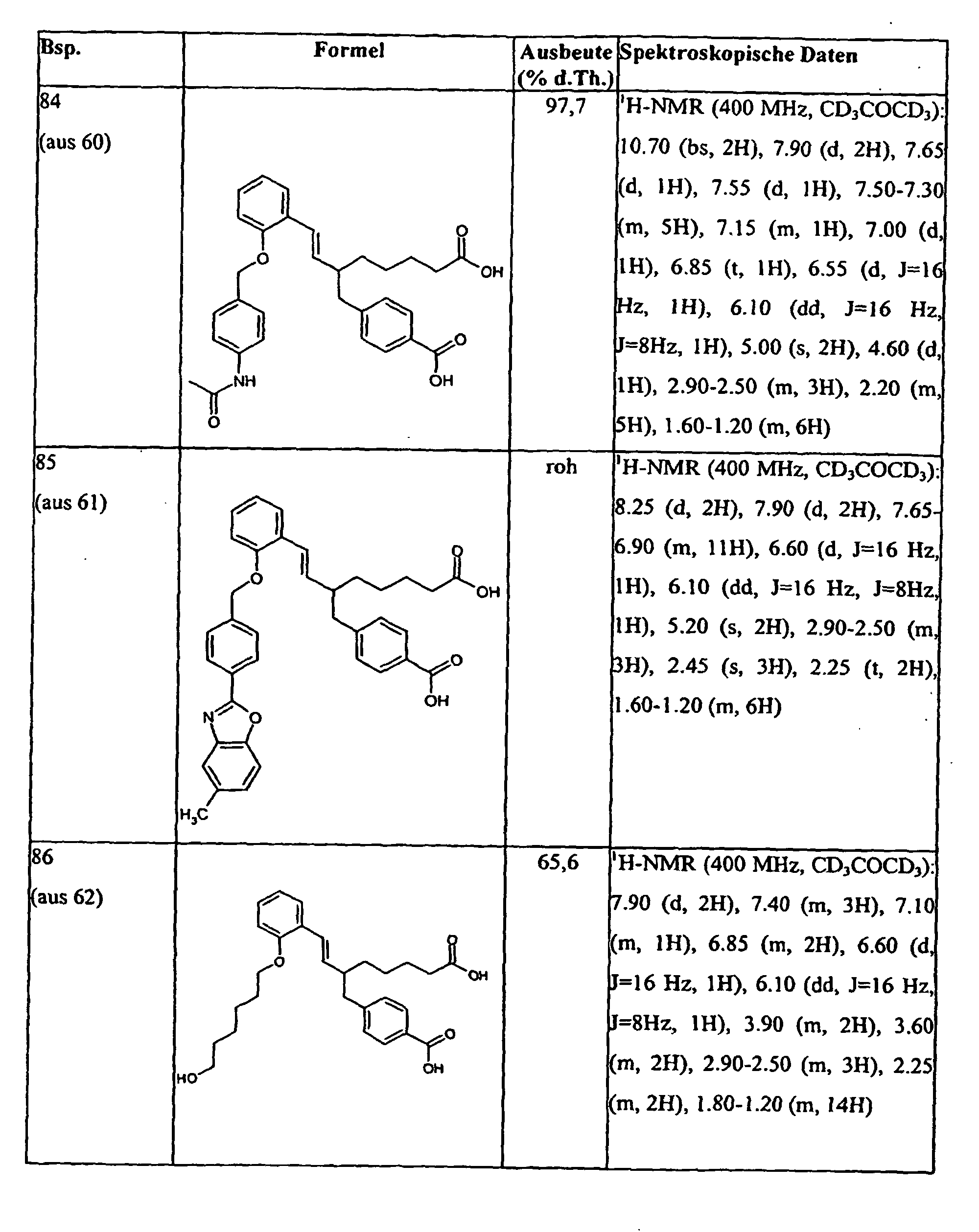

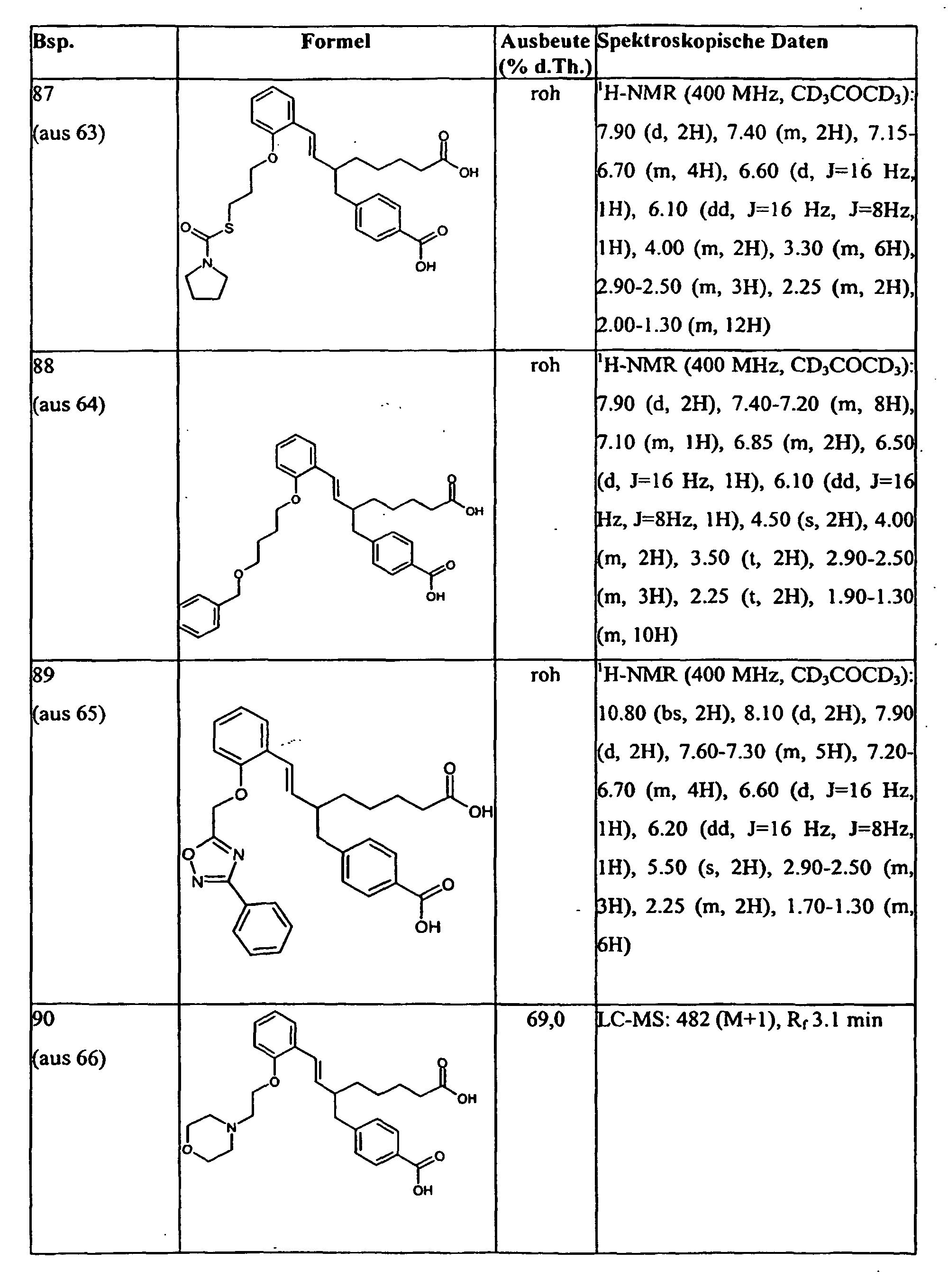

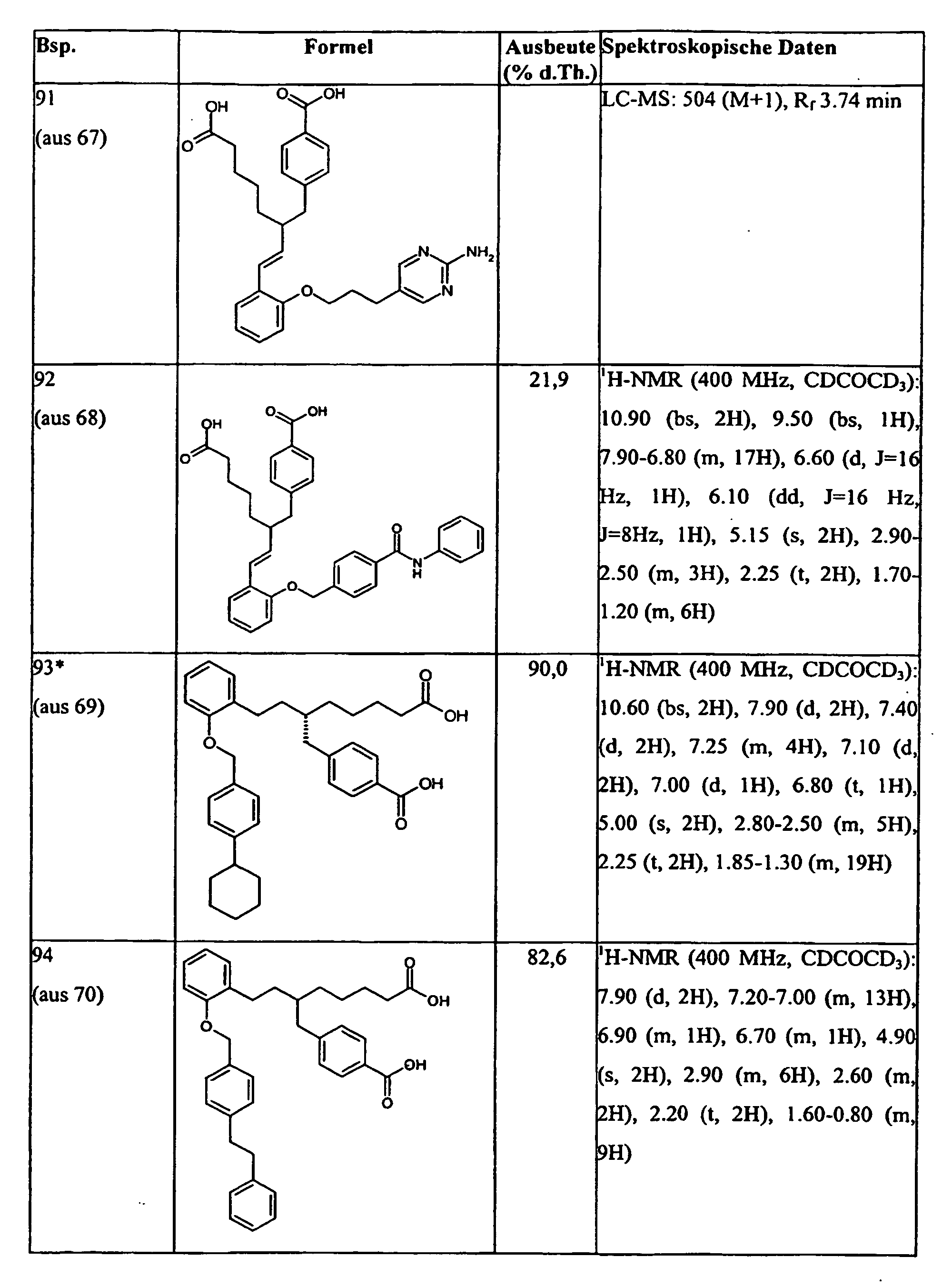

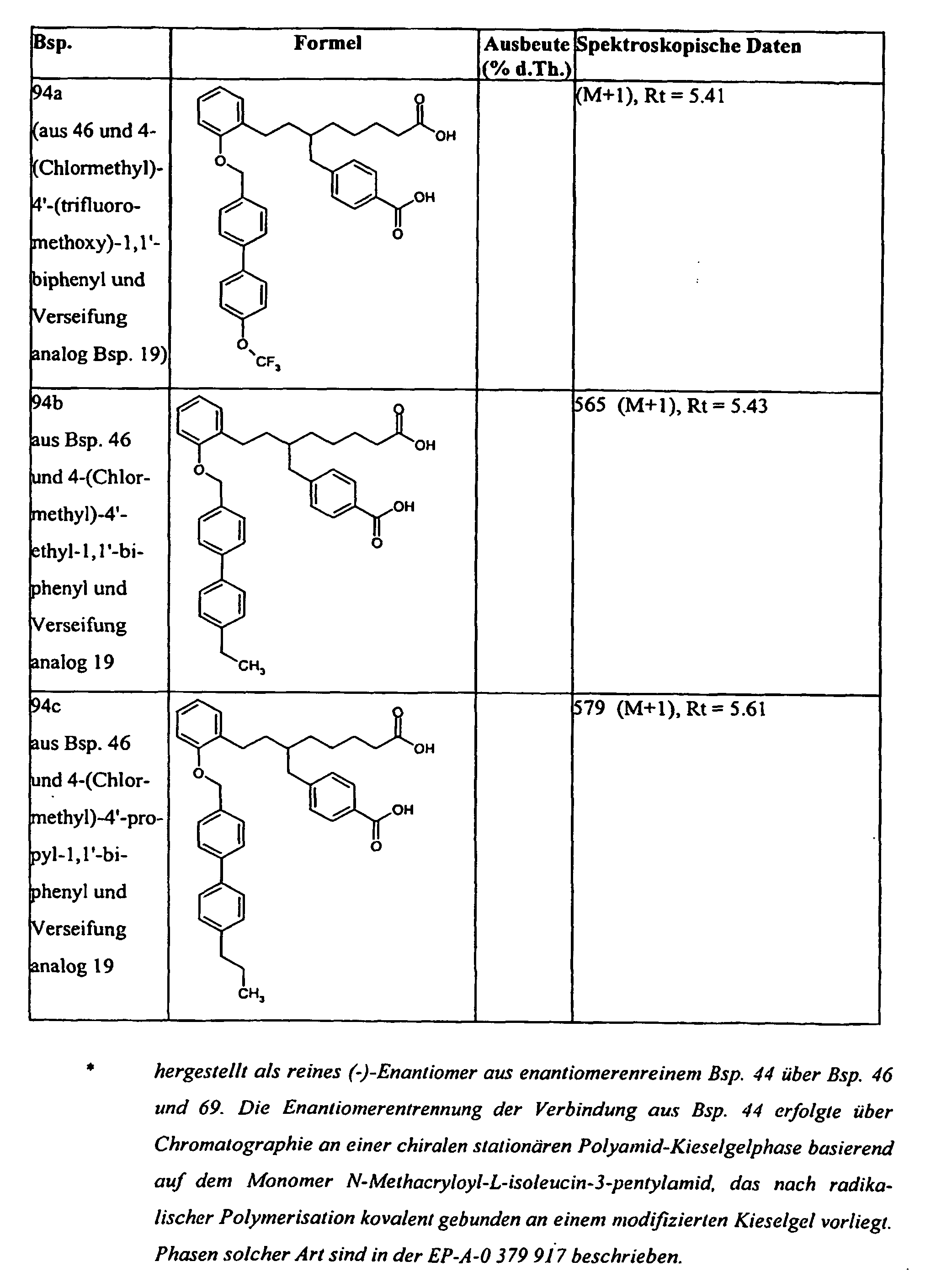

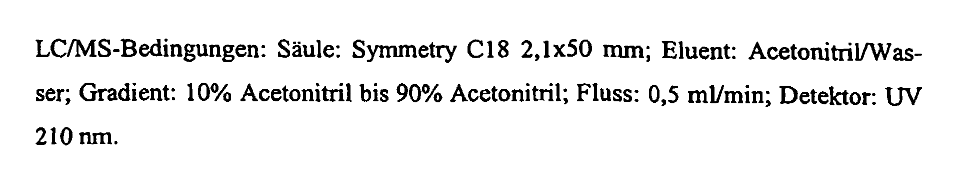

### 95: 6-(4-Carboxybenzyl)-8-(2-heptoxyphenyl)octansäure

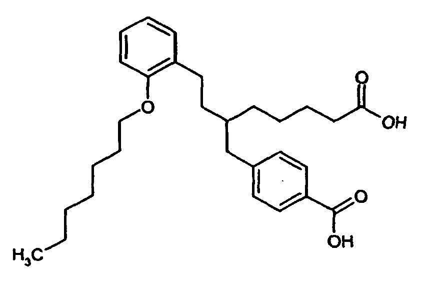

31,6 mg (0,07 mmol) 6-(4-Carboxybenzyl)-8-(2-heptoxyphenyl)-7-octensäure aus Bsp. 25 und 20 mg Palladium/A-Kohle (10%ig) werden in 5 ml Essigsäureethylester gegeben und bei Raumtemperatur unter Normaldruck mit Wasserstoff hydriert. Nach zwei Stunden wird die Mischung über Celite filtriert und im Vakuum eingedampft.
Ausbeute: 15,6 mg (80,7 d. Th.)
¹H-NMR (400 MHz, CD₂Cl₂): 7,90 (d, 2H), 7,60-7,00 (m, 4H), 6,80 (d, 2H), 3,90 (t, 2H), 2,80-2,50 (m, 4H), 2,30 (m, 2H), 1,70-1,25 (m, 19H), 0,90 (t, 3H)

Analog wurde hergestellt:

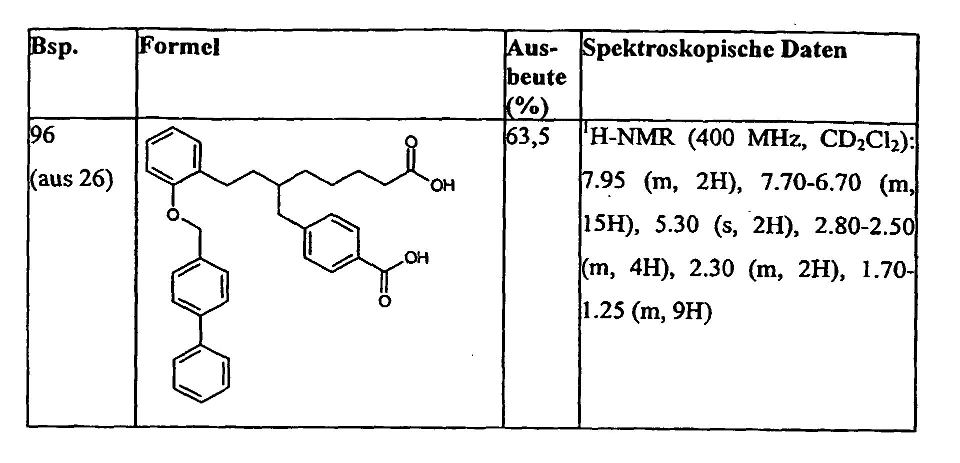

### 97: 6-(4-Ethoxycarbonylbenzyl)-8-(2-(5-N-morpholinopentyloxy)phenyl)-7-(E)-octensäureethylester

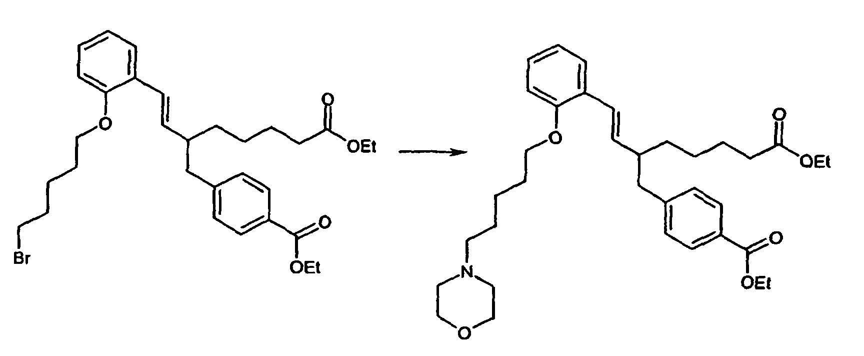

50 mg (0,09 mmol) des Bromides aus Bsp. 53, 15,2 mg (0,17 mmol) Morpholin, 13,2 mg (0,1 mmol) Kaliumcarbonat und eine katalytische Menge Kaliumiodid werden in 5 ml Acetonitril über Nacht unter Rückfluss erhitzt. 0,5 ml Wasser werden zugegeben, die Lösung wird in Dichlormethan aufgenommen, über Extrelut filtriert und im Vakuum eingedampft.
Ausbeute: 50,0 mg (98,9 % d. Th.)
¹H-NMR (300 MHz, CDCl₃): 7,90 (d, 2H), 7,40-7,10 (m, 4H), 6,80 (m, 2H), 6,55 (d, J=16 Hz, 1H), 6,00 (dd, J=16 Hz, J=8Hz, 1H), 4,35 (q, J = 6 Hz, 2H), 4,10 (q, J = 6 Hz, 2H), 3,90 (m, 2H), 3,70 (m, 4H), 2,80 (m, 2H), 2,50 (m, 7H), 2,25 (t, 2H), 1,70-1,20 (m, 18H).

Analog wurden dargestellt:

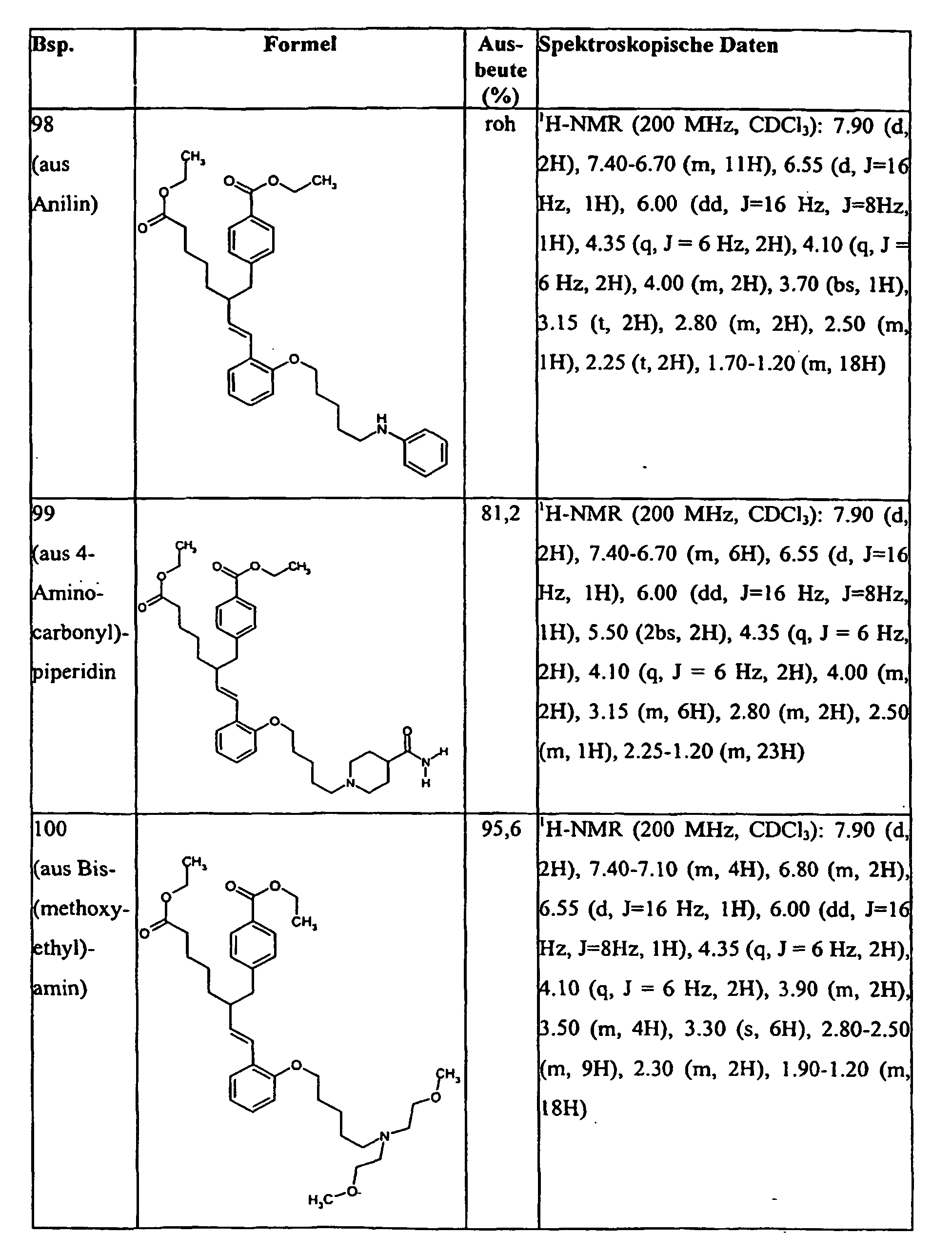

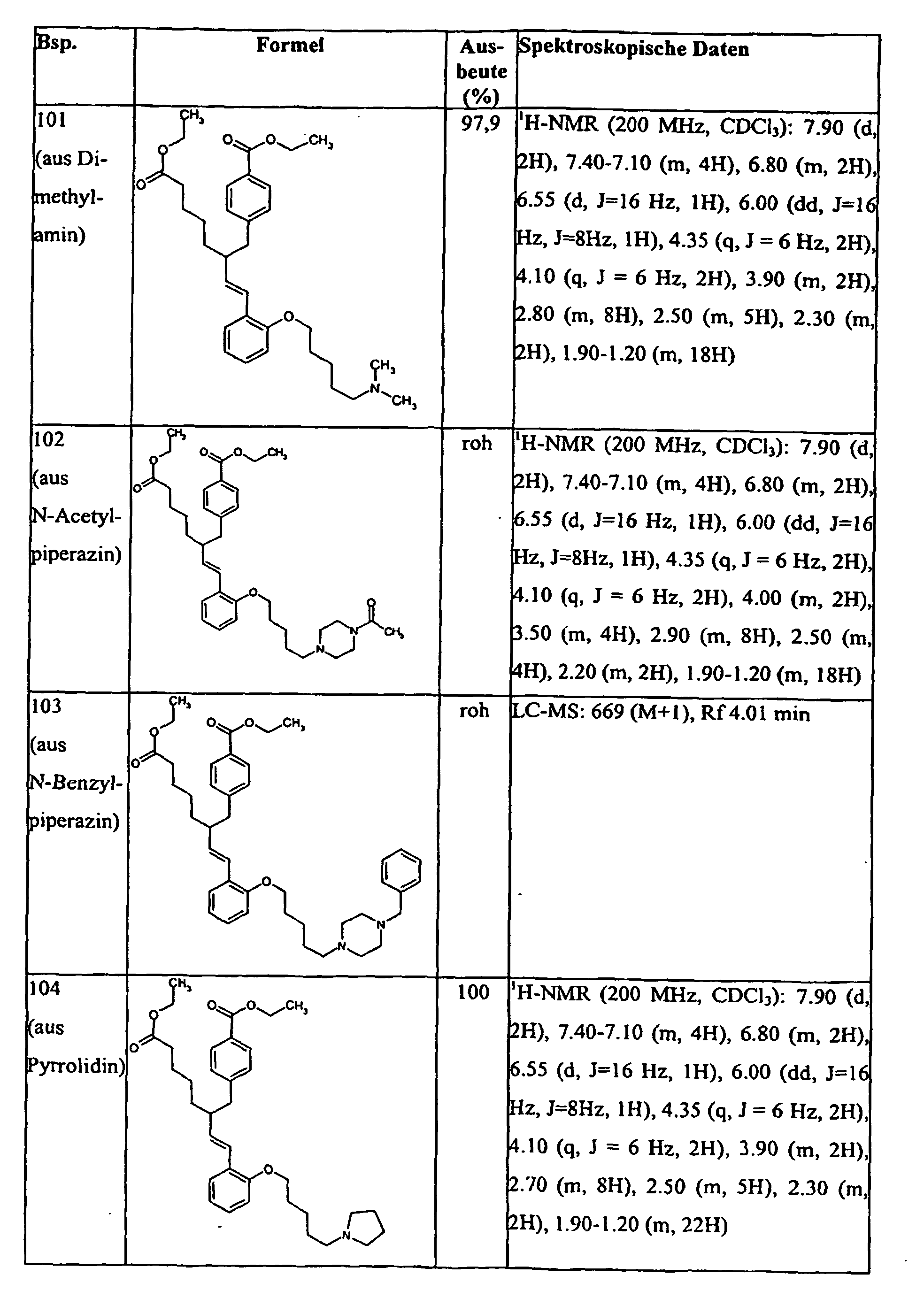

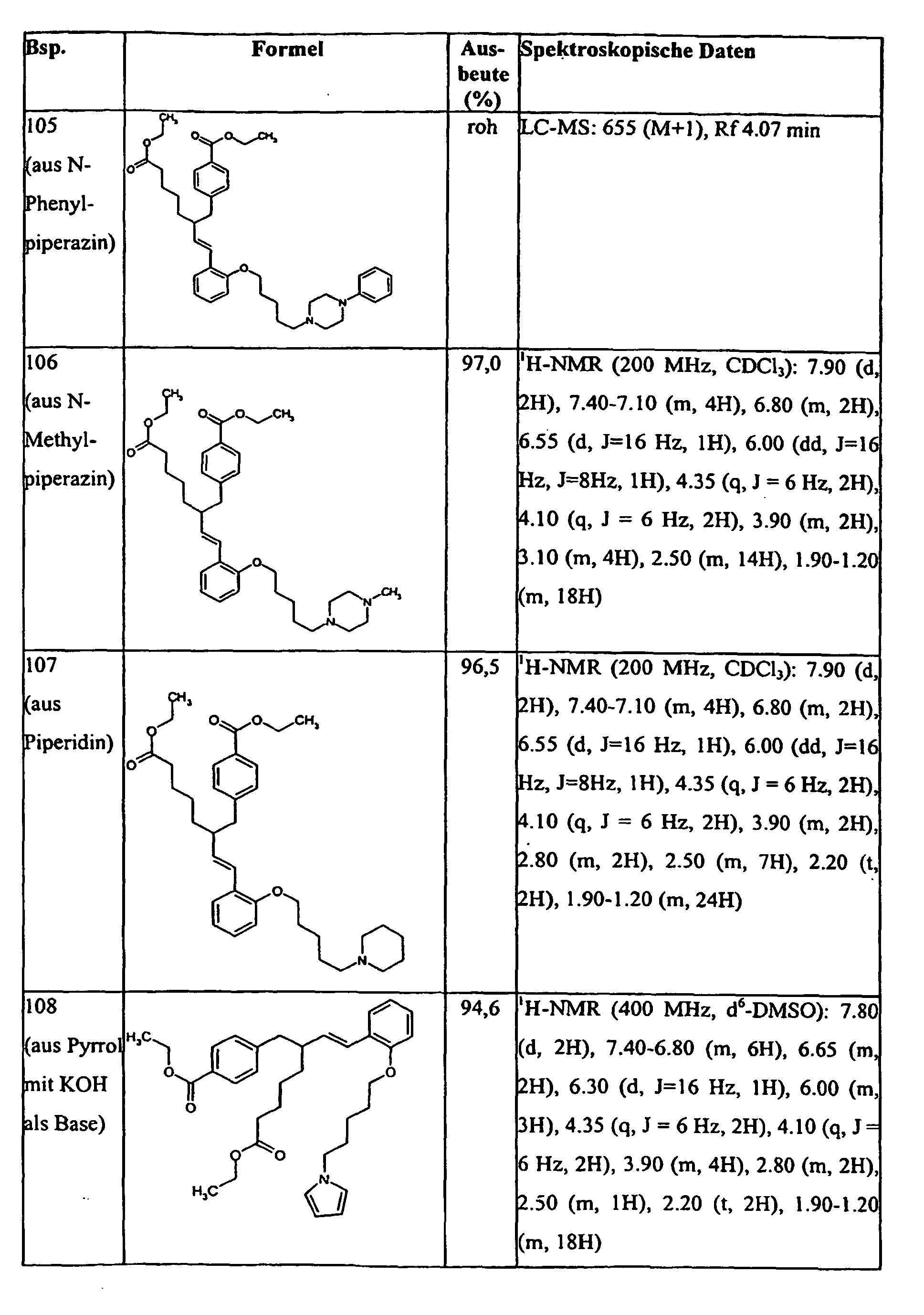

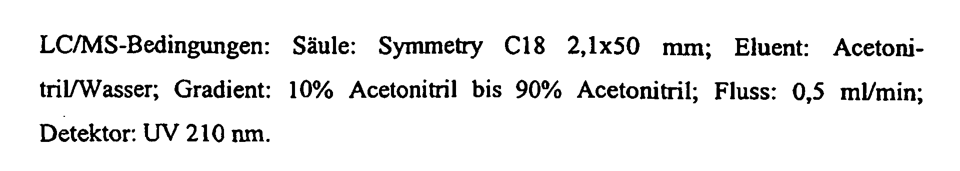

### 109: 6-(4-Carboxybenzyl)-8-(2-(5-N-morpholinopentyloxy)phenyl)-7-(E)-octensäure

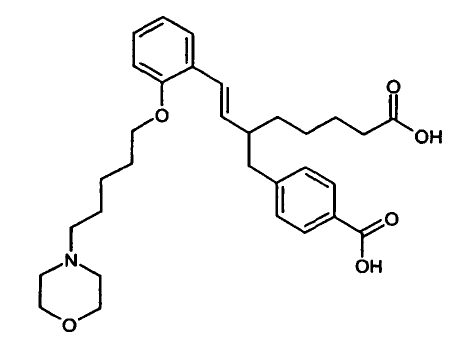

50 mg (0,09 mmol) des Diethylesters aus Bsp. 97 werden in 5 ml Methanol gelöst und mit 0,5ml 45%iger Natronlauge versetzt. Die Reaktion ist exotherm. Man lässt auf Raumtemperatur kommen und gibt 0,3 ml Dichlormethan hinzu. Nach 20 Stunden bei Raumtemperatur wird die Reaktionslösung einmal mit Ether gewaschen, mit 10%iger Schwefelsäure auf pH=4 gestellt, zweimal mit Essigester extrahiert, die vereinigten organischen Phasen über Magnesiumsulfat getrocknet, filtriert und eingeengt.

Ausbeute: 39,1 mg (86,6 % d. Th.)
¹H-NMR (400 MHz, D₂O): 7,90 (d, 2H), 7,40-7,10 (m, 6H), 6,40 (d, J=16 Hz, 1H), 6,20 (dd, J=16 Hz, J=8Hz, 1H), 3,90 (m, 2H), 3,70 (m, 4H), 2,90 (m, 1H), 2,80 (m, 1H), 2,50 (m, 5H), 2,30 (m, 2H), 2,25 (t, 2H), 1,70-1,20 (m, 12H)

Analog wurden dargestellt:

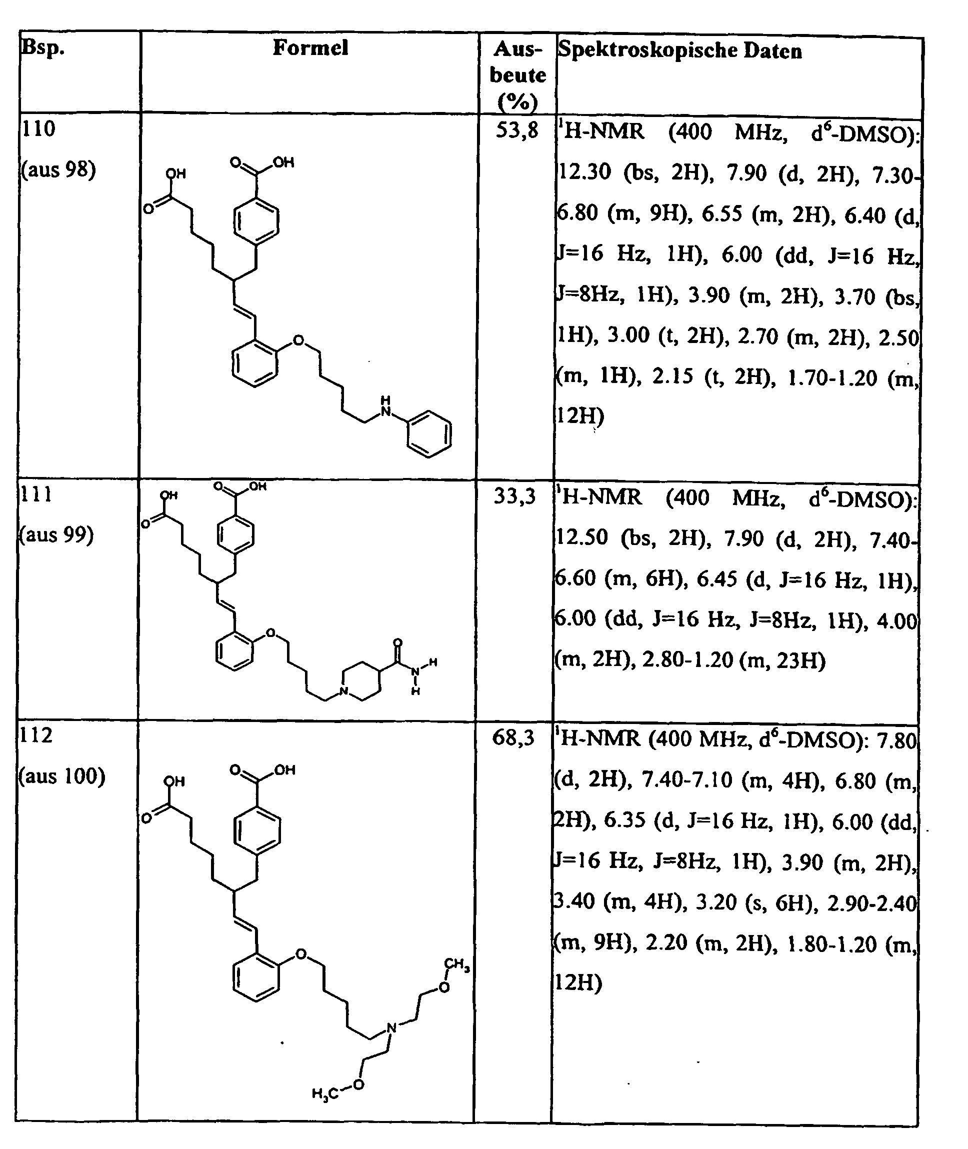

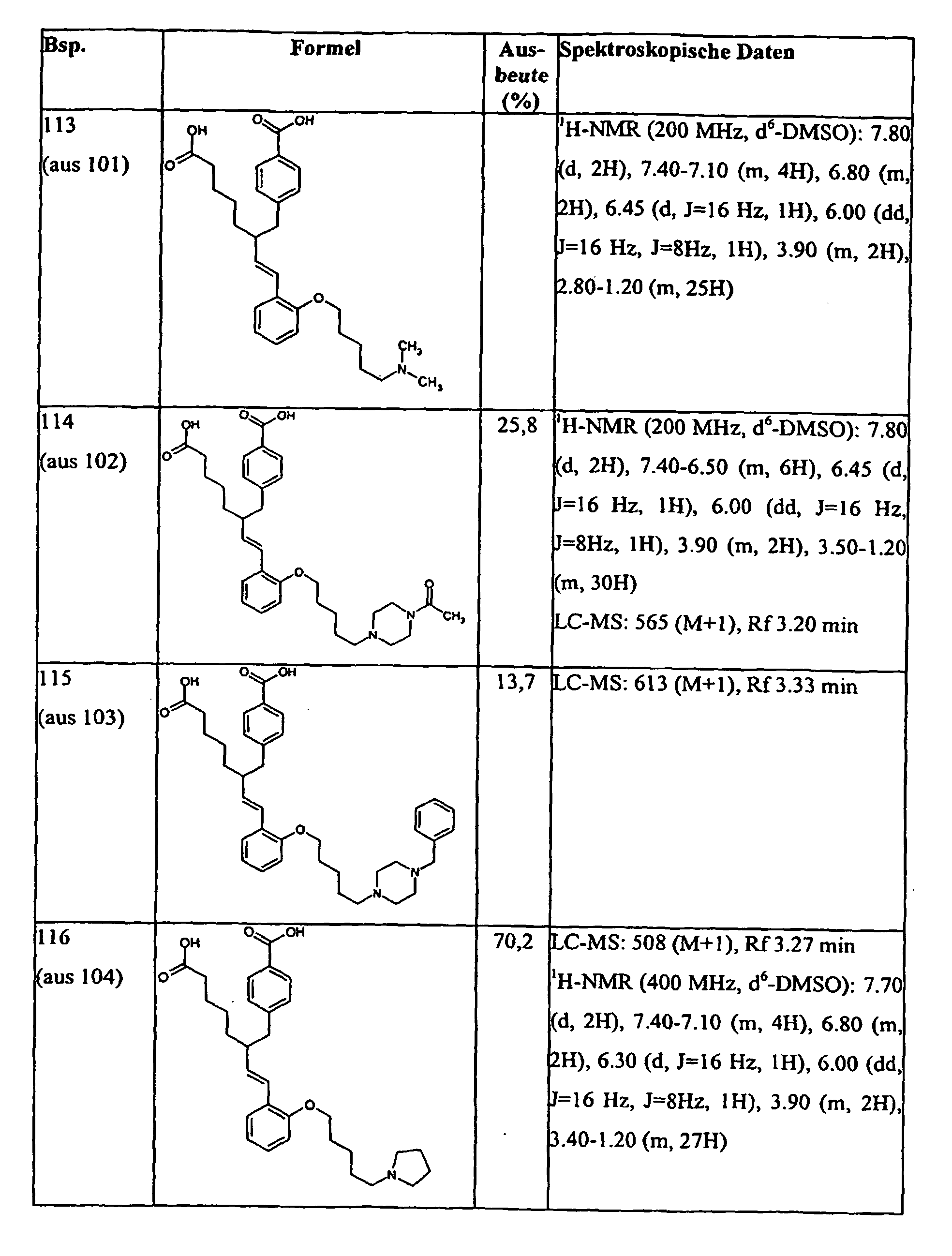

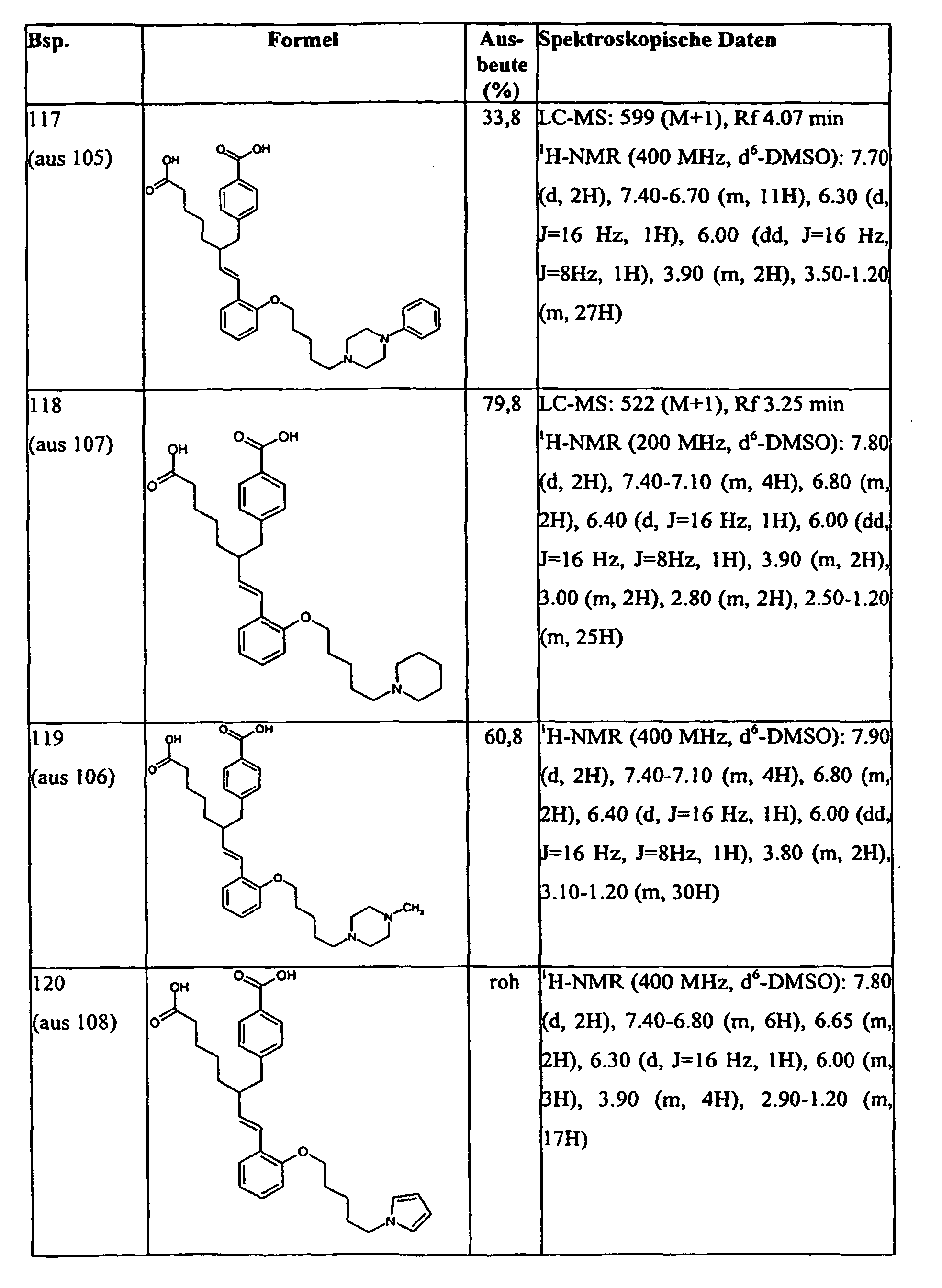

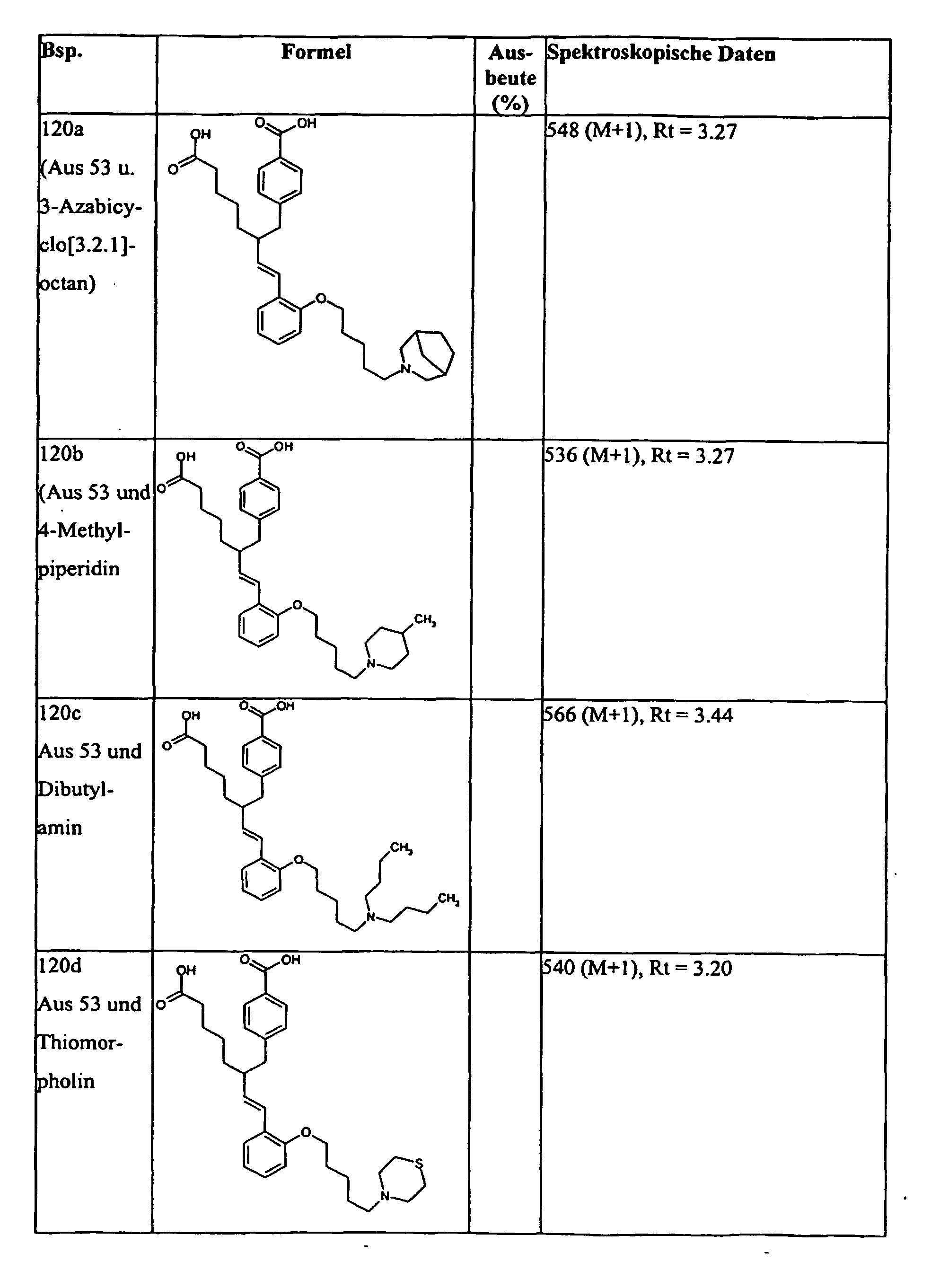

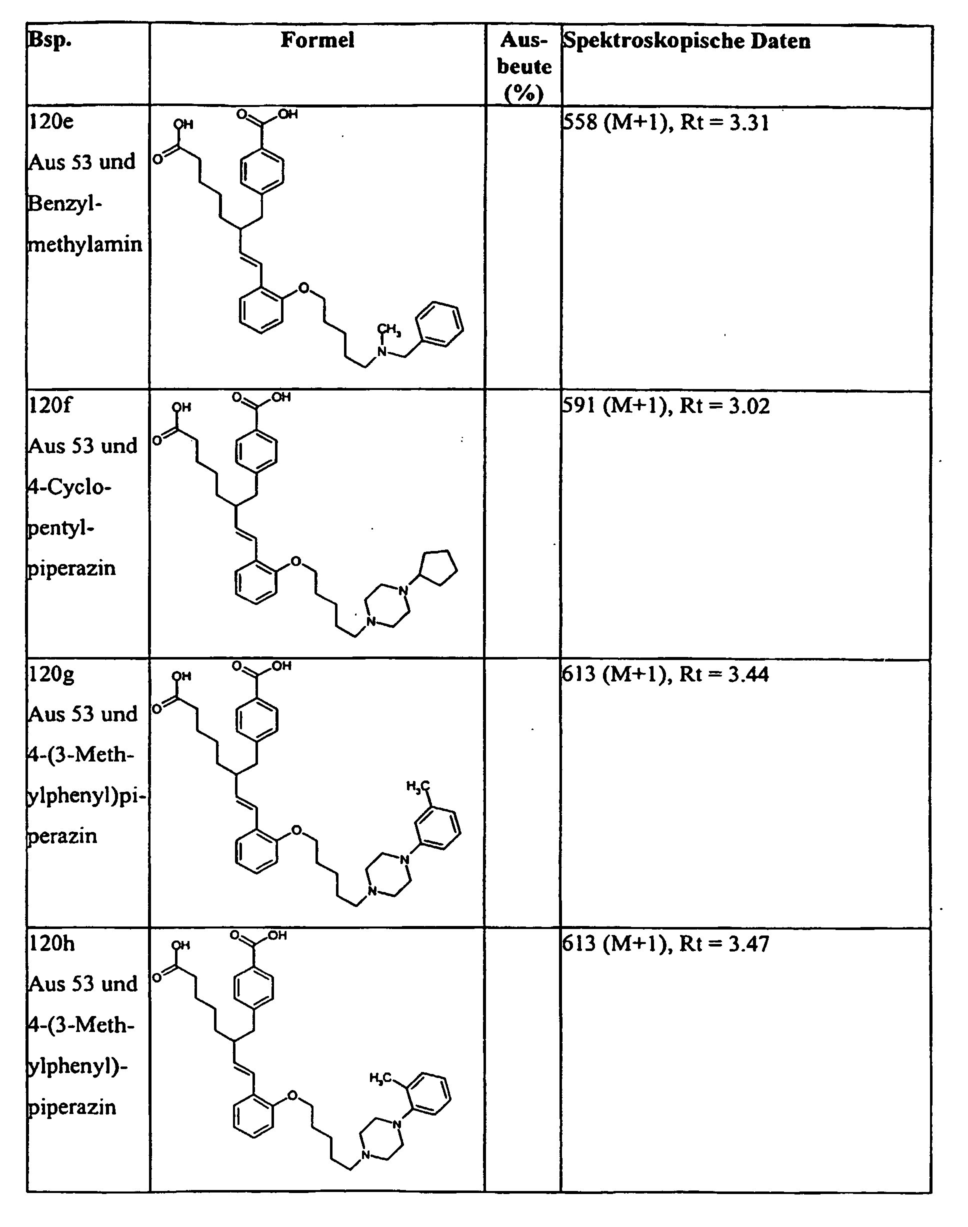

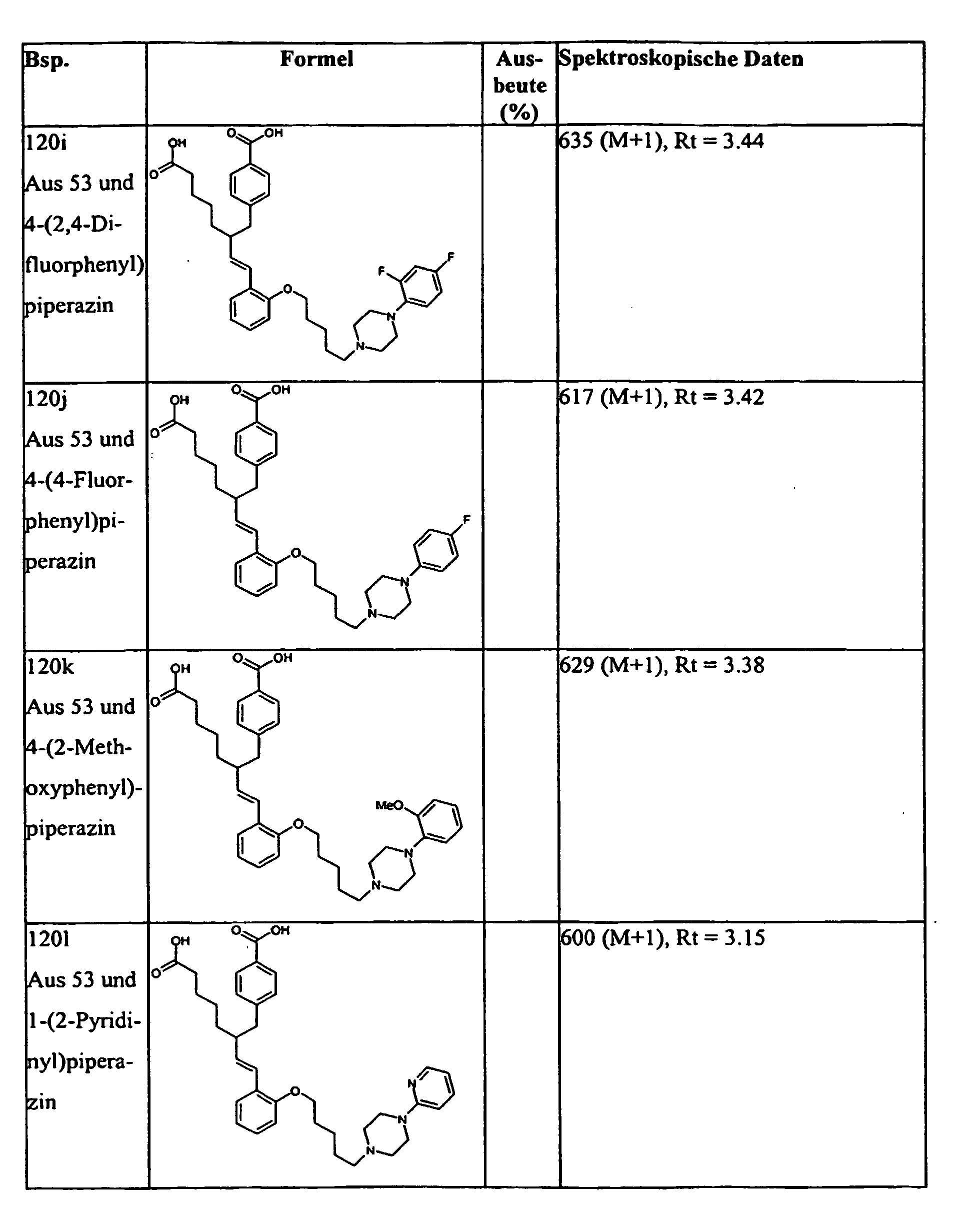

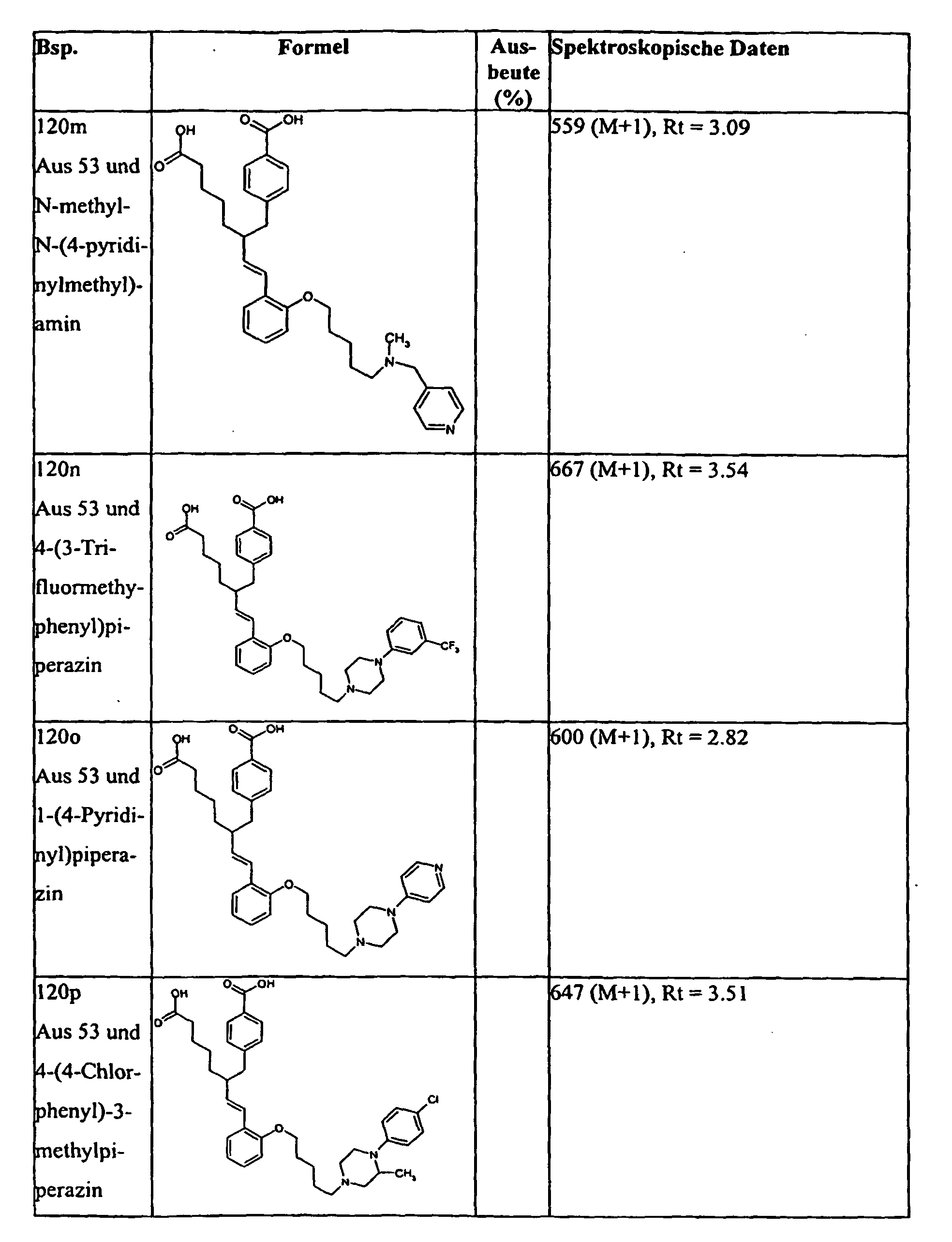

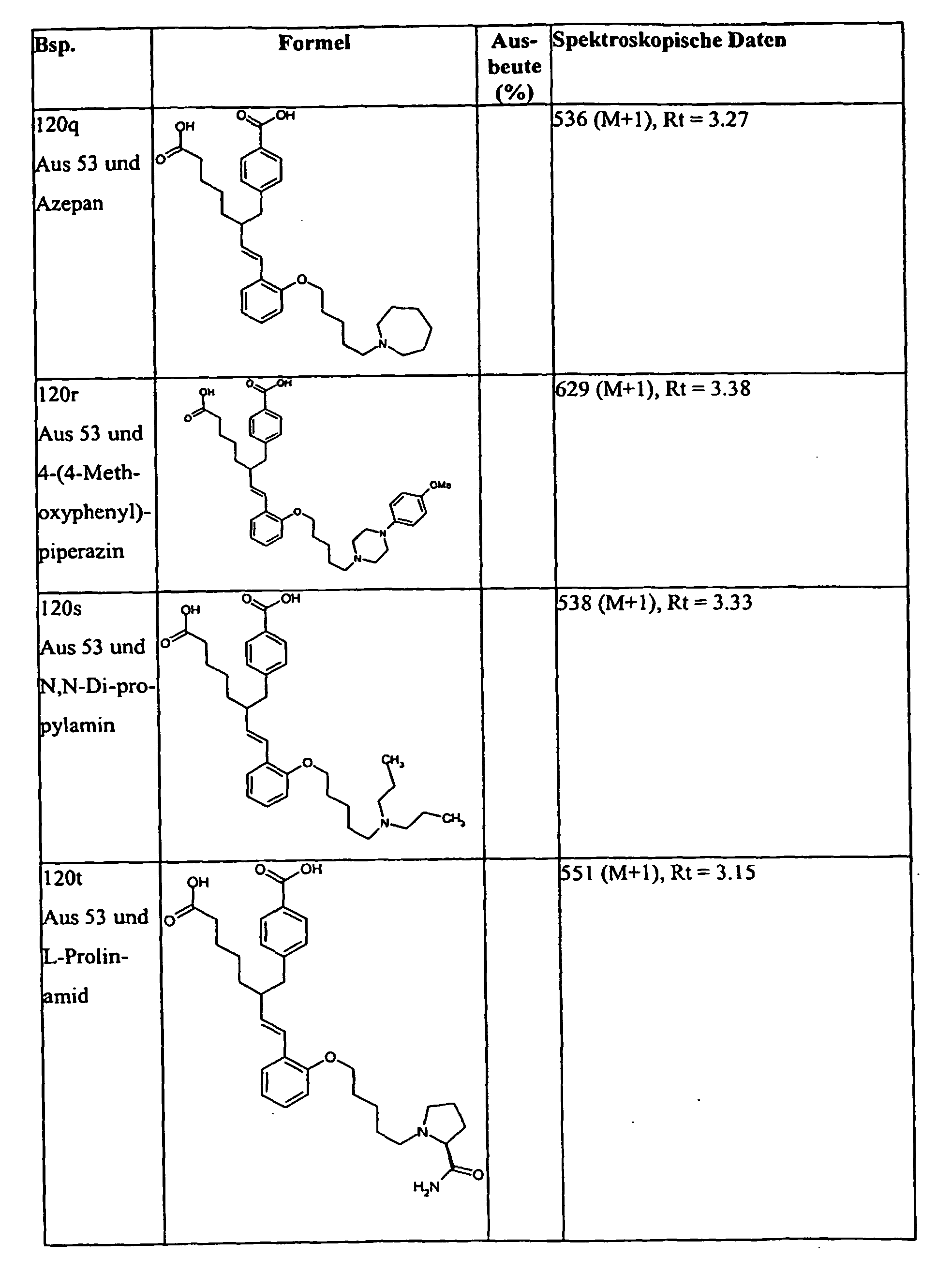

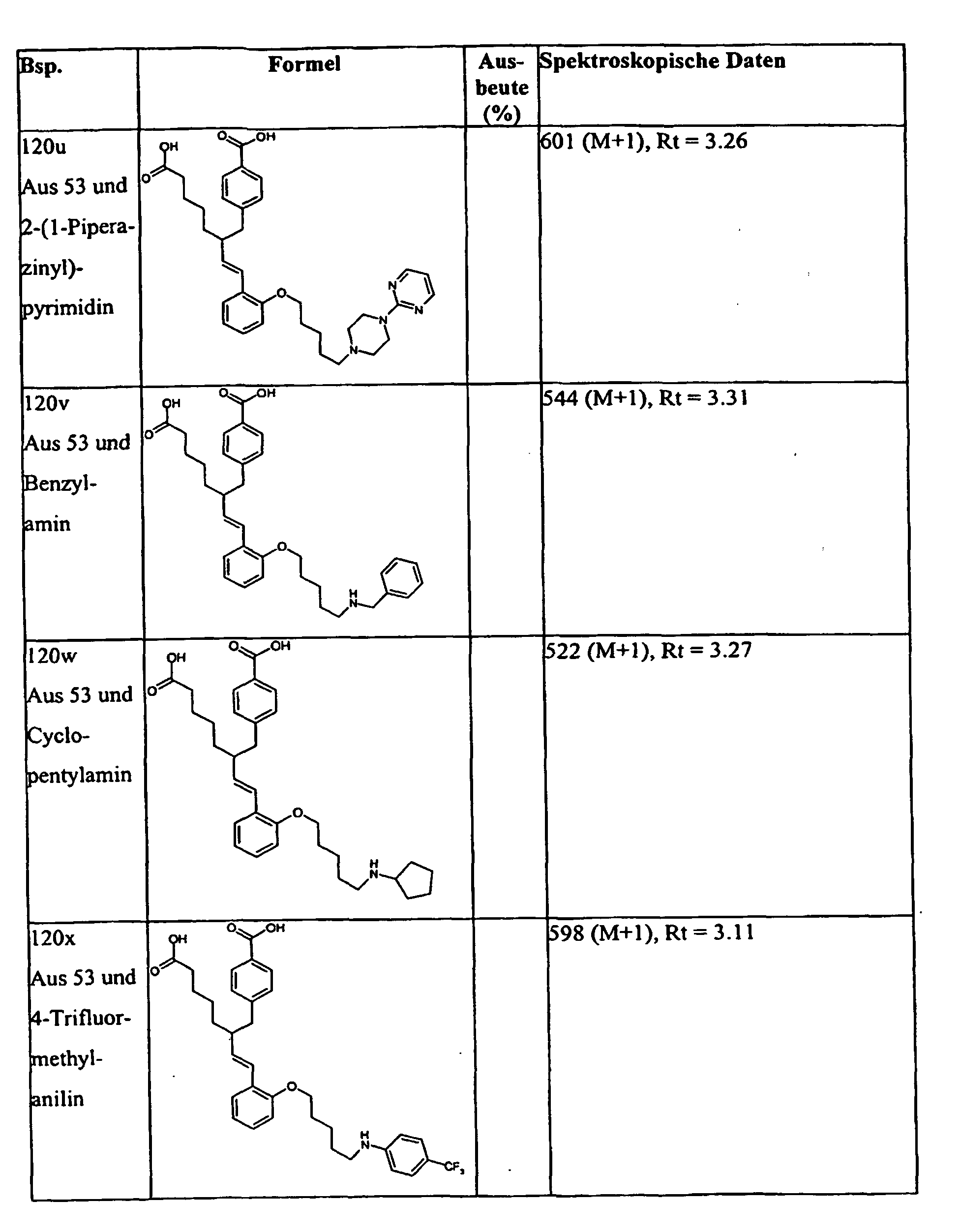

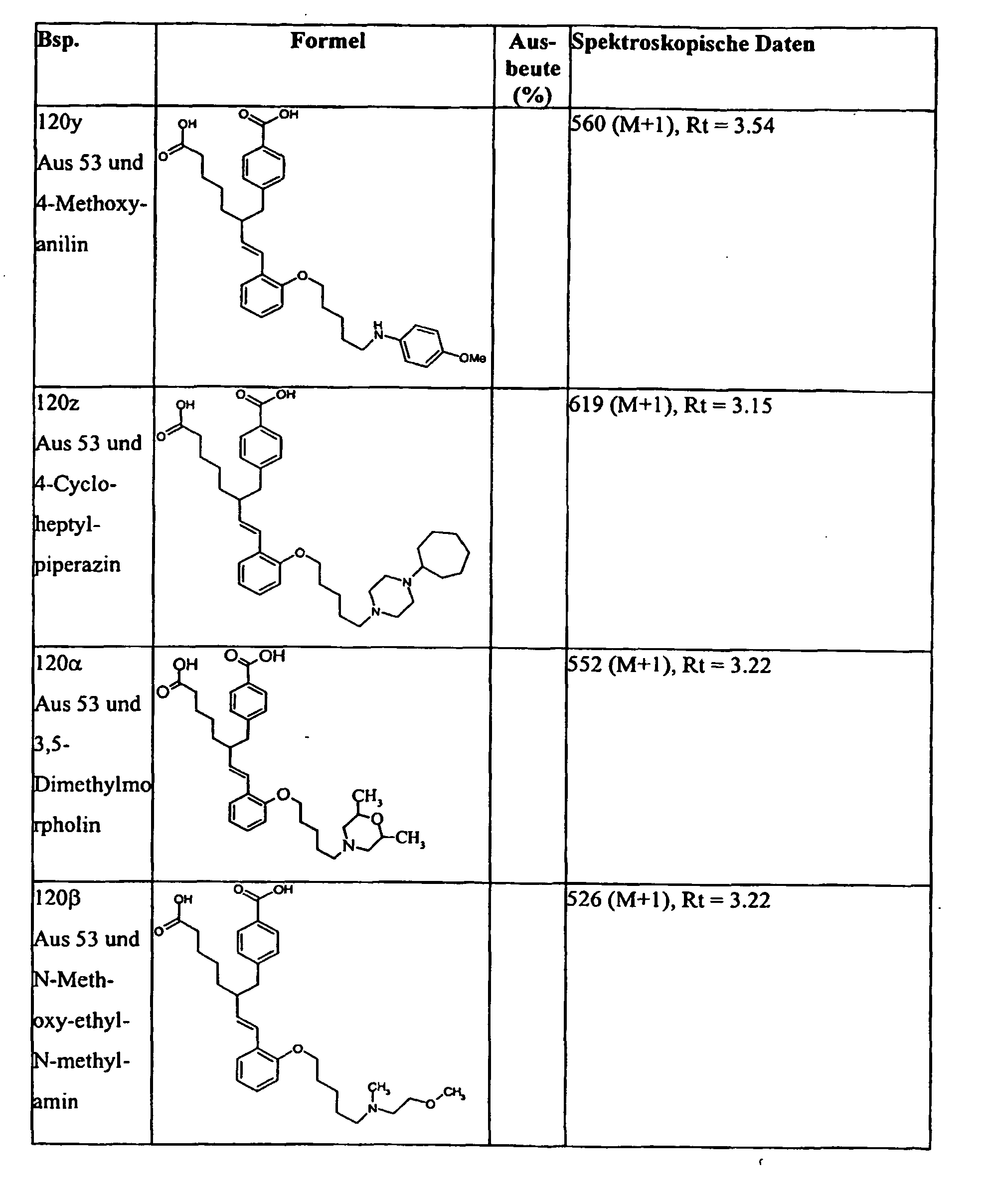

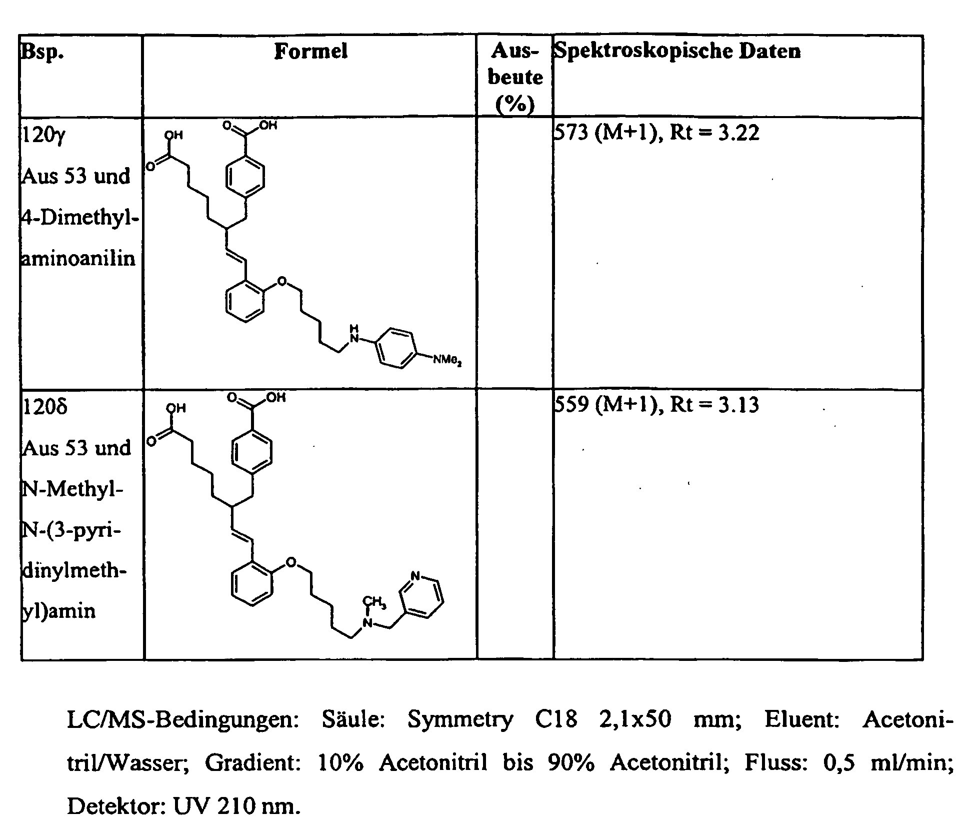

Auf analoge Weise wurden unter Verwendung unterschiedlicher Halogenderivate die folgenden Beispiele erhalten:

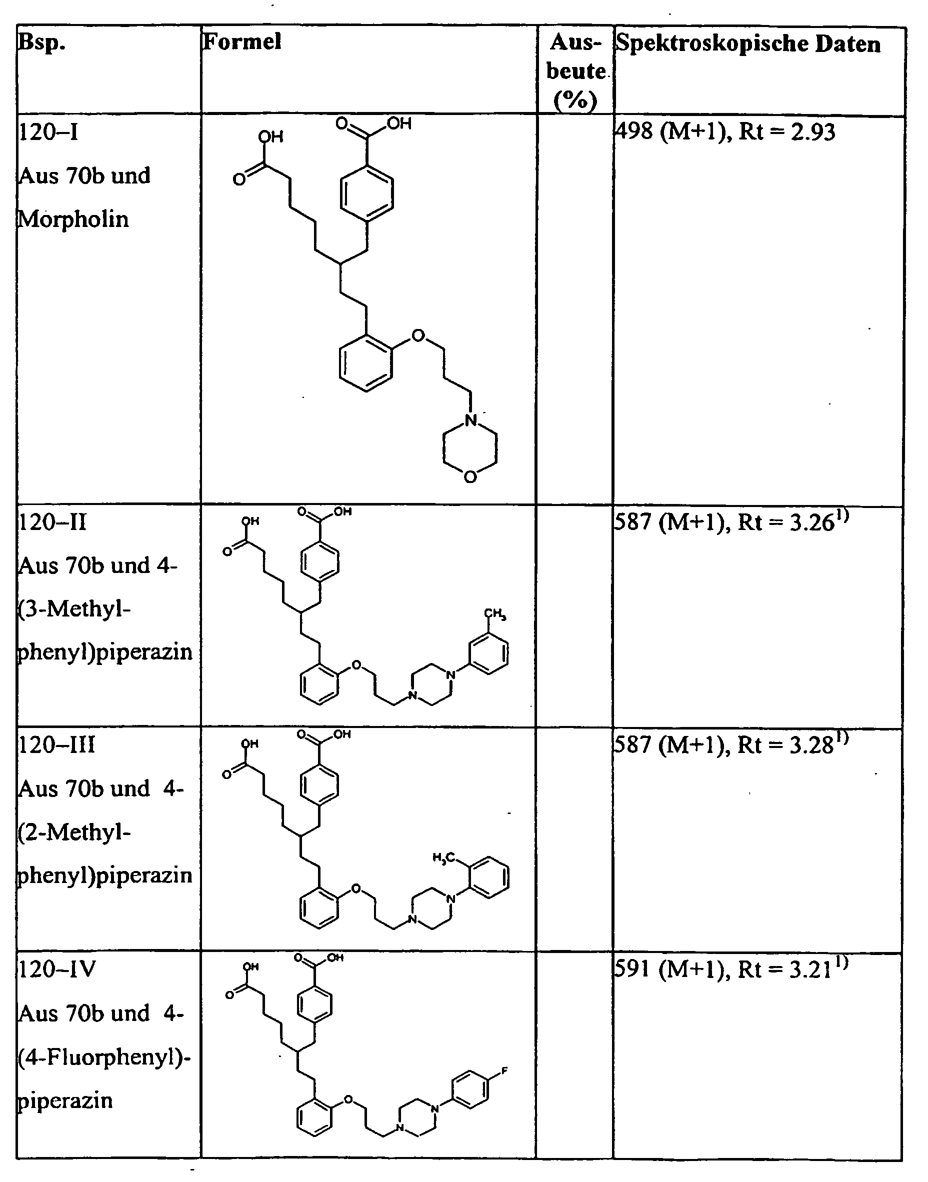

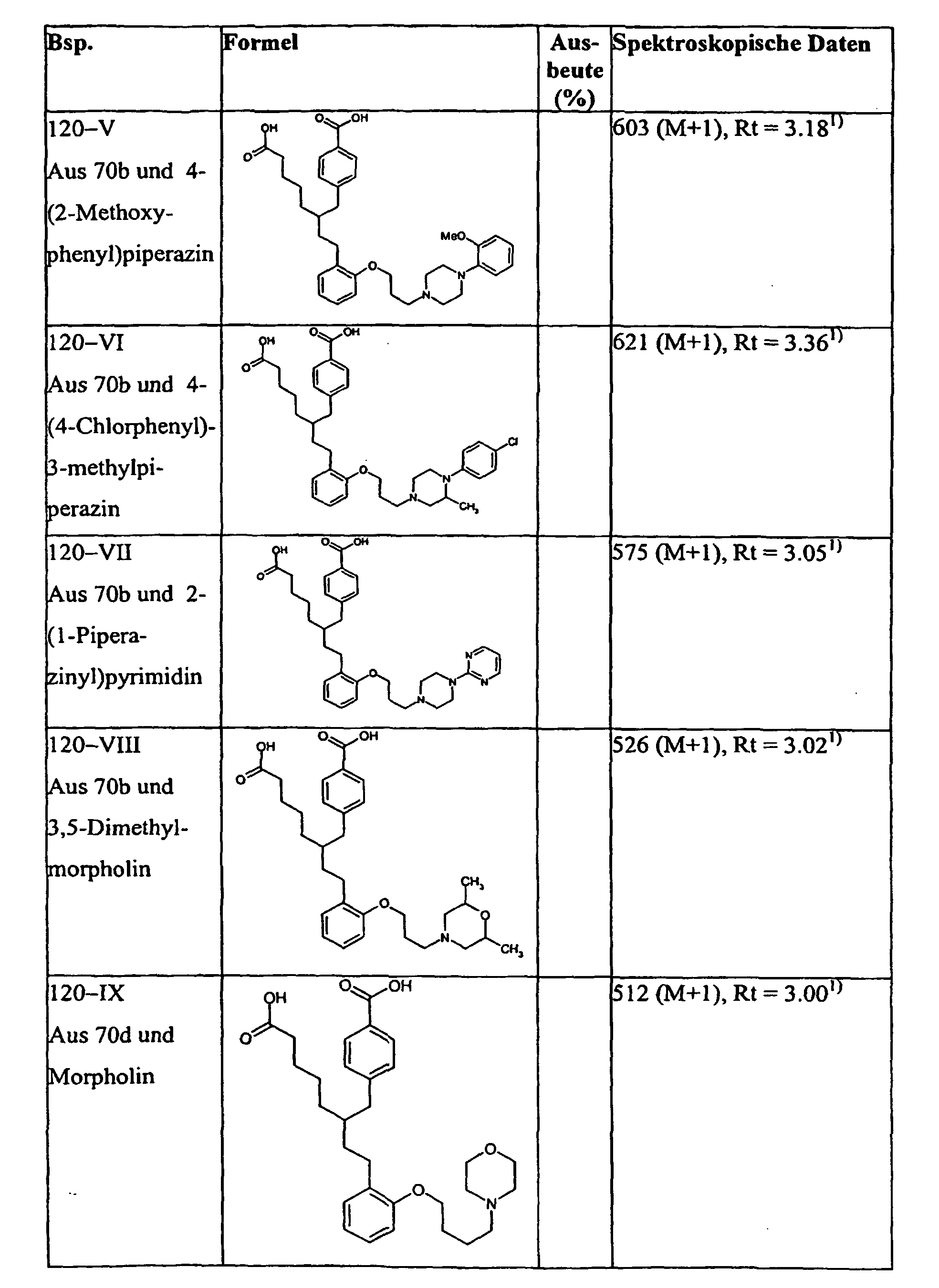

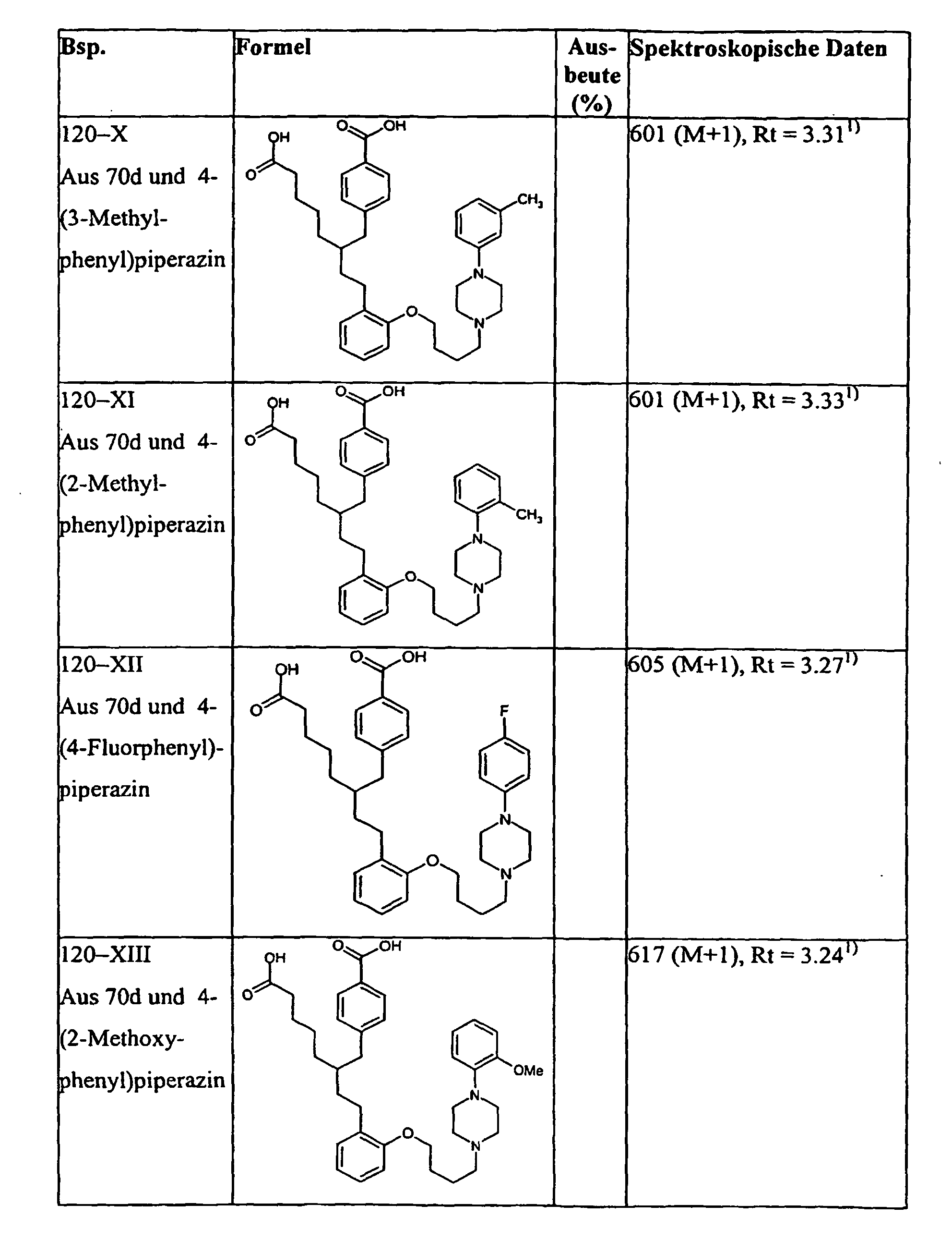

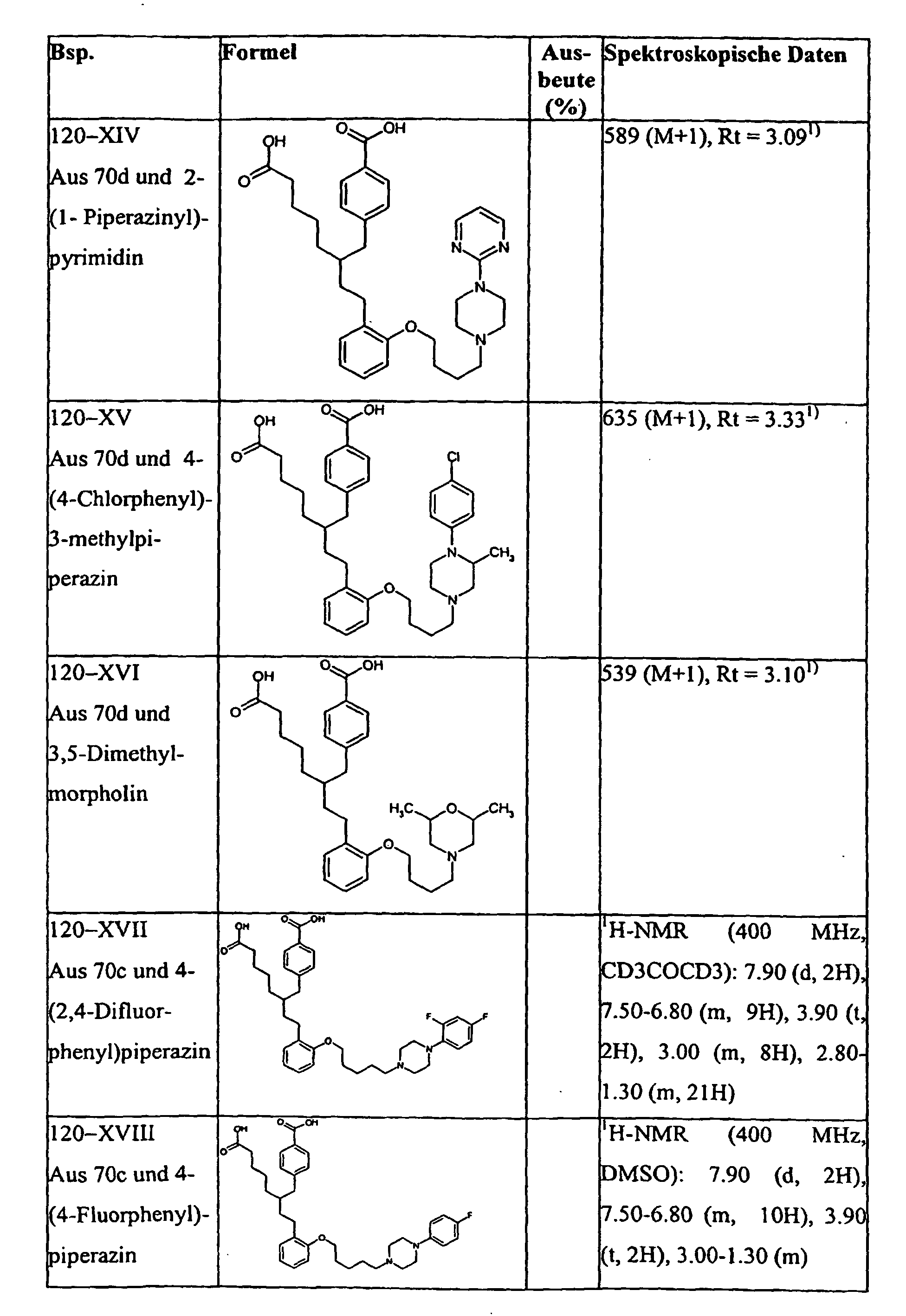

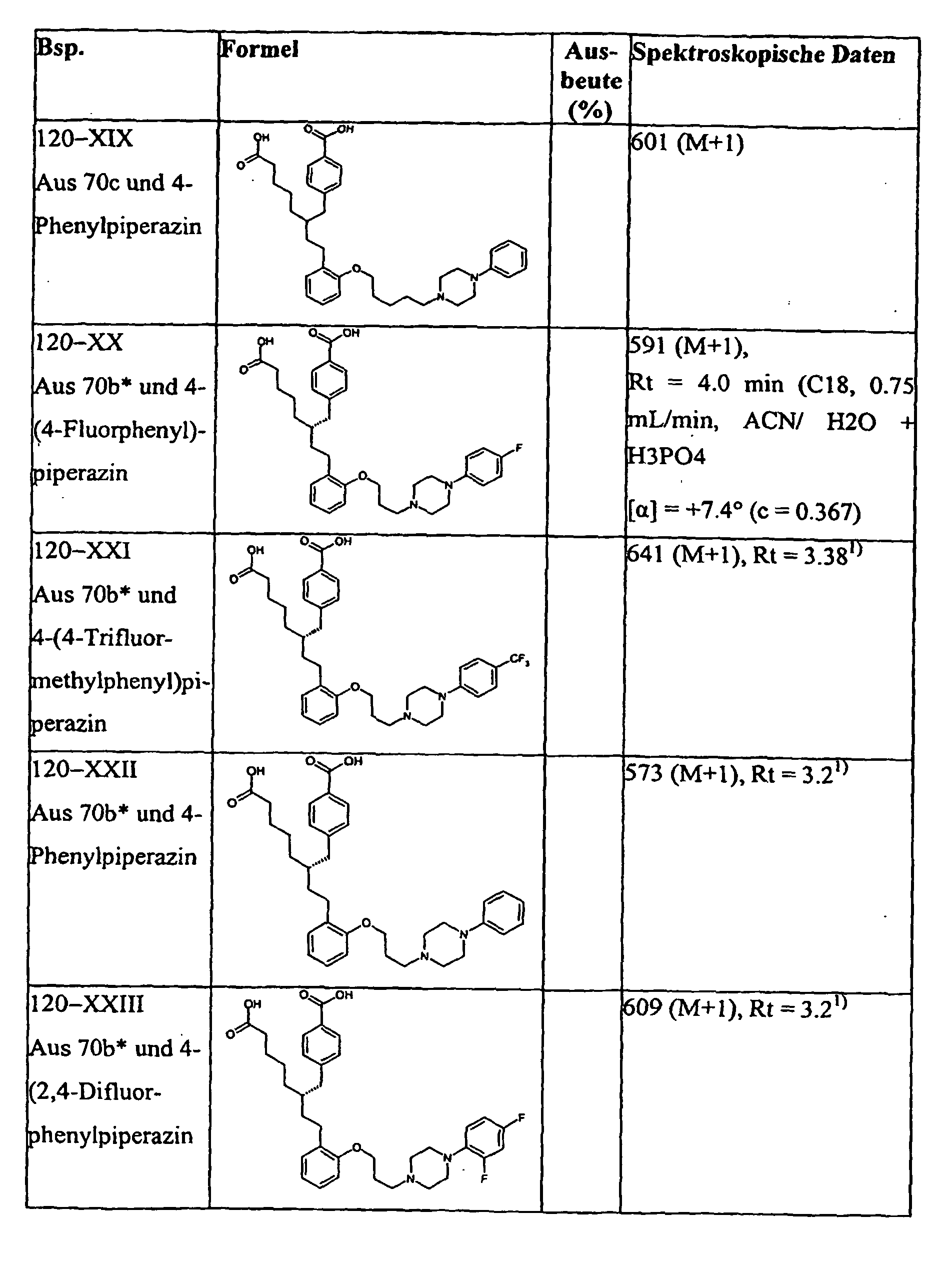

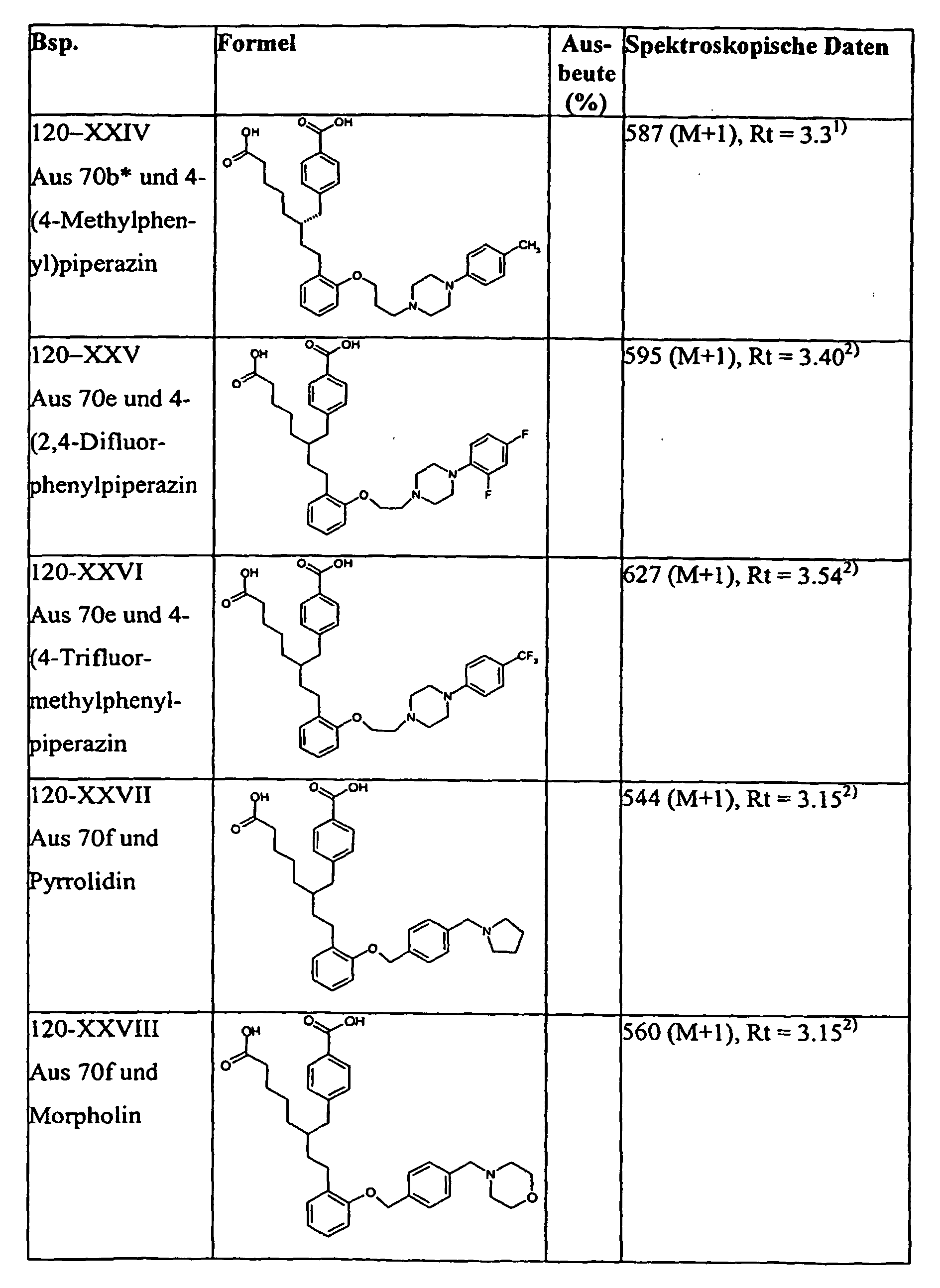

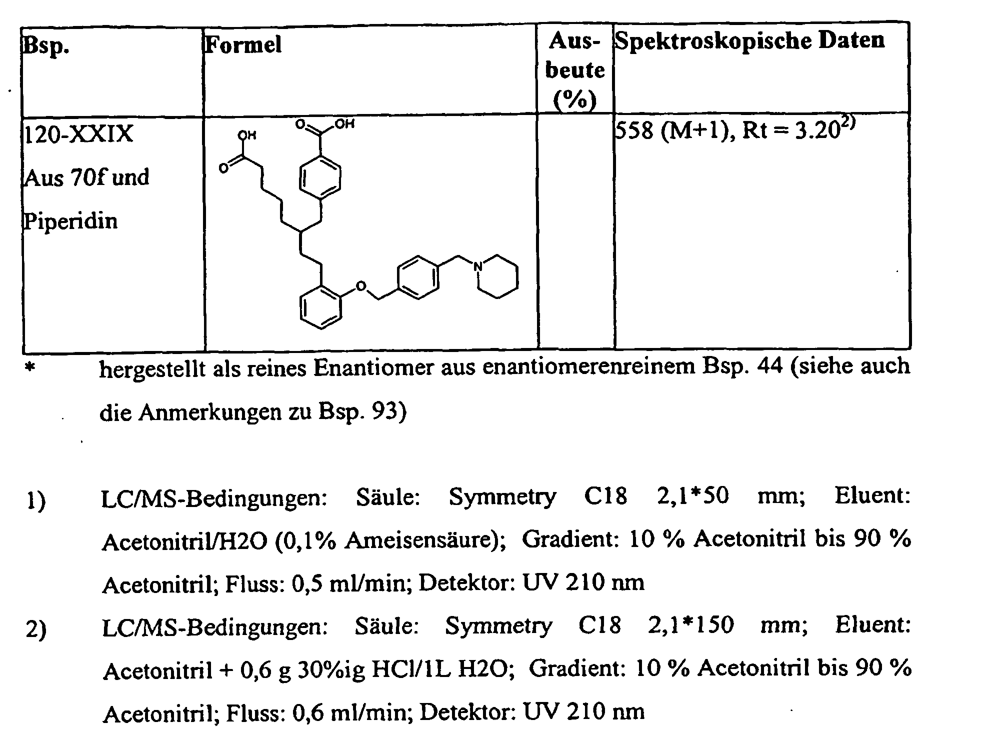

### 121: 7-{[2-(3-Fluorphenyl)-1,3-benzothiazol-4-yl]methoxy}-6-[4-(methoxycarbonyl)benzyl]heptansäuremethylester

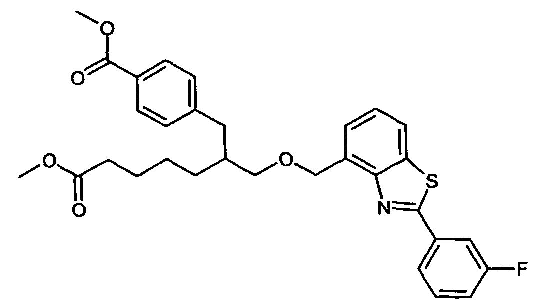

102,8 mg (0,32 mmol) 4-Brommethyl-2-(3-fluorphenyl)-benzothiazol und 300 mg MS3A werden in 5 ml Benzol unter Argon gelöst. Bei Raumtemperatur werden 82 mg (0,27 mmol) 6-Hydroxymethyl-7-(4-methoxycarbonylphenyl)heptansäuremethylester (Synthese vgl. EP-A-0 341 551, S. 31, Bsp. 42) und 92 mg (0,40 mmol) Silberoxid zugegeben. Die Mischung wird sechs Tage bei Raumtemperatur gerührt. Es wird mit ca. 0,2 ml Wasser versetzt, über Extrelut filtriert, mit Toluol nachgewaschen, im Vakuum eingeengt und chromatographiert.

Ausbeute: 64 mg (43.8% d. Th.)
¹H-NMR (200 MHz, CDCl₃): 8,00-7,10 (m, 11H), 5,10 (s, 2H), 3,90 (s, 3H), 3,70 (s, 3H), 3,50 (m, 2H), 2,70 (m, 2H), 2,30 (m, 2H), 1,80 (m, 1H), 1,70-1,20 (m, 6H)

Analog wurden die folgende Substanzen synthetisiert:

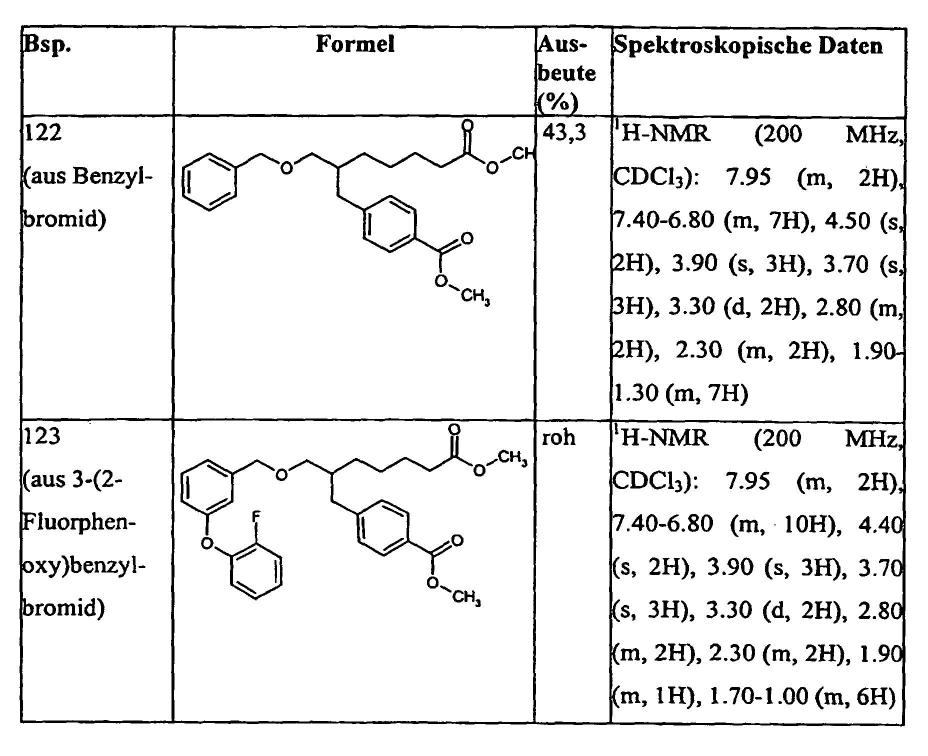

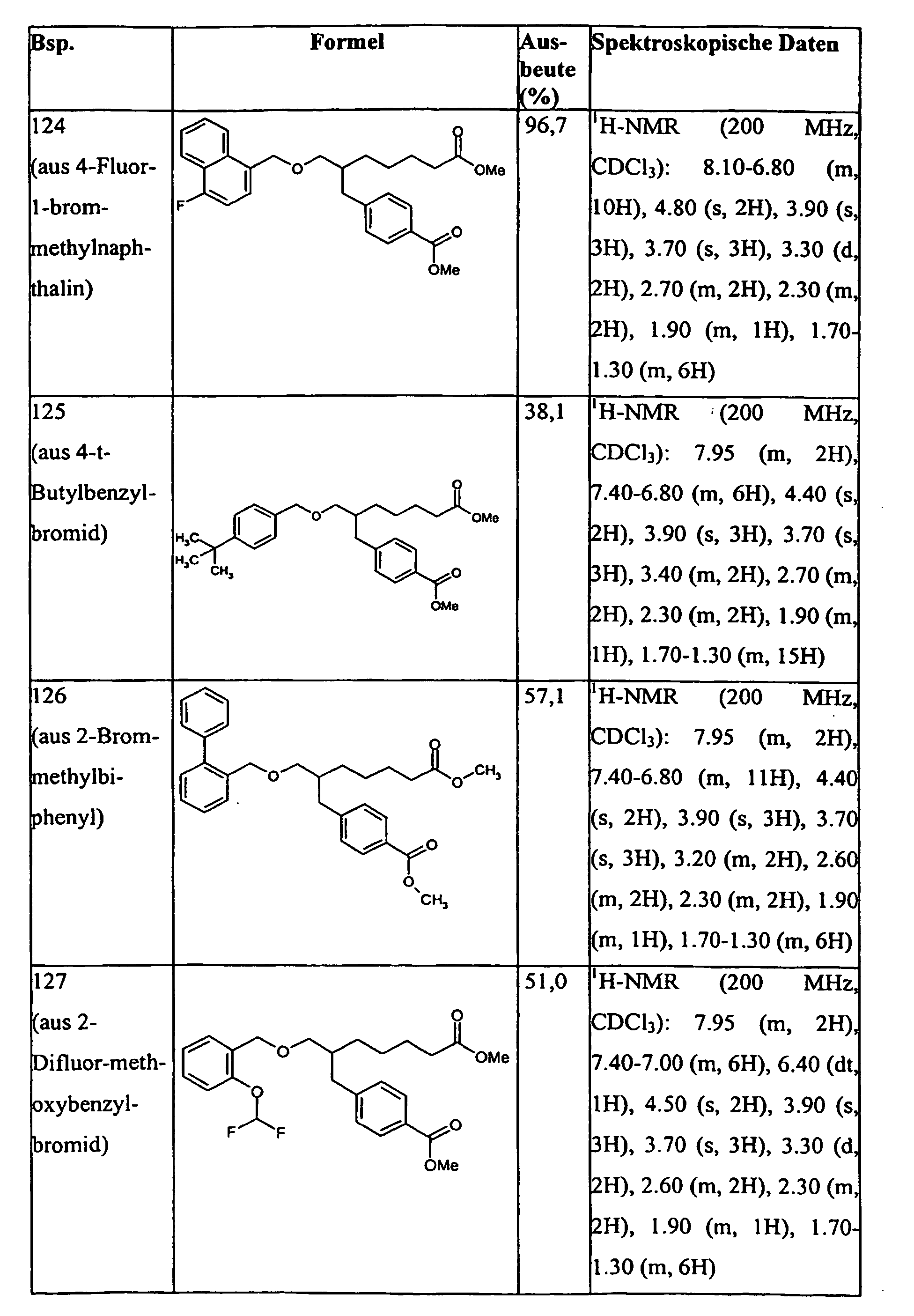

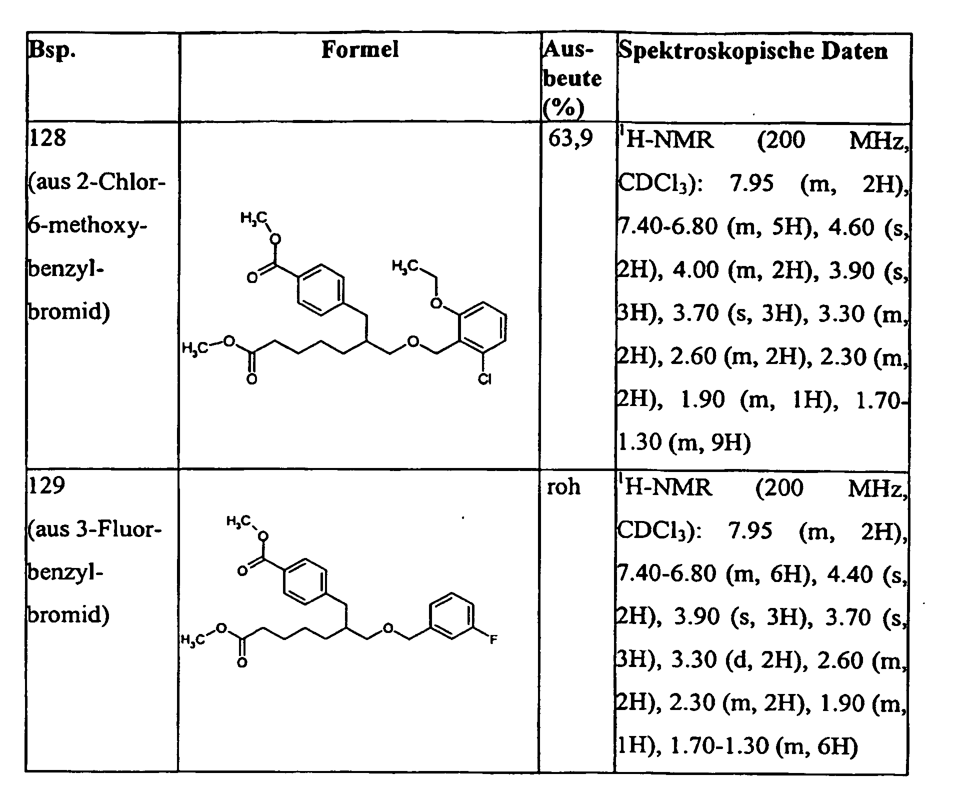

### 130: 6-(4-Carboxybenzyl)-7-{[2-(3-fluorphenyl)-1,3-benzothiazol-4-yl]methoxy}heptansäure

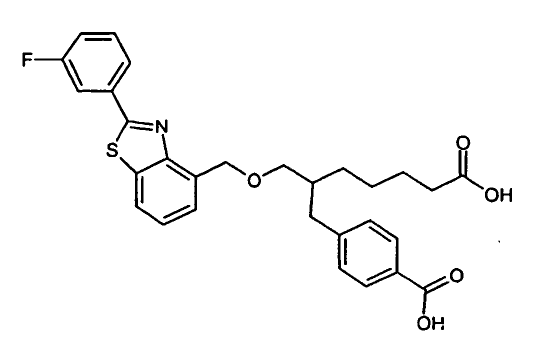

Der Diester aus Bsp. 121 wird in 5 ml Methanol gelöst und mit 0,8 ml 45%iger Natronlauge versetzt. Bei Raumtemperatur werden 0,3 ml Dichlormethan hinzugegeben. Nach 20 Stunden bei Raumtemperatur wird die Reaktionslösung einmal mit Ether gewaschen, mit 10%iger Schwefelsäure sauer gestellt, zweimal mit Essigester extrahiert, die vereinigten organischen Phasen über Extrelut filtriert und das Lösungsmittel im Vakuum eingedampft.
Ausbeute: 39,5 mg (38,5% d. Th.)
LC/MS: 522 (M+1), Rt=4.98 min

Analog werden die folgende Substanzen synthetisiert:

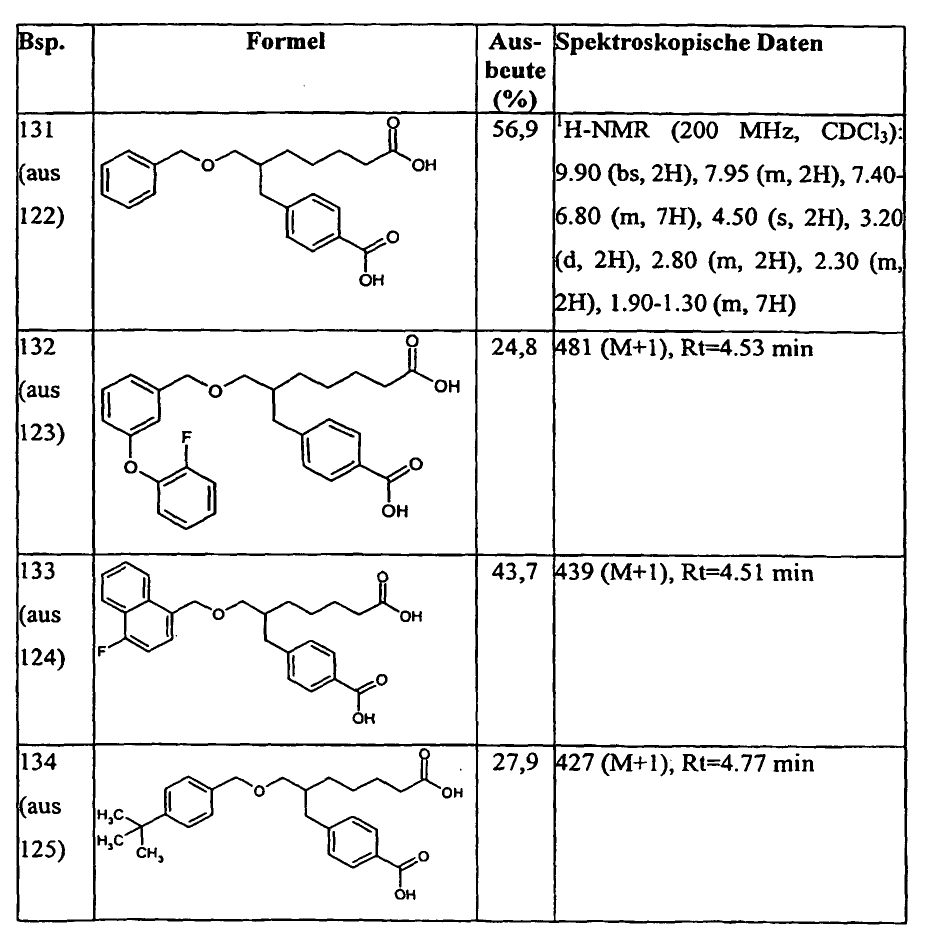

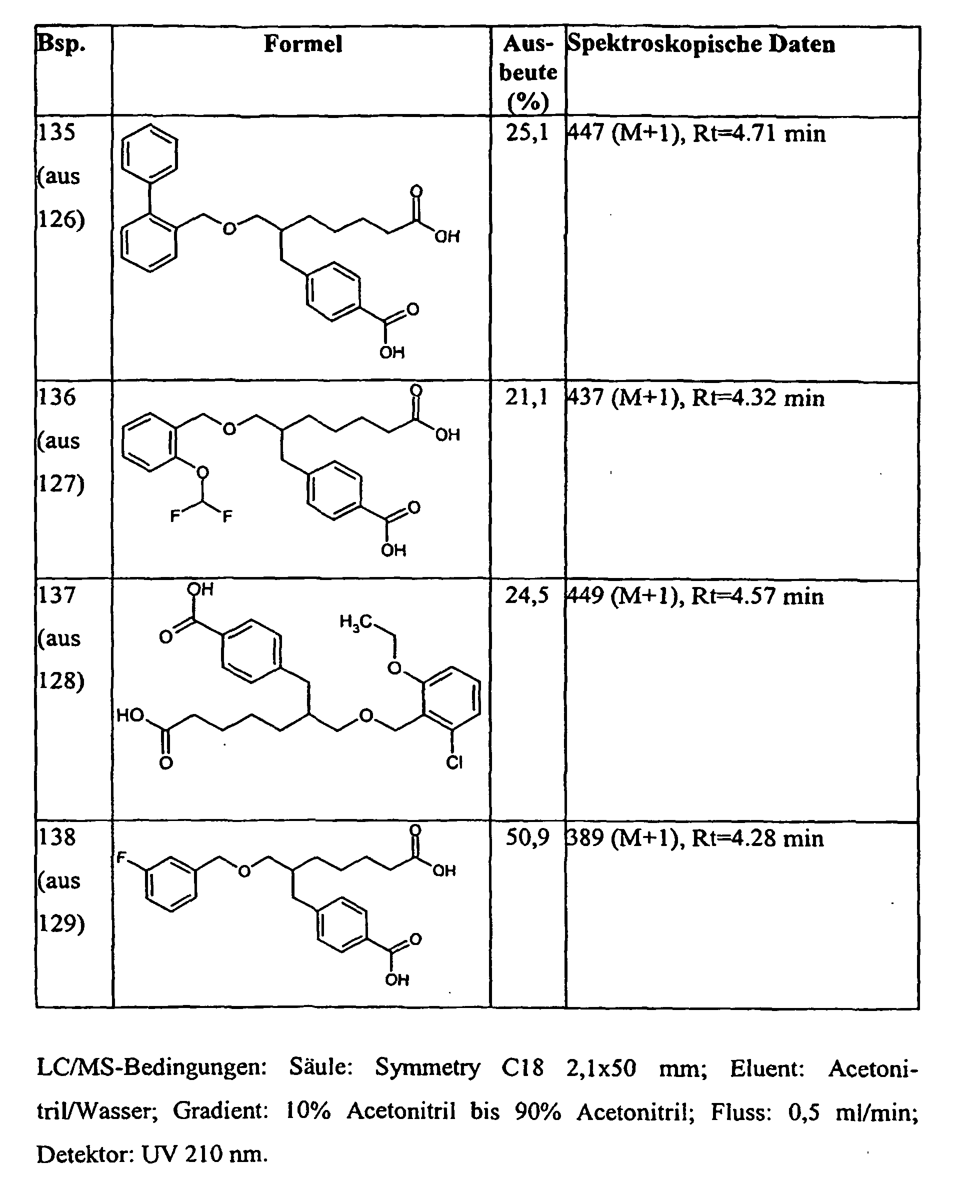

### 139: 6-(4-Carboxybenzyl)-7-(4-methoxyphenoxy)heptansäure

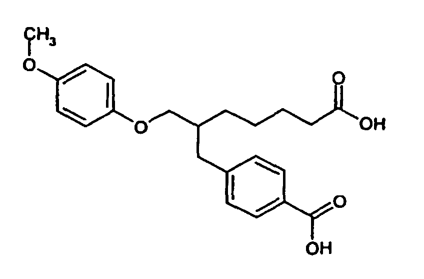

16,8 mg (0,14 mmol) 4-Methoxyphenol werden unter Argon in Dimethylformamid gelöst und bei Raumtemperatur mit 7,5 mg (0,19 mmol) Natriumhydrid (60%ig ölige Suspension) versetzt. Nach 30 Minuten Rühren bei dieser Temperatur wird eine Lösung von 41,6 mg (0,10 mmol) 7-Brom-6-(4-ethoxycarbonylbenzyl)heptansäureethylester (herstellbar aus 6-Hydroxymethyl-7-(4-methoxycarbonylphenyl)heptansäuremethylester (Synthese vgl. EP-A-0 341 551, S. 31, Bsp. 42) durch Umsetzung mit Bromierungsmitteln wie PBr₃) in DMF zugegeben. Das Reaktionsgemisch wird auf 60°C erhitzt. Nach 18 Stunden werden erneut 20 mg Natriumhydrid zugegeben und auf 100°C erhitzt. Nach 20 Stunden wird die Mischung abgekühlt, mit Wasser versetzt und mit Essigester gewaschen. Die wässrige Phase wird mit 1N Salzsäure auf pH 2 gestellt und zweimal mit Essigester extrahiert. Die organische Phase wird mit Magnesiumsulfat getrocknet und in Vakuum eingedampft.
Ausbeute: 24 mg (59,6 % d. Th.)
¹H-NMR (200 MHz, CDCl₃): 7,90 (m, 4H), 7,30 (m, 4H), 3,70 (s, 3H), 3,40 (m, 2H), 2,60 (m, 2H), 2,30 (m, 2H), 1,70-1,30 (m, 7H).

Analog wurden folgende Verbindungen hergestellt:

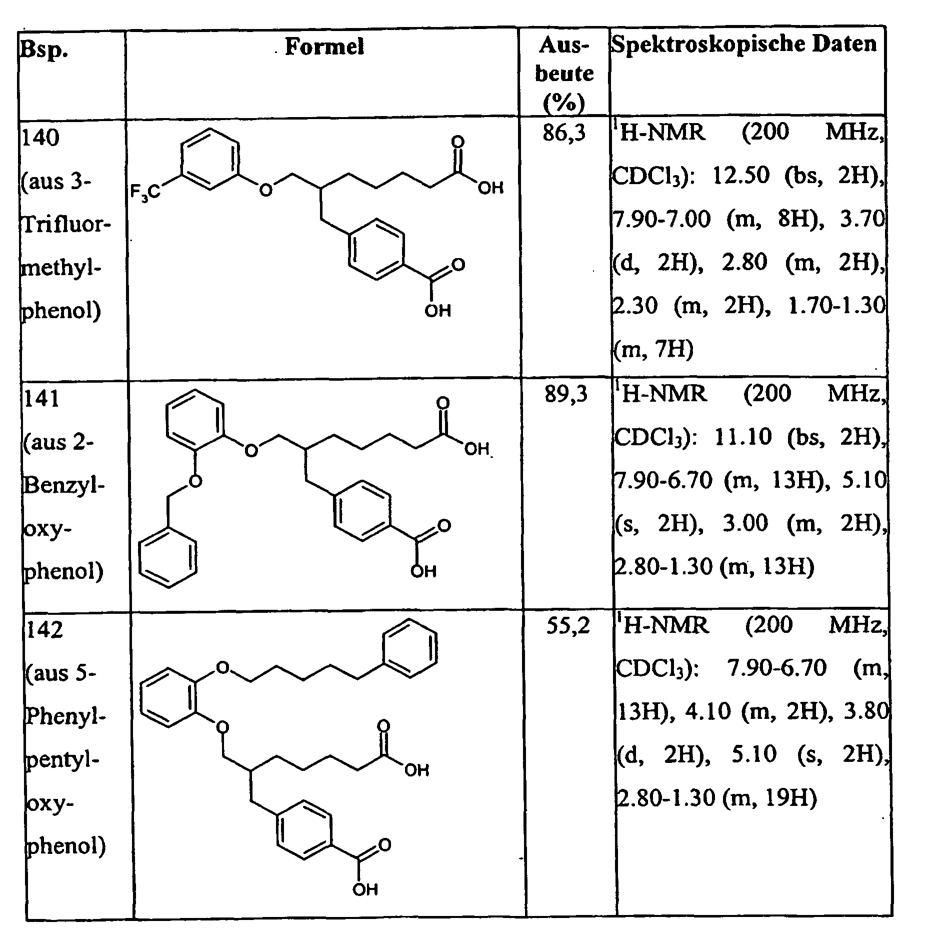

### 143: 7-Anilino-6-(4-methoxycarbonylbenzyl)heptansäuremethylester

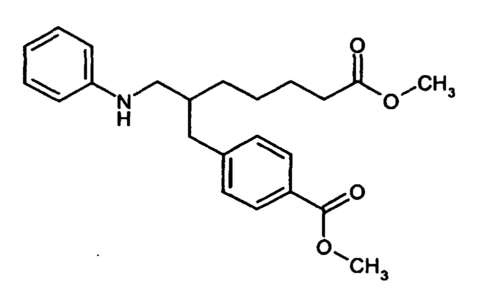

30,0 mg (0,33 mmol) Anilin werden in Dichlormethan gelöst, 0,02 ml Essigsäure und eine Lösung aus 90,6 mg (0,30 mmol) 6-Formyl-7-(4-methoxycarbonylphenyl)-heptansäureethylester (Synthese vgl. EP-A-0 341 551, S. 32, Bsp. 44) in Dichlormethan wird zugeben. Nach 30 Minuten bei Raumtemperatur wird die Lösung auf 0°C abgekühlt und 87,7 mg (0,41 mmol) Natriumtriacetoxyborhydrid zugegeben. Die Reaktionmischung wird 18 Stunden bei Raumtemperatur gerührt, 0,2 ml Wasser werden zugegeben und über Extrelut filtriert. Die Substanz wurde zur Reinigung an 10 g Kieselgel 60 (Korngröße 0,040-0,063mm) mit Cyclohexan/ Essigester 3:1 bis 1:1 als Eluenten chromatographiert.
Ausbeute: 52 mg (45,9 % d. Th.)
¹H-NMR (200 MHz, CDCl₃): 7,95 (m, 2H), 7,20 (m, 4H), 6,70 (m, 2H), 6,50 (d, 1H), 3,90 (s, 3H), 3,70 (s, 3H), 3,60 (bs, 1H), 3,00 (m, 2H), 2,70 (d, 2H), 2,30 (m, 2H), 2,00 (m, 1H), 1,70-1,30 (m, 6H).

Analog wurden die folgenden Verbindungen erhalten:

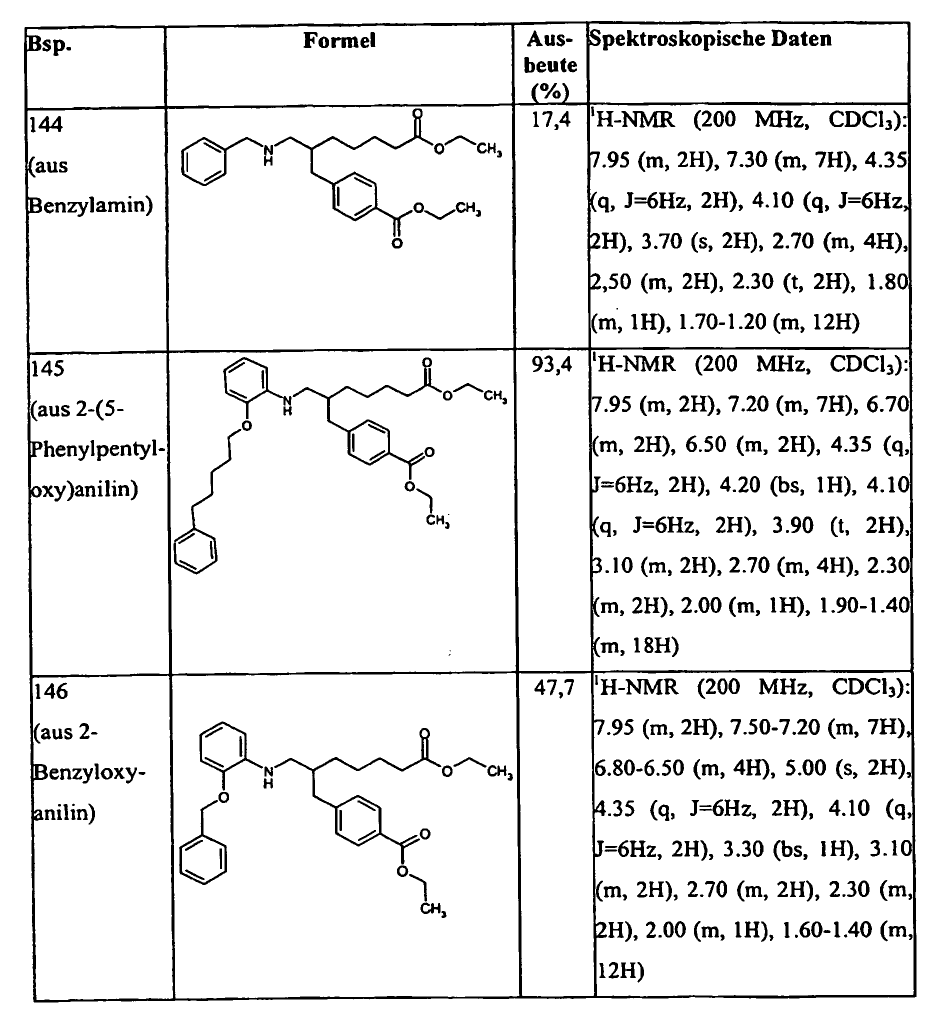

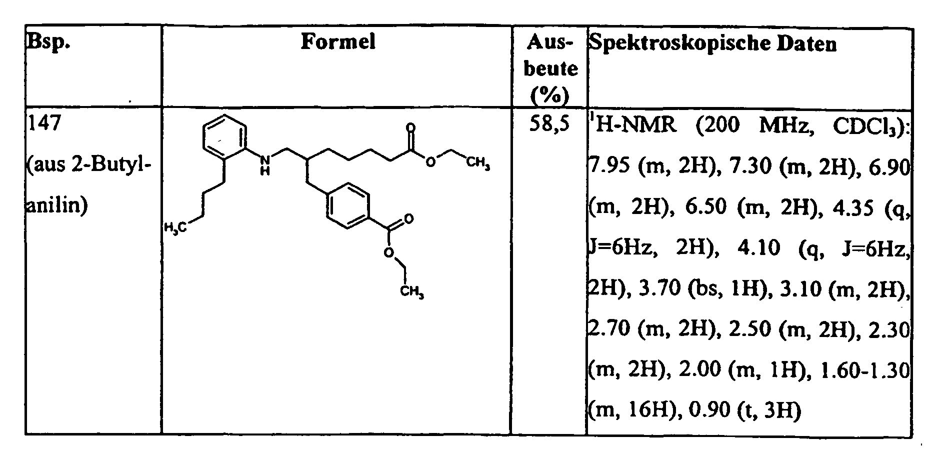

### 148: 7-Anilino-6-(4-carboxybenzyl)heptansäure

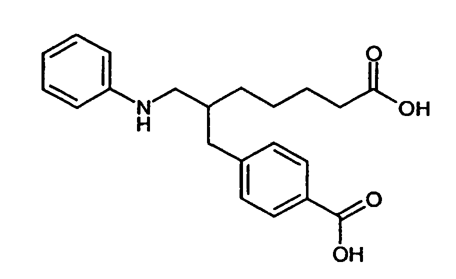

Die Substanz wird analog Beispiel 130 durch Verseifung des Esters aus Bsp. 143 hergestellt.

Ausbeute: 30,5 mg (74,8 d. Th.)
LC/MS: 356 (M+1), R 3,9 min

Analog wurden die folgenden Verbindungen erhalten:

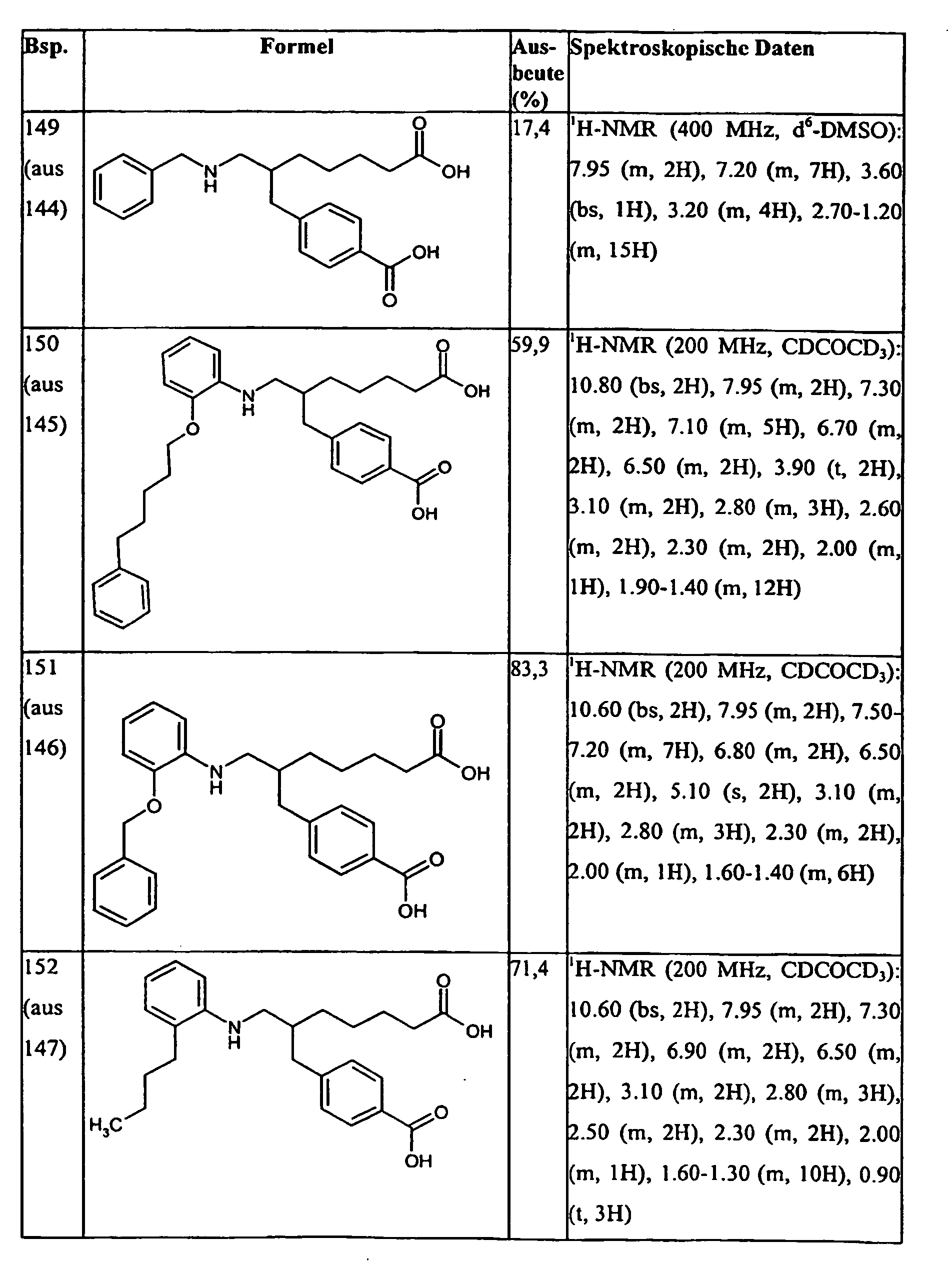

### 153: Methyl 4-((E/Z)-2-{2-[(2-ethoxy-2-oxoethyl)(methyl)amino]ethyl}-4-{2-[(5phenylpentyl)oxy]phenyl}-3-butenyl)benzoat

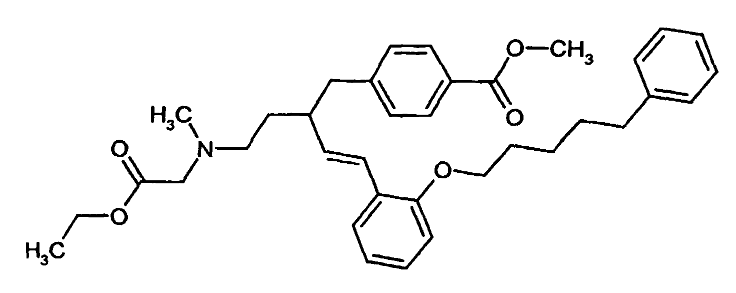

0,532 g (0,89 mmol) Triphenyl{2-[(5-phenylpentyl)oxy]benzyl}phosphoniumbromid (herstellbar analog Bsp. IId bis IVd mit 5-Phenylpentylbromid statt Butylbromid) werden in 10 ml THF suspendiert und bei -20°C mit 0,671 ml einer 1,6 M n-Butyllithiumlösung in n-Hexan versetzt. Man rührt 30 Minuten bei -20°C, woraufhin 0,300 g (0,89 mmol) Methyl 4-{4-[(2-ethoxy-2-oxoethyl)(methyl)amino]-2-formylbutyl}benzoat aus Bsp.XI, gelöst in 3 ml THF, zugesetzt werden. Man rührt 1 Stunde bei -20°C nach, setzt 20 ml Wasser zu und extrahiert mehrfach mit Ethylacetat. Die vereinigten organischen Phasen werden mit gesättigter Kochsalzlösung gewaschen, über Magnesiumsulfat getrocknet, und das Solvens wird unter vennindertem Druck abdestilliert.
Ausbeute: 192,1 mg (37,1 % der Theorie) E/Z Gemisch (85:15)
¹H-NMR (300 MHz, d⁶-DMSO): δ= 1,15 (t), 1,2-1,7 (m), 2,20 (s), 2,55 (t), 2,70 (m), 2,85 (m) 3,20 (s), 3,80 (s), 3,90 (m), 4,05 (q), 5,75 (s), 6,05 (dd), 6,35 (d), 6,90 (dd)7,1-7,4 (m), 7,85 (d).

### 154: 4-((E/Z)-2-{2-[(Carboxymethyl)(methyl)amino]ethyl}-4-{2-[(5-phenylpentyl)oxy]phenyl}-3-butenyl)benzoesäure

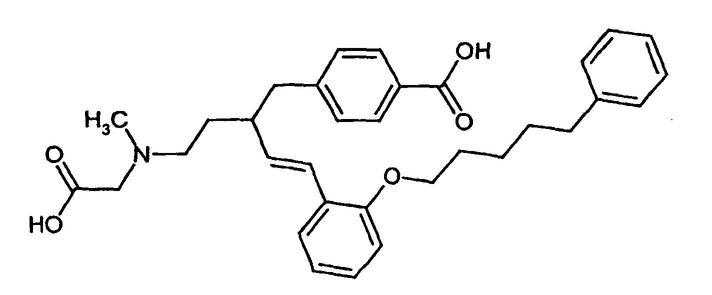

130 mg (0,230 mmol) Methyl-4-((E/Z)-2-{2-[(2-ethoxy-2-oxoethyl)(methyl)-amino]ethyl}-4-{2-[(5-phenylpentyl)oxy]phenyl}-3-butenyl)benzoat aus Bsp. 153 werden in 5 ml Methanol bei 0°C mit 1,2 ml 45 %-iger Natronlauge versetzt. Nach Erwärmen auf 22°C wird soviel Methylenchlorid zugesetzt, dass eine klare Lösung entsteht, und 18 Stunden nachgerührt. Die alkalische Lösung wird mit Wasser verdünnt und mit Methylenchlorid extrahiert. Hieraufhin wird die wässrige Pase zweimal mit 2N HCl auf pH 2-3 gestellt und mehrfach mit Methylenchlorid extrahiert. Die vereinigten organischen Phasen werden mit gesättigter Kochsalzlösung gewaschen, über Magnesiumsulfat getrocknet und das Lösungsmittel im Vakuum abdestilliert.
Ausbeute: 55,9 mg (45,1 % der Theorie) E/Z Gemisch (85:15)
¹H-NMR (300 MHz, d⁶-DMSO): δ= 1,05(d), 1,40 (m), 1,65 (m), 2,55 (m), 2,80 (m), 3,0 (m), 3,20 (s), 3,85 (m), 3,50 (s), 3,90 (m), 6,03 (dd), 6,45 (d), 6,90 (dd), 7,1-7,4 (m), 7,85 (d).

### 155: 4-((E/Z)-2-{2-[(2-methoxy-2-oxoethyl)sulfanyl]ethyl}-4-{2-[(5-phenylpentyl)-oxy]phenyl}-3-butenyl)benzoat

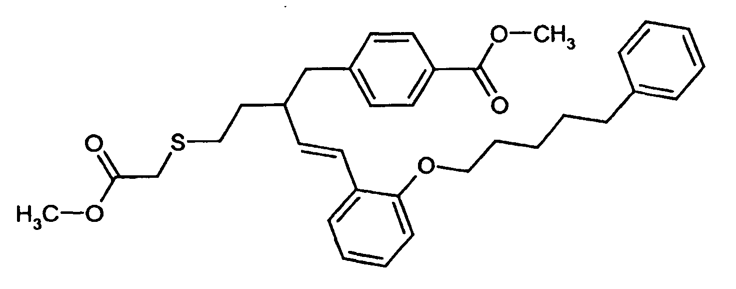

41,078 mg (1,03 mmol) Natriumhydrid (80%ig) werden in 5 ml THF vorgelegt und mit 104,32 mg (0,93 mmol) Mercaptoessigsäuremethylester versetzt. Nach 10 Minuten werden 500,0 mg (0,930 mmol) Methyl-4-((E/Z)-2-(2-bromoethyl)-4-{2-[(5-phenylpentyl)oxy]phenyl}-3-butenyl)benzoat aus Bsp. IX, gelöst in 2 ml THF, zugesetzt und 18 Stunden bei 22°C nachgerührt. Das Reaktionsgemisch wird vorsichtig mit 20 ml Wasser versetzt und mit Ethylacetat extrahiert. Die vereinigten organischen Phasen werden mit gesättigter Kochsalzlösung gewaschen, über Magnesiumsulfat getrocknet und das Lösungsmittel im Vakuum abdestilliert. Die Reinigung erfolgt an Kieselgel (0,04-0,063 nm) mit Methylenchlorid als Laufmittel.

Ausbeute: 300,10 mg (57,3 % der Theorie) E/Z-Gemisch (85:15)
¹H-NMR (300 MHz, d⁶-DMSO): δ= 1,40 (m), 1,65 (m), 7,26 (t), 2,70 (m), 2,85 (m), 3,55 (s), 3,80 (s), 3,9 (m), 6,0 (dd), 6,45 (dd), 6,90 (dd), 7,1-7,4 (m), 7,85 (d).

### 156: Methyl4-((E/Z)-2-{2-[(2-methoxy-2-oxoethyl)amino]ethyl}-4-{2-[(5-phenylpentyl)oxy]phenyl}-3-butenyl)benzoat

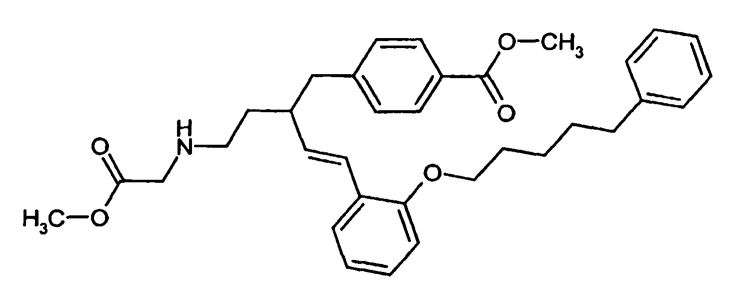

200,0 mg (0,34 mmol) Methyl-4-((E/Z)-2-(2-iodoethyl)-4-{2-[(5-phenylpentyl)-oxy]phenyl}-3-butenyl)benzoat aus Bsp. X, 43,107 mg (0,34 mmol) Glycinmethylesterhydrochlorid, 4,195 mg (0,03 mmol) 4-Dimethylaminopyridin und 0,50 ml Triethylamin werden in 2,0 ml Ethanol 48 Stunden unter Rückfluss erhitzt. Die Reaktionsmischung wird mit Wasser versetzt und mit Methylenchlorid extrahiert. Die organische Phase wird mit gesättigter Natriumchloridlösung gewaschen, über Magnesiumsulfat getrocknet und das Lösungsmittel im Vakuum abdestilliert. Der Rückstand wird an Kieselgel (0,04-0,063 nm) mit Methylenchlorid/Methanol 100:2 chromatographiert.

Ausbeute: 48,00 mg (25,7 % der Theorie) E/Z-Gemisch (85:15).
¹H-NMR (300 MHz, d⁶-DMSO): δ= 1,10 (t), 1,40 (m), 1,65 (m), 2,60 (m), 2,70 (m), 2,85 (m), 3,80 (s), 3,90 (m), 4,05 (q), 6,05 (dd), 6,35 (d), 6,85 (dd), 7,1-7,4 (m), 7,85 (d).

### 157: 4-((E/Z)-2-{2-[(Carboxymethyl)amino]ethyl}-4-{2-[(5-phenylpentyl)oxy]phenyl)-3-butenyl)benzoesäure

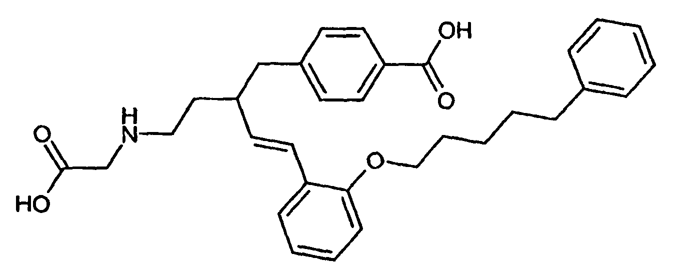

40,40 mg (0,070 mmol) Methyl-4-((E/Z)-2-{2-[(2-methoxy-2-oxoethyl)amino]-ethyl}-4-{2-[(5-phenylpentyl)oxy]phenyl}-3-butenyl)benzoat aus Bsp. 156 werden in 1,50 ml Methylenchlorid gelöst, mit 23,30 mg (0,16 mmol) Kaliumtrimethylsilanolat versetzt und 18 Stunden bei 22 °C nachgerührt. Die Lösung wird mit Wasser versetzt, mit 2N HCl auf pH 2 gestellt und mit Methylenchlorid/Methanol 2:1 extrahiert. Die organische Phase wird über Magnesiumsulfat getrocknet und das Lösungsmittel im Vakuum entfernt.
Ausbeute: 34,60 mg (86,3 % der Theorie) E/Z-Gemisch (85:15).
¹H-NMR (300 MHz, d⁶-DMSO): δ= 1,10 (t), 1,40 (m), 1,65 (m), 2,60 (m), 2,70 (m), 2,85 (m), 3,90 (m), 6,05 (dd), 6,35 (d), 6,85 (dd), 7,1-7,4 (m), 7,85 (d).

Analog der Beispiele 153 bis 157 wurden die folgenden Substanzen erhalten:

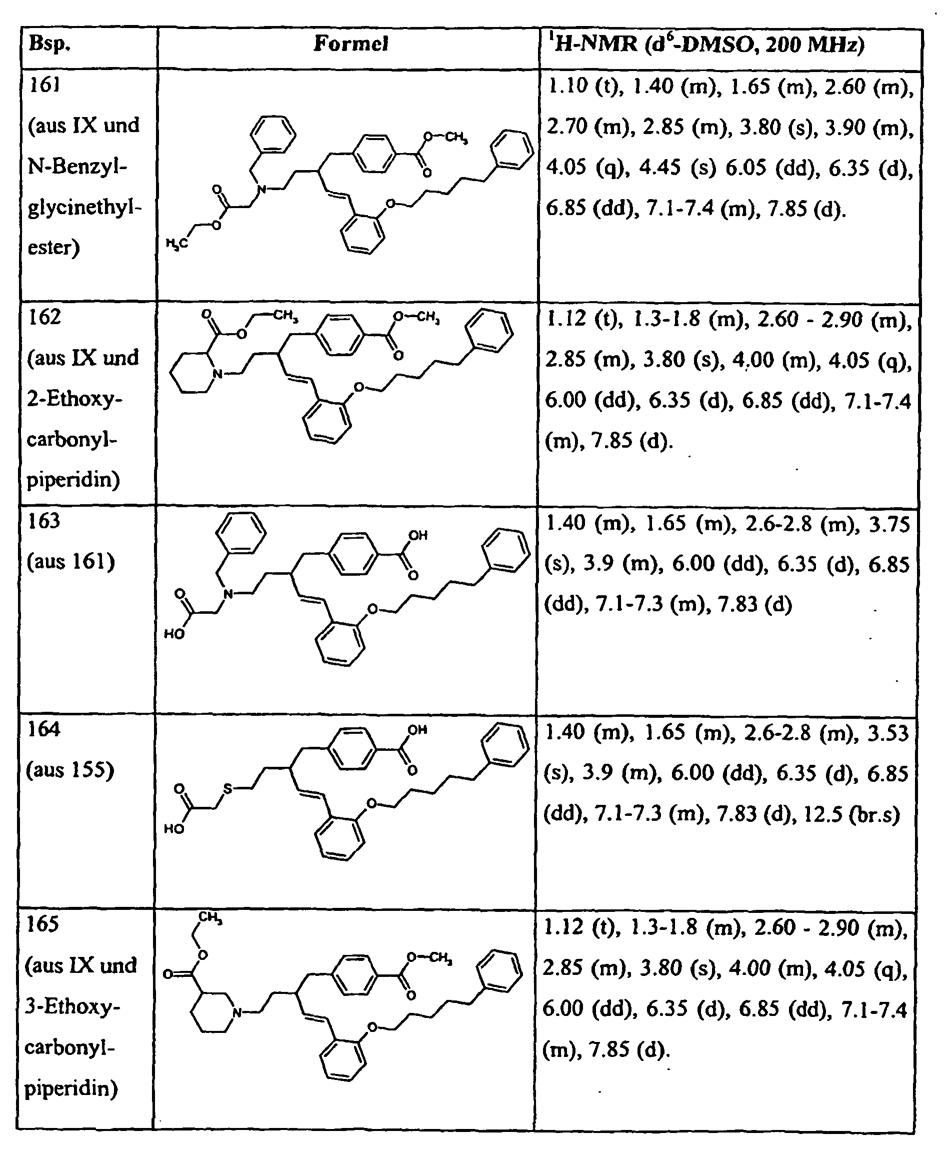

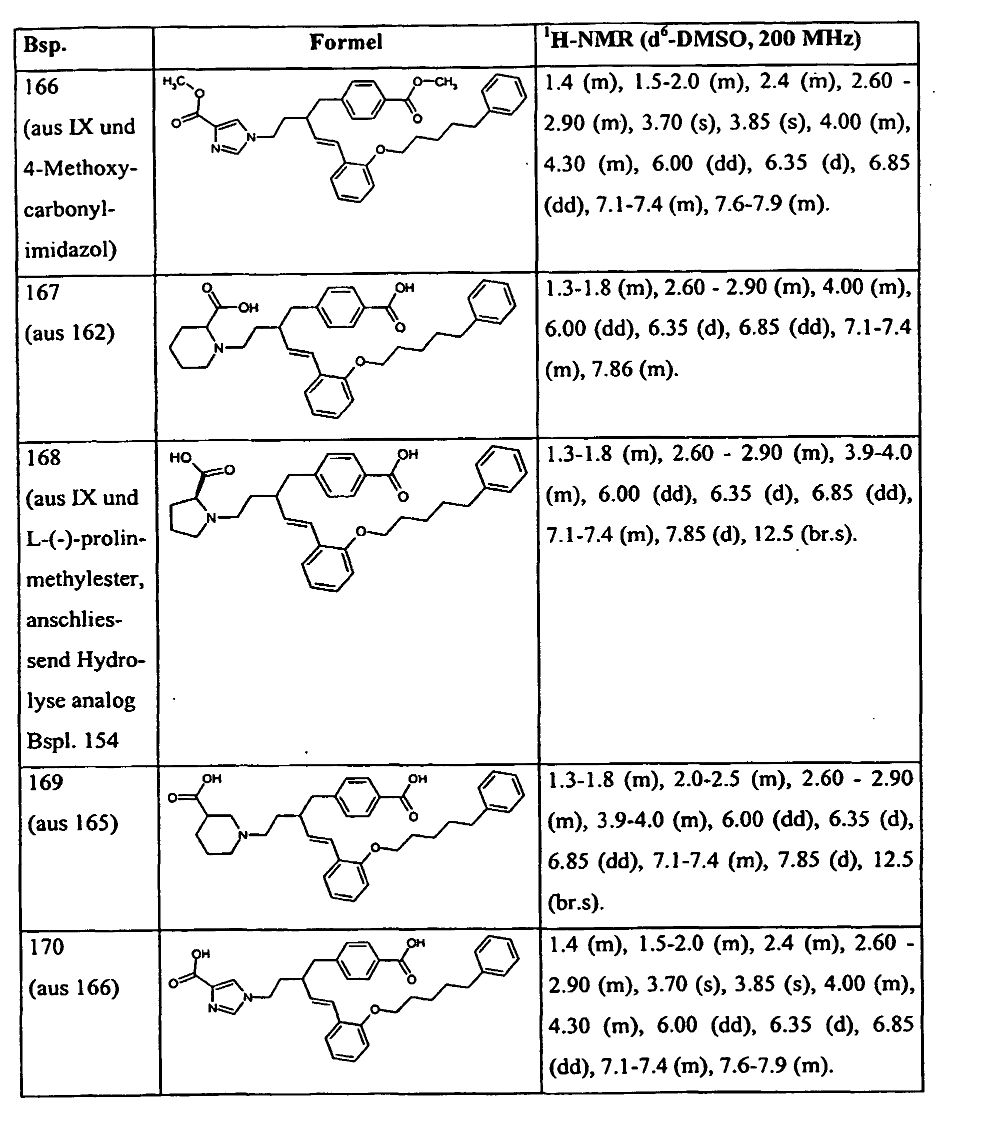

### Bsp. 171: 6-(4-Methoxycarbonylphenoxy)-8(2-(4-cyclohexylbenzyloxy)phenyl)-octansäuremethylester

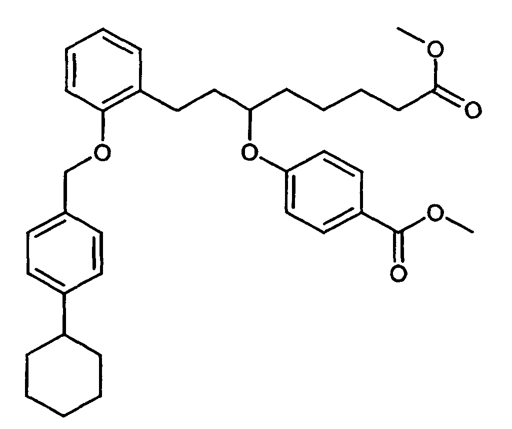

Die Synthese dieser Verbindung erfolgte analog Bsp. 47 aus der Verbindung aus Bsp. XII und 4-Cyclohexylbenzylchlorid.
Ausbeute: 81,1 %
¹H NMR (200 MHz, CDCl₃): δ = 1.14 - 2.08 (m, 20H), 2.39 - 2.97 (m, 3H), 3.63 (s, 3H), 3.87 (s, 3H), 4.29 (quint, *J* = 5.8 Hz, 1H), 5.00 (s, 2H), 6.68 - 6.97 (m, 4H), 7.03 - 7.37 (m, 6H), 7.89 (d, *J* = 8.7 Hz, 2H).

Analog wurde hergestellt:

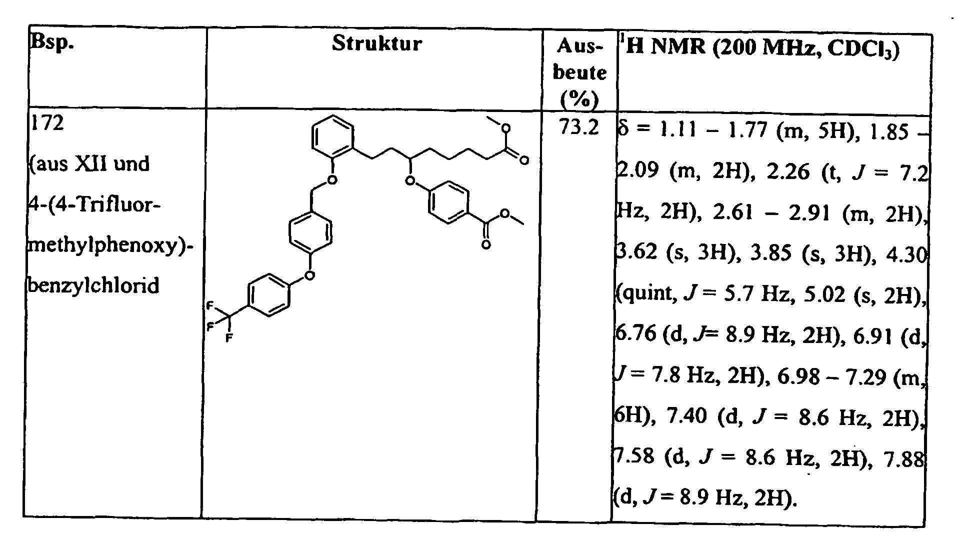

### Bsp. 173: 6-(4-Carboxyphenoxy)-8(2-(4-cyclohexylbenzyloxy)phenyl)-octansäure

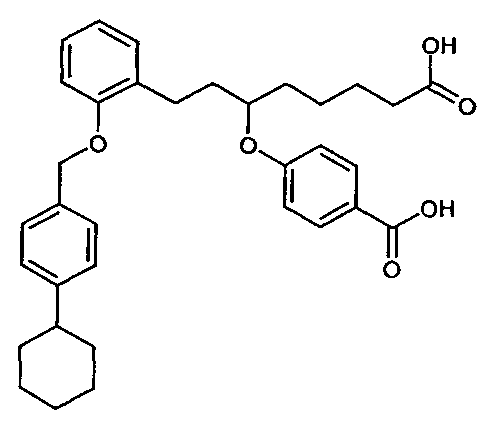

Die Synthese dieser Verbindung erfolgte analog Bsp. 19 aus der Verbindung aus Bsp. 171.
Ausbeute: 68,5 %
¹H NMR (400 MHz, CDCl₃): δ = 1.18 - 2.08 (m, 20H), 2.31 (t, *J* = 7.3 Hz, 2H)2.44 - 2.57 (m, 1H). 2.64 - 2.76 (m, 1H), 2.76 - 2.88 (m, 1H), 4.33 (quint, *J* = 5.8 Hz, 1H), 4.99 (s, 2H), 6.79 (d, *J* = 8.8 Hz, 2H), 6.82 - 6.94 (m, 2H), 7.05 - 7.34 (m, 6H), 7.94 (d, *J* = 8.8 Hz, 2H).

Analog wurde hergestellt:

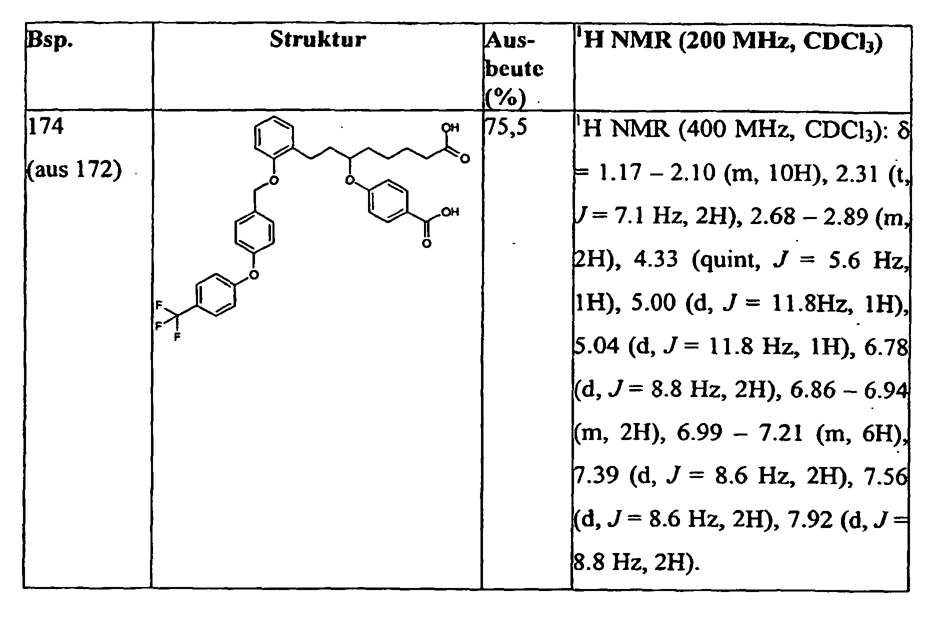

### 175: 4-[(3E)-2-(5-Ethoxy-5-oxopentyl)-4-(2-{[4-(4-morpholinyl)-benzyl]oxy}phenyl)-3-butenyl]benzoesäureethylester

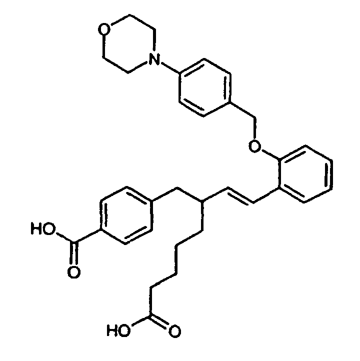

57,0 mg (0,10 mmol) der Verbindung 18a werden in 2 ml Toluol vorgelegt und nacheinander mit 11 mg (0,12 mmol) Morpholin, 23 mg (0,24 mmol) Natrium-*tert*-butylat und 3 mg (0,01 mmol) Tri-*tert*-butylphosphin versetzt. Nach Zugabe von 5,0 mg Tris(dibenzylidenaceton)dipalladium (0) wird unter Argon 18 Stunden bei 100°C erhitzt. Die Reaktionslösung wird abgekühlt, Toluol und Wasser wird zugegeben und die Mischung wird über Extrelut filtriert und das Lösungsmittel im Vakuum abdestilliert. Das Rohprodukt wird über Kieselgel mit Cyclohexan/Ethylacetat=4:1 als Laufmittel chromatographiert. Der erthaltene Diester wird analog Bsp. 109 verseift.
Ausbeute: 16 mg (28 %)
MS: 544 (M+1)

Analog wurden hergestellt:

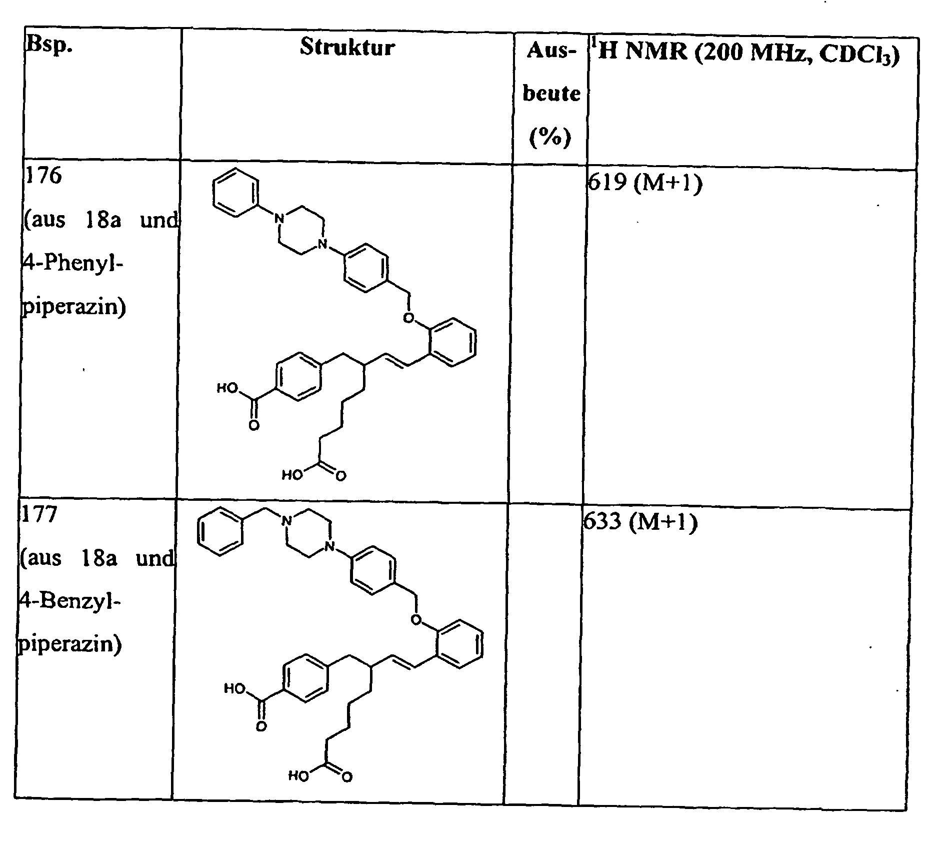

### 178: 4-((3E)-2-(4-carboxybutyl)-4-{2-[(4'-methyl-1,1'-biphenyl-4-yl)methoxy]-phenyl}-3-butenyl)benzosäure

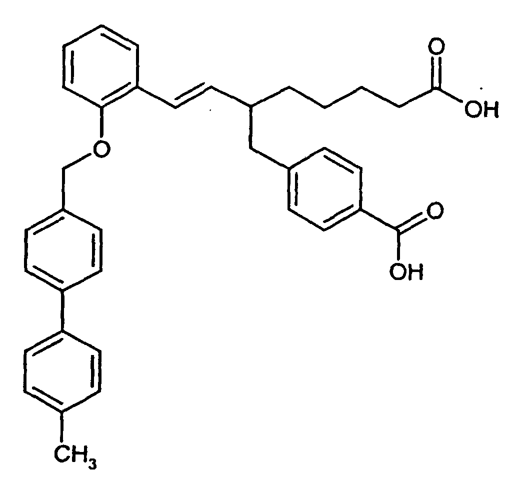

100,0 mg (0,17 mmol) 18a werden in 3 ml Dimethoxyethan vorgelegt und nacheinander mit 28 mg (0,2 mmol) 4-Methylphenylboronsäure und 0,2 ml 2M Natriumcarbonatlösung versetzt. Nach Zugabe von 5,0 mg Dichlorobis(triphenylphosphin)palladium(II) wird 18 Stunden auf Rückflußtemperatur erhitzt. Die Reaktionslösung wird abgekühlt, Dichlormethan und Wasser wird zugegeben und die Mischung wird über Extrelut filtriert und das Lösungsmittel im Vakuum abdestilliert. Das Rohprodukt wird über Kieselgel mit Cyclohexan/Ethylacetat=10.1 als Laufmittel chromatographiert. Der erhaltene Diester wird analog Bsp. 19 verseift.
Ausbeute: 80 mg (86 %)
¹H-NMR (200 MHz, CD₃COCD₃): 7.95 (m, 4H), 7.40-7.10 (m, 16H), 6.52 (m, 1H), 6.05 (m, 1H), 5.00 (m, 2H), 2.75 (m, 2H), 2.45 (m, 1H), 2.30 (s, 3H), 2.25-1.10 (m) ¹H-NMR (200 MHz, CD₃COCD3): 7.95 (m, 4H), 7.40-7.10 (m, 16H), 6.52 (m, 1H), 6.05 (m, 1H), 5.00 (m, 2H), 2.75 (m, 2H), 2.45 (m, 1H), 2.30 (s, 3H), 2.25-1.10 (m)

Analog werden hergestellt:

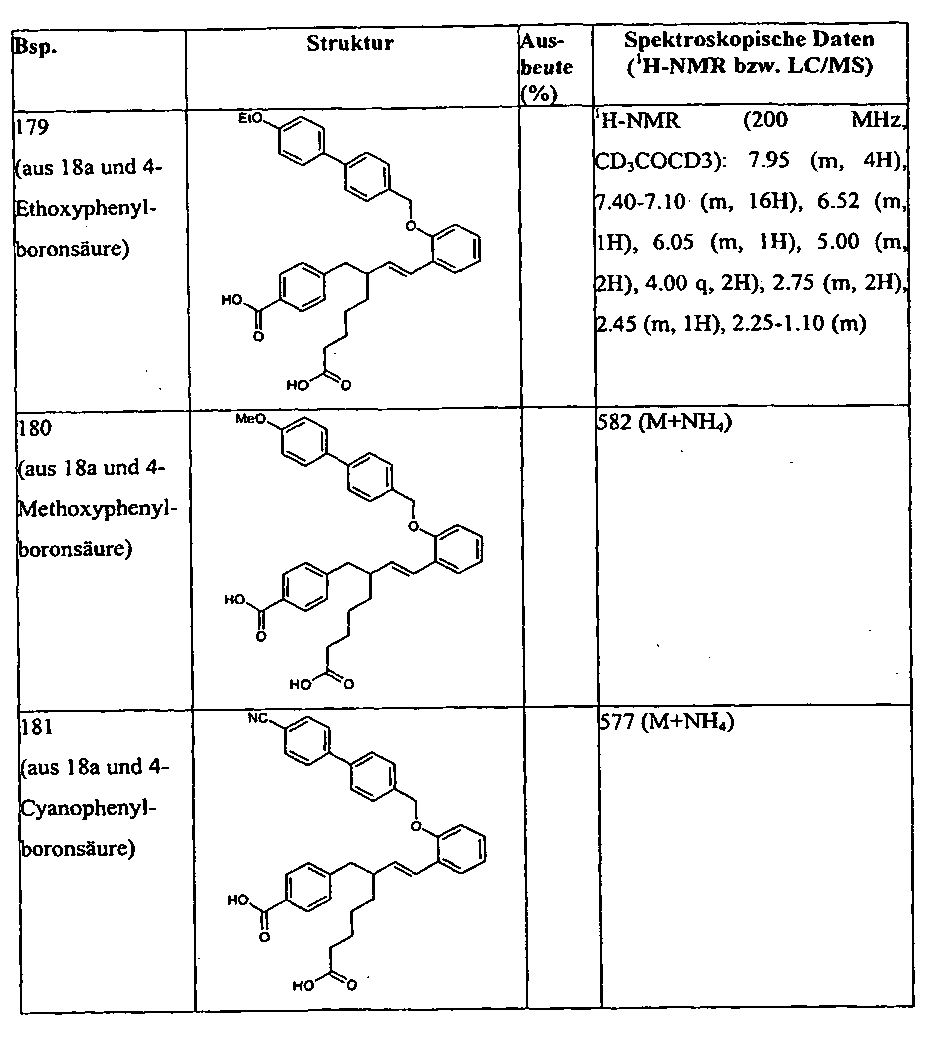

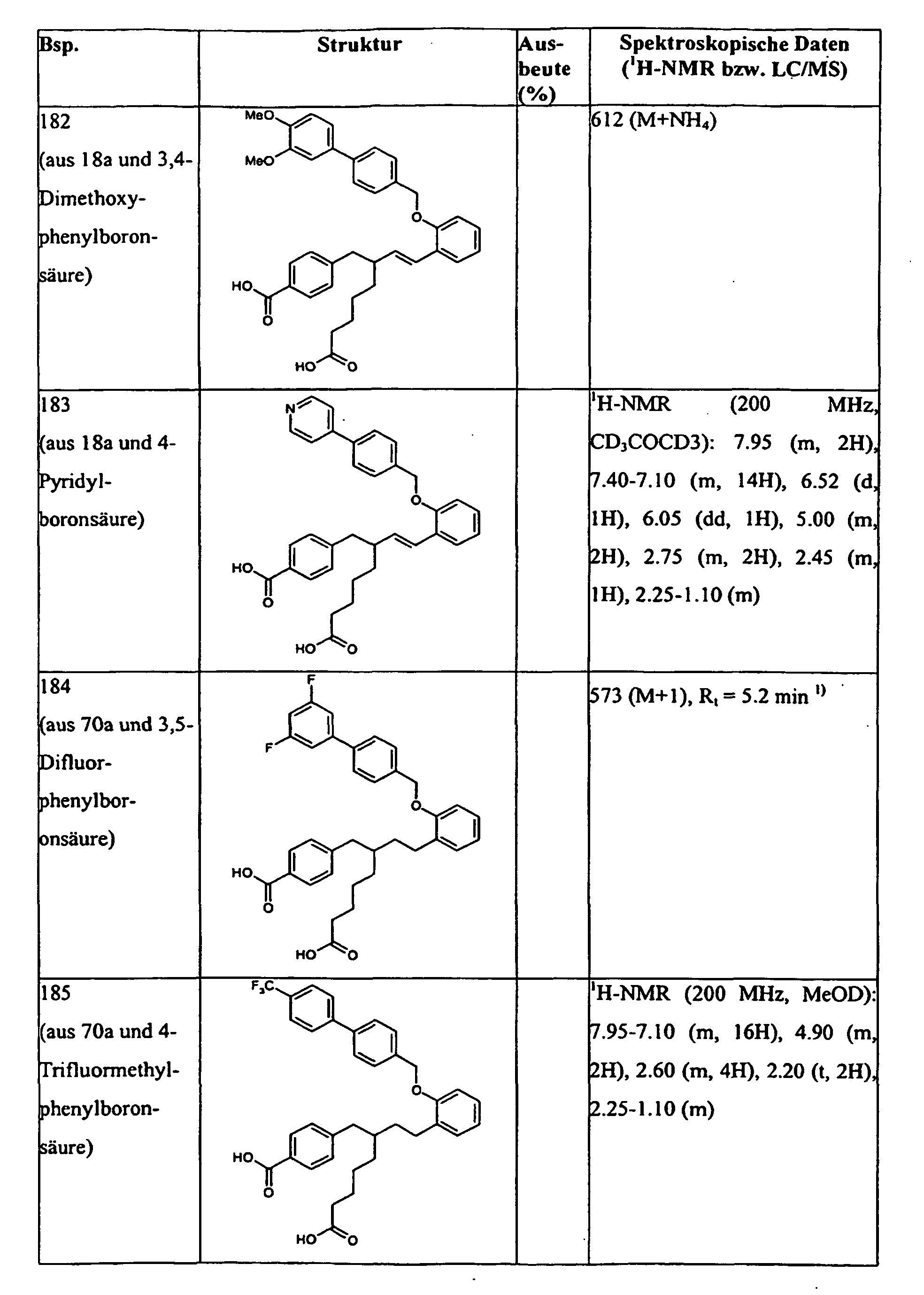

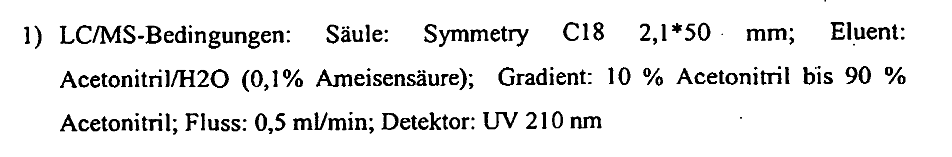

### 186: 4-{[1-(2-{2-[(4-cyclohexylbenzyl)oxy]phenyl}ethyl)-6-methoxy-6-oxohexyl]amino}benzoesäuremethylester

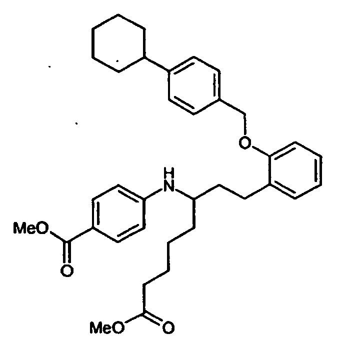

Zu einer Lösung von 500 mg (1,15 mmol) 8-{2-[(4-cyclohexylbenzyl)oxy]phenyl}-6-oxooctansäuremethylester XIV und 190 mg (1,26 mmol) 4-Aminobenzoesäuremethylester in 12,5 ml 1,2-Dichlorethan wurden bei 0°C 217 mg (1,15 mmol) TiCl₄ (1 M in CH₂Cl₂) zugegeben. Man rührte 20 min bei Raumtemperatur und gab dann 383 mg (1,72 mmol) Natriumtriacetoxyborhydrid zu. Man kontrollierte den Reaktionsverlauf per Dünnschichtchromatografie und versetzte nach beendeter Reaktion mit Wasser. Man extrahierte mit Ethylacetat und trocknete die vereinten organischen Phasen über Na₂SO₄. Das Produkt wurde chromatografisch gereinigt (Kieselgel, Gradient Cyclohexan/Ethylacetat 10:1 bis 0:100).
Ausbeute: 320 mg (48,7 %)
¹H NMR (200 MHz, CDCl₃): δ = 1.07 - 2.00 (m, 16H), 2.25 (t, *J* = 7.2 Hz, 2H), 2.41 - 2.68 (m, 2H), 2.72 - 2.91 (m, 1H), 3.10 - 3.28 (m, 1H), 3.32 - 3.51 (m, 1H), 3.63 (s, 3H), 3.73 - 3.93 (m, 2H), 3.83 (s, 3H), 4.98 (s, 2H), 6.28 (d, *J* = 8.8 Hz, 2H), 6.54 (d, *J* = 8.8 Hz, 1H), 6.81 - 6.97 (m, 2H), 7.05 - 7.39 (m, 5H), 7.76 (d, *J* = 8.8 Hz, 2H).

### 187: 4-{[1-(2-{2-[(4-cyclohexylbenzyl)oxy]phenyl}ethyl)-6-methoxy-6-oxohexyl]sulfanyl}benzoesäuremelhylester

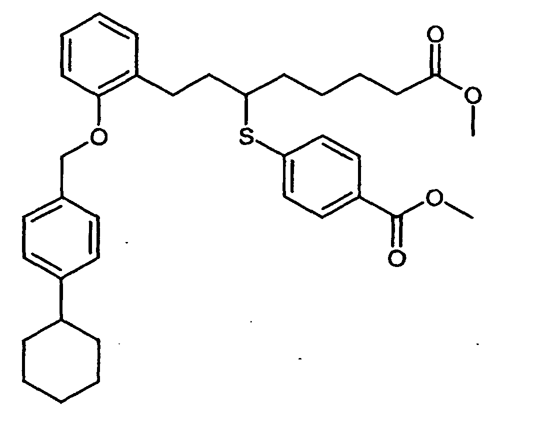

Eine Suspension von 0,30 g (0,60 mmol) 6-Bromo-8-(2-[(4-cyclohexylbenzyl)-oxy]phenyl}-octano-säuremethylester XVII, 0,15 g (0,90 mmol) 4-Sulfanylbenzoesäuremethylester und 0.17 g (1.20 mmol) Kaliumcarbonat in 15 ml DMF wurde 2 Tage bei Raumtemperatur gerührt. Die Mischung wurde mit 1 N NaOH versetzt. Man extrahierte mit Diethylether, trocknete die vereinten organischen Phasen über NaSO₄ und entfernte das Lösungsmittel. Das Produkt wurde chromatografisch gereinigt (Kieslegel, Cyclohexan/Ethylacetat 15:1).
Ausbeute: 0,17 g (50,5 %).
¹H NMR (300 MHz, CDCl₃): δ =1.20 - 1.98 (m, 22H), 2.23 (t, *J* = 7.2 Hz, 2H), 2.42 - 2.56 (m, 1H), 2.72 - 2.92 (m, 2H), 3.23 (quint, *J* = 3.2 Hz, 1H), 3.64 (s, 3H), 3.89 (s, 3H), 5.00 (s, 2H), 6.82 - 6.94 (m, 2H), 7.06 - 7.35 (m, 4H), 7.83 (d, *J* = 8.3 Hz, 2H).

### 188: 4-{[1-(2-{2-[(4-Cyclohexylbenzyl)oxy]phenyl}ethyl)-6-methoxy-6-oxohexyl]-sulfinyl}-benzoesäuremethylester

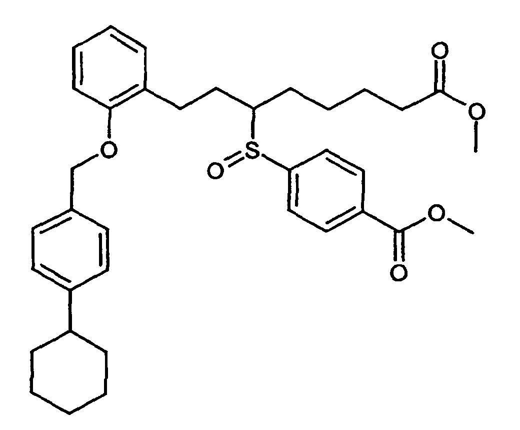

Zu einer Lösung von 113 mg (0,19 mmol) 4-{[1-(2-{2-[(4-Cyclohexylbenzyl)-oxy]-phenyl}-ethyl)-6-methoxy-6-oxohexyl]sulfanyl}benzoesäuremethylester 187 in 25 ml CH₂Cl₂ wurden bei 0°C 47 mg (0,19 mmol) Metachlorperbenzoesäure zugegeben. Man rührte 30 min bei 0°C, entfernte danach das Kühlbad und rührte weitere 16 h bei Raumtemperatur. Nach beendeter Reaktion wurde sukzessiv mit gesättigter Na₂SO₃-Lösung, gesättigter Na₂CO₃-Lösung, gesättigter NaCl-Lösung und Wasser gewaschen. Die organische Phasen wurden über Na₂SO₄ getrocknet und das Lösungsmitel entfernt. Das Produkt wurde chromatografisch gereinigt (Kieselgel, Cyclohexan/Ethylacetat 2:1)
Ausbeute: 62 mg (53.4 %).
Diastereomerengemisch dr = 55 : 45
¹H NMR (400 MHz, CDCl₃): δ = 1.17 - 1.62 (m, 10H), 1.70 - 1.93 (m, 7H), 2.09 (t, *J* = 7.3 Hz, 2H), 2.24 (t, *J* = 7.1 Hz, 1H), 2.44 - 2.61 (m, 3H), 2.77 - 2.94 (m, 1H), 3.61 (s, 3H, Dia-1), 3.66 (s, 3H, Dia-2), 3.94 (s, 3H), 4.87 (d, *J=* 16.1 Hz, 1H, Dia-2), 4.90 (d, *J =* 16.3 Hz, 1H, Dia-1), 5.01 (s, 2H, Dia-2), 6.77 - 6.95 (m, 2H), 7.08 - 7.34 (m, 6H), 7.46 (d, *J =* 8.6 Hz, 2H, Dia-2), 7.53 (d, *J =* 8.6 Hz, 2H, Dia-1), 8.01 (d, *J =* 8.3 Hz, 2H, Dia-2), 8.09 (d, *J* = 8.3 Hz, 2H, Dia-1).

### 189: 4-{[1-(2-{2-[(4-Cyclohexylbenzyl)oxy]phenyl}ethyl)-6-methoxy-6-oxohexyl]-sulfonyl}benzoesäuremethylester

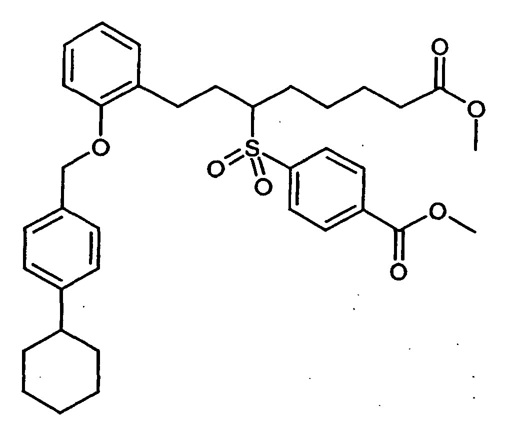

Zu einer Lösung von 113 mg (0,19 mmol) 4-{[1-(2-{2-[(4-Cyclohexylbenzyl)-oxy]-phenyl}-ethyl)-6-methoxy-6-oxohexyl]sulfanyl}benzoesäuremethylester in 25 ml CH₂Cl₂ wurden bei 0°C 149 mg (0,86 mmol) Metachlorperbenzoesäure zugegeben. Man rührte 16 h bei Raumtemperatur. Nach beendeter Reaktion wurde sukzessiv mit gesättigter Na₂SO₃-Lösung, gesättigter Na₂CO₃-Lösung, gesättigter NaCl-Lösung und Wasser gewaschen. Die organische Phasen wurden über Na₂SO₄ getrocknet und das Lösungsmitel entfernt. Das Produkt wurde chromatografisch gereinigt (Kieselgel, Cyclohexan/Ethylacetat 2: 1)
Ausbeute: 110 mg (92,3 %).
¹H NMR (400 MHz, CDCl₃): δ = 1.17 - 1.66 (m, 10H), 1.71 - 1.93 (m, 7H), 2.02 - 2.12 (m, 1H), 2.15 (t, *J* = 7.8 Hz, 2H), 2.46 - 2.56 (m, 1H), 2.58 - 2.69 (m, 1H), 2.70 - 2.81 (m, 1H), 2.90 - 2.99 (m, 1H), 3.64 (s, 3H), 3.96 (s, 3H), 4.91 (d, *J* = 13.5 Hz, 1H), 4.94 (d, *J* = 13.5 Hz, 1H), 6.81 - 6.89 (m, 2H), 6.99 - 7.05 (m, 1H), 7.12 - 7.31 (m, 5H), 7.83 (d, *J* = 8.3 Hz, 2H), 8.10 (d, *J* = 8.5 Hz, 2H).

### 190: 8-(2-(3-Brompropyloxy)-phenyl)-6-(4-(methoxycarbonylphenoxy)-octansäuremethylester

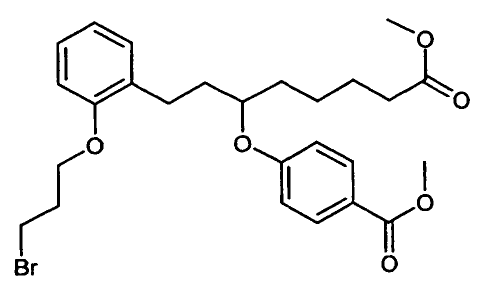

Diese Verbindung wurde analog zur Vorgehensweise bei Beispiel IId) aus der Verbindung aus Beispiel XII) und 1,3-Dibrompropan hergestellt.
Ausbeute: 68,9 %
¹H NMR (200 MHz, CDCl₃): δ = 1.32 - 1.80 (m, 6H), 1.84 - 2.01 (m, 2H), 2.13 - 2.36 (m, 4H), 2.55 - 2.84 (m, 2H), 3.54 (t, *J* = 6.3 Hz, 2H), 3.64 (s, 3H), 3.88 (s, 3H), 4.05 (t, *J* = 5.6 Hz, 2H), 4.32 (quint, *J* = 5.7 Hz, 1H), 6.74 - 6.91 (m, 4H), 7.00 - 7.22 (m, 2H), 7.94 (d, *J* = 8.8 Hz, 2H).

Die folgenden Beispiele wurden aus Bsp. 190 und den entsprechenden Aminen analog zur Vorgehensweise bei Beispiel 97 hergestellt:

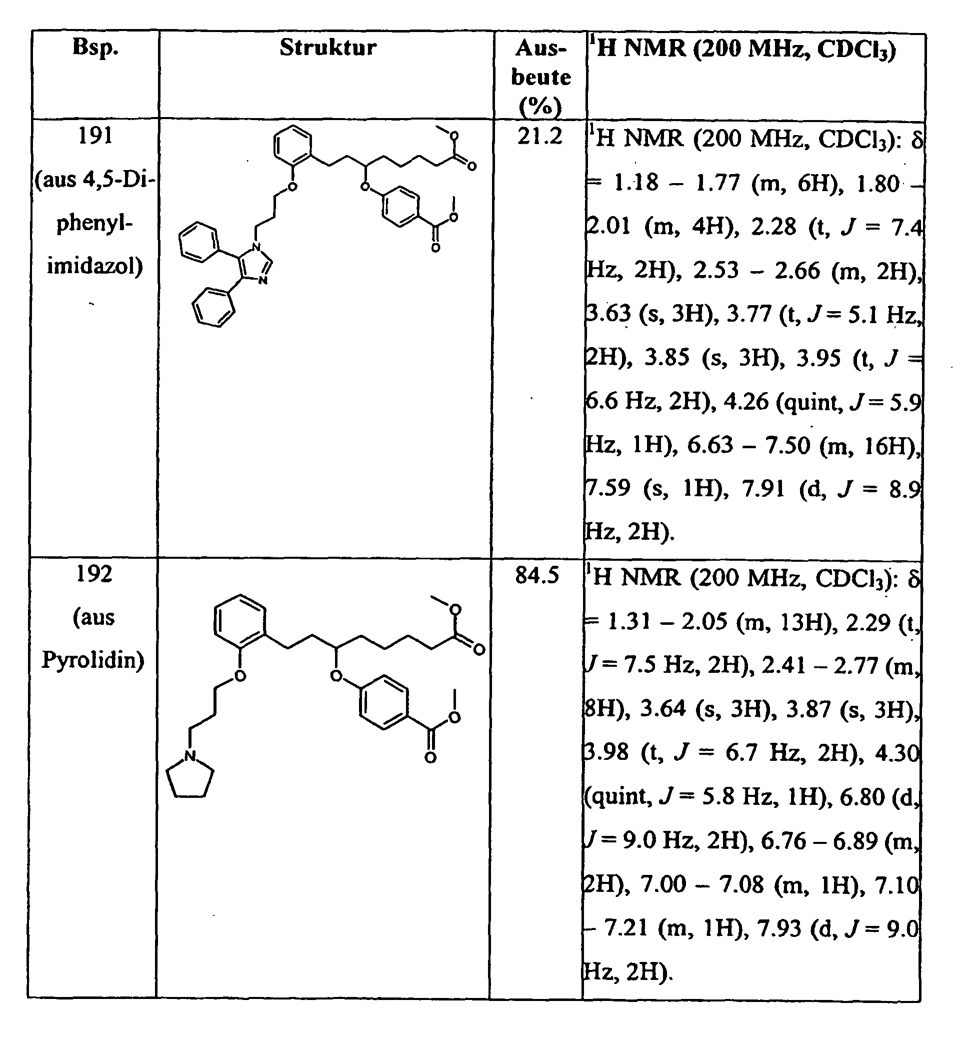

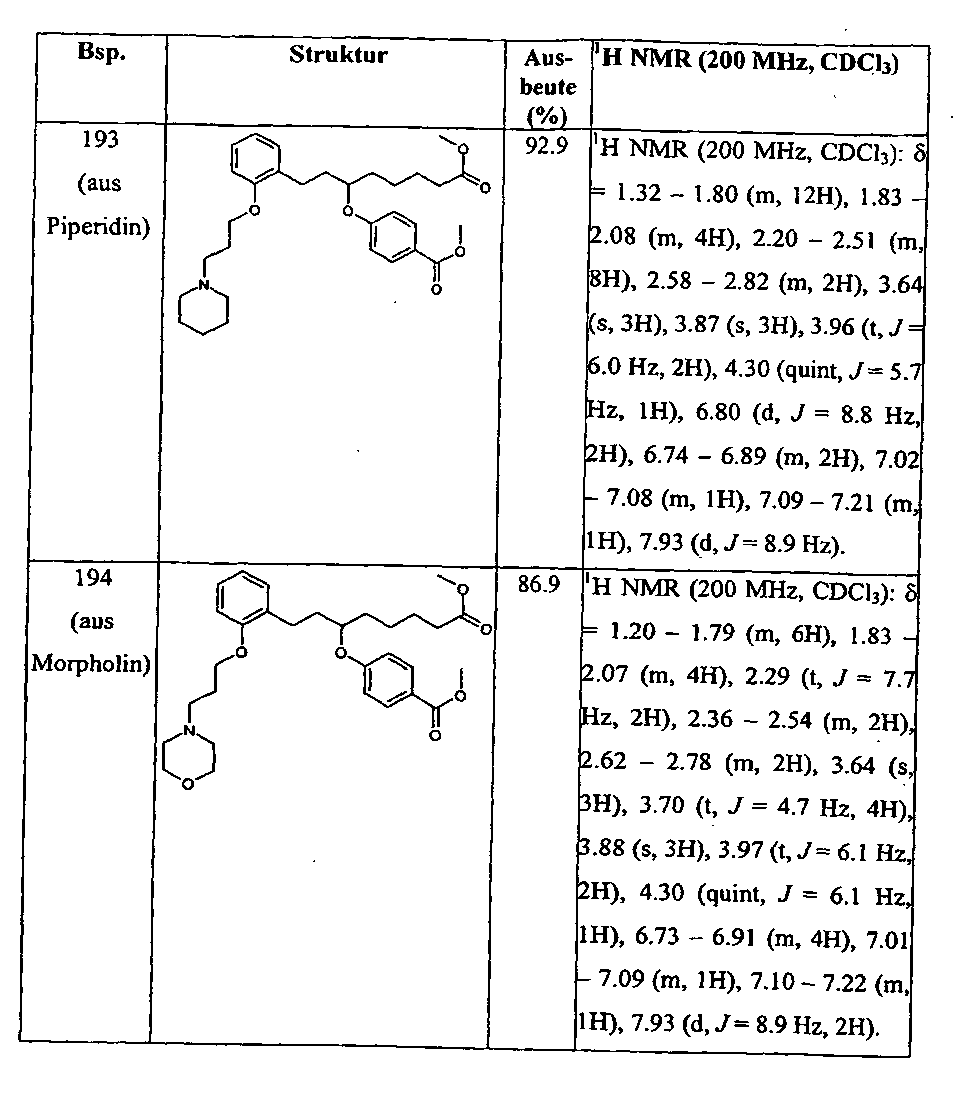

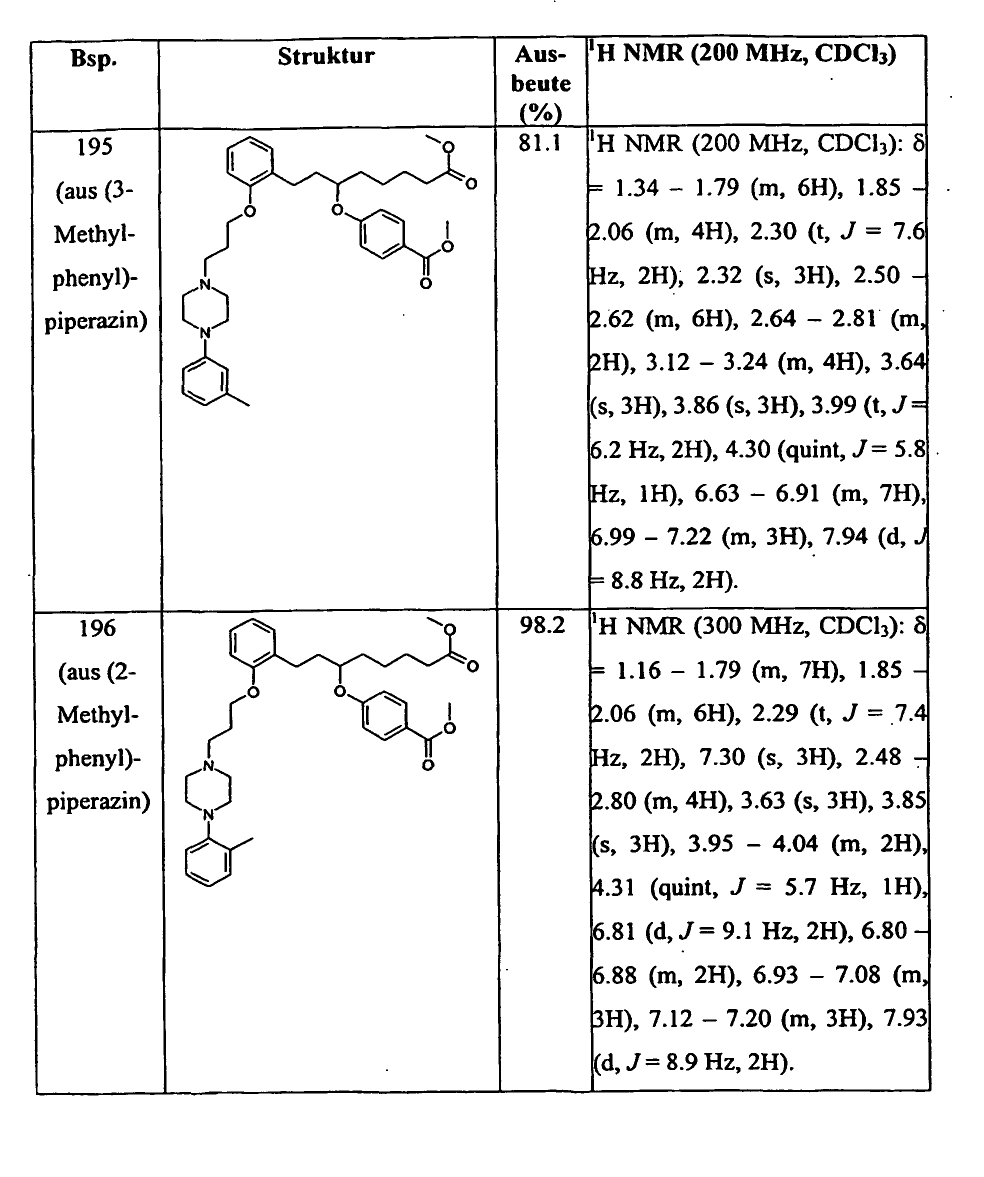

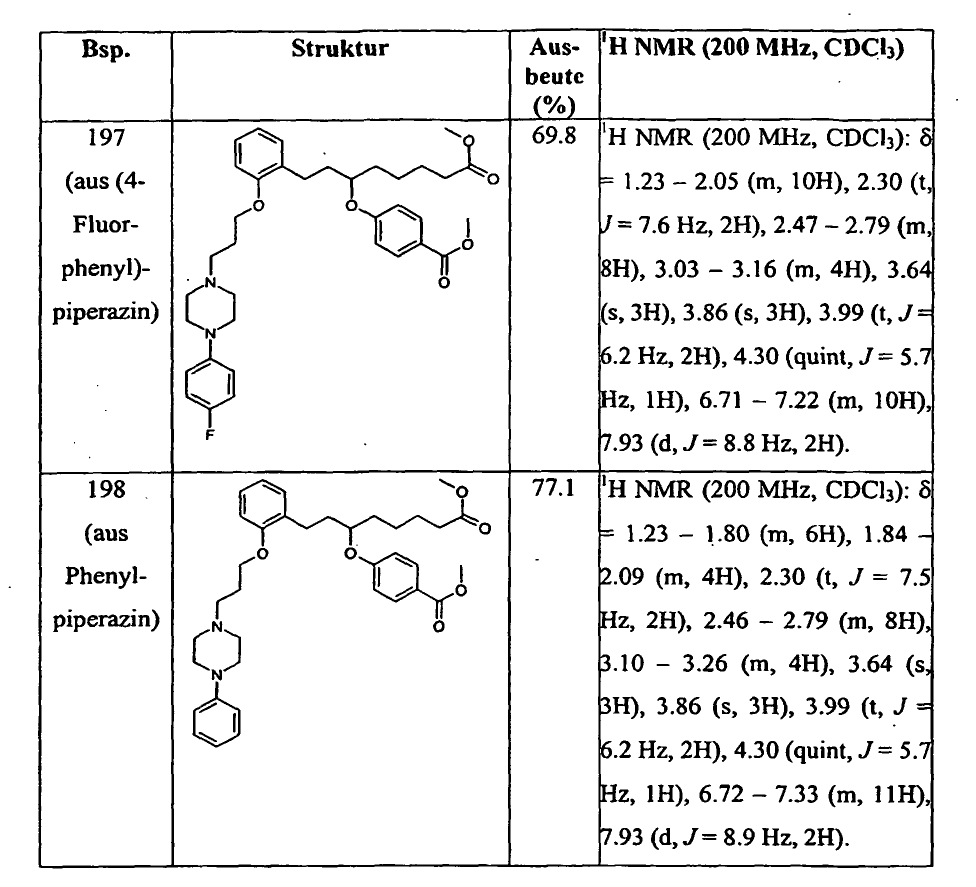

Die entsprechenden Carbonsäurederivate sind aus den Verbindungen 186 bis 198 analog der bei Bsp. 109 beschriebenen Vorgehensweise erhältlich:

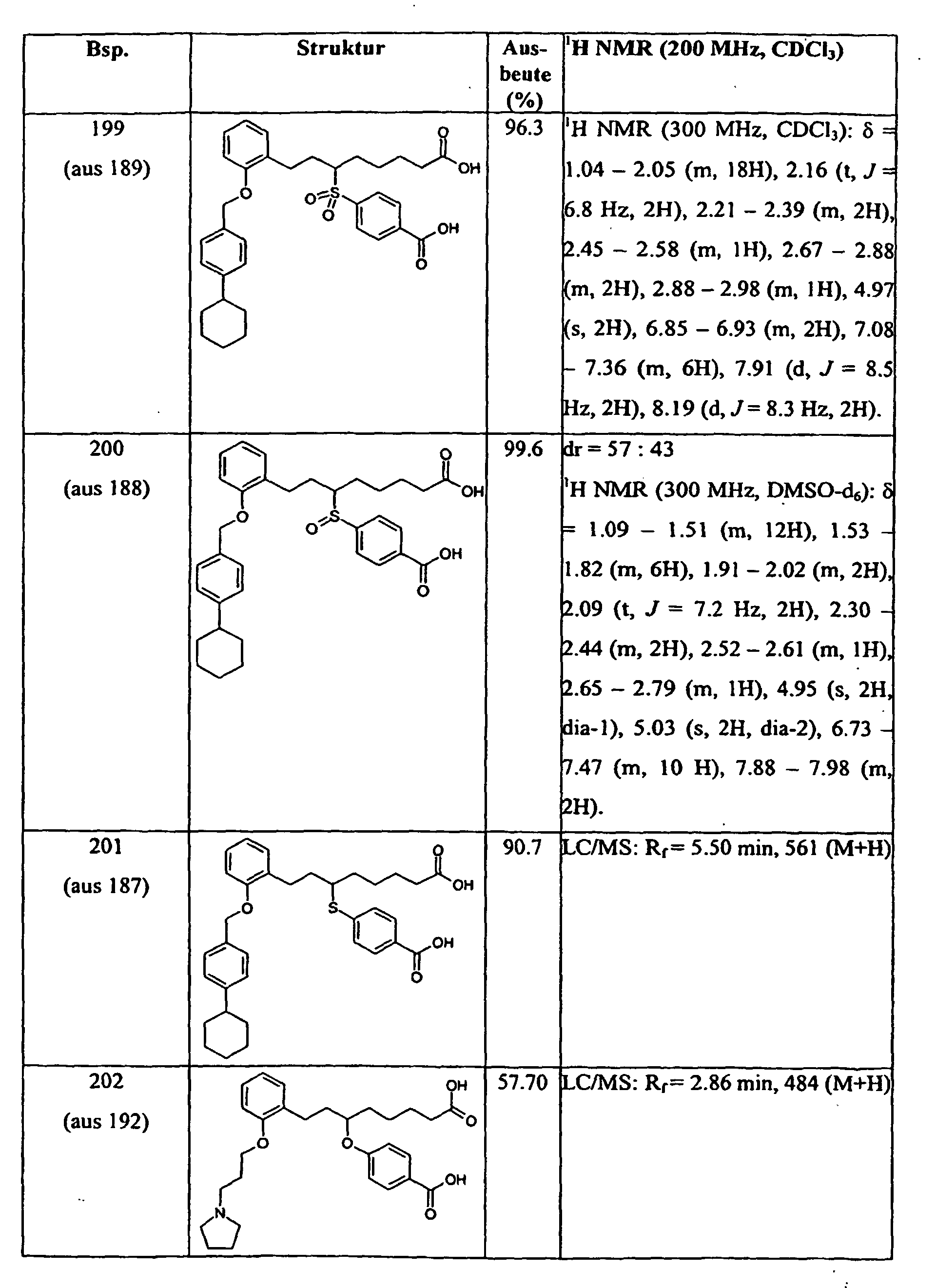

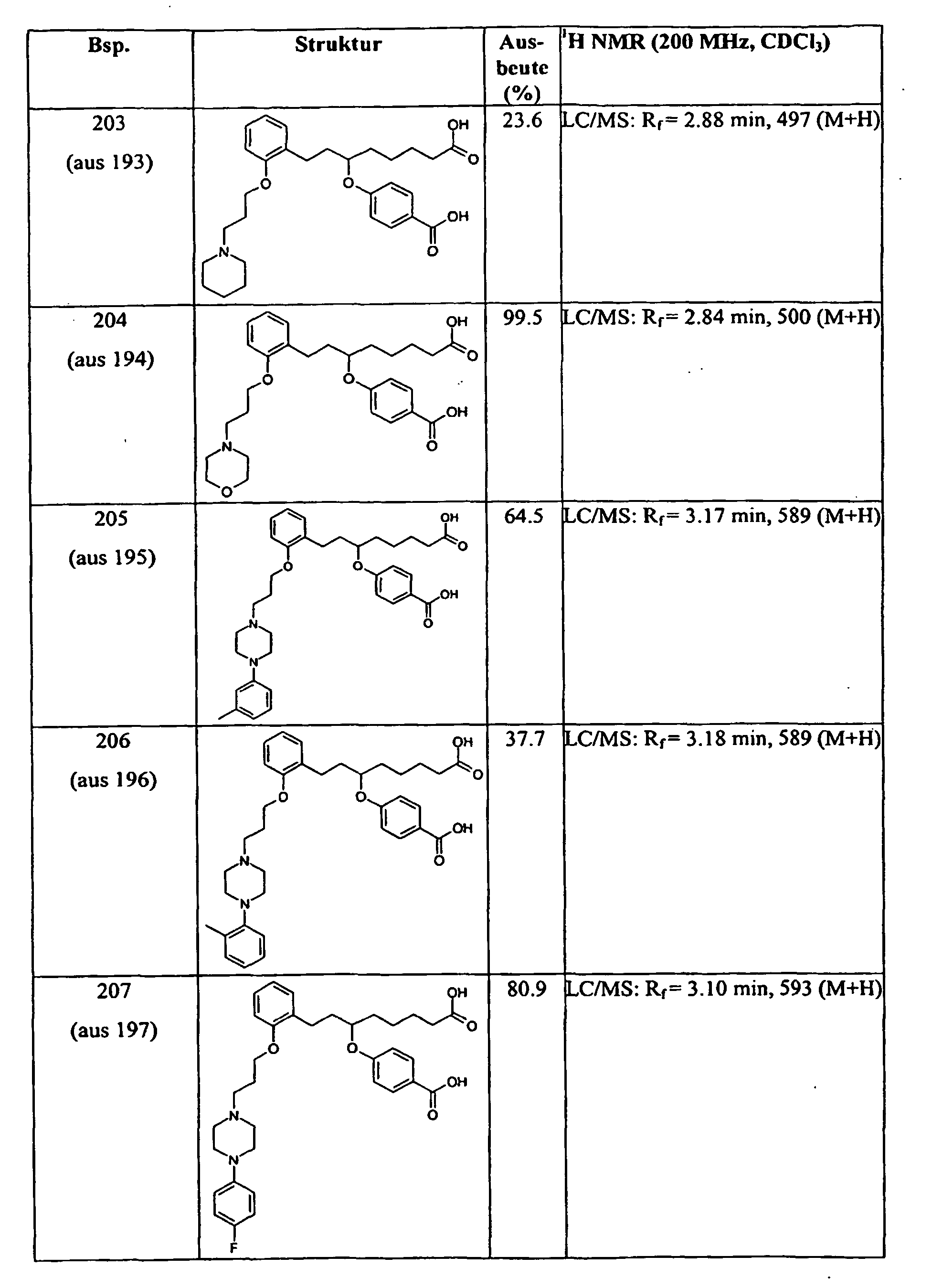

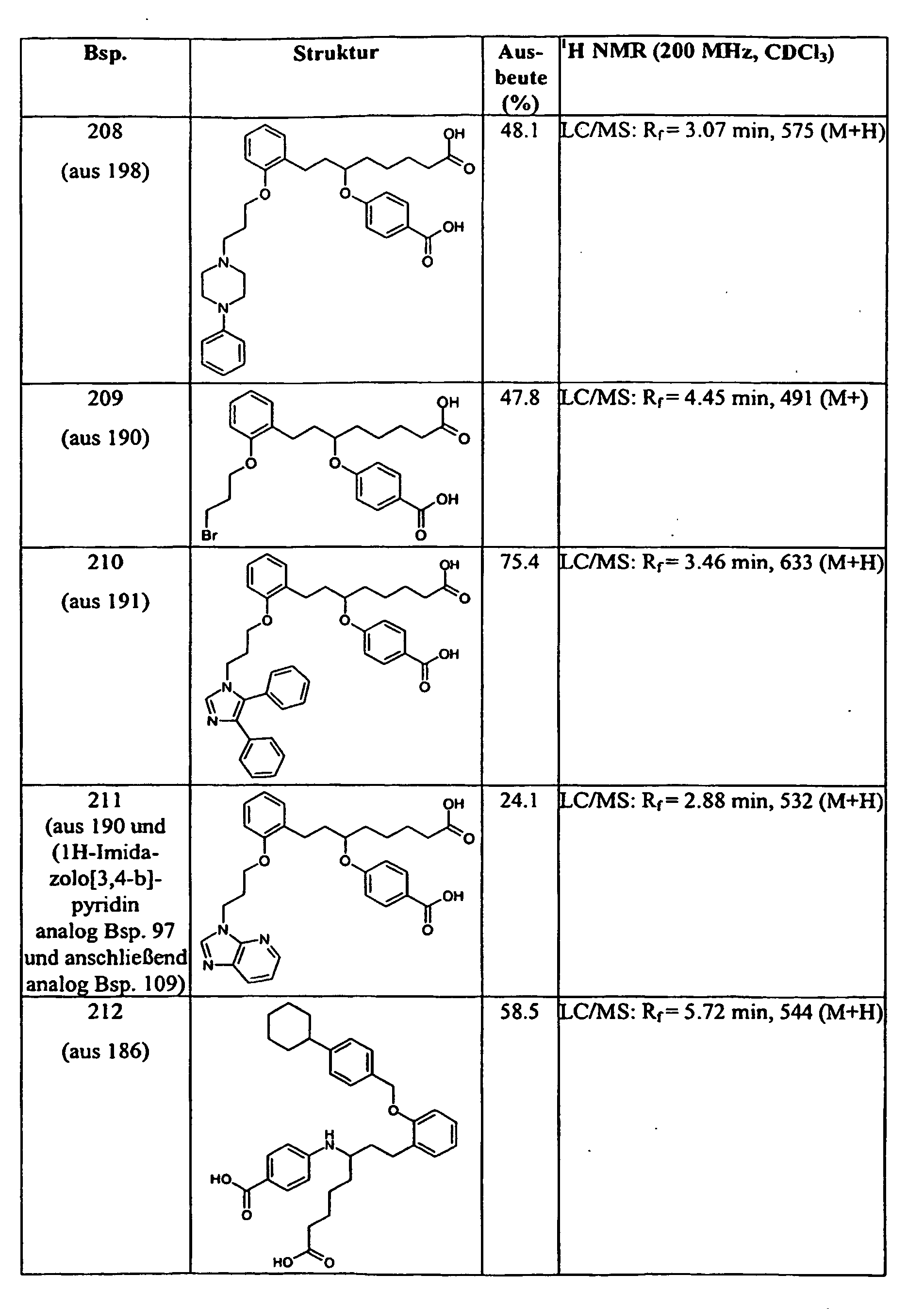

### 213: 8-(2-(4-Cyclohexyl)benzyloxy)-phenyl-6-(4-carbxybutyl)-octansäure

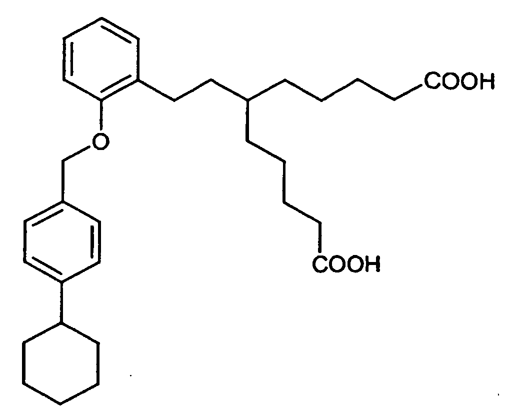

Diese Verbindung wurde analog zur Vorgehensweise bei Beispiel 47 aus dem Phenol aus Bsp. XVIII, 4-Cyclohexylbenzylchlorid und Kaliumcarbonat hergestellt.
Ausbeute: 71,3 %
¹H NMR (300 MHz, DMSO-d₆): δ = 1.11 - 1.54 (m, 22H), 1.63 - 1.84 (m, 5H), 2.14 (t, *J* = 7.2 Hz, 4H), 2.37 - 2.61 (m, 1H), 5.03 (s, 2H), 6.79 - 6.92 (m, 1H), 6.97 - 7.05 (m, 1H), 7.07 - 7.17 (m, 2H), 7.22 (d, *J* = 8.1 Hz, 2H), 7.35 (d, *J* = 7.9 Hz, 2H), 11.91 (bs, 2H).

## Patentansprüche

1. Verbindungen der allgemeinen Formel (I) 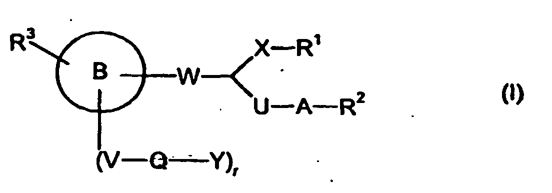 worin
B Aryl mit 6 bis 10 kohlenstoffatomen bedeutet,
r 0 oder 1 bedeutet,
V fehlt, O, NR⁴, NR⁴CONR⁴, NR⁴CO, NR⁴SO₂, COO, CONR⁴ oder S(O)ₒ bedeutet,
worin
R⁴ unabhängig von einem weiteren gegebenenfalls vorhandenen Rest R⁴ Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder Arylalkyl mit 7 bis 18 Kohlenstoffatomen bedeutet, wobei der Arylrest seinerseits ein- oder mehrfach durch Halogen, Alkyl, Alkoxy mit bis zu 6 Kohlenstoffatomen substituiert sein kann,
o 0, 1 oder 2 bedeutet,
Q fehlt, geradkettiges oder verzweigtes Alkylen, geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CO, ÖCO, S-CO-, CONR⁵ oder NR⁵SO₂ enthalten können, und ein oder mehrfach durch Halogen, Hydroxy oder Alkoxy mit bis zu 4 Kohlenstoffatomen substituiert sein können, wobei gegebenenfalls zwei beliebige Atome der vorstehenden Kette unter Bildung eines drei- bis achtgliedrigen Rings miteinander verbunden sein können, oder CONR⁵ bedeutet,
worin
R⁵ Wasserstoff, geradkettiges oder verzweigtes Alkyl mit bis zu 8 Kohlenstoffatomen oder Cycloalkyl mit 3 bis 8 Kohlenstoffatomen bedeutet, das durch Halogen oder Alkoxy mit bis zu 4 Kohlenstoffatomen substituiert sein kann,
p 0, 1 oder 2 bedeutet,
Y Wasserstoff, NR⁶R⁷, Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O oder geradkettiges oder verzweigtes Cycloalkyl mit 3 bis 8 Kohlenstoffatomen bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 8 Kohlenstoffatomen, geradkettiges oder verzweigtes Cycloalkyl mit 3 bis 8 Kohlenstoffatomen, Halogen, Hydroxy, COR⁸, CN, SR⁸, NO₂, NR¹⁰R¹¹, NR⁹COR¹², NR⁹CONR⁹R¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy, geradkettiges oder verzweigtes Alkyloxyalkyl mit bis zu 8 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 8 Kohlenstoffatomen, geradkettiges oder verzweigtes Halogenalkyl mit bis zu 8 Kohlenstoffatomen oder Cycloalkyl mit 3 bis 8 Kohlenstoffatomen bedeutet,
R⁹ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁰, R¹¹, R¹³ und R¹⁴ unabhängig voneinander Wasserstoff, geradkettiges oder verzweigtes Alkyl, geradkettiges oder verzweigtes Alkenyl mit bis zu 8 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O, Arylalkyl mit 8 bis 18 Kohlenstoffatomen, Cycloalkyl mit 3 bis 8 Kohlenstoffatomen oder einen Rest der Formel SO₂R¹⁵ bedeuten,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
oder zwei Substituenten aus R¹⁰ und R¹¹ oder R¹³ und R¹⁴ miteinander unter Bildung eines fünf- oder sechsgliedrigen Rings verbunden sein können, der O oder N enthalten kann,
worin
R¹⁵ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, CN, NO₂, Alkyl, Alkoxy, Halogenalkyl oder Halogenalkoxy mit bis zu 6 Kohlenstoffatomen substituiert sein kann,
R¹² Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können;
und/oder die cyclischen Reste jeweils ein- bis dreifach durch Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O substituiert sein können, die auch über N gebunden sein können,
welche direkt oder über eine Gruppe aus O, S, SO, SO₂, NR⁹, CONR⁹, SO₂NR⁹, geradkettigem oder verzweigtem Alkylen, geradkettigem oder verzweigtem Alkendiyl, geradkettigem oder verzweigtem Alkoxy, geradkettigem oder verzweigtem Oxyalkyloxy, geradkettigem oder verzweigtem Sulfonylalkyl, geradkettigem oder verzweigtem Thioalkyl mit jeweils bis 8 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl, geradkettiges oder verzweigtes Halogenalkoxy, Carbonylalkyl oder geradkettiges oder verzweigtes Alkenyl mit jeweils bis zu 6 Kohlenstoffatomen, Phenyl, Benzyl, Halogen, SR⁸, CN, NO₂, NR¹⁷R¹⁸, CONR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder einen Rest der Formel SO₂R²⁰ bedeuten, wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
worin
R²⁰ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlensttiffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, OH, Halogen, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
W geradkettiges oder verzweigtes Alkylen, geradkettiges oder vetzweigtes Alkendiyl mit jeweils bis zu 4 Kohlenstoffatomen bedeutet, die eine Gruppe aus O oder NR²³ enthalten können,
worin
R²³ Wasserstoff, geradkettiges oder verzweigtes Alkyl mit bis zu 8 Kohlenstoffatomen oder Cycloalkyl mit 3 bis 8 Kohlenstoffatomen bedeutet,
U geradkettiges oder verzweigtes Alkylen mit bis zu 4 Kohlenstoffatomen, O, NH, S, SO oder SO₂ bedeutet,
A fehlt, Phenyl oder einen aromatischen Heterocyclus mit 1 bis 9 Kohlenstoffatomen und bis zu 3 Heteroatomen aus der Reihe S, N und/oder O bedeutet,
welche gegebenenfalls ein- bis dreifach durch Halogen, geradkettiges oder verzweigtes Alkyl, geradkettiges oder verzweigtes Halogenalkyl oder geradkettiges oder verzweigtes Alkoxy mit jeweils bis zu 4 Kohlenstoffatomen substituiert sein können,
R² COOR²⁶ oder CN bedeutet,
worin
R²⁶ Wasserstoff oder geradkettiges oder verzweigtes Alkyl mit bis zu 8 Kohlenstoffatomen bedeutet;
X geradkettiges oder verzweigtes Alkylen, geradkettiges oder verzweigtes Alkendiyl mit jeweils bis zu 8 Kohlenstoffatomen bedeutet, die eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 6 Kohlenstoffatomen aufweisenden Carbocyclus mit gegebenenfalls einem oder zwei Heteroatomen aus der Reihe S(O)ᵣ, NR³² und/oder O enthalten können,
worin
r 0, 1 oder 2 bedeutet,
R³⁰ Wasserstoff, geradkettiges oder verzweigtes Alkyl mit bis zu 6 Kohlenstoffatomen, Phenyl oder Arylalkyl mit 7 bis 12 Kohlenstoffatomen bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ CN oder COOR³⁵ bedeutet,
worin
R³⁵ Wasserstoff oder geradkettiges oder verzweigtes Alkyl mit bis zu 6 Kohlenstoffatomen bedeutet;
mit der Maßgabe, dass Y nicht Phenyl oder ausschließlich mit einem oder zwei Resten aus der Gruppe, bestehend aus geradkettigem oder verzweigtem Alkyl oder geradkettigem oder venweigtem Alkoxy mit jeweils bis zu 8 Kohlenstoffatomen, Halogen, CF₃, OCF₃ oder CN, substituiertes Phenyl sein kann, wenn gleichzeitig B Phenyl ist, V fehlt oder O bedeutet, Q geradkettiges Alkylen mit 1 bis 4 Kohlenstoffatomen bedeutet und gegebenenfalls über ein Sauerstoffatom mit Y verknüpft ist, W eine Alkylengruppe oder eine Alkendiylgruppe mit jeweils 1 bis 6 Kohlenstoffatomen ist, U eine Alkylengruppe mit bis zu 4 Kohlenstoffatomen, O, S, SO oder SO₂ ist, A Phenyl ist und X geradkettiges Alkylen mit 1 bis 8 Kohlenstoffatomen ist und gegebenenfalls direkt über O, S, SO oder SO₂ an das die Gruppen W und U tragende Kohlenstoffatom gebunden ist;

2. Verbindungen nach Anspruch 1,
worin
B Phenyl oder Naphthyl bedeutet
r 0 oder 1 bedeutet,
V fehlt oder O, NR⁴ oder S(O)ₙ bedeutet
worin
R⁴ Wasserstoff bedeutet,
n 0 bedeutet,
Q fehlt, geradkettiges oder verzweigtes Alkylen, geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CONR⁵, S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
Y Wasserstoff, NR⁶R⁷, Phenyl, Napthyl oder einen Heterocyclus aus der Gruppe 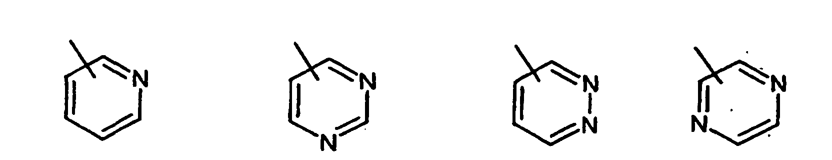 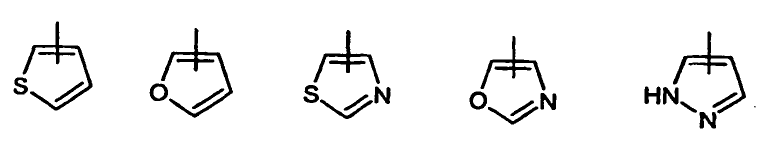 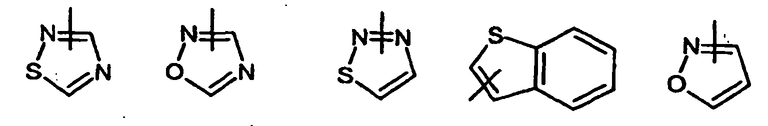 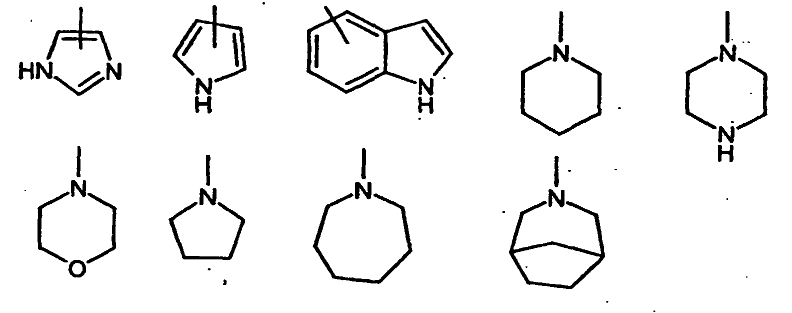 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkylöxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen; oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 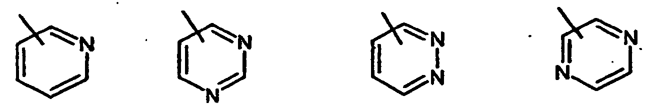 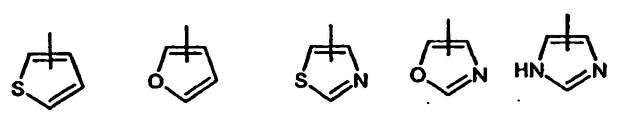 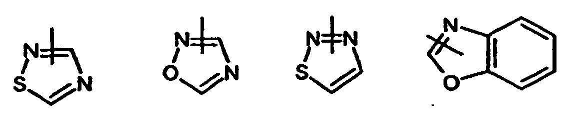 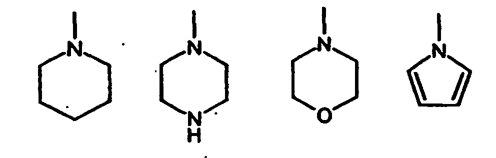 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder 0 oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, OH, F, Cl, Br, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
W CH₂CH₂, CH=CH, CH₂O, OCH₂, CH₂OCH₂, CH₂NH, NHCH₂ oder CH₂NHCH₂ bedeutet,
U geradkettiges Alkylen mit bis zu 4 Kohlenstoffatomen, O, NH, S, SO oder SO₂ bedeutet,
A fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOR²⁶ oder CN bedeutet,
worin
R²⁶ Wasserstoff oder geradkettiges oder verzweigtes Alkyl mit bis zu 4 Kohlenstoffatomen bedeutet;
X geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls einem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ CN oder COOR³⁵ bedeutet,
worin
R³⁵ Wasserstoff oder geradkettiges oder verzweigtes Alkyl mit bis zu 6 Kohlenstoffatomen bedeutet;
mit der Maßgabe, dass Y nicht Phenyl oder ausschließlich mit einem oder zwei Resten aus der Gruppe, bestehend aus geradkettigem oder verzweigtem Alkyl oder geradkettigem oder verzweigtem Alkoxy mit jeweils bis zu 4 Kohlenstoffatomen, Halogen, CF₃, oder OCF₃, substituiertes Phenyl sein kann, wenn gleichzeitig V fehlt oder O bedeutet, Q geradkettiges Alkylen mit 1 bis 10 Kohlenstoffatomen bedeutet und gegebenenfalls über ein Sauerstoffatom mit Y verknüpft ist, W eine Ethylengruppe oder eine Ethandiylgruppe ist, U eine Alkylengruppe mit bis zu 4 Kohlenstoffatomen O, S, SO oder SO₂ ist, A Phenyl ist und X geradkettiges Alkylen mit 1 bis 4 Kohlenstoffatomen ist und gegebenenfalls direkt über O, S, SO oder SO₂ an das die Gruppen W und U tragende Kohlenstoffatom gebunden ist;

3. Verbindungen nach Anspruch 1,
worin
B Phenyl oder Naphthyl bedeutet
r 0 oder 1 bedeutet,
V fehlt oder O, NR⁴ oder S(O)ₙ bedeutet
worin
R⁴ Wasserstoff bedeutet,
n 0 bedeutet,
Q fehlt, geradkettiges oder verzweigtes Alkylen; geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CONR⁵, S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
Y Wasserstoff, NR⁶R⁷, Phenyl, Napthyl oder einen Heterocyclus aus der Gruppe 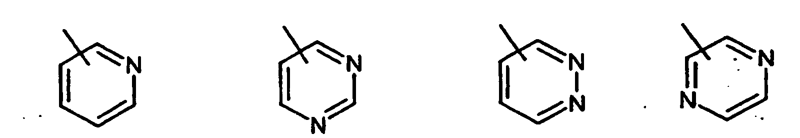 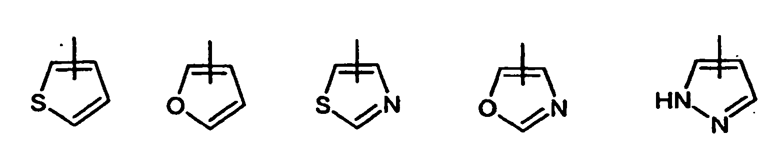 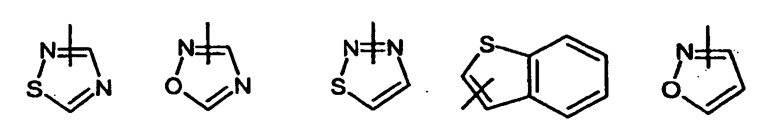 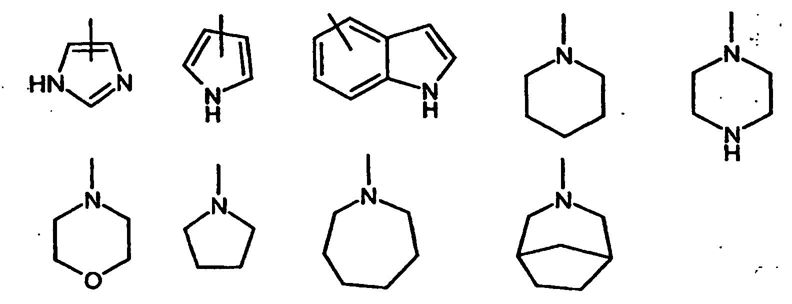 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist,bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 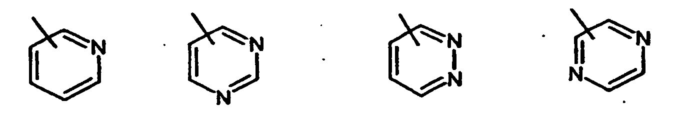 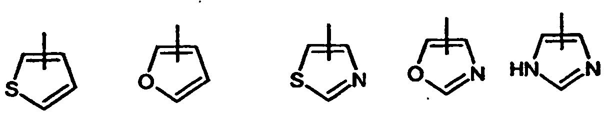 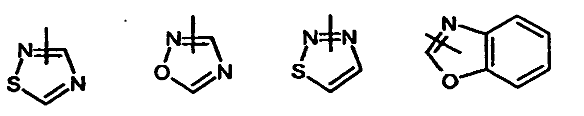 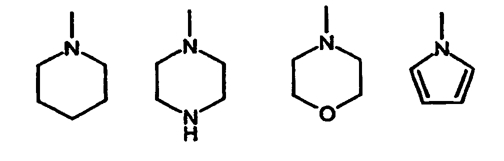 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, OH, F, Cl, Br, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
W CH₂CH₂, CH=CH, CH₂O, OCH₂, CH₂OCH₂, CH₂NH, NHCH₂ oder CH₂NHCH₂ bedeutet,
U geradkettiges Alkylen mit bis zu 4 Kohlenstoffatomen, O, NH, S, SO oder SO₂ bedeutet,
A fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOH bedeutet,
X geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls einem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ COOH bedeutet,
mit der Maßgabe, dass Y nicht Phenyl oder ausschließlich mit einem oder zwei Resten aus der Gruppe, bestehend aus geradkettigem oder verzweigtem Alkyl oder geradkettigem oder verzweigtem Allcoxy mit jeweils bis zu 4 Kohlenstoffatomen, Halogen, CF₃, oder OCF₃, substituiertes Phenyl sein kann, wenn gleichzeitig V fehlt oder O bedeutet, Q geradkettiges Alkylen mit 1 bis 10 Kohlenstoffatomen bedeutet und gegebenenfalls über ein Sauerstoffatom mit Y verknüpft ist, W eine Ethylengruppe oder eine Ethandiylgruppe ist, U eine Alkylengruppe mit bis zu 4 Kohlenstoffatomen, O, S, SO oder SO₂ ist, A Phenyl ist und X geradkettiges Alkylen mit 1 bis 4 Kohlenstoffatomen ist und gegebenenfalls direkt über O, S, SO oder SO₂ an das die Gruppen W und U tragende Kohlenstoffatom gebunden ist;

4. Verbindungen nach Anspruch 1,
worin
B Phenyl bedeutet
r 0 oder 1 bedeutet,
V fehlt oder O, NR⁴ oder S(O)ₙ bedeutet
worin
R⁴ Wasserstoff bedeutet,
n 0 bedeutet,
Q fehlt, geradkettiges oder verzweigtes Alkylen, geradkettiges oder verzweigtes Alkendiyl oder geradkettiges oder verzweigtes Alkindiyl mit jeweils bis zu 15 Kohlenstoffatomen bedeutet, die eine oder mehrere Gruppen aus O, S(O)ₚ, NR⁵, CONR⁵, S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
Y Wasserstoff, NR⁶R⁷, Phenyl, Napthyl oder einen Heterocyclus aus der Gruppe 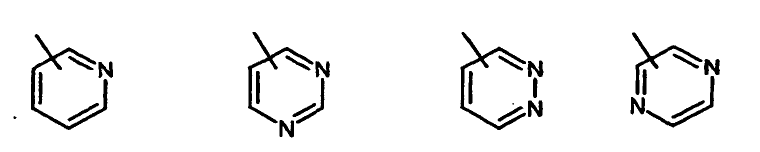 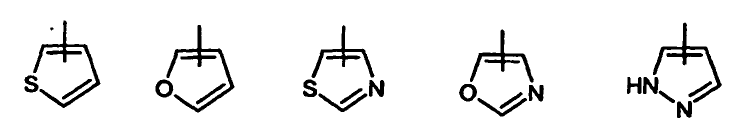 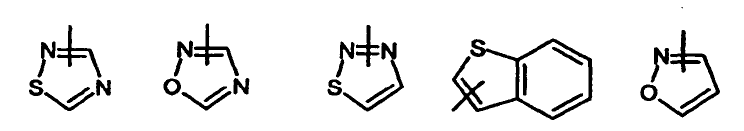 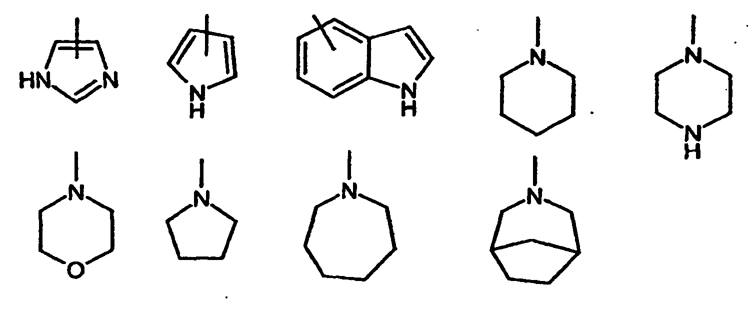 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist,bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 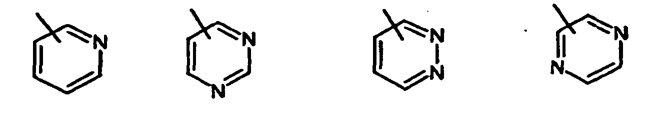 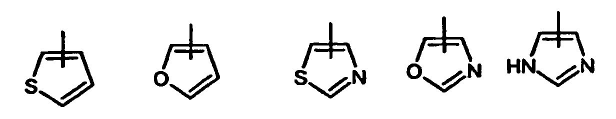 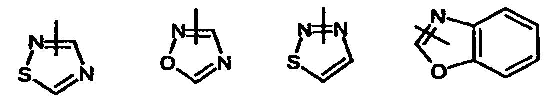 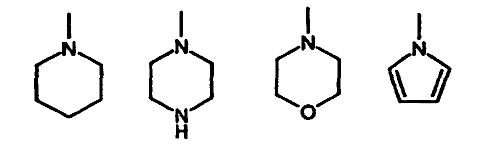 substituiert sein können,
welche direkt oder über eine Gruppe aus O, S, SO, SO₂, CONR⁹, SO₂NR⁹, geradkettigem oder verzweigtem Alkylen. geradkettigem oder verzweigtem Alkendiyi, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, bedeutet,
W CH₂CH₂ oder CH=CH bedeutet,
U CH₂ bedeutet,
A Phenyl bedeutet,
R² COOH bedeutet,
X (CH₂)₄ bedeutet,
R¹ COOH bedeutet,
mit der Maßgabe, dass Y nicht Phenyl oder ausschließlich mit einem oder zwei Resten aus der Gruppe, bestehend aus geradkettigem oder verzweigtem Alkyl oder geradkettigem oder verzweigtem Alkoxy mit jeweils bis zu 4 Kohlenstoffatomen, Halogen, CF₃, oder OCF₃, substituiertes Phenyl sein kann, wenn gleichzeitig V fehlt oder O bedeutet, Q geradkettiges Alkylen mit 1 bis 10 Kohlenstoffatomen bedeutet und gegebenenfalls über ein Sauerstoffatom mit Y verknüpft ist, W eine Ethylengruppe oder eine Ethandiylgruppe ist, U CH₂ ist, A Phenyl ist und X (CH₂)₄ ist.

5. Verbindungen der Formel (I), worin
B einen aromatischen Heterocyclus mit 1 bis 9 Kohlenstoffatomen und bis zu 3 Heteroatomen aus der Reihe S, N und/oder O bedeutet,
r 0 oder 1 bedeutet,
V fehlt, O, NR⁴, NR⁴CONR⁴, NR⁴CO, NR⁴SO₂, COO, CONR⁴ oder S(O)ₒ bedeutet,
worin
R⁴ unabhängig von einem weiteren gegebenenfalls vorhandenen Rest R⁴ Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder Arylalkyl mit 7 bis 18 Kohlenstoffatomen bedeutet, wobei der Arylrest seinerseits ein- oder mehrfach durch Halogen, Alkyl, Alkoxy mit bis zu 6 Kohlenstoffatomen substituiert sein kann,
o 0, 1 oder 2 bedeutet,
Q fehlt, geradkettiges oder verzweigtes Alkylen, geradkettiges oder verzweigtes Alkendiyl oder geradkettiges oder verzweigtes Alkindiyl mit jeweils bis zu 15 KoMenstoftatomen bedeutet, die eine oder mehrere Gruppen aus O, S(O)ₚ, NR⁵, CO, OCO, S-CO-, CONR⁵ oder NR⁵SO₂ enthalten können, und ein oder mehrfach durch Halogen, Hydroxy oder Alkoxy mit bis zu 4 Kohlenstoffatomen substituiert sein können, wobei gegebenenfalls zwei beliebige Atome der vorstehenden Kette unter Bildung eines drei- bis achtgliedrigen Rings miteinander verbunden sein können, oder CONR⁵ bedeutet,
worin
R⁵ Wasserstoff, geradkettiges oder verzweigtes Alkyl mit bis zu 8 Kohlenstoffatomen oder Cycloalkyl mit 3 bis 8 Kohlenstoffatomen bedeutet, das durch Halogen oder Alkoxy mit bis zu 4 Kohlenstoffatomen substituiert sein kann,
p 0, 1 oder 2 bedeutet,
Y Wasserstoff NR⁶R⁷, Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O oder geradkettiges oder verzweigtes Cycloalkyl mit 3 bis 8 Kohlenstoffatomen bedeutet, die auch über N gebunden sein können,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 8 Kohlenstoff atomen, geradkettiges oder verzweigtes Cycloalkyl mit 3 bis 8 Kohlenstoffatomen, Halogen, Hydroxy, COR⁸, CN, SR⁸, NO₂, NR¹⁰R¹¹, NR⁹COR¹², NR⁹CONR⁹R¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy, geradkettiges oder verzweigtes Alkyloxyalkyl mit bis zu 8 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist, bedeutet,
R⁸ Wasserstoff, geradkettiges oder verzweigtes Alkyl mit bis zu 8 Kohlenstoffatomen, geradkettiges oder verzweigtes Halogenalkyl mit bis zu 8 Kohlenstoffatomen oder Cycloalkyl mit 3 bis 8 Kohlenstoffatomen bedeutet,
R⁹ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁰, R¹¹, R¹³ und R¹⁴ unabhängig voneinander Wasserstoff, geradkettiges oder verzweigtes Alkyl, geradkettiges oder verzweigtes Alkenyl mit bis zu 8 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O, Arylalkyl mit 8 bis 18 Kohlenstoffatomen, Cycloalkyl mit 3 bis 8 Kohlenstoffatomen oder einen Rest der Formel SO₂R¹⁵ bedeuten,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
oder zwei Substituenten aus R¹⁰ und R¹¹ oder R¹³ und R¹⁴ miteinander unter Bildung eines fünf- oder sechsgliedrigen Rings verbunden sein können, der O oder N enthalten kann,
worin
R¹⁵ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, CN, NO₂, Alkyl, Alkoxy, Halogenalkyl oder Halogenalkoxy mit bis zu 6 Kohlenstoffatomen substituiert sein kann,
R¹² Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können;
und/oder die cyclischen Reste jeweils ein- bis dreifach durch Aryl mit 6 bis 10 Kohlenstoffatomen, einen aromatischen oder gesättigten Heterocyclus mit 1 bis 9 Kohlenstoffatomen und bis zu 3 Heteroatomen aus der Reihe S, N und/oder O substituiert sein können, die auch über N gebunden sein können,
welche direkt oder über eine Gruppe aus O, S, SO, SO₂, NR⁹, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylälkyl, geradkettigem oder verzweigtem Thioalkyl mit jeweils bis 8 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl, geradkettiges oder verzweigtes Halogenalkoxy, Carbonylalkyl oder geradkettiges oder verzweigtes Alkenyl mit jeweils bis zu 6 Kohlenstoffatomen, Phenyl, Benzyl, Halogen, SR⁸, CN, NO₂, NR¹⁷R¹⁸, CONR¹⁷R¹⁸oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Aryl mit 6 bis 10 Kohlenstoffatomen oder einen Rest der Formel SO₂R²⁰ bedeuten, wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
worin
R²⁰ geradkettiges oder verzweigtes Alkyl mit bis zu 4 Kohlenstoffatomen oder Aryl mit 6 bis 10 Kohlenstoffatomen bedeutet,
wobei der Arylrest seinerseits ein- oder mehrfach durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein kann,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch Halogen, Hydroxy, CN, NO₂, NH₂, NHCOR⁹, Alkyl, Alkoxy, Halogenalkyl oder Halogenalkoxy mit bis zu 6 Kohlenstoffatomen substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, Halogen, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatoinen bedeutet,
W geradkettiges oder verzweigtes Alkylen, geradkettiges oder verzweigtes Alkendiyl mit jeweils bis zu 4 Kohlenstoffatomen bedeutet, die eine Gruppe aus O oder NR²³ enthalten können,
worin
R²³ Wasserstoff, geradkettiges oder verzweigtes Alkyl mit bis zu 8 Kohlenstoffatomen oder Cycloalkyl mit 3 bis 8 Kohlenstoffatomen bedeutet,
U geradkettiges oder verzweigtes Alkylen mit bis zu 4 Kohlenstoffatomen, O, NH, S, SO oder SO₂ bedeutet,
A fehlt, Phenyl oder einen aromatischen Heterocyclus mit 1 bis 9 Kohlenstoffatomen und bis zu 3 Heteroatomen aus der Reihe S, N und/oder O bedeutet,
welche gegebenenfalls ein- bis dreifach durch Halogen, geradkettiges oder verzweigtes Alkyl, geradkettiges oder verzweigtes Halogenalkyl oder geradkettiges oder verzweigtes Alkoxy mit jeweils bis zu 4 Kohlenstoffatomen substituiert sein können,
R² COOR²⁶ oder CN bedeutet,
worin
R²⁶ Wasserstoff oder geradkettiges oder verzweigtes Alkyl mit bis zu 8 Kohlenstoffatomen bedeutet;
X geradkettiges oder verzweigtes Alkylen, geradkettiges oder verzweigtes Alkendiyl mit jeweils bis zu 8 Kohlenstoffatomen bedeutet, die eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 6 Kohlenstoffatomen aufweisenden Carbocyclus mit gegebenenfalls einem oder zwei Heteroatomen aus der Reihe S(O)ᵣ, NR³² und/oder O enthalten können,
worin
r 0, 1 oder 2 bedeutet,
R³⁰ Wasserstoff, geradkettiges oder verzweigtes Alkyl mit bis zu 6 Kohlenstoffatomen, Phenyl oder Arylalkyl mit 7 bis 12 Kohlenstoffatomen bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ CN oder COOR³⁵ bedeutet,
worin
R³⁵ Wasserstoff oder geradkettiges oder verzweigtes Alkyl mit bis zu 6 Kohlenstoffatomen bedeutet.

6. Verbindungen nach Anspruch 5,
worin
B einen Heterocyclus aus der Reihe 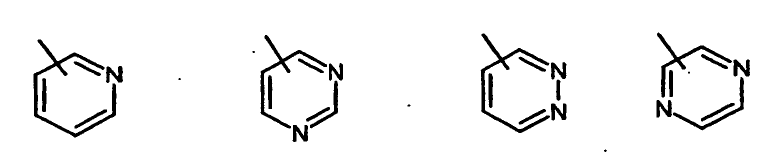 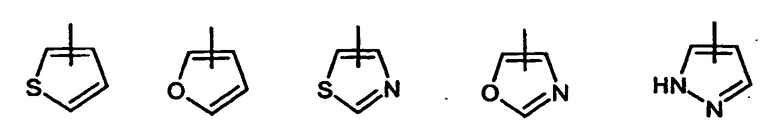 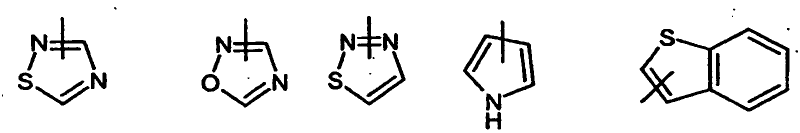 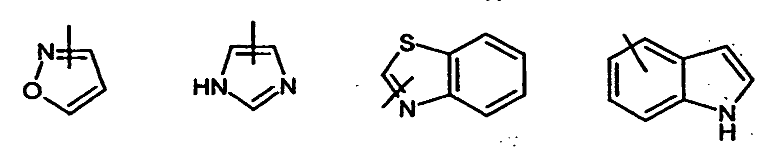 bedeutet
r 0 oder 1 bedeutet,
V fehlt oder O, NR⁴ oder S(O)ₙ bedeutet
worin
R⁴ Wasserstoff bedeutet,
n 0 bedeutet,
Q fehlt, geradkettiges oder verzweigtes Alkylen, geradkettiges oder verzweigtes Alkendiyl mit jeweils bis zu 15 Kohlenstoffatomen bedeutet, die eine oder mehrere Gruppen aus O, S(O)ₚ, NR⁵, CONR⁵, S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
Y Wasserstoff, NR⁶R⁷, Phenyl, Napthyl oder einen Heterocyclus aus der Gruppe 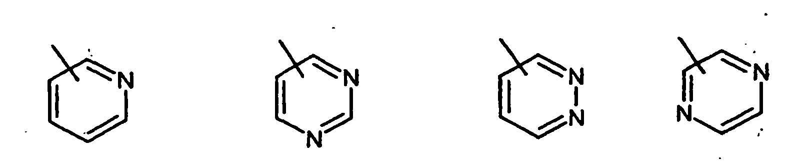 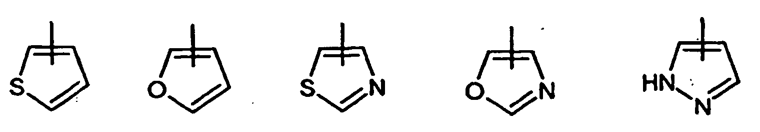 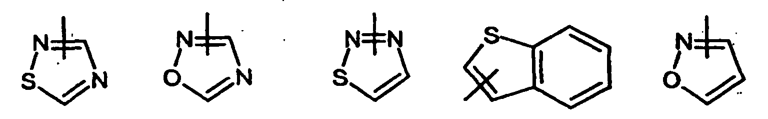 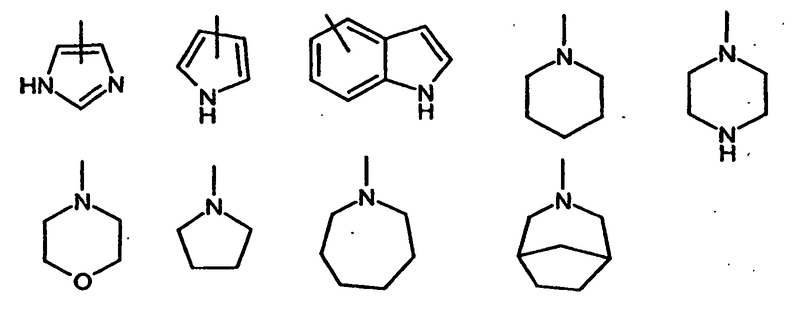 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloallcyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist,bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 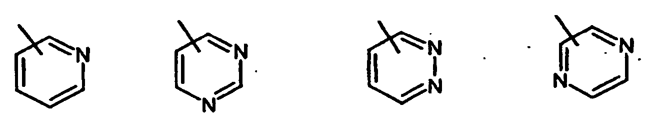 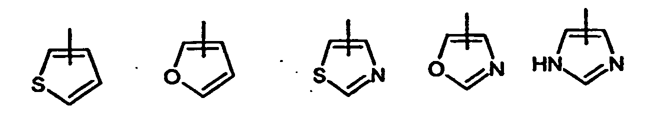 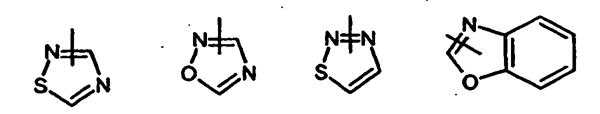 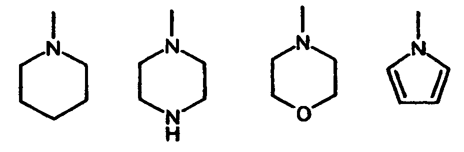 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonylalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbocyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, F, Cl, Br, geradkettiges oder verzweigtes Alkyl, geradkettiges oder verzweigtes Halogenallcyl, geradkettiges oder verzweigtes Alkoxy oder geradkettiges oder verzweigtes Halogenalkoxy mit jeweils bis zu 4 Kohlenstoffatomen bedeutet,
W CH₂CH₂, CH=CH, CH₂O, OCH₂, CH₂OCH₂, CH₂NH, NHCH₂ oder CH₂NHCH₂ bedeutet,
U geradkettiges Alkylen mit bis zu 4 Kohlenstoffatomen, O, NH, S, SO oder SO₂ bedeutet,
A fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOR²⁶ oder CN bedeutet,
worin
R²⁶ Wasserstoff oder geradkettiges oder verzweigtes Alkyl mit bis zu 4 Kohlenstoffatomen bedeutet;
X geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls einem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ CN oder COOR³⁵ bedeutet,
worin
R³⁵ Wasserstoff oder geradkettiges oder verzweigtes Alkyl mit bis zu 6 Kohlenstoffatomen bedeutet.

7. Verbindungen nach Anspruch 5,
worin
B einen Heterocyclus aus der Reihe 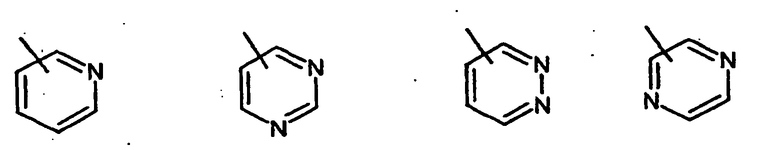 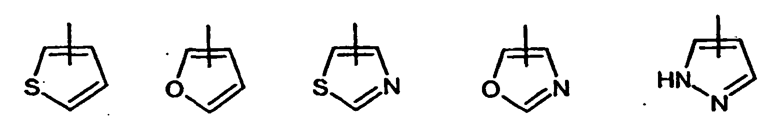 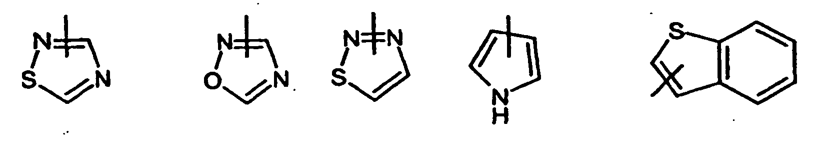 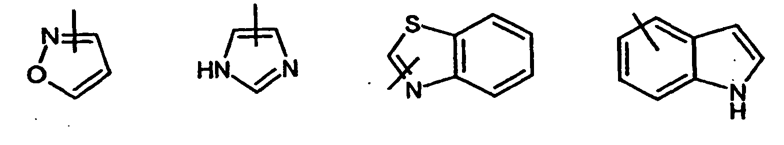 bedeutet
r 0 oder 1 bedeutet,
V fehlt oder O, NR⁴ oder S(O)ₙ bedeutet
worin
R⁴ Wasserstoff bedeutet,
n 0 bedeutet,
Q fehlt, geradkettiges oder verzweigtes Alkylen, geradkettiges oder verzweigtes Alkendiyl mit jeweils bis zu 15 Kohlenstoffatomen bedeutet, die eine oder mehrere Gruppen aus O, S(O)ₚ, NR⁵, CONR⁵, S-CO- oder OCO enthalten können, und ein- oder zweifach durch Halogen oder Hydroxy substituiert sein kann, oder CONR⁵ bedeutet,
worin
R⁵ Wasserstoff bedeutet,
p 0 oder 1 bedeutet,
Y Wasserstoff, NR⁶R⁷, Phenyl, Napthyl oder einen Heterocyclus aus der Gruppe 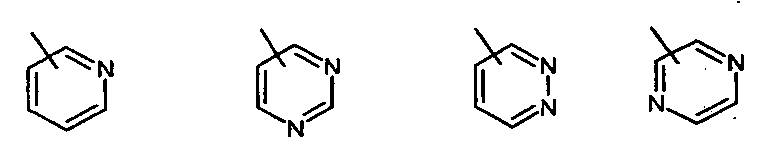 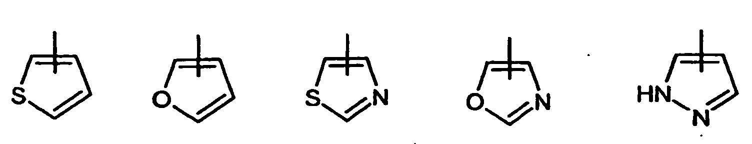 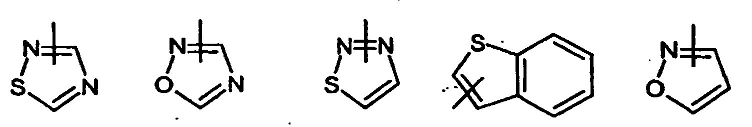 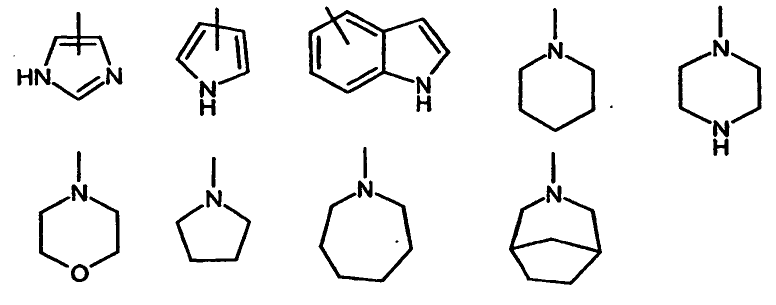 bedeutet,
wobei die cyclischen Reste jeweils ein- bis dreifach durch geradkettiges oder verzweigtes Alkyl, geradkettiges oder verzweigtes Alkenyl, geradkettiges oder verzweigtes Alkinyl, geradkettiges oder verzweigtes Alkoxy, geradkettiges oder verzweigtes Halogenalkyl, geradkettiges oder verzweigtes Halogenalkoxy mit jeweils bis zu 4 Kohlenstoffatomen, geradkettiges oder verzweigtes Cycloalkyl mit 3 bis 7 Kohlenstoffatomen, F, Cl, Br, I, NO₂, COR⁸, SR⁸, NR¹⁰R¹¹, NR⁹COR¹² oder CONR¹³R¹⁴ substituiert sein können,
worin
R⁶ und R⁷ jeweils unabhängig voneinander Wasserstoff, geradkettiges oder verzweigtes Alkyl, geradkettiges oder verzweigtes Alkoxy oder geradkettiges oder verzweigtes Alkyloxyalkyl mit jeweils bis zu 4 Kohlenstoffatomen oder Cycloalkyl mit 3 bis 8 Kohlenstoffatomen oder Aryl mit 6 bis 10 Kohlenstoffatomen, das gegebenenfalls ein- oder mehrfach mit Aryl mit 6 bis 10 Kohlenstoffatomen oder einem aromatischen Heterocyclus mit 1 bis 9 Kohlenstoffatomen und bis zu 3 Heteroatomen aus der Reihe S, N und/oder O substituiert ist,bedeutet,
R⁸ Wasserstoff, geradkettiges oder verzweigtes Alkyl mit bis zu 4 Kohlenstoffatomen, oder geradkettiges oder verzweigtes Halogenalkyl mit bis zu 4 Kohlenstoffatomen bedeutet,
R⁹ Wasserstoff, oder geradkettiges oder verzweigtes Alkyl mit bis zu 4 Kohlenstoffatomen bedeutet,
R¹⁰, R¹¹, R¹³ und R¹⁴ unabhängig voneinander Wasserstoff, geradkettiges oder verzweigtes Alkyl mit bis zu 4 Kohlenstoffatomen, oder Phenyl bedeuten,
wobei der Phenylrest ein- bis dreifach durch F, Cl Br, Hydroxy, Methyl, Ethyl, n- Propyl, i-Propyl, n- Butyl, s-Butyl, i- Butyl, t-Butyl, Methoxy, Ethoxy, Amino, Acetylamino, NO₂, CF₃, OCF₃ oder CN substituiert sein kann,
oder zwei Substituenten aus R¹⁰ und R¹¹ oder R¹³ und R¹⁴ miteinander unter Bildung eines fünf- oder sechsgliedrigen Ring verbunden sein können, der durch O oder N unterbrochen sein kann,
R¹² Wasserstoff, geradkettiges oder verzweigtes Alkyl mit bis zu 4 Kohlenstoffatomen, oder Phenyl bedeutet,
wobei der Phenylrest ein- bis dreifach durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein kann;
und/oder die cyclischen Reste jeweils ein- bis dreifach durch Phenyl oder einen Heterocyclus aus der Gruppe 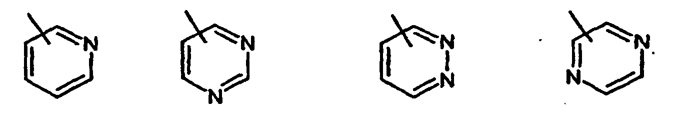 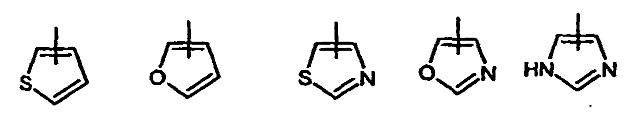 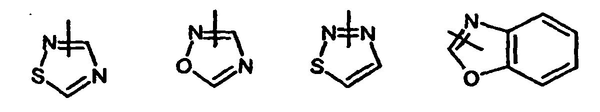 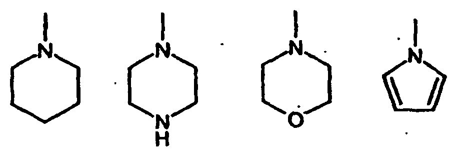 substituiert sein können,
welche direkt oder über eine Gruppe aus O, S, SO, SO₂, CONR⁹, SO₂NR⁹, geradkettigem oder verzweigtem Alkylen, geradkettigem oder verzweigtem Alkendiyl, geradkettigem oder verzweigtem Alkyloxy, geradkettigem oder verzweigtem Oxyalkyloxy, geradkettigem oder verzweigtem Sulfonytalkyl, geradkettigem oder verzweigtem Thioalkyl mit jeweils bis 4 Kohlenstoffatomen gebunden und ein- bis dreifach durch geradkettiges oder verzweigtes Alkyl, geradkettiges oder verzweigtes Alkoxy, geradkettiges oder verzweigtes Halogenalkyl oder geradkettiges oder verzweigtes Alkenyl mit jeweils bis zu 4 Kohlenstoffatomen, Phenyl, Benzyl, F, Cl, Br, I, CN, NO₂, NR¹⁷R¹⁸ oder NR¹⁶COR¹⁹ substituiert sein können,
worin
R¹⁶ Wasserstoff, geradkettiges oder verzweigtes Alkyl mit bis zu 8 Kohlenstoffatomen oder Cycloalkyl mit 3 bis 8 Kohlenstoffatomen bedeutet,
R¹⁷, R¹⁸ unabhängig voneinander Wasserstoff, geradkettiges oder verzweigtes Alkyl mit bis zu 8 Kohlenstoffatomen, Cycloalkyl mit 3 bis 8 Kohlenstoffatomen, Phenyl, wobei der Phenylrest ein- bis dreifach durch F, Cl Br, Hydroxy, Methyl, Ethyl, n-Propyl, i-Propyl, n-Butyl, s-Butyl ,i-Butyl, t-Butyl, Methoxy, Ethoxy, Amino, Acetylamino, NO₂, CF₃, OCF₃ oder CN substituiert sein kann, oder einen Rest der Formel SO₂R²⁰ bedeuten,
worin
R²⁰ geradkettiges oder verzweigtes Alkyl mit bis zu 4 Kohlenstoffatomen oder Phenyl bedeutet,
und
R¹⁹ Wasserstoff, geradkettiges oder verzweigtes Alkyl mit bis zu 12 Kohlenstoffatomen, geradkettiges oder verzweigtes Alkenyl mit bis zu 12 Kohlenstoffatomen, Aryl mit 6 bis 10 Kohlenstoffatomen, einen aromatischen Heterocyclus mit 1 bis 9 Kohlenstoffatomen und bis zu 3 Heteroatomen aus der Reihe S, N und/oder O oder Cycloalkyl mit 3 bis 8 Kohlenstoffatomen bedeutet, welche gegebenenfalls weiterhin durch F, Cl Br, Hydroxy, Methyl, Ethyl, n-Propyl, i-Propyl, n-Butyl, s-Butyl, i-Butyl, t-Butyl, Methoxy, Ethoxy, Amino, Acetylamino, NO₂, CF₃, OCF₃ oder CN substituiert sein können;
und/oder die cyclischen Reste mit einem aromatischen oder gesättigten Carbacyclus mit 1 bis 10 Kohlenstoffatomen oder einem aromatischen oder gesättigten Heterocyclus mit 1 bis 9 Kohlenstoffatomen und bis zu 3 Heteroatomen aus der Reihe S, N und/oder O anneliert sein können,
R³ Wasserstoff, F, Cl, Br, geradkettiges oder verzweigtes Alkyl, geradkettiges oder verzweigtes Halogenalkyl, geradkettiges oder verzweigtes Alkoxy oder geradkettiges oder verzweigtes Halogenalkoxy mit jeweils bis zu 4 Kohlenstoffatomen bedeutet,
W CH₂CH₂, CH=CH, CH₂O, OCH₂, CH₂OCH₂, CH₂NH, NHCH₂ oder CH₂NHCH₂ bedeutet,
U geradkettiges Alkylen mit bis zu 4 Kohlenstoffatomen, O, NH, S, SO oder SO₂ bedeutet,
A fehlt oder Phenyl, Pyridyl, Thienyl oder Thiazolyl bedeutet, das gegebenenfalls ein- bis dreifach durch Methyl, Ethyl, n-Propyl, i-Propyl, n-Butyl, i-Butyl, s-Butyl, t-Butyl, CF₃, Methoxy, Ethoxy, F, Cl, Br substituiert sein kann,
R² COOH bedeutet,
X geradkettiges oder verzweigtes Alkylen mit bis zu 4 Kohlenstoffatomen bedeutet, das eine Gruppe aus O, S(O)ᵣ, NR³⁰, oder einen drei- bis sechsgliedrigen gesättigten oder ungesättigten, gegebenenfalls einen oder mehrere geradkettige oder verzweigte Alkylreste mit 1 bis 4 Kohlenstoffatomen aufweisenden Carbocyclus mit gegebenenfalls einem oder zwei Heteroatomen aus der Reihe S(O)ᵣ, NR³² und/oder O enthalten kann,
worin
r 0, 1 oder 2 bedeutet,
R³⁰ Wasserstoff, geradkettiges oder verzweigtes Alkyl mit bis zu 6 Kohlenstoffatomen, Phenyl oder Benzyl bedeutet,
R³² Wasserstoff, geradkettiges oder verzweigtes Alkyl mit bis zu 6 Kohlenstoffatomen, Cycloalkyl mit 3 bis 6 Kohlenstoffatomen oder Phenyl bedeutet,
R¹ COOH bedeutet.

8. Verfahren zur Herstellung der Verbindungen der Formel (I) 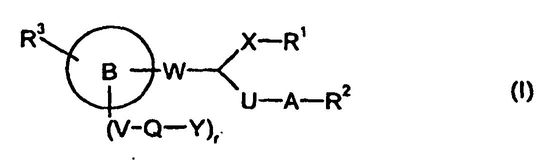 umfassend
[A] die Umsetzung von Aldehyden der allgemeinen Formel (II) 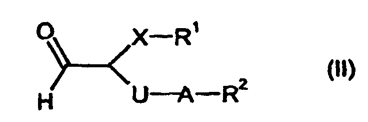 worin
R¹, R², A, U und X die vorstehend angegebene Bedeutung haben, mit der Maßgabe, dass R¹ und R² nicht für freie Carbonsäuregruppen stehen dürfen,
mit Phosphorverbindungen der allgemeinen Formel (III) 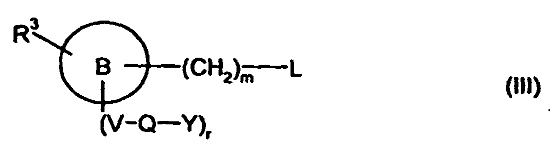 worin
R³, B, V, Q, Y und r die vorstehend angegebenen Bedeutungen haben,
m eine ganze Zahl von 1 bis 5 bedeutet, und
L für einen Rest der Formel 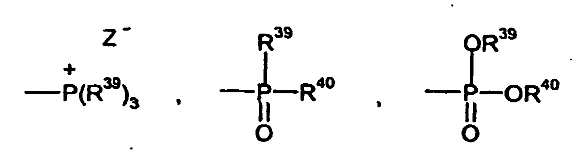 steht, worin
R³⁹ und R⁴⁰ unabhängig voneinander geradkettiges oder verzweigtes Alkyl mit bis zu 12 Kohlenstoffatomen oder Phenyl bedeuten, und
Z ein Halegenidanion oder Tosylatanion bedeutet,
in inerten Lösungsmitteln in Gegenwart einer Base,
und gegebenenfalls die anschließende teilweise oder vollständige Hydrolyse der Reste R¹ und R² zu freien Carbonsäuregruppen;
oder
[B] Verbindungen der Formel (IV), 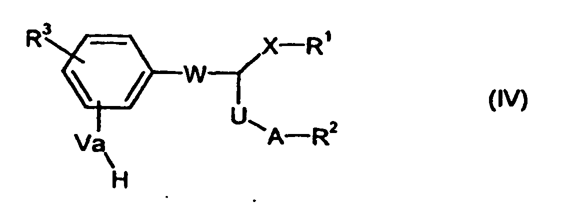 worin
Va für O oder S steht
R¹, R², R³, U, W,A, X die vorstehend angegebene Bedeutung haben
mit Verbindungen der Formel (V) 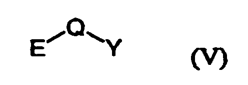 umsetzt,
worin
Q, Y die gleichen Bedeutungen wie vorstehend definiert haben,
E entweder eine Abgangsgruppe bedeutet, die in Gegenwart einer Base substituiert wird, oder eine gegebenenfalls aktivierte Hydroxyfunktion ist;
oder
[C] Verbindungen der Formel (VI), 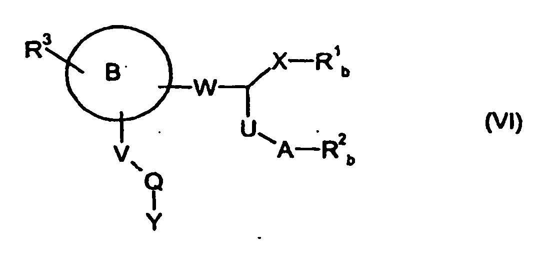 worin
R³, V, Q, Y, W, U, A, B die gleichen Bedeutungen wie vorstehend definiert haben,
R¹_{b} und R²_{b} jeweils unabhängig für CN oder COOAlk stehen, wobei Alk für einen geradkettigen oder verzweigten Alkylrest mit bis zu 6 Kohlenstoffatomen steht,
mit wässrigen Lösungen starker Säuren oder starker Basen in die entsprechenden freien Carbonsäuren überführt.
oder
[D] Verbindungen der Formel (VII) 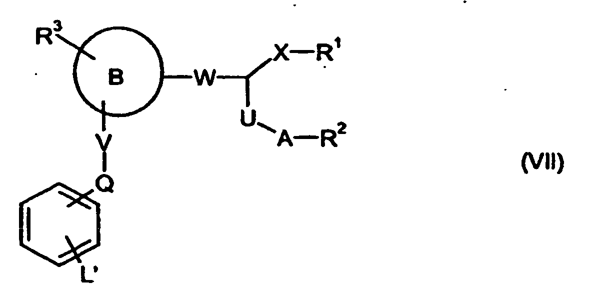 worin
R¹, R², R³, V, Q, X, W, U, A, B die gleichen Bedeutungen wie vorstehend definiert haben,
L' für Br, I oder die Gruppe CF₃SO₂-O steht,
mit Verbindungen der Formel (VIII)
M-Z' (VIII)
worin
M für einen Aryl oder Heteroarylrest, einen geradkettigen oder verzweigten Alkyl-, Alkenyl- oder Alkinylrest oder Cycloalkylrest oder für einen Arylalkyl-, einen Arylalkenyl- oder einen Arylalkinylrest steht,
Z' für die Gruppierungen -B(OH)₂, -CH≡CH, -CH=CH₂ oder -Sn(nBu)₃ steht
in Gegenwart einer Palladiumverbindung, gegebenenfalls zusätzlich in Gegenwart eines Reduktionsmittels und weiterer Zusatzstoffe und in Gegenwart einer Base umsetzt;
oder
[E] Verbindungen der Formel (VII) 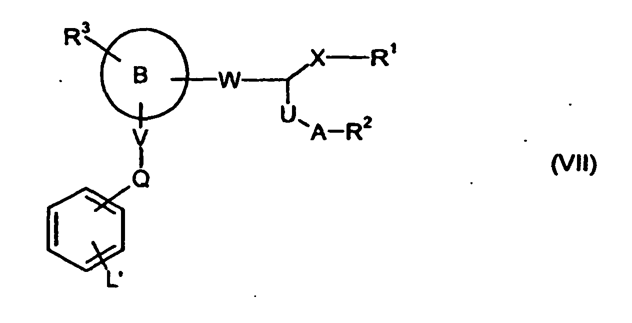 worin
R¹, R², R³, V, Q, X, W, U, A, B die gleichen Bedeutungen wie vorstehend definiert haben,
L' für Br, I oder die Gruppe CF₃SO₂-O steht,
mit Verbindungen der Formel (IX)
NHR^{a}R^{b} (IX)
worin
R^{a} und R^{b} unabhängig voneinander für Wasserstoff oder einen geradkettigen oder verzweigten Alkylrest mit bis zu 8 Kohlenstoffatomen stehen oder zusammen mit dem Stickstoffatom, an das sie gebunden sind, einen einen aromatischen Heterocyclus mit 1 bis 9 Kohlenstoffatomen und bis zu 3 Heteroatomen aus der Reihe S, N und/oder O bilden können,
in Gegenwart einer Palladiumverbindung, gegebenenfalls zusätzlich in Gegenwart eines Reduktionsmittels und weiterer Zusatzstoffe und in Gegenwart einer Base umsetzt;
oder
[F] Verbindungen der Formel (IV), 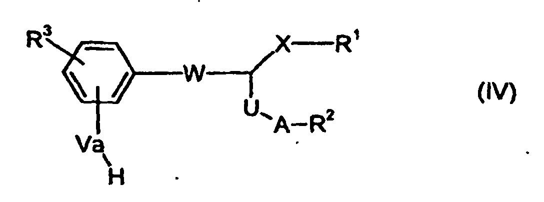 worin
Va für O oder S steht
R¹, R², R³, U, W,A, X die vorstehend angegebene Bedeutung haben
mit Verbindungen der Formel (X) 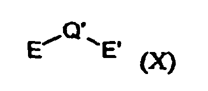 umsetzt,
worin
Q' die gleichen Bedeutung wie Q gemäß Anspruch 1 hat oder Phenyl bedeutet,
E und E' jeweils unabhängig voneinander entweder eine Abgangsgruppe bedeutet, die in Gegenwart einer Base substituiert wird, oder eine gegebenenfalls aktivierte Hydroxyfunktion ist oder ein eine derartige Gruppe enthaltender Rest ist;
und die so erhaltenen Verbindungen der Formel (XI) 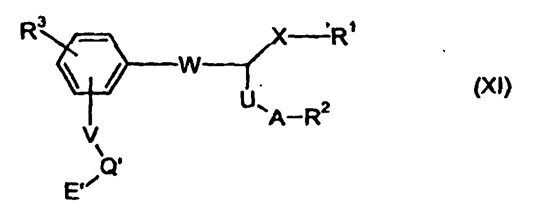 worin
R¹, R², R³, A, U, V, W, X und E' die vorstehend angegebenen Bedeutungen haben,
Q' die gleiche Bedeutung wie Q gemäß Anspruch 1 hat oder für 1,4-CH₂-Ph-CH₂- steht,
mit Aminen der Formel (XII)
NHR^{a}R^{b} (XII)
umsetzt;
worin
R^{a} und R^{b} unabhängig voneinander für Wasserstoff oder einen geradkettigen oder verzweigten Alkylrest mit bis zu 8 Kohlenstoffatomen stehen oder zusammen mit dem Stickstoffatom, an das sie gebunden sind, einen einen aromatischen Heterocyclus mit 1 bis 9 Kohlenstollatomen und bis zu 3 Heteroatomen aus der Reihe S, N und/oder O bilden können,
oder
[G] Verbindungen der Formel (XIII) 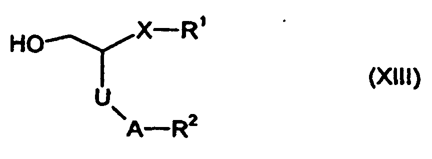 worin
R¹, R², A, U, X die vorstehend angegebenen Bedeutungen haben,
mit Verbindungen der Formel (XIV) 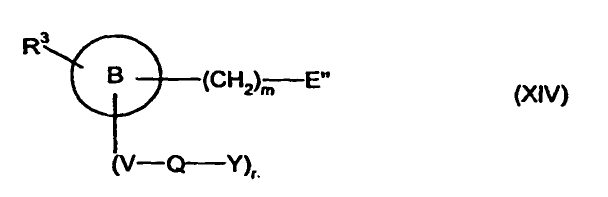 worin
R³, V, Q, Y, r und B die vorstehend angegebenen Bedeutungen haben,
m eine ganze Zahl von 1 bis 5 bedeutet, und
E" entweder eine Abgangsgruppe bedeutet, die in Gegenwart einer Base substituiert wird, oder eine gegebenenfalls aktivierte Hydroxyfunktion ist;
umsetzt;
oder
[H] Verbindungen der Formel (XV) 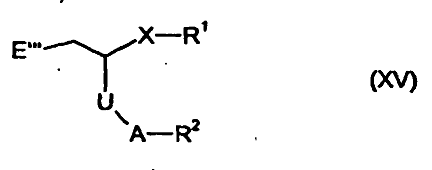 worin
R¹, R², A, U, X die vorstehend angegebenen Bedeutungen haben,
E''' entweder eine Abgangsgruppe bedeutet, die in Gegenwart einer Base substituiert wird, oder eine gegebenenfalls aktivierte Hydroxyfunktion ist;
mit Verbindungen der Formel (XVI) 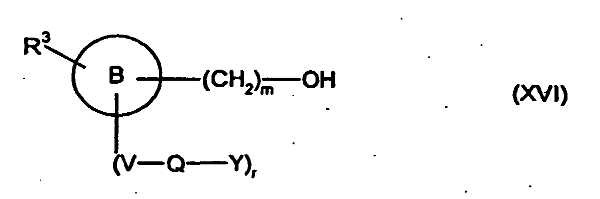 worin
R³, V, Q, Y, r und B die vorstehend angegebenen Bedeutungen haben,
m eine ganze Zahl von 1 bis 5 bedeutet,
umsetzt;
oder
[I] Verbindungen der Formel (XVII) 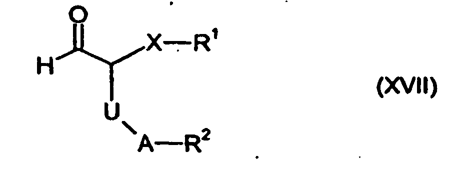 worin
R¹, R², A, U, X die vorstehend angegebenen Bedeutungen haben,
mit Verbindungen der Formel (XVIII) 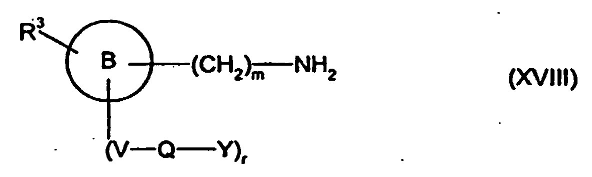 worin
R³, V, Q, Y, r und B die vorstehend angegebenen Bedeutungen haben,
m eine ganze Zahl von 0 bis 5 bedeutet,
zunächst zu einer Schiffschen Base umsetzt und diese dann mit gängigen Reduktionsmitteln reduziert oder direkt unter den Bedingungen einer reduktiven Alkylierung in Gegenwart eines Reduktionsmittels umsetzt;
oder
[J] Verbindungen der Formel (XIX) 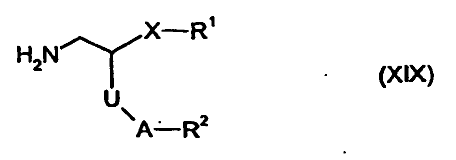 worin
R¹, R², A, U, X die vorstehend angegebenen Bedeutungen haben,
mit Verbindungen der Formel (XX) 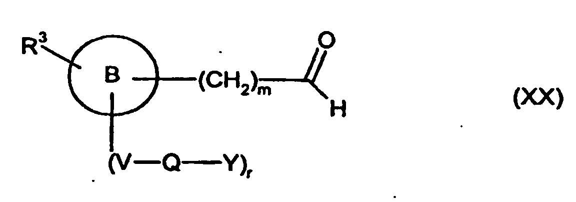 worin
R³, V, Q, Y, r und B die vorstehend angegebenen Bedeutungen haben,
m eine ganze Zahl von 0 bis 5 bedeutet,
zunächst zu einer Schiffschen Base umsetzt und diese dann mit gängigen Reduktionsmitteln reduziert oder direkt unter den Bedingungen einer reduktiven Alkylierung in Gegenwart eines Reduktionsmittels umsetzt,
oder
[K] Aldehyde der Formel (XXI) 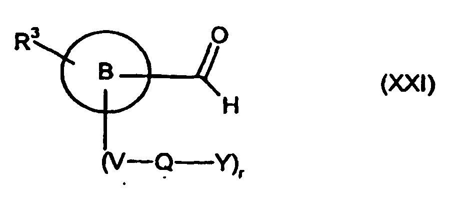 worin
R³, V, Q, Y, r und B die vorstehend angegebenen Bedeutungen haben,
mit Phosphorverbindungen der Formel (XXII) 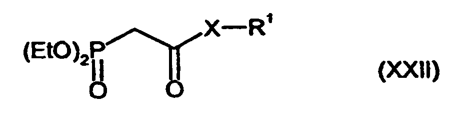 worin
X und R¹ die vorstehend angegebenen Bedeutungen haben,
zu Verbindungen der Formel (XXIII) 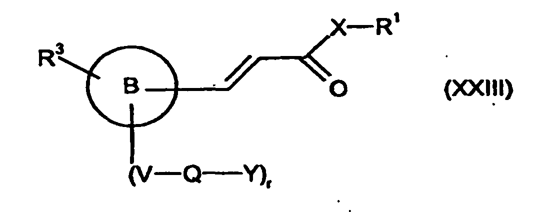 worin
R³, V, Q, Y, r, B, X und R¹ die vorstehend angegebenen Bedeutungen haben,
umgesetzt und anschließend durch aufeinanderfolgende Reduktion der Alkengruppe und der Carbonylgruppe und anschließende Substitution der durch Reduktion der Carbonylgruppe erzeugten Hydroxygruppe beziehungsweise des durch Umsetzung mit Halogenierungsmitteln aus der Hydroxygruppe erzeugten Halogenrestes mit Alkoholen, primären Aminen oder Thiolen sowie gegebenenfalls anschließende Oxidation zu den entsprechenden Sulfoxid- oder Sulfonverbindungen in Verbindungen der Formel (XXIV) überführt, 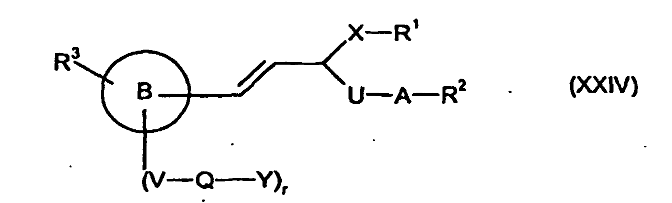 worin
R³, V, Q, Y, r, B, X, U,R² und R¹ die vorstehend angegebenen Bedeutungen haben,
U für O, NH oder S steht.

9. Arzneimittel enthaltend mindestens eine Verbindung der allgemeinen Formel (I) gemäß einem der vorhergehenden Ansprüche.

10. Verwendung von Verbindungen der Formel (I) gemäß einem der vorhergehenden Ansprüche zur Herstellung eines Arzneimittels zur Behandlung von Herz-Kreislauf-Erkrankungen.

11. Verwendung von Verbindungen der allgemeinen Formel (I) gemäß einem der vorhergehenden Ansprüche zur Herstellung von Arzneimitteln zur Behandlung von Angina pectoris, Ischämien und Herzinsuffizienz.

12. Verwendung von Verbindungen der allgemeinen Formel (I) gemäß einem der vorhergehenden Ansprüche zur Herstellung von Arzneimitteln zur Behandlung von Hypertonie, thromboembolischen Erkrankungen, Arteriosklerose und venösen Erkrankungen.

13. Verwendung von Verbindungen der allgemeinen Fonnel (I) gemäß einem der vorhergehenden Ansprüche zur Herstellung von Arzneimitteln zur Behandlung von fibrotischen Erkrankungen.

14. Verwendung nach Anspruch 15, **dadurch gekennzeichnet, dass** die fibrotische Erkrankung Leberfibrose ist.

## Claims

1. Compounds of the general formula (I) 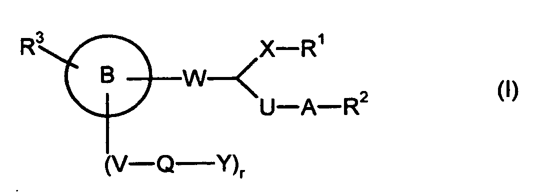 in which
B represents aryl having 6 to 10 carbon atoms,
r represents 0 or 1,
V is absent or represents O, NR⁴, NR⁴CONR⁴, NR⁴CO, NR⁴SO₂, COO, CONR⁴ or S(O)ₒ,
in which
R⁴ independently of any other radical R⁴ which may be present, represents hydrogen, straight-chain or branched alkyl having up to 8 carbon atoms, cycloalkyl having 3 to 8 carbon atoms, aryl having 6 to 10 carbon atoms or arylalkyl having 7 to 18 carbon atoms, where the aryl radical for its part may be mono- or polysubstituted by halogen, alkyl, alkoxy having up to 6 carbon atoms,
o represents 0, 1 or 2,
Q is absent or represents straight-chain or branched alkylene, straight-chain or branched alkenediyl or straight-chain or branched alkinediyl having in each case up to 15 carbon atoms, which may contain one or more groups from the group consisting of O, S(O)ₚ, NR⁵, CO, OCO, S-CO-, CONR⁵ and NR⁵SO₂ and which may be mono- or polysubstituted by halogen, hydroxyl or alkoxy having up to 4 carbon atoms, where, if appropriate, any two atoms of the chain above may be attached to one another forming a three- to eight-membered ring, or represents CONR⁵,
in which
R⁵ represents hydrogen, straight-chain or branched alkyl having up to 8 carbon atoms or cycloalkyl having 3 to 8 carbon atoms, which may be substituted by halogen or alkoxy having up to 4 carbon atoms,
p represents 0, 1 or 2,
Y represents hydrogen, NR⁶R⁷, aryl having 6 to 10 carbon atoms, an aromatic or saturated heterocycle having 1 to 9 carbon atoms and up to 3 heteroatoms from the group consisting of S, N and O or straight-chain or branched cycloalkyl having 3 to 8 carbon atoms, which may also be attached via N,
where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 8 carbon atoms, straight-chain or branched cycloalkyl having 3 to 8 carbon atoms, halogen, hydroxyl, COR⁸, CN, SR⁸, NO₂, NR¹⁰R¹¹, NR⁹COR¹² , NR⁹CONR⁹R¹² or CONR¹³R¹⁴,
in which
R⁶ and R⁷ in each case independently of one another represent hydrogen, straight-chain or branched alkyl, straight-chain or branched alkoxy, straight-chain or branched alkyloxyalkyl having up to 8 carbon atoms or cycloalkyl having 3 to 8 carbon atoms or aryl having 6 to 10 carbon atoms, which is optionally mono- or polysubstituted by aryl having 6 to 10 carbon atoms or by an aromatic heterocycle having 1 to 9 carbon atoms and up to 3 heteroatoms from the group consisting of S, N and O,
R⁸ represents hydrogen, straight-chain or branched alkyl having up to 8 carbon atoms, straight-chain or branched halogenoalkyl having up to 8 carbon atoms or cycloalkyl having 3 to 8 carbon atoms,
R⁹ represents hydrogen, straight-chain or branched alkyl having up to 8 carbon atoms or cycloalkyl having 3 to 8 carbon atoms,
R¹⁰, R¹¹, R¹³ and R¹⁴ independently of one another represent hydrogen, straight-chain or branched alkyl, straight-chain or branched alkenyl having up to 8 carbon atoms, aryl having 6 to 10 carbon atoms, an aromatic heterocycle having 1 to 9 carbon atoms and up to 3 heteroatoms from the group consisting of S, N and O, arylalkyl having 8 to 18 carbon atoms, cycloalkyl having 3 to 8 carbon atoms or a radical of the formula SO₂R¹⁵,
where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
or two substituents R¹⁰ and R¹¹ or R¹³ and R¹⁴ may be attached to one another forming a five- or six-membered ring which may contain O or N,
in which
R¹⁵ represents straight-chain or branched alkyl having up to 4 carbon atoms or aryl having 6 to 10 carbon atoms,
where the aryl radical for its part may be mono- or polysubstituted by halogen, CN, NO₂, alkyl, alkoxy, halogenoalkyl or halogenoalkoxy having up to 6 carbon atoms,
R¹² represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by halogen, hydroxyl, CN, NO₂, NH₂, NHCOR⁹, alkyl, alkoxy, halogenoalkyl or halogenoalkoxy having up to 6 carbon atoms;
and/or the cyclic radicals may in each case be mono- to trisubstituted by aryl having 6 to 10 carbon atoms, an aromatic or saturated heterocycle having 1 to 9 carbon atoms and up to 3 heteroatoms from the group consisting of S, N and O, which may also be attached via N,
which may be attached directly or via a group selected from O, S, SO, SO₂, NR⁹, CONR⁹, SO₂NR⁹, straight-chain or branched alkylene, straight-chain or branched alkenediyl, straight-chain or branched alkoxy, straight-chain or branched oxyalkyloxy, straight-chain or branched sulfonylalkyl, straight-chain or branched thioalkyl having in each case up to 8 carbon atoms and may be mono- to trisubstituted by straight-chain or branched alkyl, straight-chain or branched alkoxy, straight-chain or branched halogenoalkyl, straight-chain or branched halogenoalkoxy, carbonylalkyl or straight-chain or branched alkenyl having in each case up to 6 carbon atoms, phenyl, benzyl, halogen, SR⁸, CN, NO₂, NR¹⁷R¹⁸ CONR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, aryl having 6 to 10 carbon atoms or a radical of the formula SO₂R²⁰, where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
in which
R²⁰ represents straight-chain or branched alkyl having up to 4 carbon atoms or aryl having 6 to 10 carbon atoms,
where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by halogen, hydroxyl, CN, NO₂, NH₂, NHCOR⁹, alkyl, alkoxy, halogenoalkyl or halogenoalkoxy having up to 6 carbon atoms;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, OH, halogen, straight-chain or branched alkyl, straight-chain or branched halogenoalkyl, straight-chain or branched alkoxy or straight-chain or branched halogenoalkoxy having in each case up to 4 carbon atoms,
W represents straight-chain or branched alkylene, straight-chain or branched alkenediyl having in each case up to 4 carbon atoms, which may contain a group selected from O and NR²³,
in which
R²³ represents hydrogen, straight-chain or branched alkyl having up to 8 carbon atoms or cycloalkyl having 3 to 8 carbon atoms,
U represents straight-chain or branched alkylene having up to 4 carbon atoms, O, NH, S, SO or SO₂,
A is absent or represents phenyl or an aromatic heterocycle having 1 to 9 carbon atoms and up to 3 heteroatoms from the group consisting of S, N and O,
which may optionally be mono- to trisubstituted by halogen, straight-chain or branched alkyl, straight-chain or branched halogenoalkyl or straight-chain or branched alkoxy having in each case up to 4 carbon atoms,
R² represents COOR²⁶ or CN,
in which
R²⁶ represents hydrogen or straight-chain or branched alkyl having up to 8 carbon atoms;
X represents straight-chain or branched alkylene, straight-chain or branched alkenediyl having in each case up to 8 carbon atoms, which may contain a group selected from O, S(O)ᵣ, NR³⁰, or a three- to six-membered saturated or unsaturated carbocycle which optionally has one or more straight-chain or branched alkyl radicals having 1 to 6 carbon atoms and optionally one or two heteroatoms from the group consisting of S(O)ᵣ, NR³² and O,
in which
r represents 0, 1 or 2,
R³⁰ represents hydrogen, straight-chain or branched alkyl having up to 6 carbon atoms, phenyl or arylalkyl having 7 to 12 carbon atoms,
R³² represents hydrogen, straight-chain or branched alkyl having up to 6 carbon atoms, cycloalkyl having 3 to 6 carbon atoms or phenyl,
R¹ represents CN or COOR³⁵,
in which
R³⁵ represents hydrogen or straight-chain or branched alkyl having up to 6 carbon atoms;
with the proviso that Y may not be phenyl or phenyl substituted exclusively by one or two radicals from the group consisting of straight-chain or branched alkyl, straight-chain or branched alkoxy having in each case up to 8 carbon atoms, halogen, CF₃, OCF₃ and CN, if simultaneously B is phenyl, V is absent or represents O, Q represents straight-chain alkylene having 1 to 4 carbon atoms and is optionally attached to Y via an oxygen atom, W represents an alkylene group or an alkenediyl group having in each case 1 to 6 carbon atoms, U represents an alkylene group having up to 4 carbon atoms, O, S, SO or SO₂, A represents phenyl and X represents straight-chain alkylene having 1 to 8 carbon atoms and is optionally attached directly via O, S, SO or SO₂ to the carbon atom which carries the groups W and U.

2. Compounds according to Claim 1,
in which
B represents phenyl or naphthyl
r represents 0 or 1,
V is absent or represents O, NR⁴ or S(O)ₙ
in which
R⁴ represents hydrogen,
n represents 0,
Q is absent or represents straight-chain or branched alkylene, straight-chain or branched alkenediyl or straight-chain or branched alkinediyl having in each case up to 15 carbon atoms, which may contain one or more groups selected from O, S(O)ₚ, NR⁵, CONR⁵, S-CO- and OCO and which may be mono- or disubstituted by halogen or hydroxyl, or represents CONR⁵,
in which
R⁵ represents hydrogen,
p represents 0 or 1,
Y represents hydrogen, NR⁶R⁷, phenyl, napthyl or a heterocycle from the group 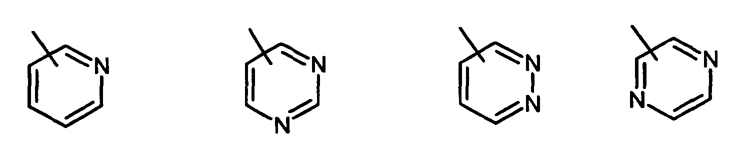 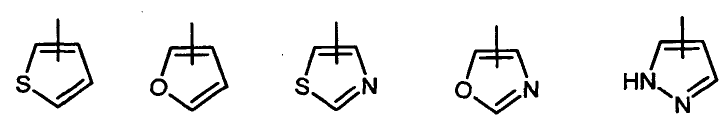 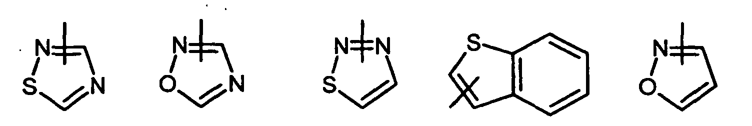 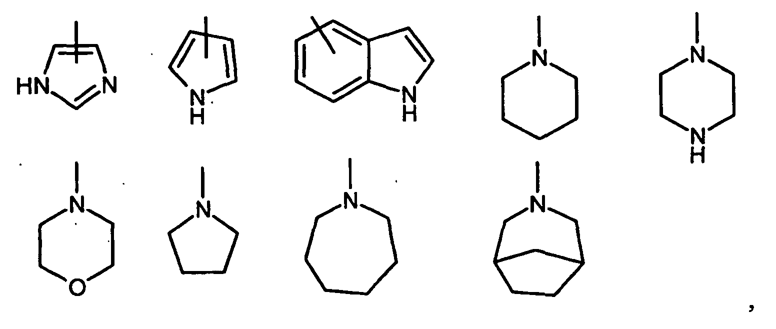 where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 4 carbon atoms, straight-chain or branched cycloalkyl having 3 to 7 carbon atoms, F, Cl, Br, I, NO₂, COR⁸, SR⁸; -NR¹⁰R¹¹, NR⁹COR¹² or CONR¹³R¹⁴,
in which
R⁶ and R⁷ in each case independently of one another represent hydrogen, straight-chain or branched alkyl, straight-chain or branched alkoxy or straight-chain or branched alkyloxyalkyl having in each case up to 4 carbon atoms or cycloalkyl having 3 to 8 carbon atoms or aryl having 6 to 10 carbon atoms, which is optionally mono- or polysubstituted by aryl having 6 to 10 carbon atoms or an aromatic heterocycle having 1 to 9 carbon atoms and up to 3 heteroatoms from the group consisting of S, N and O,
R⁸ represents hydrogen, straight-chain or branched alkyl having up to 4 carbon atoms, or straight-chain or branched halogenoalkyl having up to 4 carbon atoms,
R⁹ represents hydrogen, or straight-chain or branched alkyl having up to 4 carbon atoms,
R¹⁰, R¹¹, R¹³ and R¹⁴ independently of one another represent hydrogen, straight-chain or branched alkyl having up to 4 carbon atoms, or phenyl,
where the phenyl radical may be mono- to trisubstituted by F, Cl Br, hydroxyl, methyl, ethyl, n- propyl, i-propyl, n-butyl, s- butyl, i- butyl, t-butyl, methoxy, ethoxy, amino, acetylamino, NO₂, CF₃, OCF₃ or CN,
or two substituents R¹⁰ and R¹¹ or R¹³ and R¹⁴ may be attached to one another forming a five- or six-membered ring which may be interrupted by O or N,
R¹² represents hydrogen, straight-chain or branched alkyl having up to 4 carbon atoms, or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 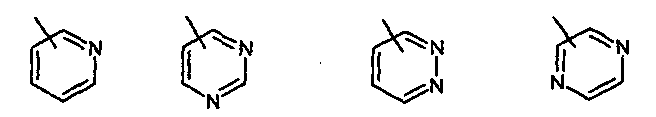 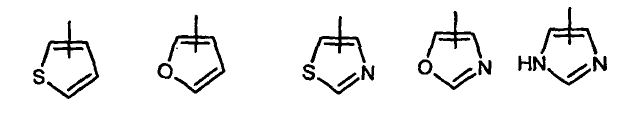 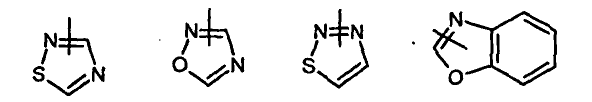 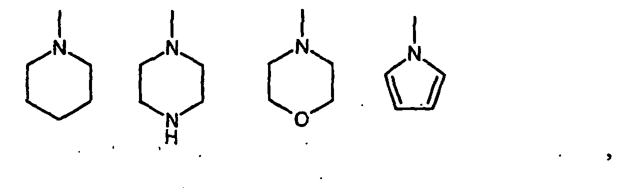 which are attached directly or via a group selected from O, S, SO, SO₂, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 4 carbon atoms and may be mono- to trisubstituted by straight-chain or branched alkyl, straight-chain or branched alkoxy, straight-chain or branched halogenoalkyl or straight-chain or branched alkenyl having in each case up to 4 carbon atoms, phenyl, benzyl, F, Cl, Br, I, CN, NO₂, NR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, phenyl, where the phenyl radical may be mono- to trisubstituted by F, Cl Br, hydroxyl, methyl, ethyl, n-propyl, i-propyl, n-butyl, s-butyl ,i-butyl, t-butyl, methoxy, ethoxy, amino, acetylamino, NO₂, CF₃, OCF₃ or CN or represent a radical of the formula SO₂R²⁰,
in which
R²⁰ represents straight-chain or branched alkyl having up to 4 carbon atoms or phenyl,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, OH, F, Cl, Br, straight-chain or branched alkyl, straight-chain or branched halogenoalkyl, straight-chain or branched alkoxy or straight-chain or branched halogenoalkoxy having in each case up to 4 carbon atoms,
W represents CH₂CH₂, CH=CH, CH₂O, OCH₂, CH₂OCH₂, CH₂NH, NHCH₂ or CH₂NHCH₂,
U represents straight-chain alkylene having up to 4 carbon atoms, O, NH, S, SO or SO₂,
A is absent or represents phenyl, pyridyl, thienyl or thiazolyl, which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² represents COOR²⁶ or CN,
in which
R²⁶ represents hydrogen or straight-chain or branched alkyl having up to 4 carbon atoms;
X represents straight-chain or branched alkylene having up to 4 carbon atoms, which may contain a group selected from O, S(O)ᵣ, NR³⁰, or a three- to six-membered saturated or unsaturated carbocycle having optionally one or more straight-chain or branched alkyl radicals having 1 to 4 carbon atoms and having optionally one or two heteroatoms from the group consisting of S(O)ᵣ, NR³² and O,
in which
r represents 0, 1 or 2,
R³⁰ represents hydrogen, straight-chain or branched alkyl having up to 6 carbon atoms, phenyl or benzyl,
R³² represents hydrogen, straight-chain or branched alkyl having up to 6 carbon atoms, cycloalkyl having 3 to 6 carbon atoms or phenyl,
R¹ represents CN or COOR³⁵,
in which
R³⁵ represents hydrogen or straight-chain or branched alkyl having up to 6 carbon atoms;
with the proviso that Y may not be phenyl or phenyl substituted exclusively by one or two radicals from the group consisting of straight-chain or branched alkyl, straight-chain or branched alkoxy having in each case up to 4 carbon atoms, halogen, CF₃, and OCF₃, if simultaneously V is absent or represents O, Q represents straight-chain alkylene having 1 to 10 carbon atoms and is optionally attached to Y via an oxygen atom, W is an ethylene group or an ethanediyl group, U represents an alkylene group having up to 4 carbon atoms, O, S, SO or SO₂, A represents phenyl and X represents straight-chain alkylene having 1 to 4 carbon atoms and is optionally attached directly via O, S, SO or SO₂ to the carbon atom which carries the groups W and U.

3. Compounds according to Claim 1,
in which
B represents phenyl or naphthyl
r represents 0 or 1,
V is absent or represents O, NR⁴ or S(O)ₙ
in which
R⁴ represents hydrogen,
n represents 0,
Q is absent or represents straight-chain or branched alkylene, straight-chain or branched alkenediyl or straight-chain or branched alkinediyl having in each case up to 15 carbon atoms, which may contain one or more groups selected from O, S(O)ₚ, NR⁵, CONR⁵, S-CO- and OCO and which may be mono- or disubstituted by halogen or hydroxyl, or represents CONR⁵,
in which
R⁵ represents hydrogen,
p represents 0 or 1,
Y represents hydrogen, NR⁶R⁷, phenyl, napthyl or a heterocycle from the group 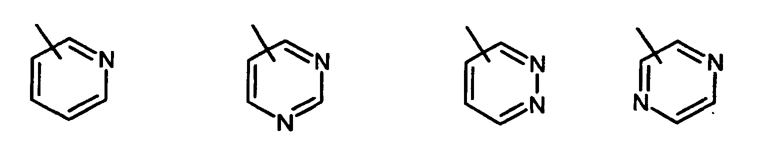 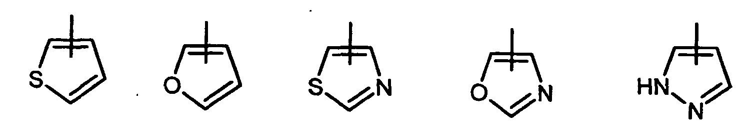 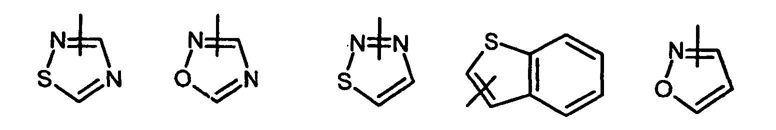 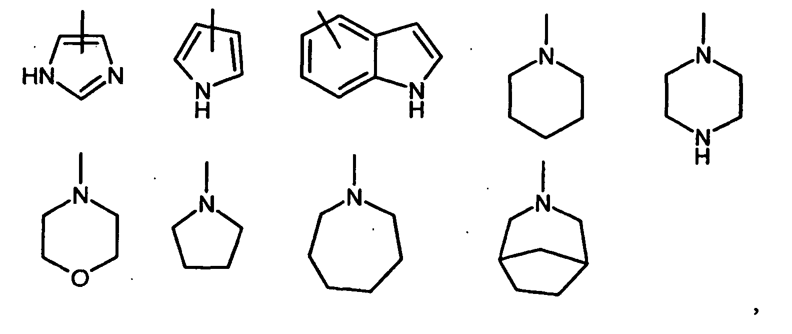 where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 4 carbon atoms, straight-chain or branched cycloalkyl having 3 to 7 carbon atoms, F, Cl, Br, I, NO₂, COR⁸, SR⁸, NR¹⁰R¹¹, NR⁹COR¹² or CONR¹³R¹⁴,
in which
R⁶ and R⁷ in each case independently of one another represent hydrogen, straight-chain or branched alkyl, straight-chain or branched alkoxy or straight-chain or branched alkyloxyalkyl having in each case up to 4 carbon atoms or cycloalkyl having 3 to 8 carbon atoms or aryl having 6 to 10 carbon atoms, which is optionally mono- or polysubstituted by aryl having 6 to 10 carbon atoms or an aromatic heterocycle having 1 to 9 carbon atoms and up to 3 heteroatoms from the group consisting of S, N and O,
R⁸ represents hydrogen, straight-chain or branched alkyl having up to 4 carbon atoms, or straight-chain or branched halogenoalkyl having up to 4 carbon atoms,
R⁹ represents hydrogen, or straight-chain or branched alkyl having up to 4 carbon atoms,
R¹⁰, R¹¹, R¹³ and R¹⁴ independently of one another represent hydrogen, straight-chain or branched alkyl having up to 4 carbon atoms, or phenyl,
where the phenyl radical may be mono- to trisubstituted by F, Cl Br, hydroxyl, methyl, ethyl, n- propyl, i-propyl, n-butyl, s- butyl, i- butyl, t-butyl, methoxy, ethoxy, amino, acetylamino, NO₂, CF₃, OCF₃ or CN,
or two substituents R¹⁰ and R¹¹ or R¹³ and R¹⁴ may be attached to one another forming a five- or six-membered ring which may be interrupted by O or N,
R¹² represents hydrogen, straight-chain or branched alkyl having up to 4 carbon atoms, or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 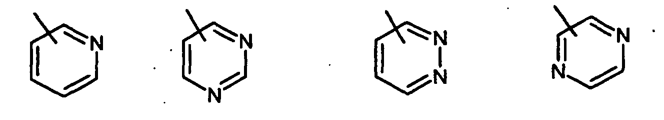 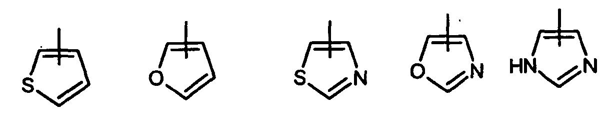 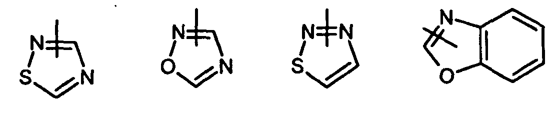 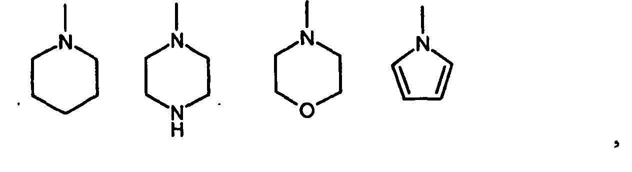 which are attached directly or via a group selected from O, S, SO, SO₂, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 4 carbon atoms and may be mono- to trisubstituted by straight-chain or branched alkyl, straight-chain or branched alkoxy, straight-chain or branched halogenoalkyl or straight-chain or branched alkenyl having in each case up to 4 carbon atoms, phenyl, benzyl, F, Cl, Br, I, CN, NO₂, NR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷ R¹⁸ independently of one another represent hydrogen, straight-chain or branched alkyl having up to 8 carbon atoms, cycloalkyl having 3 to 8 carbon atoms, phenyl, where the phenyl radical may be mono- to trisubstituted by F, Cl Br, hydroxyl, methyl, ethyl, n-propyl, i-propyl, n-butyl, s-butyl ,i-butyl, t-butyl, methoxy, ethoxy, amino, acetylamino, NO₂, CF₃, OCF₃ or CN or represent a radical of the formula SO₂R²⁰,
in which
R²⁰ represents straight-chain or branched alkyl having up to 4 carbon atoms or phenyl,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, OH, F, Cl, Br, straight-chain or branched alkyl, straight-chain or branched halogenoalkyl, straight-chain or branched alkoxy or straight-chain or branched halogenoalkoxy having in each case up to 4 carbon atoms,
W represents CH₂CH₂, CH=CH, CH₂O, OCH₂, CH₂OCH₂, CH₂NH, NHCH₂ or CH₂NHCH₂,
U represents straight-chain or branched alkylene having up to 4 carbon atoms, O, NH, S, SO or SO₂,
A is absent or represents phenyl, pyridyl, thienyl or thiazolyl, which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² represents COOH,
X represents straight-chain or branched alkylene having up to 4 carbon atoms, which may contain a group selected from O, S(O)ᵣ, NR³⁰, or a three- to six-membered saturated or unsaturated carbocycle having optionally one or more straight-chain or branched alkyl radicals having 1 to 4 carbon atoms and having optionally one or two heteroatoms from the group consisting of 8(0)ᵣ, NR³² and O,
in which
r represents 0, 1 or 2,
R³⁰ represents hydrogen, straight-chain or branched alkyl having up to 6 carbon atoms, phenyl or benzyl,
R³² represents hydrogen, straight-chain or branched alkyl having up to 6 carbon atoms, cycloalkyl having 3 to 6 carbon atoms or phenyl,
R¹ represents COOH,
with the proviso that Y may not be phenyl or phenyl substituted exclusively by one or two radicals from the group consisting of straight-chain or branched alkyl, straight-chain or branched alkoxy having in each case up to 4 carbon atoms, halogen, CF₃ and OCF₃, if simultaneously V is absent or represents O, Q represents straight-chain alkylene having 1 to 10 carbon atoms and is optionally attached to Y via an oxygen atom, W is an ethylene group or an ethanediyl group, U represents an alkylene group having up to 4 carbon atoms, O, S, SO or SO₂, A represents phenyl and X represents straight-chain alkylene having 1 to 4 carbon atoms and is optionally attached directly via O, S, SO or SO₂ to the carbon atom which carries the groups W and U;

4. Compounds according to Claim1,
in which
B represents phenyl
r represents 0 or 1,
V is absent or represents O, NR⁴ or S(O)ₙ
in which
R⁴ represents hydrogen,
n represents 0,
Q is absent or represents straight-chain or branched alkylene, straight-chain or branched alkenediyl or straight-chain or branched alkinediyl having in each case up to 15 carbon atoms, which may contain one or more groups selected from O, S(O)ₚ, NR⁵, CONR⁵, S-CO- and OCO and which may be mono- or disubstituted by halogen or hydroxyl, or represents CONR⁵,
in which
R⁵ represents hydrogen,
p represents 0 or 1,
Y represents hydrogen, NR⁶R⁷, phenyl, napthyl or a heterocycle from the group 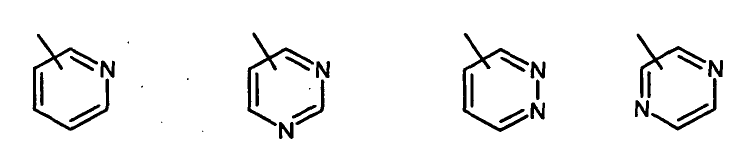 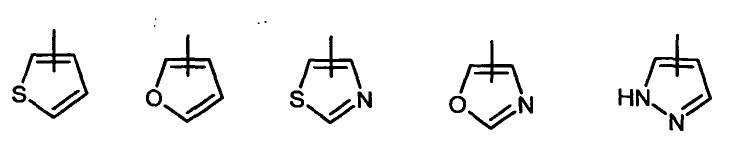 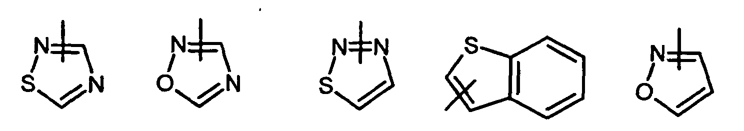 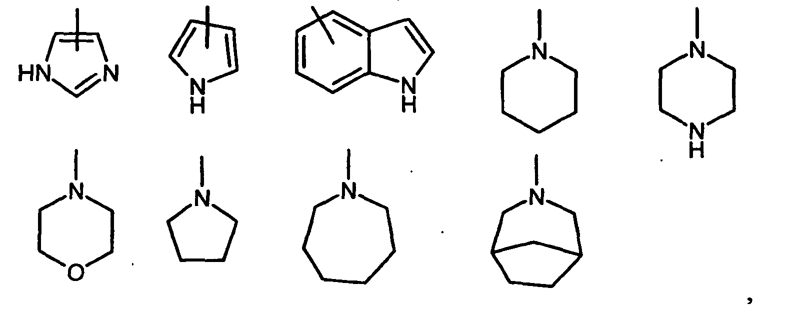 where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 4 carbon atoms, straight-chain or branched cycloalkyl having 3 to 7 carbon atoms, F, Cl, Br, I, NO₂, COR⁸, SR⁸, NR¹⁰R¹¹, NR⁹COR¹² or CONR¹³R¹⁴,
in which
R⁶ and R⁷ in each case independently of one another represent hydrogen, straight-chain or branched alkyl, straight-chain or branched alkoxy or straight-chain or branched alkyloxyalkyl having in each case up to 4 carbon atoms or cycloalkyl having 3 to 8 carbon atoms or aryl having 6 to 10 carbon atoms, which is optionally mono- or polysubstituted by aryl having 6 to 10 carbon atoms or an aromatic heterocycle having 1 to 9 carbon atoms and up to 3 heteroatoms from the group consisting of S, N and O,
R⁸ represents hydrogen, straight-chain or branched alkyl having up to 4 carbon atoms, or straight-chain or branched halogenoalkyl having up to 4 carbon atoms,
R⁹ represents hydrogen, or straight-chain or branched alkyl having up to 4 carbon atoms,
R¹⁰, R¹¹, R¹³ and R¹⁴ independently of one another represent hydrogen, straight-chain or branched alkyl having up to 4 carbon atoms, or phenyl,
where the phenyl radical may be mono- to trisubstituted by F, Cl Br, hydroxyl, methyl, ethyl, n- propyl, i-propyl, n-butyl, s- butyl, i- butyl, t-butyl, methoxy, ethoxy, amino, acetylamino, NO₂, CF₃, OCF₃ or CN,
or two substituents R¹⁰ and R¹¹ or R¹³ and R¹⁴ may be attached to one another forming a five- or six-membered ring which may be interrupted by O or N,
R¹² represents hydrogen, straight-chain or branched alkyl having up to 4 carbon atoms, or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 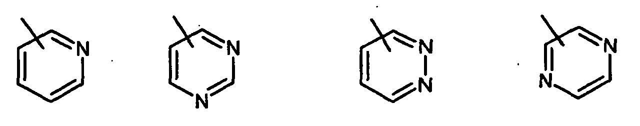 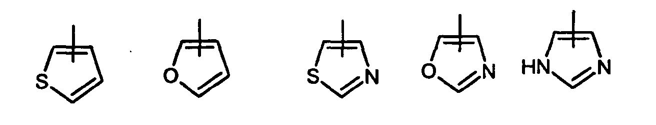 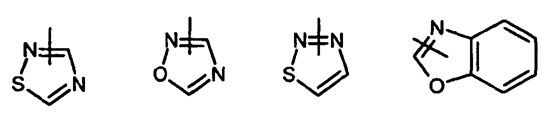 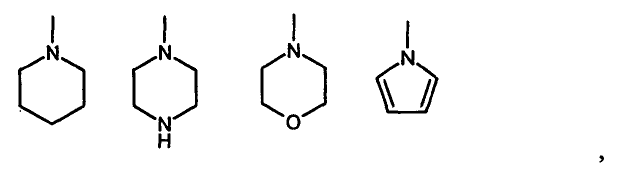 which are attached directly or via a group selected from O, S, SO, SO₂, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 4 carbon atoms and may be mono- to trisubstituted by straight-chain or branched alkyl, straight-chain or branched alkoxy, straight-chain or branched halogenoalkyl or straight-chain or branched alkenyl having in each case up to 4 carbon atoms, phenyl, benzyl, F, Cl, Br, I, CN, NO₂, NR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, phenyl, where the phenyl radical may be mono-to trisubstituted by F, Cl Br, hydroxyl, methyl, ethyl, n-propyl, i-propyl, n-butyl, s-butyl ,i-butyl, t-butyl, methoxy, ethoxy, amino, acetylamino, NO₂, CF₃, OCF₃ or CN or represent a radical of the formula SO₂R²⁰,
in which
R²⁰ represents straight-chain or branched alkyl having up to 4 carbon atoms or phenyl,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen,
W represents CH₂CH₂ or
U represents CH₂,
A represents phenyl,
R² represents COOH,
X represents (CH₂)₄
R¹ represents COOH,
with the proviso that Y may not be phenyl or phenyl substituted exclusively by one or two radicals from the group consisting of straight-chain or branched alkyl, straight-chain or branched alkoxy having in each case up to 4 carbon atoms, halogen, CF₃ and OCF₃, if simultaneously V is absent or represents O, Q represents straight-chain alkylene having 1 to 10 carbon atoms and is optionally attached to Y via an oxygen atom, W is an ethylene group or an ethanediyl group, U represents CH₂, A represents phenyl and X represents (CH₂)₄.

5. Compounds of the formula (I), in which
B represents an aromatic heterocycle having 1 to 9 carbon atoms and up to 3 heteroatoms from the group consisting of S, N and O,
r represents 0 or 1,
V is absent or represents O, NR⁴, NR⁴CONR⁴, NR⁴CO, NR⁴SO₂, COO, CONR⁴ or S(O)ₒ,
in which
R⁴ independently of any other radical R⁴ which may be present, represents hydrogen, straight-chain or branched alkyl having up to 8 carbon atoms, cycloalkyl having 3 to 8 carbon atoms, aryl having 6 to 10 carbon atoms or arylalkyl having 7 to 18 carbon atoms, where the aryl radical for its part may be mono- or polysubstituted by halogen, alkyl, alkoxy having up to 6 carbon atoms,
o represents 0, 1 or 2,
Q is absent or represents straight-chain or branched alkylene, straight-chain or branched alkenediyl or straight-chain or branched alkinediyl having in each case up to 15 carbon atoms, which may contain one or more groups from the group consisting of O, S(O)ₚ, NR⁵, CO, OCO, S-CO-, CONR⁵ and NR⁵SO₂ and which may be mono- or polysubstituted by halogen, hydroxyl or alkoxy having up to 4 carbon atoms, where, if appropriate, any two atoms of the chain above may be attached to one another forming a three- to eight-membered ring, or represents CONR⁵,
in which
R⁵ represents hydrogen, straight-chain or branched alkyl having up to 8 carbon atoms or cycloalkyl having 3 to 8 carbon atoms, which may be substituted by halogen or alkoxy having up to 4 carbon atoms,
p represents 0, 1 or 2,
Y represents hydrogen, NR⁶R⁷, aryl having 6 to 10 carbon atoms, an aromatic or saturated heterocycle having 1 to 9 carbon atoms and up to 3 heteroatoms from the group consisting of S, N and O or straight-chain or branched cycloalkyl having 3 to 8 carbon atoms, which may also be attached via N,
where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 8 carbon atoms, straight-chain or branched cycloalkyl having 3 to 8 carbon atoms, halogen, hydroxyl, COR⁸, CN, SR⁸, NO₂, NR¹⁰R¹¹, NR⁹COR¹² , NR⁹CONR⁹R¹² or CONR¹³R¹⁴,
in which
R⁶ and R⁷ in each case independently of one another represent hydrogen, straight-chain or branched alkyl, straight-chain or branched alkoxy, straight-chain or branched alkyloxyalkyl having up to 8 carbon atoms or cycloalkyl having 3 to 8 carbon atoms or aryl having 6 to 10 carbon atoms, which is optionally mono- or polysubstituted by aryl having 6 to 10 carbon atoms or by an aromatic heterocycle having 1 to 9 carbon atoms and up to 3 heteroatoms from the group consisting of S, N and O,
R⁸ represents hydrogen, straight-chain or branched alkyl having up to 8 carbon atoms, straight-chain or branched halogenoalkyl having up to 8 carbon atoms or cycloalkyl having 3 to 8 carbon atoms,
R⁹ represents hydrogen, straight-chain or branched alkyl having up to 8 carbon atoms or cycloalkyl having 3 to 8 carbon atoms,
R¹⁰, R¹¹, R¹³ and R¹⁴ independently of one another represent hydrogen, straight-chain or branched alkyl, straight-chain or branched alkenyl having up to 8 carbon atoms, aryl having 6 to 10 carbon atoms, an aromatic heterocycle having 1 to 9 carbon atoms and up to 3 heteroatoms from the group consisting of S, N and O, arylalkyl having 8 to 18 carbon atoms, cycloalkyl having 3 to 8 carbon atoms or a radical of the formula SO₂R¹⁵,
where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
or two substituents R¹⁰ and R¹¹ or R¹³ and R¹⁴ may be attached to one another forming a five- or six-membered ring which may contain O or N,
in which
R¹⁵ represents straight-chain or branched alkyl having up to 4 carbon atoms or aryl having 6 to 10 carbon atoms,
where the aryl radical for its part may be mono- or polysubstituted by halogen, CN, NO₂, alkyl, alkoxy, halogenoalkyl or halogenoalkoxy having up to 6 carbon atoms,
R¹² represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by halogen, hydroxyl, CN, NO₂, NH₂, NHCOR⁹, alkyl, alkoxy, halogenoalkyl or halogenoalkoxy having up to 6 carbon atoms;
and/or the cyclic radicals may in each case be mono- to trisubstituted by aryl having 6 to 10 carbon atoms, an aromatic or saturated heterocycle having 1 to 9 carbon atoms and up to 3 heteroatoms from the group consisting of S, N and O, which may also be attached via N,
which may be attached directly or via a group selected from O, S, SO, SO₂, NR⁹, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 8 carbon atoms and may be mono- to trisubstituted by straight-chain or branched alkyl, straight-chain or branched alkoxy, straight-chain or branched halogenoalkyl, straight-chain or branched halogenoalkoxy, carbonylalkyl or straight-chain or branched alkenyl having in each case up to 6 carbon atoms, phenyl, benzyl, halogen, SR⁸, CN, NO₂, NR¹⁷R¹⁸, CONR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, aryl having 6 to 10 carbon atoms or a radical of the formula SO₂R²⁰, where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
in which
R²⁰ represents straight-chain or branched alkyl having up to 4 carbon atoms or aryl having 6 to 10 carbon atoms,
where the aryl radical for its part may be mono- or polysubstituted by halogen, hydroxyl, CN, NO₂, NH₂, NHCOR⁹, alkyl, alkoxy, halogenoalkyl or halogenoalkoxy having up to 6 carbon atoms,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by halogen, hydroxyl, CN, NO₂, NH₂, NHCOR⁹, alkyl, alkoxy, halogenoalkyl or halogenoalkoxy having up to 6 carbon atoms;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, halogen, straight-chain or branched alkyl, straight-chain or branched halogenoalkyl, straight-chain or branched alkoxy or straight-chain or branched halogenoalkoxy having in each case up to 4 carbon atoms,
W represents straight-chain or branched alkylene, straight-chain or branched alkenediyl having in each case up to 4 carbon atoms, which may contain a group selected from O and NR²³,
in which
R²³ represents hydrogen, straight-chain or branched alkyl having up to 8 carbon atoms or cycloalkyl having 3 to 8 carbon atoms,
U represents straight-chain or branched alkylene having up to 4 carbon atoms, O, NH, S, SO or SO₂,
A is absent or represents phenyl or an aromatic heterocycle having 1 to 9 carbon atoms and up to 3 heteroatoms from the group consisting of S, N and O,
which may optionally be mono- to trisubstituted by halogen, straight-chain or branched alkyl, straight-chain or branched halogenoalkyl or straight-chain or branched alkoxy having in each case up to 4 carbon atoms,
R² represents COOR²⁶ or CN,
in which
R²⁶ represents hydrogen or straight-chain or branched alkyl having up to 8 carbon atoms;
X represents straight-chain or branched alkylene, straight-chain or branched alkenediyl having in each case up to 8 carbon atoms, which may contain a group selected from O, S(O)ᵣ, NR³⁰, or a three- to six-membered saturated or unsaturated carbocycle which optionally has one or more straight-chain or branched alkyl radicals having 1 to 6 carbon atoms and optionally one or two heteroatoms from the group consisting of S(O)ᵣ, NR³² and O,
in which
r represents 0, 1 or 2,
R³⁰ represents hydrogen, straight-chain or branched alkyl having up to 6 carbon atoms, phenyl or arylalkyl having 7 to 12 carbon atoms,
R³² represents hydrogen, straight-chain or branched alkyl having up to 6 carbon atoms, cycloalkyl having 3 to 6 carbon atoms or phenyl,
R¹ represents CN or COOR³⁵,
in which
R³⁵ represents hydrogen or straight-chain or branched alkyl having up to 6 carbon atoms.

6. Compounds according to Claim 5,
in which
B represents a heterocycle from the group consisting of 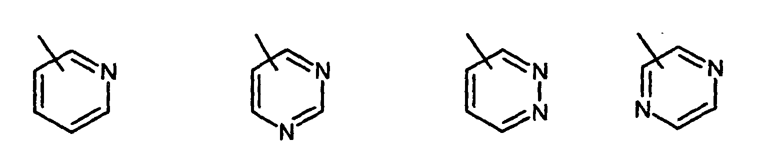 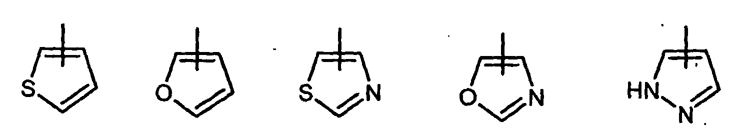 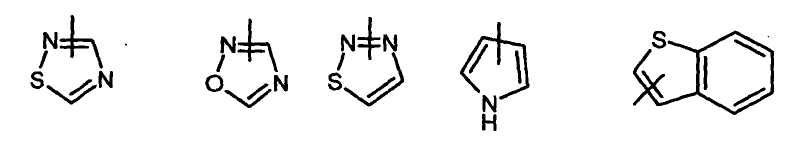 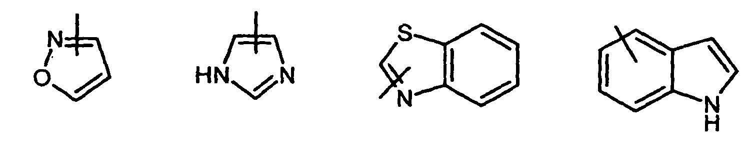
r represents 0 or 1,
V is absent or represents O, NR⁴ or S(O)ₙ
in which
R⁴ represents hydrogen,
n represents 0,
Q is absent or represents straight-chain or branched alkylene, straight-chain or branched alkenediyl having in each case up to 15 carbon atoms, which may contain one or more groups selected from O, S(O)ₚ, NR⁵, CONR⁵, S-CO- and OCO and which may be mono- or disubstituted by halogen or hydroxyl, or represents CONR⁵,
in which
R⁵ represents hydrogen,
p represents 0 or 1,
Y represents hydrogen, NR⁶R⁷, phenyl, napthyl or a heterocycle from the group 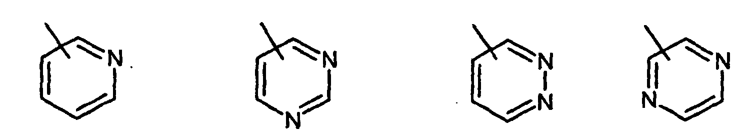 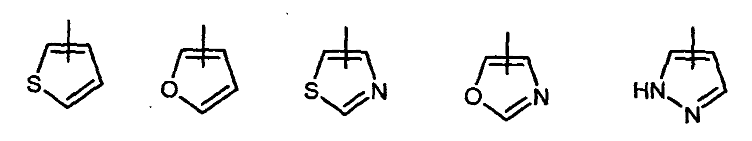 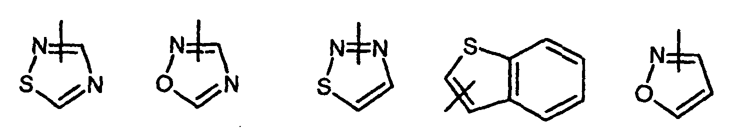 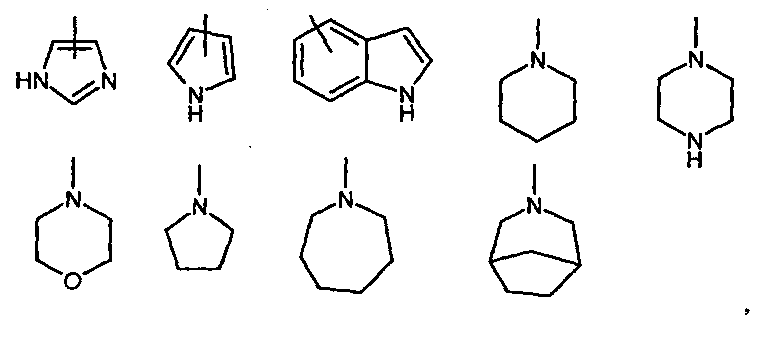 where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 4 carbon atoms, straight-chain or branched cycloalkyl having 3 to 7 carbon atoms, F, Cl, Br, I, NO₂, COR⁸, SR⁸, NR¹⁰R¹¹, NR⁹COR¹² or CONR¹³R¹⁴,
in which
R⁶ and R⁷ in each case independently of one another represent hydrogen, straight-chain or branched alkyl, straight-chain or branched alkoxy or straight-chain or branched alkyloxyalkyl having in each case up to 4 carbon atoms or cycloalkyl having 3 to 8 carbon atoms or aryl having 6 to 10 carbon atoms, which is optionally mono- or polysubstituted by aryl having 6 to 10 carbon atoms or an aromatic heterocycle having 1 to 9 carbon atoms and up to 3 heteroatoms from the group consisting of S, N and O,
R⁸ represents hydrogen, straight-chain or branched alkyl having up to 4 carbon atoms, or straight-chain or branched halogenoalkyl having up to 4 carbon atoms,
R⁹ represents hydrogen, or straight-chain or branched alkyl having up to 4 carbon atoms,
R¹⁰, R¹¹, R¹³ and R¹⁴ independently of one another represent hydrogen, straight-chain or branched alkyl having up to 4 carbon atoms, or phenyl,
where the phenyl radical may be mono- to trisubstituted by F, Cl Br, hydroxyl, methyl, ethyl, n- propyl, i-propyl, n-butyl, s- butyl, i- butyl, t-butyl, methoxy, ethoxy, amino, acetylamino, NO₂, CF₃, OCF₃ or CN,
or two substituents R¹⁰ and R¹¹ or R¹³ and R¹⁴ may be attached to one another forming a five- or six-membered ring which may be interrupted by O or N,
R¹² represents hydrogen, straight-chain or branched alkyl having up to 4 carbon atoms, or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 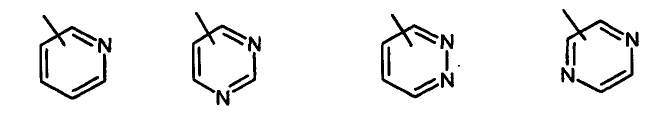 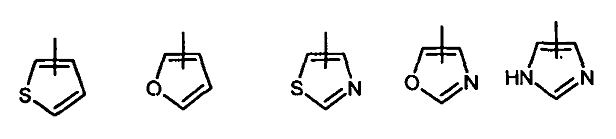 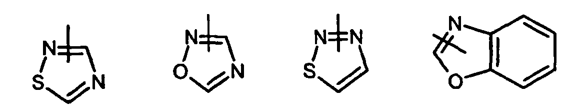 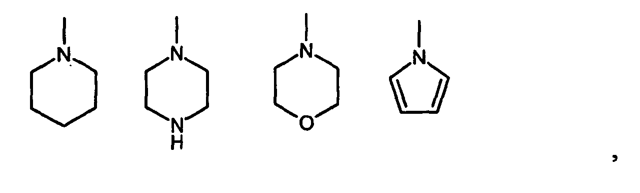 which are attached directly or via a group selected from O, S, SO, SO₂, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 4 carbon atoms and may be mono- to trisubstituted by straight-chain or branched alkyl, straight-chain or branched alkoxy, straight-chain or branched halogenoalkyl or straight-chain or branched alkenyl having in each case up to 4 carbon atoms, phenyl, benzyl, F, Cl, Br, I, CN, NO₂, NR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, phenyl, where the phenyl radical may be mono- to trisubstituted by F, Cl Br, hydroxyl, methyl, ethyl, n-propyl, i-propyl, n-butyl, s-butyl ,i-butyl, t-butyl, methoxy, ethoxy, amino, acetylamino, NO₂, CF₃, OCF₃ or CN or represent a radical of the formula SO₂R²⁰,
in which
R²⁰ represents straight-chain or branched alkyl having up to 4 carbon atoms or phenyl,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, F, Cl, Br, straight-chain or branched alkyl, straight-chain or branched halogenoalkyl, straight-chain or branched alkoxy or straight-chain or branched halogenoalkoxy having in each case up to 4 carbon atoms,
W represents CH₂CH₂, CH=CH, CH₂O, OCH₂, CH₂OCH₂, CH₂NH, NHCH₂ or CH₂NHCH₂,
U represents straight-chain alkylene having up to 4 carbon atoms, O, NH, S, SO or SO₂,
A is absent or represents phenyl, pyridyl, thienyl or thiazolyl, which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² represents COOR²⁶ or CN,
in which
R²⁶ represents hydrogen or straight-chain or branched alkyl having up to 4 carbon atoms;
X represents straight-chain or branched alkylene having up to 4 carbon atoms, which may contain a group selected from O, S(O)ᵣ, NR³⁰, or a three- to six-membered saturated or unsaturated carbocycle having optionally one or more straight-chain or branched alkyl radicals having 1 to 4 carbon atoms and having optionally one or two heteroatoms from the group consisting of S(O)ᵣ, NR³² and O,
in which
r represents 0, 1 or 2,
R³⁰ represents hydrogen, straight-chain or branched alkyl having up to 6 carbon atoms, phenyl or benzyl,
R³² represents hydrogen, straight-chain or branched alkyl having up to 6 carbon atoms, cycloalkyl having 3 to 6 carbon atoms or phenyl,
R¹ represents CN or COOR³⁵,
in which
R³⁵ represents hydrogen or straight-chain or branched alkyl having up to 6 carbon atoms.

7. Compounds according to Claim 5,
in which
B represents a heterocycle from the group consisting of 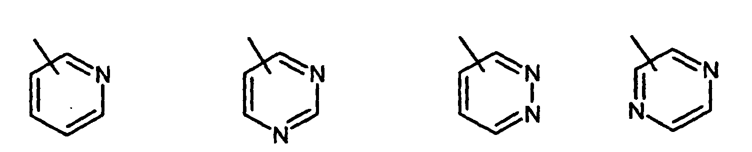 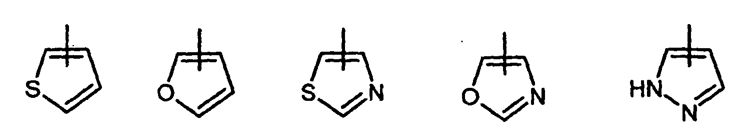 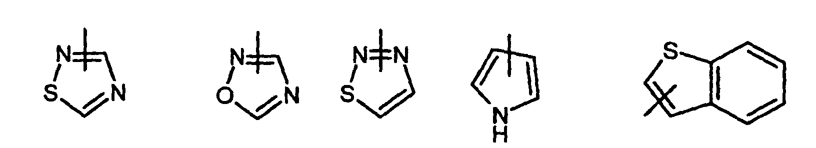 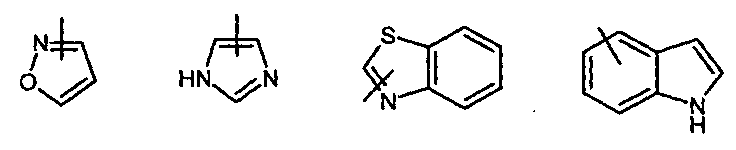
r represents 0 or 1,
V is absent or represents O, NR⁴ or S(O)ₙ
in which
R⁴ represents hydrogen,
n represents 0,
Q is absent or represents straight-chain or branched alkylene, straight-chain or branched alkenediyl having in each case up to 15 carbon atoms, which may contain one or more groups selected from O, S(O)ₚ, NR⁵, CONR⁵, S-CO- and OCO and which may be mono- or disubstituted by halogen or hydroxyl, or represents CONR⁵,
in which
R⁵ represents hydrogen,
p represents 0 or 1,
Y represents hydrogen, NR⁶R⁷, phenyl, napthyl or a heterocycle from the group 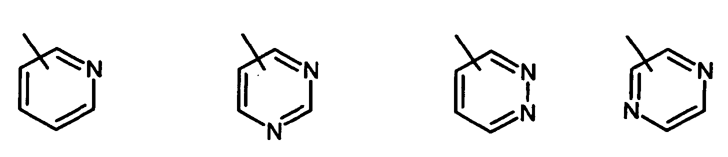 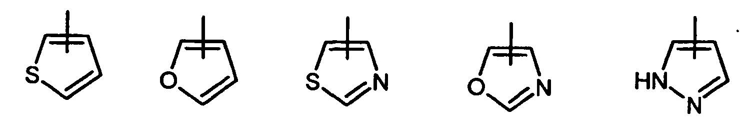 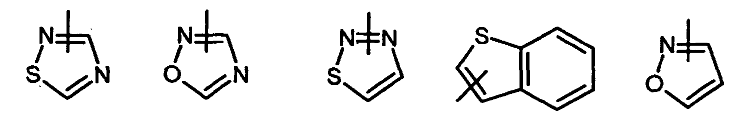 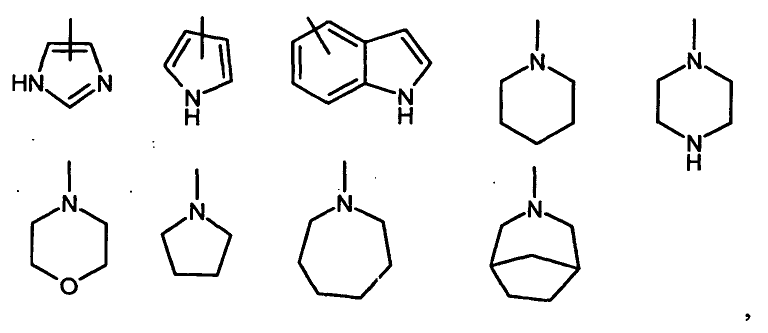 where the cyclic radicals may in each case be mono- to trisubstituted by straight-chain or branched alkyl, straight-chain or branched alkenyl, straight-chain or branched alkinyl, straight-chain or branched alkoxy, straight-chain or branched halogenoalkyl, straight-chain or branched halogenoalkoxy having in each case up to 4 carbon atoms, straight-chain or branched cycloalkyl having 3 to 7 carbon atoms, F, Cl, Br, I, NO₂, COR⁸, SR⁸, NR¹⁰R¹¹, NR⁹COR¹² or CONR¹³R¹⁴,
in which
R⁶ and R⁷ in each case independently of one another represent hydrogen, straight-chain or branched alkyl, straight-chain or branched alkoxy or straight-chain or branched alkyloxyalkyl having in each case up to 4 carbon atoms or cycloalkyl having 3 to 8 carbon atoms or aryl having 6 to 10 carbon atoms, which is optionally mono- or polysubstituted by aryl having 6 to 10 carbon atoms or an aromatic heterocycle having 1 to 9 carbon atoms and up to 3 heteroatoms from the group consisting of S, N and O,
R⁸ represents hydrogen, straight-chain or branched alkyl having up to 4 carbon atoms, or straight-chain or branched halogenoalkyl having up to 4 carbon atoms,
R⁹ represents hydrogen, or straight-chain or branched alkyl having up to 4 carbon atoms,
R¹⁰, R¹¹, R¹³ and R¹⁴ independently of one another represent hydrogen, straight-chain or branched alkyl having up to 4 carbon atoms, or phenyl,
where the phenyl radical may be mono- to trisubstituted by F, Cl Br, hydroxyl, methyl, ethyl, n- propyl, i-propyl, n-butyl, s- butyl, i- butyl, t-butyl, methoxy, ethoxy, amino, acetylamino, NO₂, CF₃, OCF₃ or CN,
or two substituents R¹⁰ and R¹¹ or R¹³ and R¹⁴ may be attached to one another forming a five- or six-membered ring which may be interrupted by O or N,
R¹² represents hydrogen, straight-chain or branched alkyl having up to 4 carbon atoms, or phenyl,
where the phenyl radical may be mono- to trisubstituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may in each case be mono- to trisubstituted by phenyl or a heterocycle from the group consisting of 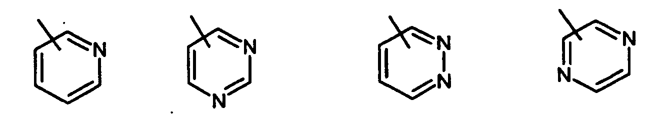 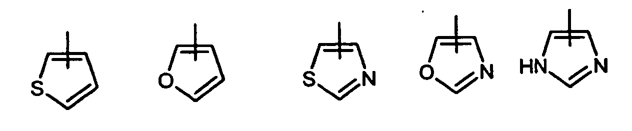 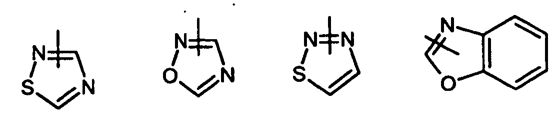 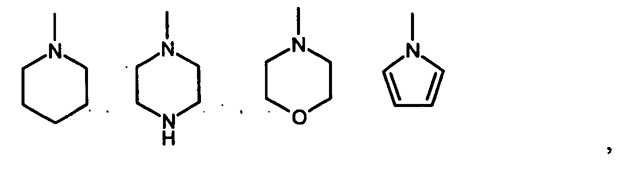 which are attached directly or via a group selected from O, S, SO, SO₂, CONR⁹, SO₂NR⁹, straight-chain or branched alkylene, straight-chain or branched alkenediyl, straight-chain or branched alkyloxy, straight-chain or branched oxyalkyloxy, straight-chain or branched sulfonylalkyl, straight-chain or branched thioalkyl having in each case up to 4 carbon atoms and may be mono- to trisubstituted by straight-chain or branched alkyl, straight-chain or branched alkoxy, straight-chain or branched halogenoalkyl or straight-chain or branched alkenyl having in each case up to 4 carbon atoms, phenyl, benzyl, F, Cl, Br, I, CN, NO₂, NR¹⁷R¹⁸ or NR¹⁶COR¹⁹,
in which
R¹⁶ represents hydrogen, straight-chain or branched alkyl having up to 8 carbon atoms or cycloalkyl having 3 to 8 carbon atoms,
R¹⁷, R¹⁸ independently of one another represent hydrogen, straight-chain or branched alkyl having up to 8 carbon atoms, cycloalkyl having 3 to 8 carbon atoms, phenyl, where the phenyl radical may be mono- to trisubstituted by F, Cl Br, hydroxyl, methyl, ethyl, n-propyl, i-propyl, n-butyl, s-butyl ,i-butyl, t-butyl, methoxy, ethoxy, amino, acetylamino, ·NO₂, CF₃, OCF₃ or CN or represent a radical of the formula SO₂R²⁰,
in which
R²⁰ represents straight-chain or branched alkyl having up to 4 carbon atoms or phenyl,
and
R¹⁹ represents hydrogen, straight-chain or branched alkyl having up to 12 carbon atoms, straight-chain or branched alkenyl having up to 12 carbon atoms, aryl having 6 to 10 carbon atoms, an aromatic heterocycle having 1 to 9 carbon atoms and up to 3 heteroatoms from the group consisting of S, N and O or cycloalkyl having 3 to 8 carbon atoms, which may optionally furthermore be substituted by F, Cl Br, hydroxyl, methyl, ethyl, n-propyl, i-propyl, n-butyl, s-butyl, i-butyl, t-butyl, methoxy, ethoxy, amino, acetylamino, NO₂, CF₃, OCF₃ or CN;
and/or the cyclic radicals may be fused with an aromatic or saturated carbocycle having 1 to 10 carbon atoms or an aromatic or saturated heterocycle having 1 to 9 carbon atoms and up to 3 heteroatoms from the group consisting of S, N and O,
R³ represents hydrogen, F, Cl, Br, straight-chain or branched alkyl, straight-chain or branched halogenoalkyl, straight-chain or branched alkoxy or straight-chain or branched halogenoalkoxy having in each case up to 4 carbon atoms,
W represents CH₂CH₂, CH=CH, CH₂O, OCH₂, CH₂OCH₂, CH₂NH, NHCH₂ or CH₂NHCH₂,
U represents straight-chain alkylene having up to 4 carbon atoms, O, NH, S, SO or SO₂,
A is absent or represents phenyl, pyridyl, thienyl or thiazolyl, which may optionally be mono- to trisubstituted by methyl, ethyl, n-propyl, i-propyl, n-butyl, i-butyl, s-butyl, t-butyl, CF₃, methoxy, ethoxy, F, Cl, Br,
R² represents COOH,
X represents straight-chain or branched alkylene having up to 4 carbon atoms, which may contain a group selected from O, S(O)ᵣ, NR³⁰, or a three- to six-membered saturated or unsaturated carbocycle having optionally one or more straight-chain or branched alkyl radicals having 1 to 4 carbon atoms and having optionally one or two heteroatoms from the group consisting of S(O)ᵣ, NR³² and O,
in which
r represents 0, 1 or 2,
R³⁰ represents hydrogen, straight-chain or branched alkyl having up to 6 carbon atoms, phenyl or benzyl,
R³² represents hydrogen, straight-chain or branched alkyl having up to 6 carbon atoms, cycloalkyl having 3 to 6 carbon atoms or phenyl,
R¹ represents COOH.

8. Process for preparing the compounds of the formula (I) 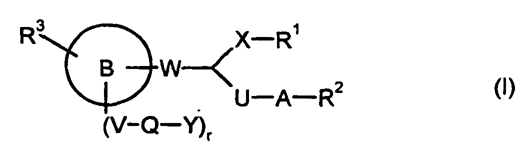 comprising
[A] the reaction of aldehydes of the general formula (II) 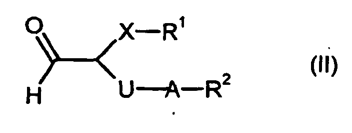 in which
R¹, R², A, U and X have the meaning given above, with the proviso that R¹ and R² may not represent free carboxylic acid groups,
with phosphorus compounds of the general formula (III) 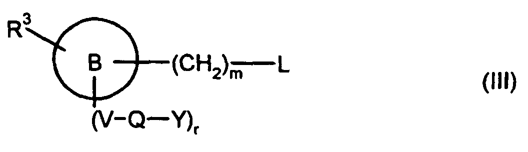 in which
R³, B, V, Q, Y and r have the meanings given above,
m represents an integer from 1 to 5, and
L represents a radical of the formula 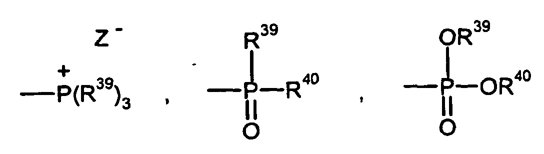
in which
R³⁹ and R⁴⁰ independently of one another represent straight-chain or branched alkyl having up to 12 carbon atoms or phenyl, and
Z represents a halide anion or tosylate anion,
in inert solvents in the presence of a base,
and, if appropriate, the subsequent partial or complete hydrolysis of the radicals R¹ and R² to free carboxylic acid groups;
or
[B] compounds of the formula (IV), 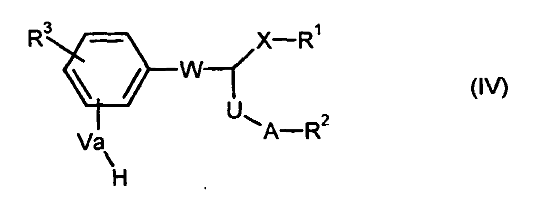 in which
Va represents O or S
R¹, R², R³, U, W,A, X have the meaning given above
are reacted with compounds of the formula (V) 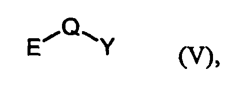 in which
Q, Y have the same meanings as defined above,
E represents either a leaving group which is substituted in the presence of a base or an optionally activated hydroxyl function;
or
[C] compounds of the formula (VI), 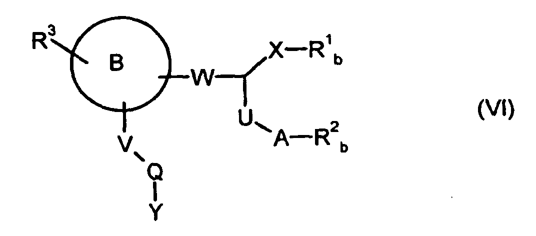 in which
R³, V, Q, Y, W, U, A, B have the same meanings as defined above,
R¹_{b} and R²_{b} each independently represent CN or COOAlk, where Alk represents a straight-chain or branched alkyl radical having up to 6 carbon atoms,
are converted with aqueous solutions of strong acids or strong bases into the corresponding free carboxylic acids.
or
[D] compounds of the formula (VII) 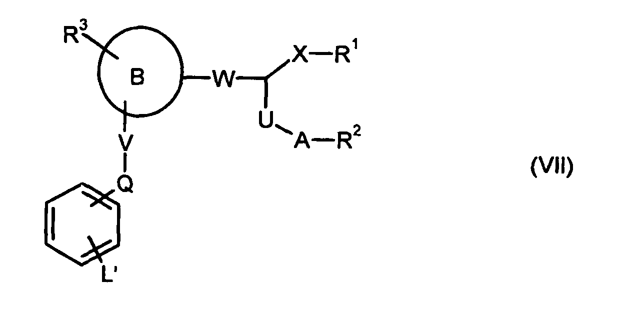 in which
R¹, R², R³, V, Q, X, W, U, A, B have the same meanings as defined above,
L' represents Br, I or the group CF₃SO₂-O,
are reacted with compounds of the formula (VIII)
M-Z' (VIII)
in which
M represents an aryl or heteroaryl radical, a straight-chain or branched alkyl, alkenyl or alkinyl radical or cycloalkyl radical or represents an arylalkyl, an arylalkenyl or arylalkinyl radical,
Z' represents the groupings -B(OH)₂, -CH≡CH, -CH=CH₂ or -Sn(nBu)₃
in the presence of a palladium compound, if appropriate additionally in the presence of a reducing agent and further additives and in the presence of a base;
or
[E] compounds of the formula (VII) 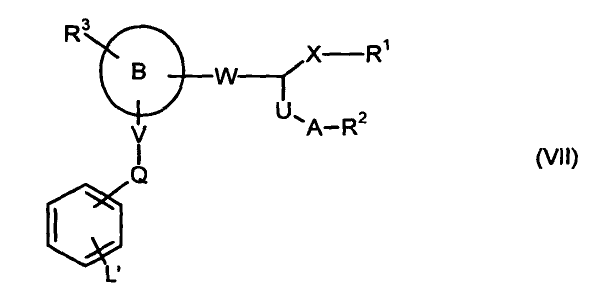 in which
R¹, R², R³, V, Q, X, W, U, A, B have the same meanings as defined above,
L' represents Br, I or the group CF₃SO₂-O,
are reacted with compounds of the formula (IX)
NHR^{a}R^{b} (IX)
in which
R^{a} and R^{b} independently of one another represent hydrogen or a straight-chain or branched alkyl radical having up to 8 carbon atoms or together with the nitrogen atom to which they are attached may form an an aromatic heterocycle having 1 to 9 carbon atoms and up to 3 heteroatoms from the group consisting of S, N and O,
in the presence of a palladium compound, if appropriate additionally in the presence of a reducing agent and further additives and in the presence of a base;
or
[F] compounds of the formula (IV), 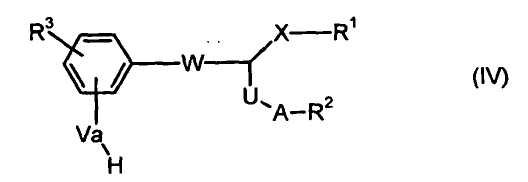 in which
Va represents O or S
R¹, R², R³, U, W,A, X have the meaning given above
are reacted with compounds of the formula (X) 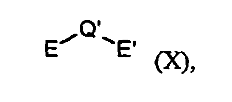 in which
Q' has the same meaning as Q according to Claim 1 or represents phenyl,
E and E' in each case independently of one another represent either a leaving group which is substituted in the presence of a base or an optionally activated hydroxyl function or a radical containing such a group;
and the resulting compounds of the formula (XI) 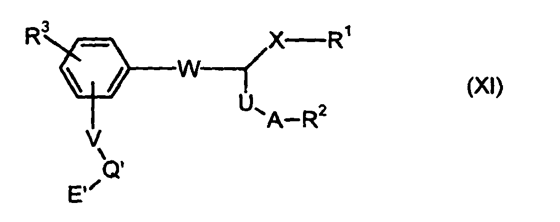 in which
R¹, R², R³, A, U, V, W, X and E' have the meanings given above,
Q' has the same meaning as Q according to Claim 1 or represents 1,4-CH₂-Ph-CH₂-,
are reacted with amines of the formula (XII)
NHR^{a}R^{b} (XII)
in which
R^{a} and R^{b} independently of one another represent hydrogen or a straight-chain or branched alkyl radical having up to 8 carbon atoms or together with the nitrogen atom to which they are attached may form an an aromatic heterocycle having 1 to 9 carbon atoms and up to 3 heteroatoms from the group consisting of S, N and O,
or
[G] compounds of the formula (XIII) 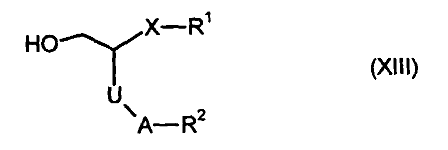 in which
R¹, R², A, U, X have the meanings given above,
are reacted with compounds of the formula (XIV) 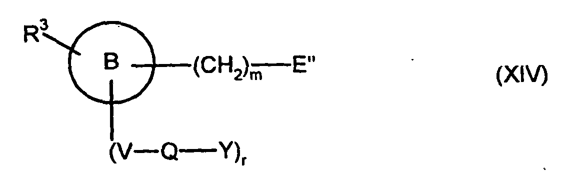 in which
R³, V, Q, Y, r and B have the meanings given above,
m represents an integer from 1 to 5, and
E" represents either a leaving group which is substituted in the presence of a base or an optionally activated hydroxyl function;
or
[H] compounds of the formula (XV) 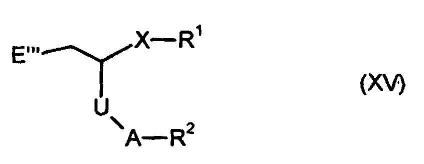 in which
R¹, R², A, U, X have the meanings given above,
E''' represents either a leaving group which is substituted in the presence of a base or an optionally activated hydroxyl function;
are reacted with compounds of the formula (XVI) 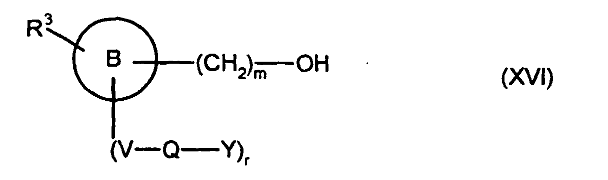 in which
R³, V, Q, Y, rand B have the meanings given above,
m represents an integer from 1 to 5,
or
[I] compounds of the formula (XVII) 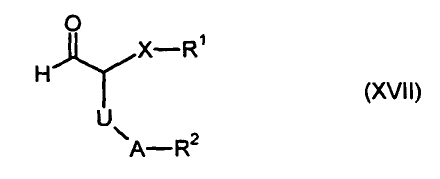 in which
R¹, R², A, U, X have the meanings given above,
are reacted with compounds of the formula (XVIII) 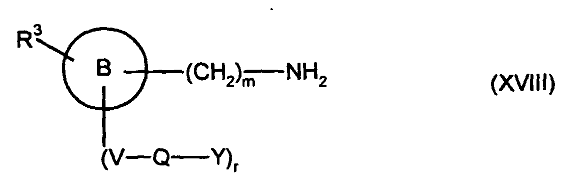 in which
R³, V, Q, Y, r and B have the meanings given above,
m represents an integer from 0 to 5,
giving initially a Schiff's base, which is then reduced with customary reducing agents or reacted directly under the conditions of a reductive alkylation in the presence of a reducing agent;
or
[J] compounds of the formula (XIX) 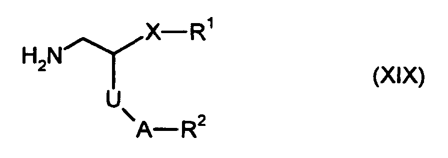 in which
R¹, R², A, U, X have the meanings given above,
are reacted with compounds of the formula (XX) 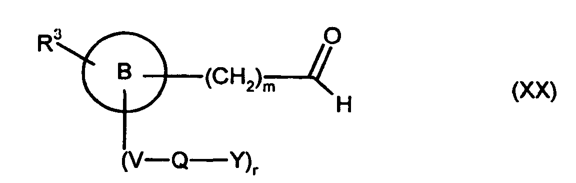 in which
R³, V, Q, Y, r and B have the meanings given above,
m represents an integer from 0 to 5,
giving initially a Schiff's base, which is then reduced with customary reducing agents or reacted directly under the conditions of a reductive alkylation in the presence of a reducing agent,
or
[K] aldehydes of the formula (XXI) 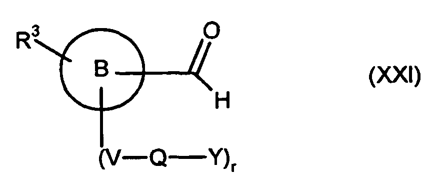 in which
R³, V, Q, Y, r and B have the meanings given above,
are reacted with phosphorus compounds of the formula (XXII) 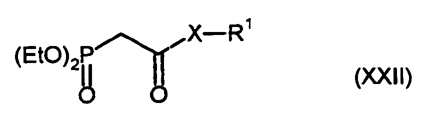 in which
X and R¹ have the meanings given above,
to give compounds of the formula (XXIII) 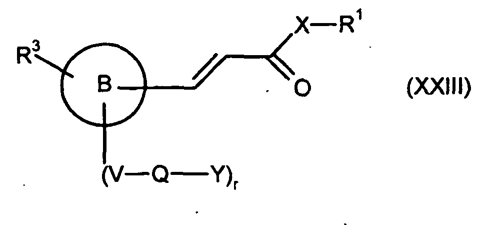 in which
R³, V, Q, Y, r, B, X and R¹ have the meanings given above,
and subsequently, by successive reduction of the alkene group and the carbonyl group and subsequent substitution of the hydroxyl group generated by reduction of the carbonyl group or by reaction of the halogen radical generated from the hydroxyl group using halogenating agents with alcohols, primary amines or thiols and, if appropriate, subsequent oxidation to the corresponding sulfoxide or sulfone compounds, converted into compounds of the formula (XXIV), 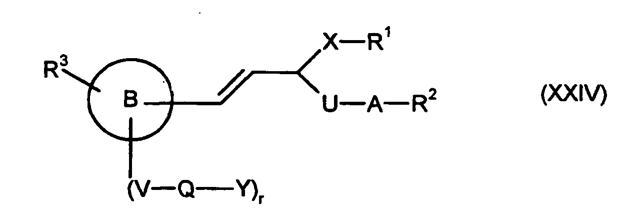 in which
R³, V, Q, Y, r, B, X, U,R² and R¹ have the meanings given above,
U represents O, NH or S.

9. Medicament, comprising at least one compound of the general formula (I) according to any of the preceding claims.

10. Use of compounds of the formula (I) according to any of the preceding claims for preparing a medicament for the treatment of cardiovascular disorders.

11. Use of compounds of the formula (I) according to any of the preceding claims for preparing medicaments for the treatment of angina pectoris, ischemias and cardiac insufficiency.

12. Use of compounds of the formula (I) according to any of the preceding claims for preparing medicaments for the treatment of hypertension, thromboembolic disorders, arteriosclerosis and venous disorders.

13. Use of compounds of the formula (I) according to any of the preceding claims for preparing medicaments for the treatment of fibrotic disorders.

14. Use according to Claim 15, **characterized in that** the fibrotic disorder is hepatic fibrosis.

## Revendications

1. Composés de formule générale (I) 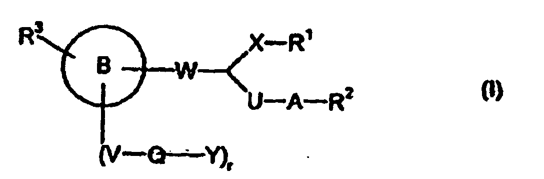 dans laquelle
B est un reste aryle ayant 6 à 10 atomes de carbone,
r a la valeur 0 ou 1,
V est manquant, ou représente O, NR⁴, NR⁴CONR⁴, NR⁴CO, NR⁴SO₂, COO, CONR⁴ ou S(O)ₒ,
où
R⁴ représente, indépendamment d'un autre reste R⁴ éventuellement présent, l'hydrogène, un reste alkyle linéaire ou ramifié ayant jusqu'à 8 atomes de carbone, un reste cycloalkyle ayant 3 à 8 atomes de carbone, un reste aryle ayant 6 à 10 atomes de carbone ou un reste arylalkyle ayant 7 à 18 atomes de carbone, le reste aryle pouvant lui-même être substitué une ou plusieurs fois par un halogène, un radical alkyle, alkoxy ayant jusqu'à 6 atomes de carbone,
o a la valeur 0, 1 ou 2,
Q est manquant ou représente un reste alkylène linéaire ou ramifié, un reste alcènediyle linéaire ou ramifié ou un reste alcynediyle linéaire ou ramifié, ayant chacun jusqu'à 15 atomes de carbone, ces restes pouvant contenir un ou plusieurs groupes de la série O, S(O)ₚ, NR⁵, CO, OCO, S-CO-, CONR⁵ ou NR⁵SO₂, et pouvant être substitués une ou plusieurs fois par un halogène, un radical hydroxy, ou alkoxy ayant jusqu'à 4 atomes de carbone, deux atomes quelconques de la chaîne existante pouvant être liés le cas échéant en formant un noyau de trois à huit chaînons, ou représente CONR⁵,
où
R⁵ représente l'hydrogène, un reste alkyle linaire ou ramifié ayant jusqu'à 8 atomes de carbone ou un reste cycloalkyle ayant 3 à 8 atomes de carbone, qui peut être substitué par un halogène ou par un radical alkoxy ayant jusqu'à 4 atomes de carbone,
p a la valeur 0, 1 ou 2,
Y représente l'hydrogène, un groupe NR⁶R⁷, un reste aryle ayant 6 à 10 atomes de carbone, un hétérocycle aromatique ou saturé ayant 1 à 9 atomes de carbone et jusqu'à 3 hétéroatomes de la série S, N et/ou 0, ou un reste cycloalkyle linéaire ou ramifié ayant 3 à 8 atomes de carbone, qui peuvent aussi être liés par l'intermédiaire de N,
les restes cycliques pouvant chacun être substitués une à trois fois par un radical alkyle linéaire ou ramifié, un radical alcényle linéaire ou ramifié, un radical alcynyle linéaire ou ramifié, un radical alkoxy linéaire ou ramifié, un radical halogénalkyle linéaire ou ramifié, un radical halogénalkoxy linéaire ou ramifié ayant dans chaque cas jusqu'à 8 atomes de carbone, un radical cycloalkyle linéaire ou ramifié ayant 3 à 8 atomes de carbone, un halogène, un groupe hydroxy, COR⁸, CN, SR⁸, NO₂, NR¹⁰R¹¹, NR⁹COR¹², NR⁹CONR⁹R¹² ou CONR¹³R¹⁴,
où
R⁶ et R⁷ désignent chacun, indépendamment l'un de l'autre, l'hydrogène, un reste alkyle linéaire ou ramifié, un reste alkoxy linéaire ou ramifié, un reste alkoxyalkyle linéaire ou ramifié ayant jusqu'à 8 atomes de carbone ou un reste cycloalkyle ayant 3 à 8 atomes de carbone ou un reste aryle ayant 6 à 10 atomes de carbone, qui est éventuellement substitué une ou plusieurs fois avec un radical aryle ayant 6 à 10 atomes de carbone ou avec un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 8 atomes de carbone, un reste halogénalkyle linéaire ou ramifié ayant jusqu'à 8 atomes de carbone ou un reste
R⁹ cycloalkyle ayant 3 à 8 atomes de carbone, représente l'hydrogène, un reste alkyle linéaire ou ramifié ayant jusqu'à 8 atomes de carbone ou un reste cycloalkyle ayant 3 à 8 atomes de carbone,
R¹⁰, R¹¹, R¹³ et R¹⁴ représentent, indépendamment les uns des autres, l'hydrogène, un reste alkyle linéaire ou ramifié, un reste alcényle linéaire ou ramifié ayant jusqu'à 8 atomes de carbone, un reste aryle ayant 6 à 10 atomes de carbone, un hétérocycle aromatique ayant 1 à 9 atomes de carbone et jusqu'à 3 hétéroatomes de la série S, N et/ou O, un reste arylalkyle ayant 8 à 18 atomes de carbone, un reste cycloalkyle ayant 3 à 8 atomes de carbone ou un reste de formule SO₂R¹⁵, le reste alkyle pouvant lui-même être substitué une ou plusieurs fois par un radical halogéno, hydroxy, CN, NO₂, NH₂, NHCOR⁹, alkyle, alkoxy, halogénalkyle ou halogénalkoxy ayant jusqu'à 6 atomes de carbone,
ou bien
deux substituants formés de R¹⁰ et R¹¹ ou de R¹³ et R¹⁴ peuvent être liés ensemble avec formation d'un noyau pentagonal ou hexagonal qui peut contenir O ou N,
où
R¹⁵ est un reste alkyle linéaire ou ramifié ayant jusqu'à 4 atomes de carbone ou un reste aryle ayant 6 à 10 atomes de carbone,
le reste aryle pouvant lui-même être substitué une ou plusieurs fois par un radical halogéno, CN, NO₂, alkyle, alkoxy, halogénalkyle ou halogénalkoxy ayant jusqu'à 6 atomes de carbone,
R¹² représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O, ou un reste cycloalkyle ayant 3 à 8 atomes de carbone, pouvant être substitués le cas échéant par un radical halogéno, hydroxy, CN, NO₂, NH₂, NHCOR⁹, alkyle, alkoxy, halogénalkyle ou halogénalkoxy ayant jusqu'à 6 atomes de carbone ;
et/ou les restes cycliques peuvent être substitués dans chaque cas une à trois fois par un radical aryle ayant 6 à 10 atomes de carbone, un hétérocycle aromatique ou saturé ayant 1 à 9 atomes de carbone et jusqu'à 3 hétéroatomes de la série S, N et/ou O, pouvant aussi être liés par l'intermédiaire de N,
qui peuvent être liés directement ou par l'intermédiaire d'un groupe 0, S, SO, SO₂, NR⁹, CONR⁹, SO₂NR⁹, un reste alkylène linéaire ou ramifié, un reste alcènediyle linéaire ou ramifié, un reste alkoxy linéaire ou ramifié, un reste oxyalkyloxy linéaire ou ramifié, un reste sulfonylalkyle linéaire ou ramifié, un reste thioalkyle linéaire ou ramifié ayant jusqu'à 8 atomes de carbone, et qui peuvent être substitués une à trois fois par un radical alkyle linéaire ou ramifié, alkoxy linéaire ou ramifié, halogénalkyle linéaire ou ramifié, halogénalkoxy linéaire ou ramifié, carbonylalkyle ou alcényle linéaire ou ramifié ayant dans chaque cas jusqu'à 6 atomes de carbone, phényle, benzyle, halogéno, SR⁸, CN, NO₂, NR¹⁷R¹⁸, CONR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent indépendamment l'un de l'autre l'hydrogène, un reste alkyle linéaire ou ramifié ayant jusqu'à 8 atomes de carbone, un reste cycloalkyle ayant 3 à 8 atomes de carbone, un reste aryle ayant 6 à 10 atomes de carbone ou un reste de formule SO₂R²⁰, le reste aryle pouvant lui-même être substitué une ou plusieurs fois par un radical halogéno, hydroxy, CN, NO₂, NH₂, NHCOR⁹, alkyle, alkoxy, halogénalkyle ou halogénalkoxy ayant jusqu'à 6 atomes de carbone,
où
R²⁰ désigne un reste alkyle linéaire ou ramifié ayant jusqu'à 4 atomes de carbone ou un reste aryle ayant 6 à 10 atomes de carbone,
le reste aryle pouvant lui-même être substitué une ou deux plusieurs fois par un radical halogéno, hydroxy, CN, NO₂, NH₂, NHCOR⁹, alkyle, alkoxy, halogénalkyle ou halogénalkoxy ayant jusqu'à 6 atomes de carbone,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O, ou un reste cycloalkyle ayant 3 à 8 atomes de carbone, qui peuvent être substitués en outre le cas échéant par un radical halogéno, hydroxy, CN, NO₂, NH₂, NHCOR⁹, alkyle, alkoxy, halogénalkyle ou halogénalkoxy ayant jusqu'à 6 atomes de carbone ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène, un groupe OH, un halogène, un reste alkyle linéaire ou ramifié, un reste halogénalkyle linéaire ou ramifié, un reste alkoxy linéaire ou ramifié ou un reste halogénalkoxy linéaire ou ramifié, ayant chacun jusqu'à 4 atomes de carbone,
W représente un reste alkylène linéaire ou ramifié, un reste alcènediyle linéaire ou ramifié ayant jusqu'à 4 atomes de carbone, qui peuvent contenir un groupe choisi entre O et NR²³,
où
R²³ xreprésente l'hydrogène, un reste alkyle linéaire ou ramifié ayant jusqu'à 8 atomes de carbone ou un reste cycloalkyle ayant 3 à 8 atomes de carbone,
U désigne un reste alkylène linéaire ou ramifié ayant jusqu'à 4 atomes de carbone, O, NH, S, SO ou SO₂,
A est manquant ou représente un reste phényle ou un reste hétérocyclique aromatique ayant 1 à 9 atomes de carbone et jusqu'à 3 hétéroatomes de la série S, N et/ou O,
qui peuvent éventuellement être substitués une à trois fois par un radical halogéno, alkyle linéaire ou ramifié, halogénalkyle linéaire ou ramifié ou alkoxy linéaire ou ramifié ayant chacun jusqu'à 4 atomes de carbone,
R² représente un groupe COOR²⁶ ou CN,
où
R²⁶ représente l'hydrogène ou un reste alkyle linéaire ou ramifié ayant jusqu'à 8 atomes de carbone ;
X représente un reste alkylène linéaire ou ramifié, un reste alcènediyle linéaire ou ramifié, ayant chacun jusqu'à 8 atomes de carbone, qui peuvent contenir un groupe de la série O, S(O)ᵣ, NR³⁰ ou un carbocycle de trois à six chaînons saturé ou non saturé, présentant éventuellement un ou plusieurs restes alkyle linéaires ou ramifiés ayant 1 à 6 atomes de carbone, avec le cas échéant un ou deux hétéroatomes de la série S(O)ᵣ, NR³² et/ou O,
où
r a la valeur 0, 1 ou 2,
R³⁰ représente l'hydrogène, un reste alkyle linéaire ou ramifié ayant jusqu'à 6 atomes de carbone, un reste phényle ou arylalkyle ayant 7 à 12 atomes de carbone,
R³² représente l'hydrogène, un reste alkyle linéaire ou ramifié ayant jusqu'à 6 atomes de carbone, un reste cycloalkyle de 3 à 6 atomes de carbone ou un reste phényle,
R¹ est un groupe CN ou un groupe COOR³⁵,
dans lequel
R³⁵ représente l'hydrogène ou un reste alkyle linéaire ou ramifié ayant jusqu'à 6 atomes de carbone ;
sous réserve que Y ne puisse pas être un reste phényle ou puisse être un reste phényle exclusivement substitué avec un ou deux restes du groupe constitué de radicaux alkyle linéaires ou ramifiés ou alkoxy linéaires ou ramifiés ayant jusqu'à 8 atomes de carbone, halogéno, CF₃, OCF₃ ou CN en même temps que B est un reste phényle, V est manquant ou représente O, Q représente un reste alkylène linéaire ayant 1 à 4 atomes de carbone et est éventuellement lié à Y par l'intermédiaire d'un atome d'oxygène, W représente un groupe alkylène ou un groupe alkylènediyle ayant chacun 1 à 6 atomes de carbone, U représente un groupe alkylène ayant jusqu'à 4 atomes de carbone, O, S, SO ou SO₂, A représente un reste phényle et X représente un reste alkylène linéaire ayant 1 à 8 atomes de carbone éventuellement en liaison directe par l'intermédiaire de 0, S, SO ou SO₂ à l'atome de carbone portant les groupes W et U.

2. Composés suivant la revendication 1,
dans lesquels
B est un reste phényle ou naphtyle,
r a la valeur 0 ou 1,
V est manquant ou représente O, NR⁴ ou S(O)ₙ,
où
R⁴ représente l'hydrogène,
n a la valeur 0,
Q est manquant ou représente un reste alkylène linéaire ou ramifié, un reste alcènediyle linéaire ou ramifié ou un reste alcynediyle linéaire ou ramifié, ayant chacun jusqu'à 15 atomes de carbone, qui peuvent contenir un ou plusieurs groupes de la série O, S(O)ₚ, NR⁵, CONR⁵, S-CO- ou OCO, et peut être substitué une ou deux fois par un halogène ou un radical hydroxy, ou représente CONR⁵,
où
R⁵ désigne l'hydrogène,
p a la valeur 0 ou 1,
Y représente l'hydrogène, un groupe NR⁶R⁷, un reste phényle, naphtyle ou un hétérocycle du groupe 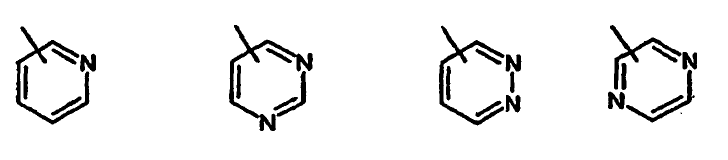 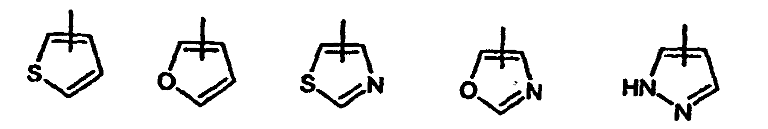 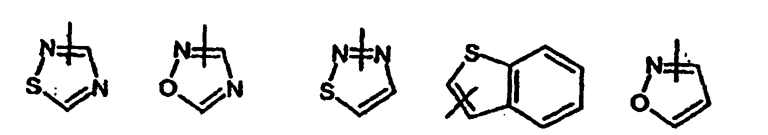 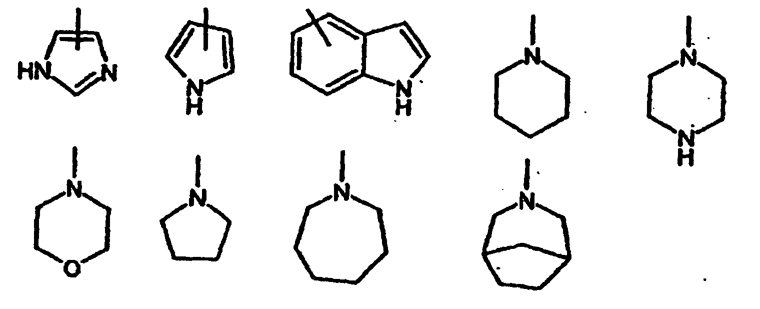 les restes cycliques pouvant chacun être substitués une à trois fois par un radical alkyle linéaire ou ramifié, alcényle linéaire ou ramifié, alcynyle linéaire ou ramifié, alkoxy linéaire ou ramifié, halogénalkyle linéaire ou ramifié, halogénalkoxy linéaire ou ramifié ayant dans chaque cas jusqu'à 4 atomes de carbone, cycloalkyle linéaire ou ramifié ayant 3 à 7 atomes de carbone, F, Cl, Br, I, NO₂, COR⁸, SR⁸, NR¹⁰R¹¹, NR⁹COR¹² ou CONR¹³R¹⁴,
où
R⁶ et R⁷ désignent chacun, indépendamment l'un de l'autre, l'hydrogène, un reste alkyle linéaire ou ramifié, un reste alkoxy linéaire ou ramifié ou un reste alkyloxyalkyle linéaire ou ramifié ayant chacun jusqu'à 4 atomes de carbone ou un reste cycloalkyle ayant 3 à 8 atomes de carbone ou un reste aryle ayant 6 à 10 atomes de carbone, qui est éventuellement substitué une ou plusieurs fois avec un radical aryle ayant 6 à 10 atomes de carbone ou avec un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁹ représente l'hydrogène ou un reste alkyle linéaire ou ramifié ayant jusqu'à 4 atomes de carbone,
R¹⁰, R¹¹, R¹³ et R¹⁴ représentent, indépendamment les uns des autres, l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants formés de R¹⁰ et R¹¹ ou de R¹³ et R¹⁴ peuvent être liés ensemble en formant un noyau pentagonal ou hexagonal qui peut être interrompu par O ou N,
R¹² représente l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques pouvant chacun être substitués une à trois fois par un radical phényle ou par un hétérocycle du groupe 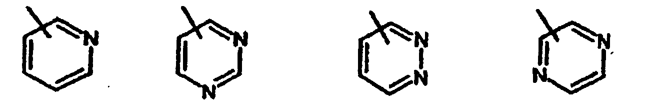 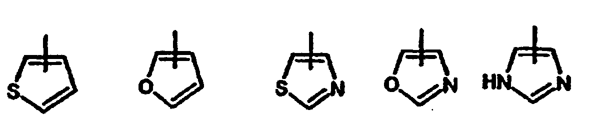 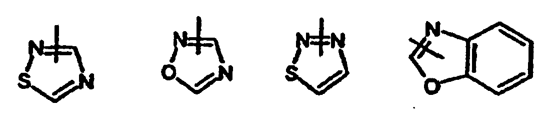 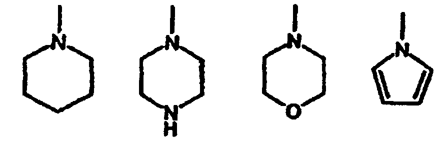 ces hétérocycles pouvant être liés directement ou par l'intermédiaire d'un groupe O, S, SO, SO₂, CONR⁹, SO₂NR⁹, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant dans chaque cas jusqu'à 4 atomes de carbone et pouvant être substitués une à trois fois par un radical alkyle linéaire ou ramifié, alkoxy linéaire ou ramifié, halogénalkyle linéaire ou ramifié ou alcényle linéaire ou ramifié ayant chacun jusqu'à 4 atomes de carbone, phényle, benzyle, F, Cl, Br, I, CN, NO₂, NR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent indépendamment l'un de l'autre l'hydrogène, un reste alkyle linéaire ou ramifié ayant jusqu'à 8 atomes de carbone, un reste cycloalkyle ayant 3 à 8 atomes de carbone, un reste phényle, le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN, ou représentent un reste de formule SO₂R²⁰
dans laquelle
R²⁰ désigne un reste alkyle linéaire ou ramifié ayant jusqu'à 4 atomes de carbone ou un reste phényle,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O, ou un reste cycloalkyle ayant 3 à 8 atomes de carbone, ces restes pouvant en outre être éventuellement substitués par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou 0,
R³ représente l'hydrogène, OH, F, Cl, Br, un reste alkyle linéaire ou ramifié, un reste halogénalkyle linéaire ou ramifié, un reste alkoxy linéaire ou ramifié ou un reste halogénalkoxy linéaire ou ramifié, ayant dans chaque cas jusqu'à 4 atomes de carbone,
W représente CH₂CH₂, CH=CH, CH₂O, OCH₂, CH₂OCH₂, CH₂NH, NHCH₂ ou CH₂NHCH₂,
U représente un reste alkylène linéaire ayant jusqu'à 4 atomes de carbone, O, NH, S, SO ou SO₂,
A est manquant ou représente un reste phényle, pyridyle, thiényle ou thiazolyle qui peut être substitué éventuellement une à trois fois par un radical méthyle, éthyle, n-propyle, isopropyle, n-butyle, isobutyle, sec.-butyle, tertiobutyle, CF₃, méthoxy, éthoxy, F, Cl, Br,
R² est un groupe COOR²⁶ ou CN,
où
R²⁶ représente l'hydrogène ou un reste alkyle linéaire ou ramifié ayant jusqu'à 4 atomes de carbone ;
X désigne un reste alkylène linéaire ou ramifié ayant jusqu'à 4 atomes de carbone, qui peut contenir un groupe de la série O, S(O)ᵣ, NR³⁰, ou un carbocycle de trois à six chaînons saturé ou non saturé, portant le cas échéant un ou plusieurs restes alkyle linéaires ou ramifiés ayant 1 à 4 atomes de carbone, avec le cas échéant un ou deux hétéroatomes de la série S(O)ᵣ, NR³² et/ou O,
où
r a la valeur 0, 1 ou 2,
R³⁰ représente l'hydrogène, un reste alkyle linéaire ou ramifié ayant jusqu'à 6 atomes de carbone, un reste phényle ou benzyle,
R³² représente l'hydrogène, un reste alkyle linéaire ou ramifié ayant jusqu'à 6 atomes de carbone, un reste cycloalkyle ayant 3 à 6 atomes de carbone ou phényle,
R¹ est un groupe CN ou COOR³⁵,
où
R³⁵ représente l'hydrogène ou un reste alkyle linéaire ou ramifié ayant jusqu'à 6 atomes de carbone ;
sous réserve que Y ne puisse pas être un reste phényle ou puisse être un reste phényle exclusivement substitué avec un ou deux restes du groupe constitué de radicaux alkyle linéaires ou ramifiés ou alkoxy linéaires ou ramifiés ayant chacun jusqu'à 4 atomes de carbone, halogéno, CF₃ ou OCF₃ en même temps que V est manquant ou représente O, Q représente un reste alkylène linéaire ayant 1 à 10 atomes de carbone et est éventuellement lié par l'intermédiaire d'un atome d'oxygène à Y, W représente un groupe éthylène ou un groupe éthanediyle, U représente un groupe alkylène ayant jusqu'à 4 atomes de carbone, O, S, SO ou SO₂, A représente un reste phényle et X représente un reste alkylène linéaire ayant 1 à 4 atomes de carbone, éventuellement lié directement par l'intermédiaire de 0, S, SO ou SO₂ à l'atome de carbone portant les groupes W et U.

3. Composés suivant la revendication 1,
dans lesquels
B est un reste phényle ou naphtyle,
r a la valeur 0 ou 1,
V est manquant ou représente O, NR⁴ ou S(O)ₙ,
où
R⁴ représente l'hydrogène,
n a la valeur 0,
Q est manquant ou représente un reste alkylène linéaire ou ramifié, alcènediyle linéaire ou ramifié ou alcynediyle linéaire ou ramifié ayant chacun jusqu'à 15 atomes de carbone, qui peuvent contenir un ou plusieurs groupes de la série O, S(O)ₚ, NR⁵, CONR⁵, S-CO- ou OCO, et peut être substitué une ou deux fois par un radical halogéno ou hydroxy, ou représente CONR⁵,
où
R⁵ représente l'hydrogène,
p a la valeur 0 ou 1,
Y représente l'hydrogène, un groupe NR⁶R⁷, un reste phényle, naphtyle ou un hétérocycle du groupe 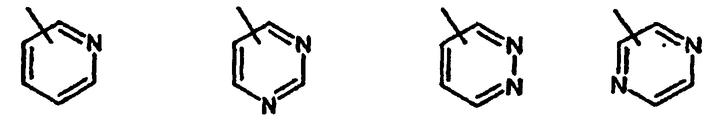 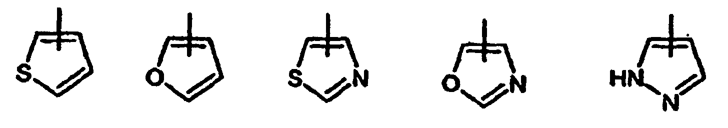 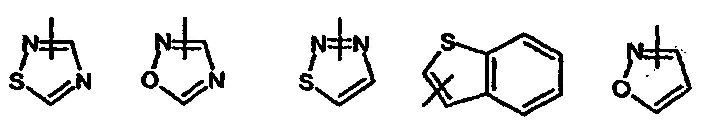 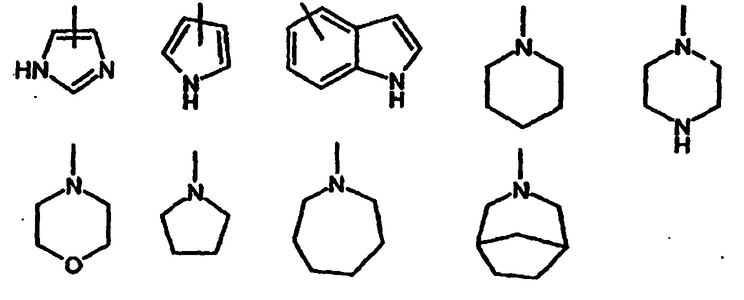 les restes cycliques pouvant être substitués chacun une à trois fois par un radical alkyle linéaire ou ramifié, alcényle linéaire ou ramifié, alcynyle linéaire ou ramifié, alkoxy linéaire ou ramifié, halogénalkyle linéaire ou ramifié, halogénalkoxy linéaire ou ramifié ayant jusqu'à 4 atomes de carbone, cycloalkyle linéaire ou ramifié ayant 3 à 7 atomes de carbone, F, Cl, Br, I, NO₂, COR⁸, SR⁸, NR¹⁰R¹¹, NR⁹COR¹² ou CONR¹³R¹⁴,
où
R⁶ et R⁷ représentent chacun, indépendamment l'un de l'autre, l'hydrogène, un reste alkyle linéaire ou ramifié, un reste alkoxy linéaire ou ramifié ou un reste alkyloxyalkyle linéaire ou ramifié ayant chacun jusqu'à 4 atomes de carbone ou un reste cycloalkyle ayant 3 à 8 atomes de carbone ou un reste aryle ayant 6 à 10 atomes de carbone, qui est éventuellement substitué une ou plusieurs fois avec un radical aryle ayant 6 à 10 atomes de carbone ou avec un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁹ représente l'hydrogène ou un reste alkyle linéaire ou ramifié ayant jusqu'à 4 atomes de carbone,
R¹⁰ R¹¹, R¹³ et R¹⁴ représentent, indépendamment les uns des autres, l'hydrogène, un reste alkyle linéaire ou ramifié ayant jusqu'à 4 atomes de carbone ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants formés de R¹⁰ et R¹¹ ou de R¹³ et R¹⁴ peuvent être liés ensemble en formant un noyau pentagonal ou hexagonal qui peut être interrompu par O ou N,
R¹² représente l'hydrogène, un reste alkyle linéaire ou ramifié ayant jusqu'à 4 atomes de carbone ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou par un hétérocycle du groupe 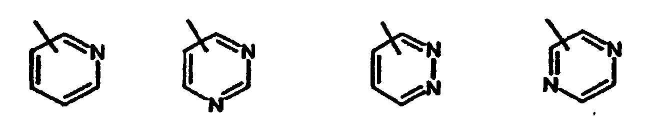 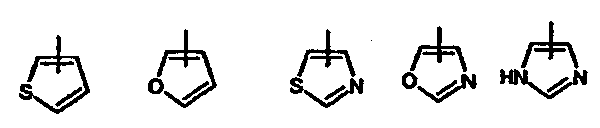 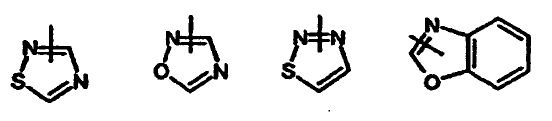 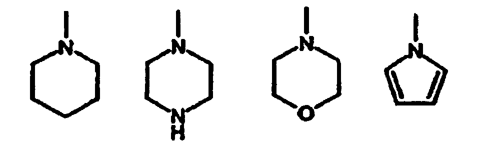 ces hétérocycles pouvant être liés directement ou par l'intermédiaire d'un groupe O, S, SO, SO₂, CONR⁹, SO₂NR⁹, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant chacun jusqu'à 4 atomes de carbone et qui peuvent être substitués une à trois fois par un radical alkyle linéaire ou ramifié, alkoxy linéaire ou ramifié, halogénalkyle linéaire ou ramifié ou alcényle linéaire ou ramifié ayant chacun jusqu'à 4 atomes de carbone, phényle, benzyle, F, Cl, Br, I, CN, NO₂, NR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent, indépendamment l'un de l'autre, l'hydrogène, un reste alkyle linéaire ou ramifié ayant jusqu'à 8 atomes de carbone, un reste cycloalkyle ayant 3 à 8 atomes de carbone, un reste phényle, le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN, ou désignent un reste de formule SO₂R²⁰,
où
R²⁰ est un reste alkyle linéaire ou ramifié ayant jusqu'à 4 atomes de carbone ou un reste phényle,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 8 atomes de carbone, ces restes pouvant en outre être substitués le cas échéant par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 8 hétéroatomes de la série S, N et/ou O,
R³ représente l'hydrogène, OH, F, Cl, Br, un reste alkyle linéaire ou ramifié, un reste halogénalkyle linéaire ou ramifié, un reste alkoxy linéaire ou ramifié ou un reste halogénalkoxy linéaire ou ramifié ayant chacun jusqu'à 4 atomes de carbone,
W représente CH₂CH₂, CH=CH, CH₂O, OCH₂, CH₂OCH₂, CH₂NH, NHCH₂ ou CH₂NHCH₂,
U est un reste alkylène linéaire ayant jusqu'à 4 atomes de carbone, O, NH, S, SO ou SO₂,
A est manquant ou représente un reste phényle, pyridyle, thiényle ou thiazolyle qui peut être substitué une à trois fois par un radical méthyle, éthyle, n-propyle, isopropyle, n-butyle, isobutyle, sec.-butyle, tertiobutyle, CF₃, méthoxy, éthoxy, F, Cl, Br,
R² représente COOH,
X désigne un reste alkylène linéaire ou ramifié ayant jusqu'à 4 atomes de carbone, qui peut contenir un groupe de la série 0, S(O)ᵣ, NR³⁰, ou un carbocycle de trois à six chaînons saturé ou non saturé, portant éventuellement un ou plusieurs restes alkyle linéaires ou ramifiés ayant 1 à 4 atomes de carbone, avec le cas échéant un ou deux hétéroatomes de la série S(O)ᵣ, NR³² et/ou O,
où
r a la valeur O, 1 ou 2,
R³⁰ représente l'hydrogène, un reste alkyle linéaire ou ramifié ayant jusqu'à 6 atomes de carbone, un reste phényle ou benzyle,
R³² représente l'hydrogène, un reste alkyle linéaire ou ramifié ayant jusqu'à 6 atomes de carbone, un reste cycloalkyle ayant 3 à 6 atomes de carbone ou un reste phényle,
R¹ est un groupe COOH,
sous réserve que Y ne puisse pas être un reste phényle ou puisse être un reste phényle exclusivement substitué avec un ou deux restes du groupe constitué d'un radical alkyle linéaire ou ramifié ou alkoxy linéaire ou ramifié ayant chacun jusqu'à 4 atomes de carbone, halogéno, CF₃ ou OCF₃ en même temps que V est manquant ou représente O, Q représente un reste alkylène ayant 1 à 10 atomes de carbone et est éventuellement lié à Y par l'intermédiaire d'un atome d'oxygène, W représente un groupe éthylène ou un groupe éthanediyle, U représente un groupe alkylène ayant jusqu'à 4 atomes de carbone, O, S, SO ou SO₂, A représente un reste phényle et X représente un reste alkylène linéaire ayant 1 à 4 atomes de carbone et est éventuellement lié directement, par l'intermédiaire de O, S, SO ou SO₂, à l'atome de carbone portant les groupes W et U.

4. Composés suivant la revendication 1,
dans lesquels
B désigne un reste phényle,
r a la valeur 0 ou 1,
V est manquant ou représente O, NR⁴ ou S(O)ₙ,
où
R⁴ représente l'hydrogène,
n a la valeur 0,
Q est manquant ou représente un reste alkylène linéaire ou ramifié, alcènediyle linéaire ou ramifié, alcynediyle linéaire ou ramifié ayant chacun jusqu'à 15 atomes de carbone, qui peuvent contenir un ou plusieurs groupes de la série O, S(O)ₚ, NR⁵, CONR⁵, S-CO- ou OCO, et peut être substitué une ou deux fois par un radical halogéno ou hydroxy, ou représente CONR⁵,
où
R⁵ représente l'hydrogène,
P a la valeur 0 ou 1,
Y représente l'hydrogène, un groupe NR⁶R⁷, un reste phényle, naphtyle ou un hétérocycle du groupe 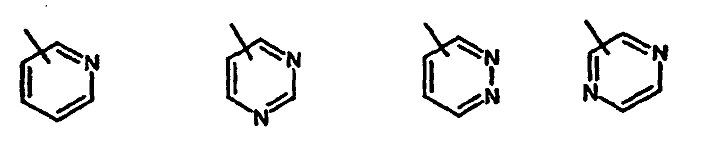 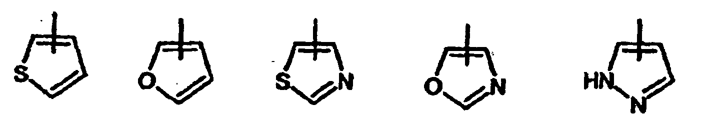 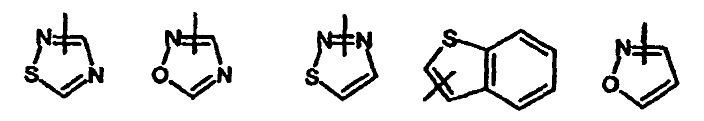 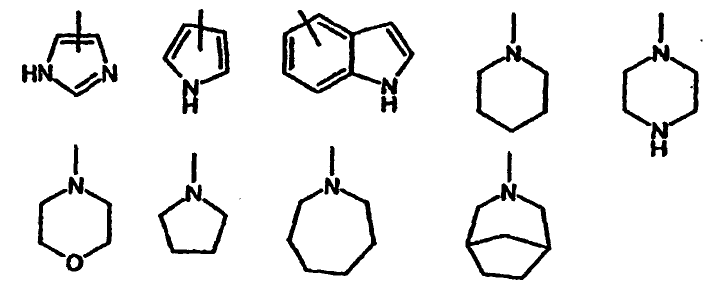 où les restes cycliques peuvent être substitués chacun une à trois fois par un radical alkyle linéaire ou ramifié, alcényle linéaire ou ramifié, alcynyle linéaire ou ramifié, alkoxy linéaire ou ramifié, halogénalkyle linéaire ou ramifié, halogénalkoxy linéaire ou ramifié ayant chacun jusqu'à 4 atomes de carbone, cycloalkyle linéaire ou ramifié ayant 3 à 7 atomes de carbone, F, Cl, Br, I, NO₂, COR⁸, SR⁸, NR¹⁰R¹¹, NR⁹COR¹² ou CONR¹³R¹⁴,
où
R⁶ et R⁷ représentent chacun, indépendamment l'un de l'autre, l'hydrogène, un reste alkyle linéaire ou ramifié, un reste alkoxy linéaire ou ramifié ou un reste alkyloxyalkyle linéaire ou ramifié ayant chacun jusqu'à 4 atomes de carbone ou un reste cycloalkyle ayant 3 à 8 atomes de carbone ou un reste aryle ayant 6 à 10 atomes de carbone, qui est éventuellement substitué une ou plusieurs fois avec un radical aryle ayant 6 à 10 atomes de carbone ou avec un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁹ représente l'hydrogène ou un reste alkyle linéaire ou ramifié ayant jusqu'à 4 atomes de carbone,
R¹⁰, R¹¹, R¹³ et R¹⁴ représentent indépendamment les uns des autres l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants formés de R¹⁰ et R¹¹ ou de R¹³ et R¹⁴ peuvent être liés ensemble en formant un noyau pentagonal ou hexagonal qui peut être interrompu par O ou N,
R¹² représente l'hydrogène, un reste alkyle linéaire ou ramifié ayant jusqu'à 4 atomes de carbone ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou par un hétérocycle du groupe des hétérocycles 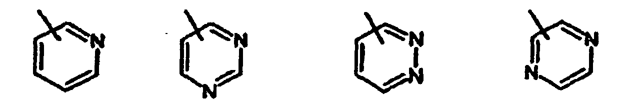 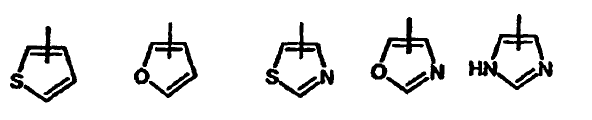 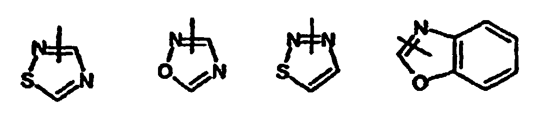 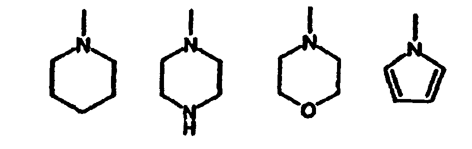 qui sont liés directement ou par l'intermédiaire d'un groupe de la série O, S, SO, SO₂, CONR⁹, SO₂NR⁹, alkyle linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant chacun jusqu'à 4 atomes de carbone et qui peuvent être substitués une à trois fois par un radical alkyle linéaire ou ramifié, alkoxy linéaire ou ramifié, halogénalkyle linéaire ou ramifié ou alcényle linéaire ou ramifié ayant chacun jusqu'à 4 atomes de carbone, phényle, benzyle, F, Cl, Br, I, CN, NO₂, NR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent indépendamment l'un de l'autre l'hydrogène, un reste alkyle linéaire ou ramifié ayant jusqu'à 8 atomes de carbone, un reste cycloalkyle ayant 3 à 8 atomes de carbone, un reste phényle, le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN, ou bien représentant un reste de formule SO₂R²⁰,
où
R²⁰ désigne un reste alkyle linéaire ou ramifié ayant jusqu'à 4 atomes de carbone ou un reste phényle,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou 0 ou un reste cycloalkyle ayant 3 à 8 atomes de carbone, qui peuvent éventuellement être substitués en outre par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène,
W représente CH₂CH₂ ou CH=CH,
U représente CH₂,
A est un reste phényle,
R² est un groupe COOH,
X est un groupe (CH₂)₄,
R¹ est un groupe COOH,
sous réserve que Y ne puisse pas être un reste phényle ou puisse être un reste phényle exclusivement substitué avec un ou deux restes du groupe constitué d'un radical alkyle linéaire ou ramifié ou d'un radical alkoxy linéaire ou ramifié ayant chacun jusqu'à 4 atomes de carbone, halogéno, CF₃ ou OCF₃ en même temps que V est manquant ou représente O, Q représente un reste alkylène linéaire ayant 1 à 10 atomes de carbone et est éventuellement lié à Y par l'intermédiaire de l'atome d'oxygène, W représente un groupe éthylène ou un groupe éthanediyle, U représente CH₂, A représente un reste phényle et X représente un groupe (CH₂)₄.

5. Composés de formule (I), dans laquelle
B représente un hétérocycle aromatique ayant 1 à 9 atomes de carbone et jusqu'à 3 hétéroatomes de la série S, N et/ou O,
r a la valeur 0 ou 1,
V est manquant ou représente 0, NR⁴, NR⁴CONR⁴, NR⁴CO, NR⁴SO₂, COO, CONR⁴ ou S(O)ₒ,
où
R⁴ représente, indépendamment d'un autre reste R⁴ éventuellement présent, l'hydrogène, un reste alkyle linéaire ou ramifié ayant jusqu'à 8 atomes de carbone, un reste cycloalkyle ayant 3 à 8 atomes de carbone, un reste aryle ayant 6 à 10 atomes de carbone ou un reste arylalkyle ayant 7 à 18 atomes de carbone, le reste aryle pouvant lui-même être substitué une ou plusieurs fois par un radical halogéno, alkyle, alkoxy ayant jusqu'à 6 atomes de carbone,
o a la valeur 0, 1 ou 2,
Q est manquant ou représente un reste alkylène linéaire ou ramifié, un reste alcènediyle linéaire ou ramifié ou un reste alcynediyle linéaire ou ramifié ayant chacun jusqu'à 15 atomes de carbone, qui peuvent contenir un ou plusieurs groupes de la série O, S(O)ₚ, NR⁵, CO, OCO, S-CO-, CONR⁵ ou NR⁵SO₂ et qui peuvent être substitués une ou plusieurs fois par un halogène, un radical hydroxy ou un radical alkoxy ayant jusqu'à 4 atomes de carbone, deux atomes quelconques de la chaîne précédente pouvant éventuellement être liés ensemble en formant un noyau de trois à huit chaînons, ou représente un groupe CONR⁵,
où
R⁵ représente l'hydrogène, un reste alkyle linéaire ou ramifié ayant jusqu'à 8 atomes de carbone ou un reste cycloalkyle ayant 3 à 8 atomes de carbone, qui peut être substitué par un halogène ou un radical alkoxy ayant jusqu'à 4 atomes de carbone,
p a la valeur 0, 1 ou 2,
Y représente l'hydrogène, un groupe NR⁶R⁷, un reste aryle ayant 6 à 10 atomes de carbone, un hétérocycle aromatique ou saturé ayant 1 à 9 atomes de carbone et jusqu'à 3 hétéroatomes de la série S, N et/ou O ou un reste cycloalkyle linéaire ou ramifié ayant 3 à 8 atomes de carbone, qui peuvent aussi être liés par l'intermédiaire de N,
les restes cycliques pouvant être substitués chacun une à trois fois par un radical alkyle linéaire ou ramifié, un radical alcényle linéaire ou ramifié, un radical alcynyle linéaire ou ramifié, un radical alkoxy linéaire ou ramifié, un radical halogénalkyle linéaire ou ramifié, un radical halogénalkoxy linéaire ou ramifié, ayant chacun jusqu'à 8 atomes de carbone, un radical cycloalkyle linéaire ou ramifié ayant 3 à 8 atomes de carbone, un radical halogéno, hydroxy, COR⁸, CN, SR⁸, NO₂, NR¹⁰R¹¹, NR⁹COR¹², NR⁹CONR⁹R¹² ou CONR¹³R¹⁴,
où
R⁶ et R⁷ représentent chacun, indépendamment l'un de l'autre, l'hydrogène, un reste alkyle linéaire ou ramifié, un reste alkoxy linéaire ou ramifié, un reste alkyloxyalkyle linéaire ou ramifié ayant jusqu'à 8 atomes de carbone ou un reste cycloalkyle ayant 3 à 8 atomes de carbone, ou un reste aryle ayant 6 à 10 atomes de carbone, qui est éventuellement substitué une ou plusieurs fois par un radical aryle ayant 6 à 10 atomes de carbone ou par un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 8 atomes de carbone, un reste halogénalkyle linéaire ou ramifié ayant jusqu'à 8 atomes de carbone ou un reste cycloalkyle ayant 3 à 8 atomes de carbone,
R⁹ représente l'hydrogène, un reste alkyle linéaire ou ramifié ayant jusqu'à 8 atomes de carbone ou un reste cycloalkyle ayant 3 à 8 atomes de carbone,
R¹⁰, R¹¹, R¹³ et R¹⁴ représentent, indépendamment les uns des autres, l'hydrogène, un reste alkyle linéaire ou ramifié, un reste alcényle linéaire ou ramifié ayant jusqu'à 8 atomes de carbone, un reste aryle ayant 6 à 10 atomes de carbone, un hétérocycle aromatique ayant 1 à 9 atomes de carbone et jusqu'à 3 hétéroatomes de la série S, N et/ou O, un reste arylalkyle ayant 8 à 18 atomes de carbone, un reste cycloalkyle ayant 3 à 8 atomes de carbone ou un reste de formule SO₂R¹⁵, le reste aryle pouvant lui-même être substitué une ou plusieurs fois par un radical halogéno, hydroxy, CN, NO₂, NH₂, NHCOR⁹, alkyle, alkoxy, halogénalkyle ou halogénalkoxy ayant jusqu'à 6 atomes de carbone,
ou bien deux substituants formés de R¹⁰ et R¹¹ ou de R¹³ et R¹⁴ peuvent être liés ensemble en formant un noyau pentagonal ou hexagonal qui peut contenir O ou N,
où
R¹⁵ représente un reste alkyle linéaire ou ramifié ayant jusqu'à 4 atomes de carbone ou un reste aryle ayant 6 à 10 atomes de carbone,
le reste aryle pouvant lui-même être substitué une ou plusieurs fois par un radical halogéno, CN, NO₂, alkyle, alkoxy, halogénalkyle ou halogénalkoxy ayant jusqu'à 6 atomes de carbone,
R¹² représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou 0 ou un reste cycloalkyle ayant 3 à 8 atomes de carbone, qui peuvent éventuellement être substitués en outre par un radical halogéno, hydroxy, CN, NO₂, NH₂, NHCOR⁹, alkyle, alkoxy, halogénalkyle ou halogénalkoxy ayant jusqu'à 6 atomes de carbone ;
et/ou les restes cycliques peuvent être substitués chacun une à trois fois par un radical aryle ayant 6 à 10 atomes de carbone, un hétérocycle aromatique ou saturé ayant 1 à 9 atomes de carbone et jusqu'à 3 hétéroatomes de la série S, N et/ou O, pouvant aussi être liés par l'intermédiaire de N,
qui peuvent être liés directement ou par l'intermédiaire d'un groupe de la série 0, S, SO, SO₂, NR⁹, CONR⁹, SO₂NR⁹, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant chacun jusqu'à 8 atomes de carbone et peuvent être substitués une à trois fois par un radical alkyle linéaire ou ramifié, alkoxy linéaire ou ramifié, halogénalkyle linéaire ou ramifié, halogénalkoxy linéaire ou ramifié, carbonylalkyle ou alcényle linéaire ou ramifié ayant chacun jusqu'à 6 atomes de carbone, phényle, benzyle, halogéno, SR⁸, CN, NO₂, NR¹⁷R¹⁸, CONR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent, indépendamment l'un de l'autre, l'hydrogène, un reste alkyle linéaire ou ramifié ayant jusqu'à 8 atomes de carbone, un reste cycloalkyle ayant 3 à 8 atomes de carbone, un reste aryle ayant 6 à 10 atomes de carbone ou un reste de formule SO₂R²⁰, le reste aryle pouvant lui-même être substitué une ou plusieurs fois par un radical halogéno, hydroxy, CN, NO₂, NH₂, NHCOR⁹, alkyle, alkoxy, halogénalkyle ou halogénalkoxy ayant chacun jusqu'à 6 atomes de carbone,
où
R²⁰ représente un reste alkyle linéaire ou ramifié ayant jusqu'à 4 atomes de carbone ou un reste aryle ayant 6 à 10 atomes de carbone,
le reste aryle pouvant lui-même être substitué une ou plusieurs fois par un radical halogéno, hydroxy, CN, NO₂, NH₂, NHCOR⁹, alkyle, alkoxy, halogénalkyle ou halogénalkoxy ayant jusqu'à 6 atomes de carbone,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 8 atomes de carbone, qui peuvent éventuellement être substitués en outre par un radical halogéno, hydroxy, CN, NO₂, NH₂, NHCOR⁹, alkyle, alkoxy, halogénalkyle ou halogénalkoxy ayant jusqu'à 6 atomes de carbone ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène, un halogène, un reste alkyle linéaire ou ramifié, un reste halogénalkyle linéaire ou ramifié, un reste alkoxy linéaire ou ramifié ou un reste halogénalkoxy linéaire ou ramifié ayant chacun jusqu'à 4 atomes de carbone,
W représente un reste alkylène linéaire ou ramifié, un reste alcènediyle linéaire ou ramifié ayant chacun jusqu'à 4 atomes de carbone, qui peuvent contenir un groupe O ou NR²³,
où
R²³ représente l'hydrogène, un reste alkyle linéaire ou ramifié ayant jusqu'à 8 atomes de carbone ou un reste cycloalkyle ayant 3 à 8 atomes de carbone,
U représente un reste alkylène linéaire ou ramifié ayant jusqu'à 4 atomes de carbone, O, NH, S, SO ou SO₂,
A est manquant ou représente un reste phényle ou un hétérocycle aromatique ayant 1 à 9 atomes de carbone et jusqu'à 3 hétéroatomes de la série S, N et/ou O, qui peuvent éventuellement être substitués une à trois fois par un halogène, un radical alkyle linéaire ou ramifié, un radical halogénalkyle linéaire ou ramifié ou un radical alkoxy linéaire ou ramifié ayant chacun jusqu'à 4 atomes de carbone,
R² représente COOR²⁶ ou CN,
où
R²⁶ représente l'hydrogène ou un reste alkyle linéaire ou ramifié ayant jusqu'à 8 atomes de carbone ;
X représente un reste alkylène linéaire ou ramifié, un reste alcènediyle linéaire ou ramifié ayant chacun jusqu'à 8 atomes de carbone, qui peuvent contenir un groupe de la série O, S(O)ᵣ, NR³⁰ ou un carbocycle de trois à six chaînons saturé ou non saturé, présentant éventuellement un ou plusieurs restes alkyle linéaires ou ramifiés ayant 1 à 6 atomes de carbone, avec éventuellement un ou deux hétéroatomes de la série S(O)ᵣ, NR³² et/ou 0,
où
r a la valeur 0, 1 ou 2,
R³⁰ représente l'hydrogène, un reste alkyle linéaire ou ramifié ayant jusqu'à 6 atomes de carbone, un reste phényle ou un reste arylalkyle ayant 7 à 12 atomes de carbone,
R³² représente l'hydrogène, un reste alkyle linéaire ou ramifié ayant jusqu'à 6 atomes de carbone, un reste cycloalkyle ayant 3 à 6 atomes de carbone ou un reste phényle,
R¹ représente CN ou COOR³⁵,
où
R³⁵ représente l'hydrogène ou un reste alkyle linéaire ou ramifié ayant jusqu'à 6 atomes de carbone.

6. Composés suivant la revendication 5,
dans lesquels
B représente un hétérocycle de la série 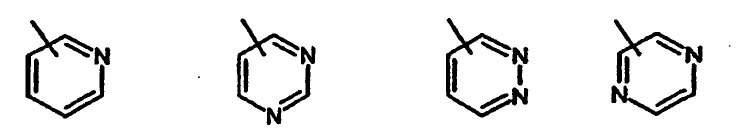 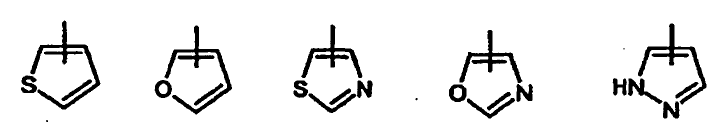 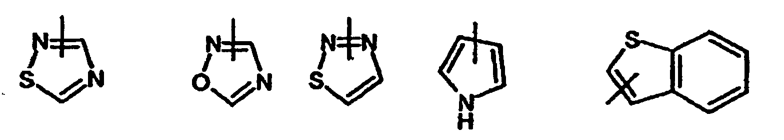 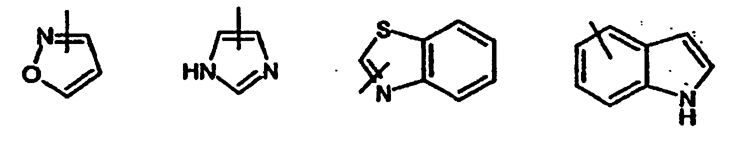
r a la valeur 0 ou 1,
V est manquant ou représente O, NR⁴ ou S(O)ₙ,
où
R⁴ représente l'hydrogène,
n a la valeur 0,
Q est manquant ou représente un reste alkylène linéaire ou ramifié, alcènediyle linéaire ou ramifié ayant chacun jusqu'à 15 atomes de carbone, qui peuvent contenir un ou plusieurs groupes de la série O, S(O)ₚ, NR⁵, CONR⁵, S-CO- ou OCO, et peut être substitué une ou deux fois par un radical halogéno ou hydroxy, ou représente un groupe CONR⁵,
où
R⁵ représente l'hydrogène,
p a la valeur 0 ou 1,
Y représente l'hydrogène, un groupe NR⁶R⁷, un reste phényle, naphtyle ou un hétérocycle du groupe 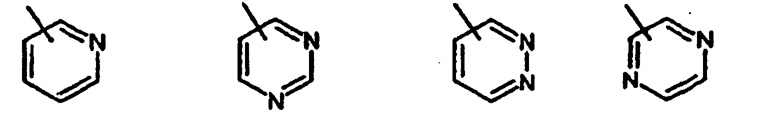 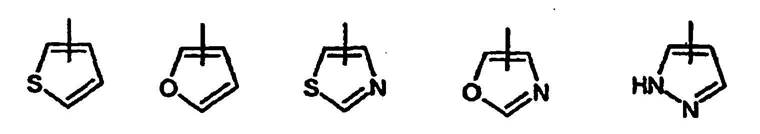 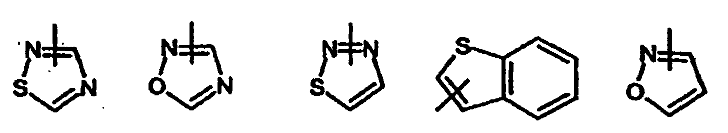 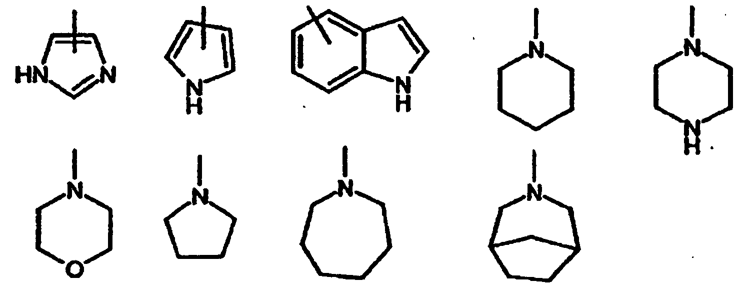 les restes cycliques pouvant être substitués chacun une à trois fois par un radical alkyle linéaire ou ramifié, alcényle linéaire ou ramifié, alcynyle linéaire ou ramifié, alkoxy linéaire ou ramifié, halogénalkyle linéaire ou ramifié, halogénalkoxy linéaire ou ramifié ayant chacun jusqu'à 4 atomes de carbone, cycloalkyle linéaire ou ramifié ayant 3 à 7 atomes de carbone, F, Cl, Br, I, NO₂, COR⁸, SR⁸, NR¹⁰R¹¹, NR⁹COR¹² ou CONR¹³R¹⁴,
où
R⁶ et R⁷ représentent, indépendamment l'un de l'autre, l'hydrogène, un reste alkyle linéaire ou ramifié, un reste alkoxy linéaire ou ramifié ou un reste alkyloxyalkyle linéaire ou ramifié ayant chacun jusqu'à 4 atomes de carbone, ou un reste aryle ayant 6 à 10 atomes de carbone, qui est éventuellement substitué une ou plusieurs fois par un radical aryle ayant 6 à 10 atomes de carbone ou un hétérocycle aromatique ayant 1 à 9 atomes de carbone et jusqu'à 3 hétéroatomes de la série S, N et/ou 0,
R⁸ représente l'hydrogène, un reste alkyle linéaire ou ramifié ayant jusqu'à 4 atomes de carbone ou un reste halogénalkyle ayant jusqu'à 4 atomes de carbone,
R⁹ représente l'hydrogène ou un reste alkyle linéaire ou ramifié ayant jusqu'à 4 atomes de carbone,
R¹⁰, R¹¹, R¹³ et R¹⁴ représentent, indépendamment les uns des autres, l'hydrogène, un reste alkyle linéaire ou ramifié ayant jusqu'à 4 atomes de carbone ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants formés de R¹⁰ et R¹¹ ou de R¹³ et R¹⁴ peuvent être liés ensemble en formant un noyau pentagonal ou hexagonal qui peut être interrompu par O ou N,
R¹² représente l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou par un hétérocycle du groupe 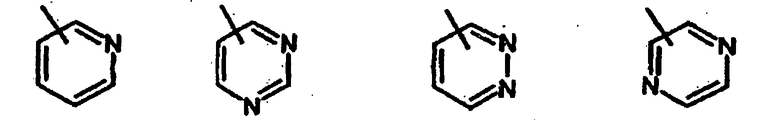 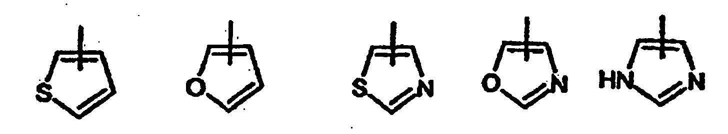 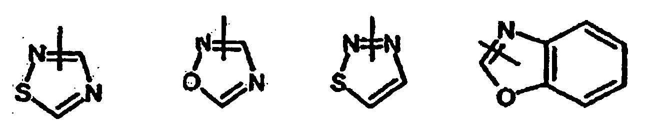 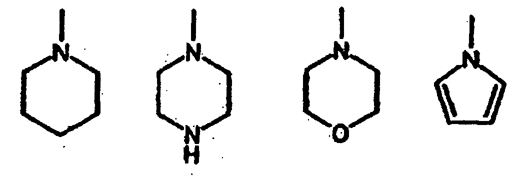 qui peuvent être liés directement ou par l'intermédiaire d'un groupe de la série O, S, SO, SO₂, CONR⁹, SO₂NR⁹, alkylène linéaire ou ramifié, alcènediyle linéaire ou ramifié, alkyloxy linéaire ou ramifié, oxyalkyloxy linéaire ou ramifié, sulfonylalkyle linéaire ou ramifié, thioalkyle linéaire ou ramifié ayant chacun jusqu'à 4 atomes de carbone et qui peuvent être substitués une à trois fois par un radical alkyle linéaire ou ramifié, alkoxy linéaire ou ramifié, halogénalkyle linéaire ou ramifié ou alcényle linéaire ou ramifié ayant chacun jusqu'à 4 atomes de carbone, phényle, benzyle, F, Cl, Br, I, CN, NO₂, NR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent, indépendamment l'un de l'autre, l'hydrogène, un reste alkyle linéaire ou ramifié ayant jusqu'à 8 atomes de carbone, un reste cycloalkyle ayant 3 à 8 atomes de carbone, un reste phényle, le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN, ou désignent un reste de formule SO₂R²⁰,
où
R²⁰ représente un reste alkyle linéaire ou ramifié ayant jusqu'à 4 atomes de carbone ou un reste phényle,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 8 atomes de carbone, qui peuvent être substitués en outre, le cas échéant, par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou 0,
R³ représente l'hydrogène, F, Cl, Br, un reste alkyle linéaire ou ramifié, un reste halogénalkyle linéaire ou ramifié, un reste alkoxy linéaire ou ramifié ou un reste halogénalkoxy linéaire ou ramifié ayant chacun jusqu'à 4 atomes de carbone,
W représente CH₂CH₂, CH=CH, CH₂O, OCH₂, CH₂OCH₂, CH₂NH, NHCH₂ ou CH₂NHCH₂,
U est un reste alkylène linéaire ayant jusqu'à 4 atomes de carbone, O, NH, S, SO ou SO₂,
A est manquant ou représente un reste phényle, pyridyle, thiényle ou thiazolyle, qui peut être substitué le cas échéant une à trois fois par un radical méthyle, éthyle, n-propyle, isopropyle, n-butyle, isobutyle, sec.-butyle, tertiobutyle, CF₃, méthoxy, éthoxy, F, Cl, Br,
R² est un groupe COOR²⁶ ou CN,
où
R²⁶ représente l'hydrogène ou un reste alkyle linéaire ou ramifié ayant jusqu'à 4 atomes de carbone ;
X est un reste alkylène linéaire ou ramifié ayant jusqu'à 4 atomes de carbone, qui peut contenir un groupe de la série 0, S(O)ᵣ, NR³⁰ ou un carbocycle de trois à six chaînons saturé ou non saturé, présentant éventuellement un ou plusieurs restes alkyle linéaires ou ramifiés ayant 1 à 4 atomes de carbone, avec éventuellement un ou deux hétéroatomes de la série S(O)ᵣ, NR³² et/ou O,
où
r a la valeur 0, 1 ou 2,
R³⁰ représente l'hydrogène, un reste alkyle linéaire ou ramifié ayant jusqu'à 6 atomes de carbone, un reste phényle ou benzyle,
R³² représente l'hydrogène, un reste alkyle linéaire ou ramifié ayant jusqu'à 6 atomes de carbone, un reste cycloalkyle ayant 3 à 6 atomes de carbone ou un reste phényle,
R¹ représente CN ou COOR³⁵,
où
R³⁵ représente l'hydrogène ou un reste alkyle linéaire ou ramifié ayant jusqu'à 6 atomes de carbone.

7. Composés suivant la revendication 5,
dans lesquels
B représente un hétérocycle de la série 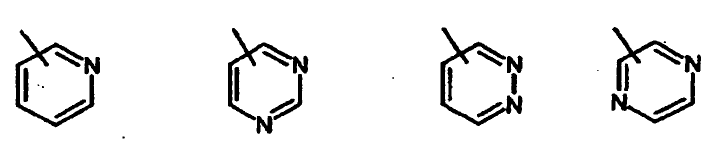 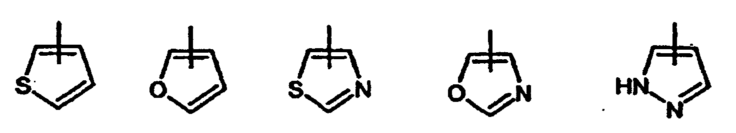 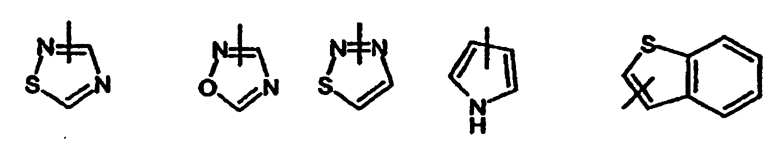 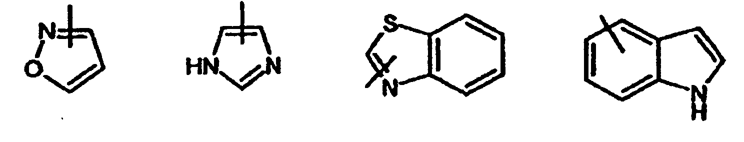
r a la valeur 0 ou 1,
V est manquant ou représente O, NR⁴ ou S(O)ₙ,
où
R⁴ représente l'hydrogène,
n a la valeur 0,
Q est manquant ou représente un reste alkylène linéaire ou ramifié, alcènediyle linéaire ou ramifié ayant chacun jusqu'à 15 atomes de carbone, qui peuvent contenir un ou plusieurs groupes de la série O, S(O)ₚ, NR⁵, CONR⁵, S-CO- ou OCO, et peut être substitué une ou deux fois par un radical halogéno ou hydroxy, ou représente CONR⁵,
où
R⁵ représente l'hydrogène,
p a la valeur 0 ou 1,
Y représente l'hydrogène, un groupe NR⁶R⁷, un reste phényle, naphtyle ou un hétérocycle du groupe 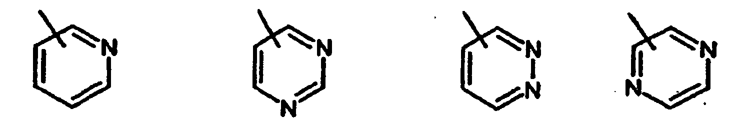 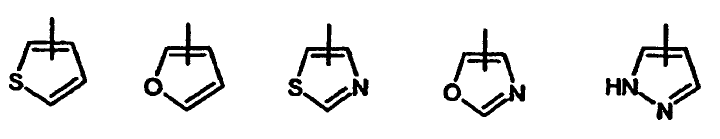 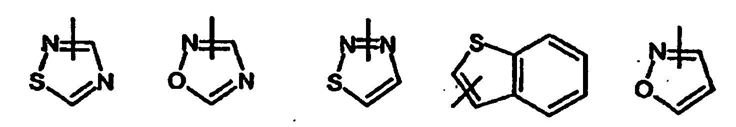 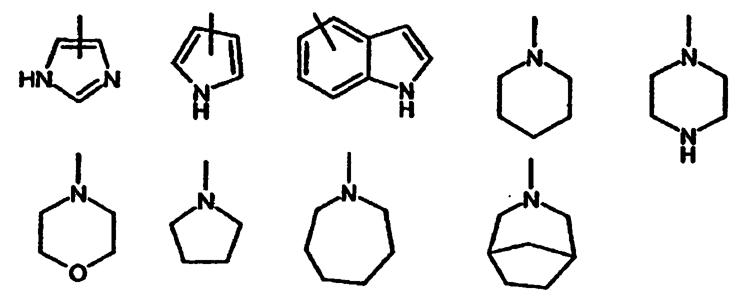 les restes cycliques pouvant chacun être substitués une à trois fois par un reste alkyle linéaire ou ramifié, un reste alcényle linéaire ou ramifié, un reste alcynyle linéaire ou ramifié, un reste alkoxy linéaire ou ramifié, un reste halogénalkyle linéaire ou ramifié, un reste halogénalkoxy linéaire ou ramifié ayant chacun jusqu'à 4 atomes de carbone, un reste cycloalkyle linéaire ou ramifié ayant 3 à 7 atomes de carbone, F, Cl, Br, I, NO₂, COR⁸, SR⁸, NR¹⁰R¹¹, NR⁹COR¹² ou CONR¹³R¹⁴,
où
R⁶ et R⁷ représentent chacun, indépendamment l'un de l'autre, l'hydrogène, un reste alkyle linéaire ou ramifié, un reste alkoxy linéaire ou ramifié ou un reste alkyloxyalkyle linéaire ou ramifié ayant jusqu'à 4 atomes de carbone ou un reste cycloalkyle ayant 3 à 8 atomes de carbone, ou un reste aryle ayant 6 à 10 atomes de carbone, qui est éventuellement substitué une ou plusieurs fois avec un radical aryle ayant 6 à 10 atomes de carbone ou un hétérocycle aromatique ayant 1 à 9 atomes de carbone et jusqu'à 3 hétéroatomes de la série S, N et/ou O,
R⁸ représente l'hydrogène, un reste alkyle linéaire ou ramifié ayant jusqu'à 4 atomes de carbone ou un reste halogénalkyle linéaire ou ramifié ayant jusqu'à 4 atomes de carbone,
R⁹ représente l'hydrogène ou un reste alkyle linéaire ou ramifié ayant jusqu'à 4 atomes de carbone,
R¹⁰, R¹¹, R¹³ et R¹⁴ représentent, indépendamment les uns des autres, l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN,
ou bien deux substituants formés de R¹⁰ et R¹¹ ou de R¹³ et R¹⁴ peuvent être liés ensemble en formant un noyau pentagonal ou hexagonal qui peut être interrompu par O ou N,
R¹² représente l'hydrogène, un reste alkyle linéaire ou ramifié ayant jusqu'à 4 atomes de carbone, ou un reste phényle,
le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être substitués chacun une à trois fois par un radical phényle ou par un hétérocycle du groupe des hétérocycles 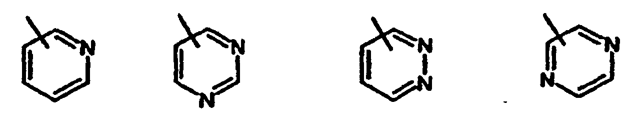 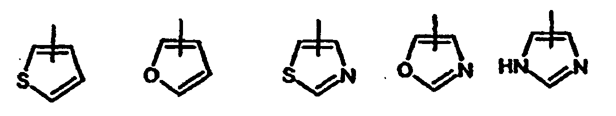 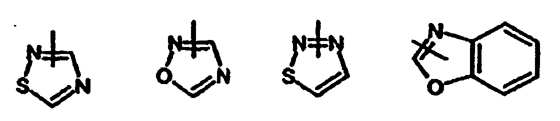 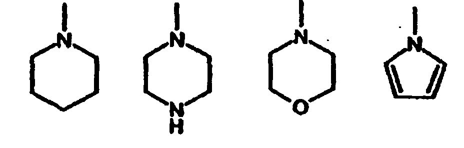 qui sont liés directement ou par l'intermédiaire d'un groupe de la série O, S, SO, SO₂, CONR⁹, SO₂NR⁹, un reste alkylène linéaire ou ramifié, un reste alcènediyle linéaire ou ramifié, un reste alkyloxy linéaire ou ramifié, un reste oxyalkyloxy linéaire ou ramifié, un reste sulfonylalkyle linéaire ou ramifié, un reste thioalkyle linéaire ou ramifié ayant chacun jusqu'à 4 atomes de carbone et qui peuvent être substitués une à trois fois par un radical alkyle linéaire ou ramifié, alkoxy linéaire ou ramifié, halogénalkyle linéaire ou ramifié ou alcényle linéaire ou ramifié ayant chacun jusqu'à 4 atomes de carbone, phényle, benzyle, F, Cl, Br, I, CN, NO₂, NR¹⁷R¹⁸ ou NR¹⁶COR¹⁹,
où
R¹⁶ représente l'hydrogène, un reste alkyle linéaire ou ramifié ayant jusqu'à 8 atomes de carbone ou un reste cycloalkyle ayant 3 à 8 atomes de carbone,
R¹⁷, R¹⁸ représentent, indépendamment l'un de l'autre, l'hydrogène, un reste alkyle linéaire ou ramifié ayant jusqu'à 8 atomes de carbone, un reste cycloalkyle ayant 3 à 8 atomes de carbone, un reste phényle, le reste phényle pouvant être substitué une à trois fois par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN, ou représentent un reste de formule SO₂R²⁰,
où
R²⁰ est un reste alkyle linéaire ou ramifié ayant jusqu'à 4 atomes de carbone ou un reste phényle,
et
R¹⁹ représente l'hydrogène, un reste alkyle linéaire ou ramifié ayant jusqu'à 12 atomes de carbone, un reste alcényle linéaire ou ramifié ayant jusqu'à 12 atomes de carbone, un reste aryle ayant 6 à 10 atomes de carbone, un hétérocycle aromatique ayant 1 à 9 atomes de carbone et jusqu'à 3 hétéroatomes de la série S, N et/ou O ou un reste cycloalkyle ayant 3 à 8 atomes de carbone, qui peuvent éventuellement être substitués en outre par F, Cl, Br, un radical hydroxy, méthyle, éthyle, n-propyle, isopropyle, n-butyle, sec.-butyle, isobutyle, tertiobutyle, méthoxy, éthoxy, amino, acétylamino, NO₂, CF₃, OCF₃ ou CN ;
et/ou les restes cycliques peuvent être condensés avec un carbocycle aromatique ou saturé ayant 1 à 10 atomes de carbone ou avec un hétérocycle aromatique ou saturé ayant 1 à 9 atomes de carbone et jusqu'à 3 hétéroatomes de la série S, N et/ou O,
R³ représente l'hydrogène, F, Cl, Br, un reste alkyle linéaire ou ramifié, un reste halogénalkyle linéaire ou ramifié, un reste alkoxy linéaire ou ramifié ou un reste halogénalkoxy linéaire ou ramifié ayant chacun jusqu'à 4 atomes de carbone,
W représente CH₂CH₂, CH=CH, CH₂O, OCH₂, CH₂OCH₂, CH₂NH, NHCH₂ ou CH₂NHCH₂,
où
U est un reste alkylène linéaire ayant jusqu'à 4 atomes de carbone, O, NH, S, SO ou SO₂,
A est manquant ou représente un reste phényle, pyridyle, thiényle ou thiazolyle, qui peut éventuellement être substitué une à trois fois par un radical méthyle, éthyle, n-propyle, isopropyle, n-butyle, isobutyle, sec.-butyle, tertiobutyle, CF₃, méthoxy, éthoxy, F, Cl, Br,
R² est un groupe COOH,
X est un reste alkylène linéaire ou ramifié ayant jusqu'à 4 atomes de carbone, qui peut contenir un groupe de la série O, S(O)ᵣ, NR³⁰ ou un carbocycle de trois à six chaînons saturé ou non saturé, présentant éventuellement un ou plusieurs restes alkyle linéaires ou ramifiés ayant 1 à 4 atomes de carbone, avec éventuellement un ou deux hétéroatomes de la série S(O)ᵣ, NR³² et/ou 0,
où
r a la valeur 0, 1 ou 2,
R³⁰ représente l'hydrogène, un reste alkyle linéaire ou ramifié ayant jusqu'à 6 atomes de carbone, un reste phényle ou benzyle,
R³² représente l'hydrogène, un reste alkyle linéaire ou ramifié ayant jusqu'à 6 atomes de carbone, un reste cycloalkyle ayant 3 à 6 atomes de carbone ou un reste phényle,
R¹ représente le groupe COOH.

8. Procédé de production des composés de formule (I), 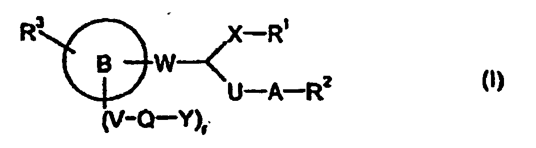 comprenant
[A] la réaction d'aldéhydes de formule générale (II), 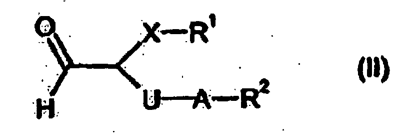 dans laquelle
R¹, R², A, U et X ont la définition indiquée ci-dessus, sous réserve que R¹ et R² ne puissent pas représenter des groupes acides carboxyliques libres,
avec des composés de phosphore de formule générale (III), 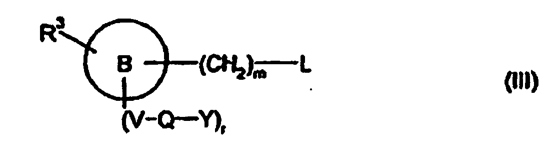 dans laquelle
R³, B, V, Q, Y et r ont les définitions indiquées ci-dessus,
m est un nombre entier de 1 à 5, et
L représente un reste de formule 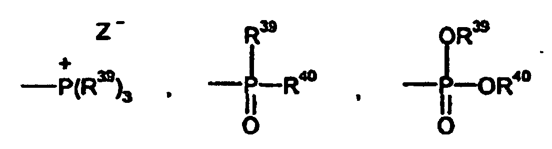 dans laquelle
R³⁹ et R⁴⁰ représentent, indépendamment l'un de l'autre, un reste alkyle linéaire ou ramifié ayant jusqu'à 12 atomes de carbone ou un reste phényle, et
Z est un anion halogénure ou un anion.tosylate,
dans des solvants inertes en présence d'une base,
et, le cas échéant, l'hydrolyse partielle ou totale subséquente des restes R¹ et R² en groupes acides carboxyliques libres ;
ou bien
[B] la réaction de composés de formule (IV), 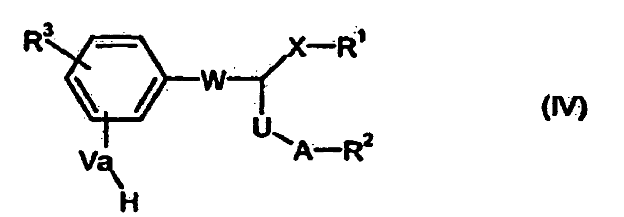 dans laquelle
Va représente O ou S,
R¹, R², R³, U, W, A, X ont la définition indiquée ci-dessus,
avec des composés de formule (V), 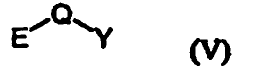 dans laquelle
Q, Y ont les mêmes définitions que ci-dessus,
E désigne un groupe partant qui est substitué en présence d'une base, ou représente une fonction hydroxy éventuellement activée ;
ou bien
[C] la transformation de composés de formule (VI), 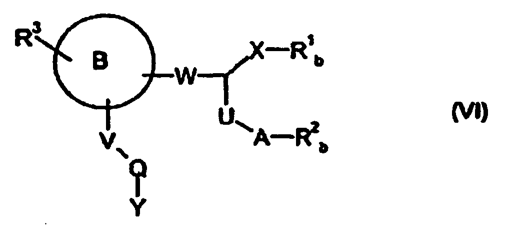 dans laquelle
R³, V, Q, Y, W, U, A, B ont les définitions indiquées ci-dessus,
R¹_{b} et R²_{b} représentent chacun, indépendamment, CN ou COOAlk où Alk désigne un reste alkyle linéaire ou ramifié ayant jusqu'à 6 atomes de carbone,
avec des solutions aqueuses d'acides forts ou de bases fortes en les acides carboxyliques libres correspondants,
ou bien
[D] la réaction de composés de formule (VII), 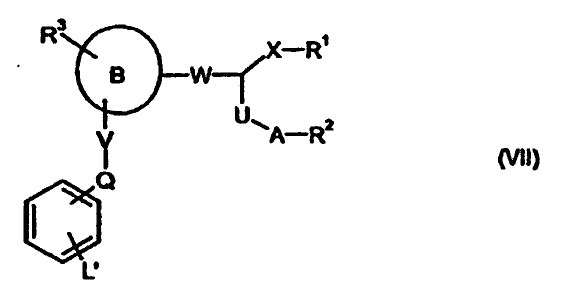 dans laquelle
R¹, R², R³, V, Q, X, W, U, A, B ont les mêmes définitions que ci-dessus,
L' représente Br, I ou le groupe CF₃SO₂-O,
avec des composés de formule (VIII),
**M-Z' (VIII)**
dans laquelle
M est un reste aryle ou hétéroaryle, un reste alkyle, alcényle ou alcynyle linéaire ou ramifié ou un reste cycloalkyle ou un reste arylalkyle, un reste arylalcényle ou un reste arylalcynyle,
Z' représente les groupements -B(OH)₂, -CH≡CH, -CH=H₂ ou -Sn(nBu)₃,
en présence d'un composé de palladium, en outre le cas échéant, en présence d'un agent réducteur et d'autres additifs et en présence d'une base ;
ou bien
[E] la réaction de composés de formule (VII), 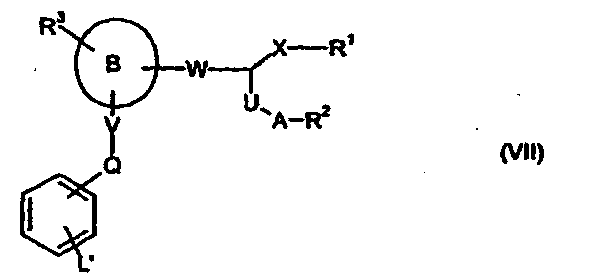 dans laquelle
R¹, R², R³, V, Q, X, W, U, A, B ont les mêmes définitions que ci-dessus,
L' représente Br, I ou le groupe CF₃SO₂-O,
avec des composés de formule (IX),
**NHR**^{**a**}**R**^{**b**} **(IX)**
dans laquelle
R^{a} et R^{b} représentent, indépendamment l'un de l'autre, l'hydrogène ou un reste alkyle linéaire ou ramifié ayant jusqu'à 8 atomes de carbone ou peuvent former conjointement avec l'atome d'azote auquel ils sont liés un hétérocycle aromatique ayant 1 à 9 atomes de carbone et jusqu'à 3 hétéroatomes de la série S, N et/ou 0,
en présence d'un composé de palladium, en outre le cas échéant, en présence d'un agent réducteur et d'autres additifs et en présence d'une base ;
ou bien
[F] la réaction de composés de formule (IV), 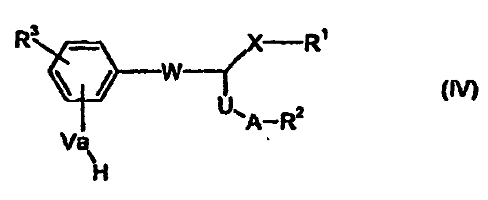 dans laquelle
Va représente O ou S,
R¹, R², R³, U, W, A, X ont la définition indiquée ci-dessus,
avec des composés de formule (X), 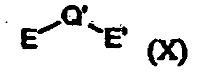 dans laquelle
Q' a la même définition que Q selon la revendication 1 ou désigne un reste phényle,
E et E' désignent chacun indépendamment l'un de l'autre un groupe partant qui est substitué en présence d'une base ou représentent une fonction hydroxy éventuellement activée ou représentent un reste contenant un tel groupe ;
et la réaction des composés ainsi obtenus de formule (XI), 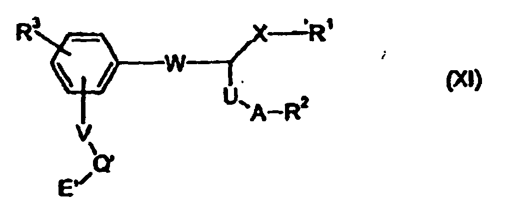 dans laquelle
R¹, R², R³, A, U, W, X et E' ont les définitions indiquées ci-dessus,
Q' a la même définition que Q selon la revendication 1, ou représente un groupe 1,4-CH₂-Ph-CH₂-,
avec des amines de formule (XII),
**NHR**^{**a**}**R**^{**b**} **(XII)**
dans laquelle
R^{a} et R^{b} représentent indépendamment l'un de l'autre l'hydrogène ou un reste alkyle linéaire ou ramifié ayant jusqu'à 8 atomes de carbone ou peuvent former conjointement avec l'atome d'azote auquel ils sont liés un hétérocycle aromatique ayant 1 à 9 atomes de carbone et jusqu'à 3 hétéroatomes de la série S, N et/ou O,
ou bien
[G] la réaction de composés de formule (XIII), 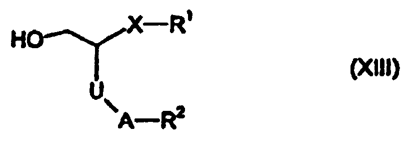 dans laquelle
R¹, R², A, U, X ont les définitions indiquées ci-dessus,
avec des composés de formule (XIV), 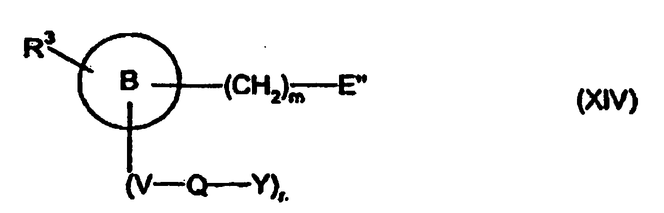 dans laquelle
R³, V, Q, Y, r et B ont les définitions indiquées ci-dessus,
m représente un nombre entier de 1 à 5, et
E" représente un groupe partant qui est substitué en présence d'une base, ou une fonction hydroxy éventuellement activée ;
ou bien
[H] la réaction de composés de formule (XV), 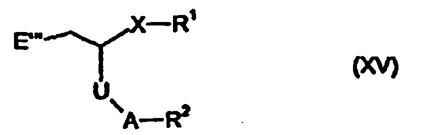 dans laquelle
R¹, R², A, U, X ont les définitions indiquées ci-dessus,
E"' désigne un groupe partant qui est substitué en présence d'une base ou représente une fonction hydroxy éventuellement activée ;
avec des composés de formule (XVI), 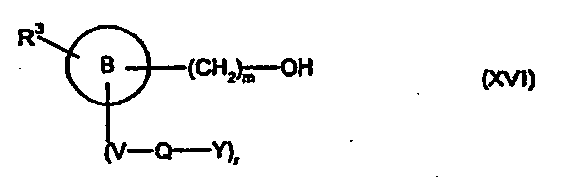 dans laquelle
R³, V, Q, Y, r et B ont les définitions indiquées ci-dessus,
m désigne un nombre entier de 1 à 5 ;
ou bien
[I] la réaction de composés de formule (XVII), 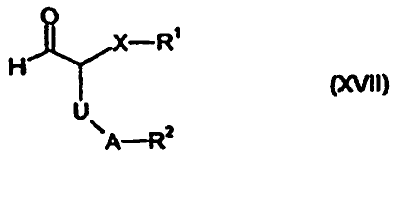 dans laquelle
R¹, R², A, U, X ont les définitions indiquées ci-dessus,
avec des composés de formule (XVIII), 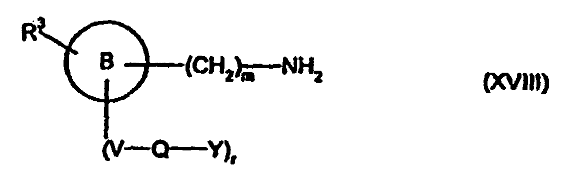 dans laquelle
R³, V, Q, Y, r et B ont les définitions indiquées ci-dessus,
m est un nombre entier de 0 à 5,
pour former tout d'abord une base de Schiff, puis la réduction de cette base avec des agents réducteurs usuels,
ou sa réaction directe dans les conditions d'une alkylation par voie de réduction en présence d'un agent réducteur ;
ou bien
[J] la réaction de composés de formule (XIX), 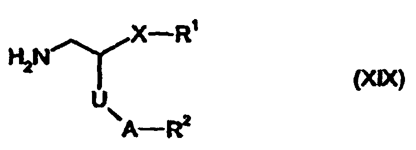 dans laquelle
R¹, R², A, U, X ont les définitions indiquées ci-dessus,
avec des composés de formule (XX), 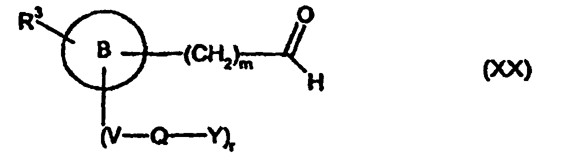 dans laquelle
R³, V, Q, Y, r et B ont les définitions indiquées ci-dessus,
m est un nombre entier de 0 à 5,
pour former tout d'abord une base de Schiff, puis la réduction de cette base avec des agents réducteurs usuels ou sa réaction directement dans les conditions d'une alkylation par voie de réduction en présence d'un agent réducteur, ou bien
[K] la réaction d'aldéhydes de formule (XXI), 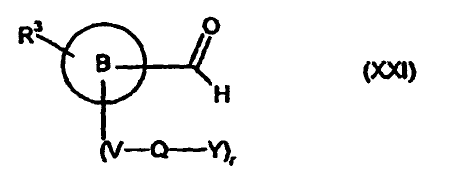 dans laquelle
R³, V, Q, Y, r et B ont les définitions indiquées ci-dessus,
avec des composés de phosphore de formule (XXII), 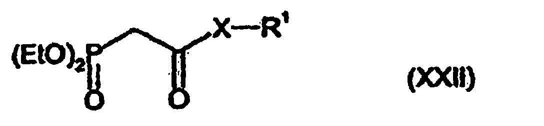 dans laquelle
X et R¹ ont les définitions indiquées ci-dessus, pour former des composés de formule (XXIII), 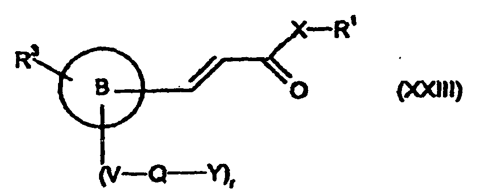
dans laquelle
R³, V, Q, Y, r, B, X et R¹ ont les définitions indiquées ci-dessus,
puis la transformation par réduction successive du groupe alcène et du groupe carbonyle, suivie de la substitution du groupe hydroxy produit par réduction du groupe carbonyle et respectivement du reste halogéno produit à partir du groupe hydroxy par réaction avec des agents d'halogénation, au moyen d'alcools, d'amines primaires ou de thiols ainsi que, le cas échéant, l'oxydation subséquente en sulfoxydes ou sulfones correspondants, pour obtenir des composés de formule (XXIV), 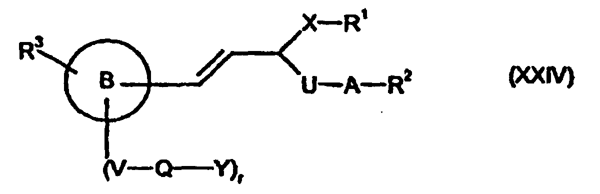 dans laquelle
R³, V, Q, Y, r, B, X, U, R² et R¹ ont les définitions indiquées ci-dessus,
U représente O, NH ou S.

9. Médicament contenant au moins un composé de formule générale (I) suivant l'une des revendications précédentes.

10. Utilisation de composés de formule (I) suivant l'une des revendications précédentes pour la préparation d'un médicament destiné au traitement de maladies cardiovasculaires.

11. Utilisation de composés de formule générale (I) suivant l'une des revendications précédentes, pour la préparation de médicaments destinés au traitement de l'angor, d'ischémies et de l'insuffisance cardiaque.

12. Utilisation de composés de formule générale (I) suivant l'une des revendications précédentes, pour la préparation de médicaments destinés au traitement de l'hypertonie, de maladies thromboemboliques, de l'artériosclérose et d'affections veineuses.

13. Utilisation de composés de formule générale (I) suivant l'une des revendications précédentes, pour la préparation de médicaments destinés au traitement d'affections fibrotiques.

14. Utilisation suivant la revendication 15, **caractérisée en ce que** l'affection fibrotique est la fibrose hépatique.
